# EUROPEAN PATENT APPLICATION

(11) **EP 1 676 915 A2**
(43) Date of publication of application: **05.07.2006**
(21) Application number: 05028449.6
(22) Date of filing: 26.10.2000
(51) Int. Cl.: C12N 15/11, A61K 31/713, A61P 17/00, A61P 17/06, A61P 35/00

(54) **Ribozyme therapy for the treatment of proliferative skin diseases**

(30) Priority: 26.10.1999 US 161532 P
(62) Divisional of application: 00975399.7
(71) Applicant: Immusol Incorporated, San Diego, CA 92121 (US)
(72) Inventor: Robbins, Joan M., San Diego, CA 92131 (US); Tritz, Richard, San Diego, CA (US)
(74) Representative: Walton, Seán Malcolm

(57) **Abstract**

As an effective therapy for proliferative skin and eye diseases such as psoriasis and proliferative diabetic retinopathy, this invention provides ribozymes and ribozyme delivery systems which cleave RNA encoding cytokines involved in inflammation, matrix metalloproteinases, a cyclin, a cell-cycle dependent kinase, a growth factor, or a reductase.

## Description

### TECHNICAL FIELD

The present invention relates generally to therapeutics, and more specifically, to compositions and methods which may be utilized in the treatment and/or prevention of proliferative skin and eye diseases such as psoriasis and proliferative retinopathy.

### BACKGROUND OF THE INVENTION

Proliferative skin diseases such as psoriasis affect as many as 2 to 3% of the entire U.S. population, with more than 250,000 new cases being diagnosed each year. The cost of treating psoriasis in the United States alone is estimated to range between three and five billion dollars per year, thus representing a major health cost to the health care system. Although the cause of psoriasis is presently unknown, there is evidence which tends to suggest that it is a polygenetic autoimmune disorder.

Proliferative skin diseases such as psoriasis are characterized by itchy, inflamed, scaly lesions which bleed easily. Although there are a wide variety of topical treatments (e.g., coal tar preparations, steroid-based creams and ointments) and systemic treatments (e.g., ultraviolet radiation, PUVA, steroids, and chemotherapeutic agents such as methotrexate), at present there is no cure for psoriasis. Further, presently available treatments are generally unsatisfactory, in that remission rates are high and certain therapeutic regimens have potentially serious side effects.

Proliferative diseases of the eye such as proliferative diabetic retinopathy (PDR) affect as many as 700,000 in the U.S., with more than 65,000 new cases diagnosed each year. Annually, as many as 25,000 people go blind from the disorder, making it a leading cause of blindness among working-age Americans. The cost savings from successful intervention could be as high as 100 million dollars per year. The disease does not stem from a single retinal change. Rather, it may be triggered by a combination of biochemical, metabolic, and hematological abnormalities.

There are no early symptoms. There is no pain, no blurred vision, and no ocular inflammation. In fact, many people do not develop any visual impairment until the disease has advanced well into its proliferative stage. At this point, the vision that has been lost cannot be restored. Laser surgery, also called photocoagulation, is the only current therapy to treat PDR. At present there is no cure for this disease.

Scarring is a significant source of disfigurement, pain, and increased medical costs for affected patients. It is the result of abnormal events during wound healing that can result in tissue overgrowth characterized by hyperproliferation of fibroblasts and excessive deposition of matrix. In surgery, scar tissue formation and contraction is a major clinical problem. Likewise, scarring following accidental burning or other injuries or trauma often has serious results, causing impaired function and unsightly aesthetic effects. Currently, there are no satisfactory treatments to prevent scarring.

Thus, a need exists for an effective therapy to treat proliferative diseases *(e.g*., psoriasis, PDR and scarring). The present invention satisfies this need and further provides other related advantages as well.

### SUMMARY OF THE INVENTION

Briefly stated, the present invention provides ribozymes and ribozyme delivery systems which are able to inhibit the mechanisms involved in proliferation of cells associated with proliferative skin and eye diseases such as psoriasis, PDR and scarring. Accordingly, the present invention ribozymes are provided which are suitable for treating scarring and proliferative diseases by inhibiting a cytokine involved in inflammation, a matrix metalloproteinase involved in extracellular matrix elaboration, a cyclin, a cell-cycle dependent kinase, a growth factor involved in cell cycle regulation, or a reductase.

Within one aspect of the invention, such methods generally comprise the step of administering to a patient a therapeutically effective amount of ribozyme which cleaves RNA encoding a cytokine involved in inflammation, a matrix metalloproteinase, a cyclin, a cell-cycle dependent kinase, a growth factor, or a reductase such that said proliferative skin disease is treated. Within a related aspect, methods of treating proliferative skin disease are provided comprising the step of administering to a patient an effective amount of nucleic acid molecule comprising a promoter operably linked to a nucleic acid segment encoding a ribozyme which cleaves RNA encoding a cytokine involved in inflammation, a matrix metalloproteinase, a cyclin, a cell-cycle dependent kinase, a growth factor, or a reductase such that said proliferative skin disease is treated. Representative examples of proliferative skin diseases include psoriasis, atopic dermatitis, actinic keratosis, squamous or basal cell carcinoma, viral or seborrheic wart.

Within other aspects, such methods generally comprise the step of administering to a patient a therapeutically effective amount of ribozyme which cleaves RNA encoding a cytokine involved in inflammation, a matrix metalloproteinase, a cyclin, a cell-cycle dependent kinase, or a growth factor such that said proliferative eye disease is treated. Within a related aspect, methods of treating proliferative eye disease are provided comprising the step of administering to a patient an effective amount of nucleic acid molecules comprising a promoter operably linked to a nucleic acid segment encoding a ribozyme which cleaves RNA encoding a cytokine involved in inflammation, a matrix metalloproteinase, a cyclin, a cell-cycle dependent kinase, a growth factor, or a reductase such that said proliferative eye disease is treated. Representative examples of proliferative eye diseases include proliferative diabetic retinopathy, proliferative vitreoretinopathy, proliferative sickle cell retinopathy, retinopathy of prematurity and retinal detachment.

Within other aspects, methods are provided for treating or preventing scarring, comprising administering to a patient a therapeutically effective amount of ribozyme which cleaves RNA encoding a cytokine involved in inflammation, a matrix metalloproteinase, a cyclin, a cell-cycle dependent kinase, a growth factor, or a reductase such that said scarring is treated or prevented. Within another related aspect, methods are provided for treating or preventing scarring, comprising administering to a patient an effective amount of nucleic acid molecule comprising a promoter operably linked to a nucleic acid segment encoding a ribozyme which cleaves RNA encoding a cytokine involved in inflammation, a matrix metalloproteinase, a cyclin, a cell-cycle dependent kinase, a growth factor, or a reductase such that said scarring is treated or prevented. Representative examples of diseases or injuries which cause scarring include keloids, adhesions (*e.g*., surgical adhesions), hypertrophic burn scars, and trauma (e.g., blunt trauma or surgical trauma).

Cyclins and cell-cycle dependent kinases are directly involved in the check point control of cell division leading to proliferation. Particularly preferred cyclins or cell-cycle dependent kinases include CDK1, CDK2, CDK4, Cyclin B1, Cyclin D and PCNA.

Infection or injury induces a complex cascade of events including activation of the clotting pathway, adherence of immune cells to the endothelium, induction of proliferation and migration of cells into the injury site to rapidly repair the insult. These responses are mediated by certain cytokines and growth factors released from the injured tissue and by hematopoetic cells. Chronic inflammation develops when cytokines and growth factors continue to be produced, resulting in an exaggerated healing response. Particularly preferred cytokines include the inflammatory cytokines interleukin 1 alpha and beta, interleukin 2, interleukin 6, interleukin 8, interferon gamma, and tumor necrosis factor. Particularly preferred growth factors include vascular endothelial growth factor (VEGF), and platelet derived growth factor (PDGF).

Matrix metalloproteinases, along with their corresponding inhibitors, tissue inhibitors of metalloproteinases or TIMP, are involved in the repair of injured tissue. The balance between the proteinases and the inhibitors regulates the pattern and extent of wound healing. Excessive production of MMP or insufficient production of TIMP results in abnormal wound healing. Particularly preferred matrix metalloproteinases include MMP 1, MMP 2, MMP 3 and MMP 9.

Preferably, the ribozyme is a hammerhead or hairpin ribozyme, representative examples of which recognize the target site sequences set forth below and in the Examples. In preferred embodiments, the present invention also provides nucleic acid molecule encoding such ribozymes; further preferably, the nucleic acid is DNA or cDNA. Even further preferably, the nucleic acid molecule is under the control of a promoter to transcribe the nucleic acid.

In one embodiment of the invention, nucleic acid molecules are described which encode the ribozymes provided herein. Preferably, the vector is a plasmid, a virus, retrotransposon, a cosmid or a retrovirus. In one embodiment where the vector is a retroviral vector, the nucleic acid molecule encoding the ribozyme under the control of a promoter, which is preferably a pol III promoter, further preferably a human tRNA^{Val} promoter or an adenovirus VA1 promoter, is inserted between the 5' and 3' long terminal repeat sequences of the retrovirus.

These and other aspects of the present invention will become evident upon reference to the following detailed description and attached drawings. In addition, various references are set forth herein that describe in more detail certain procedures or compositions (e.g., plasmids, etc.), and are therefore incorporated by reference in their entirety as if each were individually noted for incorporation.

### BRIEF DESCRIPTION OF THE DRAWINGS

Figure 1 is a schematic illustration of which shows the general structure of a chimeric DNA/RNA ribozyme (SEQ ID NOs: 4385 and 4386).
Figure 2 is a photograph of a gel which shows the stability of chimeric ribozymes PN30003, 30004, and 30005 in human vascular smooth muscle cell lysate.
Figure 3 is a photograph of a gel which shows the stability of chimeric ribozymes PN30003 and 30005 in serum.
Figure 4 is a schematic illustration of a propanediol linker.
Figure 5 is a schematic illustration of vector pLNT-Rz.
Figure 6 is a schematic illustration of a representative hairpin ribozyme (SEQ ID NOs: 4387 and 4388).
Figure 7 is a photograph of treated and control skin from a porcine model of scar formation.
Figure 8 is a photograph of control eyes and eyes treated with chimeric PCNA targeted ribozyme from a rabbit dispase model of proliferative vitreoretinopathy.
Figure 9 is a chart of the cumulative transdermal absorption of ³²P-ribozyme presented as the percent of the applied dose recovered in the reservoir of diffusion chambers.
Figure 10 is a chart of the cumulative transdermal absorption of ³²P-ribozyme presented as total micrograms recovered in the reservoir of diffusion chambers.
Figure 11 is a chart of the localization of ³²P-ribozyme in various skin compartments presented as percent of the applied dose recovered in each compartment.
Figure 12 is a chart of the localization of ³²P-ribozyme in various skin compartments presented as total micrograms in each compartment.
Figure 13 is a chart of histopathologic scoring of chimeric PCNA targeted ribozyme versus control treated rabbit eyes. Scoring is based on a modified Fastenberg scale (Table 22) and performed blindly by two trained observers. P is less than 0.0008 by T test.
Figure 14A and Figure 14B are two photographs of a surgical wound model in pig skin. Figure 14A shows the dimensions of the wound (approximately 3 cm long, 5 mm deep and 6 mm wide). Figure 14B shows excision of the skin and closure with a single suture.

### DETAILED DESCRIPTION OF THE INVENTION

### DEFINITIONS

Prior to setting forth the invention, it may be helpful to an understanding thereof to first set forth definitions of certain terms that will be used hereinafter.

"Ribozyme" refers to a nucleic acid molecule which is capable of cleaving a specific nucleic acid sequence. Ribozymes may be composed of RNA, DNA, nucleic acid analogues (e.g., phosphorothioates), or any combination of these (e.g., DNA/RNA chimerics). Within particularly preferred embodiments, a ribozyme should be understood to refer to RNA molecules that contain anti-sense sequences for specific recognition, and an RNA-cleaving enzymatic activity.

"Ribozyme gene" refers to a nucleic acid molecule (e.g., DNA) consisting of the ribozyme sequence which, when transcribed into RNA, will yield the ribozyme.

"Vector" refers to an assembly which is capable of expressing a ribozyme of interest. The vector may be composed of either deoxyribonucleic acids ("DNA") or ribonucleic acids ("RNA"). Optionally, the vector may include a polyadenylation sequence, one or more restriction sites, as well as one or more selectable markers such as neomycin phosphotransferase, hygromycin phosphotransferase or puromycin-N-acetyl-transferase. Additionally, depending on the host cell chosen and the vector employed, other genetic elements such as an origin of replication, additional nucleic acid restriction sites, enhancers, sequences conferring inducibility of transcription, and selectable markers, may also be incorporated into the vectors described herein.

"Nucleic acid" or "nucleic acid molecule" refers to any of deoxyribonucleic acid (DNA), ribonucleic acid (RNA), oligonucleotides, fragments generated by the polymerase chain reaction (PCR), and fragments generated by any of ligation, scission, endonuclease action, and exonuclease action. Nucleic acids can be composed of monomers that are naturally-occurring nucleotides (such as deoxyribonucleotides and ribonucleotides), or analogs of naturally-occurring nucleotides (e.g., α-enantiomeric forms of naturally-occurring nucleotides), or a combination of both. Modified nucleotides can have modifications in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid" also includes so-called "peptide nucleic acids," which comprise naturally-occurring or modified nucleic acid bases attached to a polyamide backbone. Nucleic acids can be either single stranded or double stranded.

"Isolated nucleic acid molecule" is a nucleic acid molecule that is not integrated in the genomic DNA of an organism. For example, a DNA molecule that encodes a gene that has been separated from the genomic DNA of a eukaryotic cell is an isolated DNA molecule. Another example of an isolated nucleic acid molecule is a chemically-synthesized nucleic acid molecule that is not integrated in the genome of an organism.

"Promoter" is a nucleotide sequence that directs the transcription of a structural gene. Typically, a promoter is located in the 5' region of a gene, proximal to the transcriptional start site of a structural gene. If a promoter is an inducible promoter, then the rate of transcription increases in response to an inducing agent. In contrast, the rate of transcription is not regulated by an inducing agent if the promoter is a constitutive promoter.

As noted above, proliferative diseases such as psoriasis, PDR, and scarring affect many individuals and represent a major cost to the health care system. As discussed in more detail below, by interfering with cell-cycle control of cells which might otherwise proliferate, proliferative diseases such as psoriasis or PDR can be effectively treated. This invention accomplishes such by providing ribozymes and methods of using ribozymes that inhibit the activity of cytokines which are involved in inflammation, matrix metalloproteinases, cyclins, cell-cycle dependent kinases, growth factors, reductases, all of which directly or indirectly contribute to proliferation.

Cyclins and cell-cycle dependent kinases are directly involved in the signaling and synthesis that results in cells progressing through the cell cycle to replicate. Representative examples of suitable ribozyme targets include cdk1 ribozyme binding sites (SEQ ID NOS: 1-149); cdk2 ribozyme binding sites (SEQ ID NOS: 150-3010); cdk3 ribozyme binding sites (SEQ ID NOS: 302-405); cdk4 ribozyme binding sites (SEQ ID NOS: 406-526); cdk6 ribozyme binding sites (SEQ ID NOS: 527-665); cdk7 ribozyme binding sites (SEQ ID NOS: 666-866); cdk8 ribozyme binding sites (SEQ ID NOS: 867-1112); cdk-we-hu ribozyme binding sites (SEQ ID NOS: 1113-1408); cyclin A2 ribozyme binding sites (SEQ ID NOS: 1409-1614); cyclin C ribozyme binding sites (SEQ ID NOS: 1615-1819); cyclin D1 ribozyme binding sites (SEQ ID NOS: 1820-1889); cyclin D2 ribozyme binding sites (SEQ ID NOS: 1890-1975); cyclin D3 ribozyme binding sites (SEQ ID NOS: 1976-2053); cyclin E ribozyme binding sites (SEQ ID NOS: 2054-2318); cyclin F ribozyme binding sites (SEQ ID NOS: 2319-2561); cyclin G1 ribozyme binding sites (SEQ ID NOS: 2562-2787); cyclin H ribozyme binding sites (SEQ ID NOS: 2788-2964); cyclin A1 ribozyme binding sites (SEQ ID NOS: 2965-3257); cyclin B1 ribozyme binding sites (SEQ ID NOS: 3258-3478); cdc25 hs ribozyme binding sites (SEQ ID NOS: 3479-3854); PCNA HH ribozyme binding sites (SEQ ID NOS: 3855-4115); and chimeric hairpin ribozymes: SEQ ID NOS: 4116-4119).

As noted above, infection or injury induces a complex cascade of events including activation of the clotting pathway, adherence of immune cells to the endothelium, induction of proliferation and migration of cells into the injury site to rapidly repair the insult. These responses are mediated by certain cytokines and growth factors released from the injured tissue and by hematopoetic cells. Chronic inflammation develops when cytokines and growth factors continue to be produced, resulting in an exaggerated healing response. Particularly preferred cytokines include the inflammatory cytokines interleukin 1 alpha and beta, interleukin 2, interleukin 6, interleukin 8, interferon gamma, and tumor necrosis factor. Particularly preferred growth factors include vascular endothelial growth factor (VEGF), and platelet derived growth factor (PDGF).

Matrix metalloproteinases, along with their corresponding inhibitors, tissue inhibitors of metalloproteinases or TIMP, are involved in a the repair of injured tissue. The balance between the proteinases and the inhibitors regulates the pattern and extent of wound healing. Excessive production of MMP or insufficient production of TIMP results in abnormal wound healing. Particularly preferred matrix metalloproteinases include MMP 1, MMP 2, MMP 3 and MMP 9.

### RIBOZYMES

As noted above, the present invention provides ribozymes having the ability to cleave or otherwise inhibit nucleic acid molecules which are either directly, or indirectly (e.g., they encode proteins) involved in cell-cycle control. Several different types of ribozymes may be constructed for use within the present invention, including for example, hammerhead ribozymes (Rossi, J.J. et al., *Pharmac. Ther. 50*:245-254, 1991) (Forster and Symons, *Cell 48*:211-220, 1987; Haseloff and Gerlach, *Nature 328*:596-600, 1988; Walbot and Bruening, *Nature 334*:196, 1988; Haseloff and Gerlach, *Nature 334*:585, 1988; Haseloff et al., U.S. Patent No. 5,254,678), hairpin ribozymes (Hampel et al., *Nucl. Acids Res. 18*:299-304, 1990, and U.S. Patent No. 5,254,678), hepatitis delta virus ribozymes (Perrotta and Been, *Biochem. 31:16,* 1992), Group I intron ribozymes (Cech et al., U.S. Patent No. 4,987,071) and RNase P ribozymes (Takada et al., *Cell 35*:849, 1983); (*see* also, WO 95/29241, entitled "Ribozymes with Product Ejection by Strand Displacement"; and WO 95/31551, entitled "Novel Enzymatic RNA Molecules."

Cech et al. (U.S. Patent No. 4,987,071, issued January 22, 1991) has disclosed the preparation and use of ribozymes which are based on the properties of the *Tetrahymena* ribosomal RNA self-splicing reaction. These ribozymes require an eight base pair target site and free guanosine (or guanosine derivatives). A temperature optimum of 50°C is reported for the endoribonuclease activity. The fragments that arise from cleavage contain 5'-phosphate and 3'-hydroxyl groups and a free guanosine nucleotide added to the 5'-end of the cleaved RNA.

In contrast to the ribozymes of Cech et al., particularly preferred ribozymes of the present invention hybridize efficiently to target sequences at physiological temperatures, making them suitable for use *in vivo,* and not merely as research tools *(see* column 15, lines 18 to 42, of Cech et al., U.S. Patent No. 4,987,071). Thus, particularly preferred ribozymes for use within the present invention include hairpin ribozymes (for example, as described by Hampel et al., European Patent Publication No. 0 360 257, published March 26, 1990) and hammerhead ribozymes. Briefly, the sequence requirement for the hairpin ribozyme is any RNA sequence consisting of NNNBN*GUC(N)ₓ (Sequence ID Nos.4120-4124) (where x is any number from 6 to 10, N*G is the cleavage site, B is any of G, C, or U, and N is any of G, U, C, or A). Representative examples of recognition or target sequences for hairpin ribozymes are set forth below in the Examples. Additionally, the backbone or common region of the hairpin ribozyme can be designed using the nucleotide sequence of the native hairpin ribozyme (Hampel et al., *Nucl. Acids Res. 18*:299-304, 1990) or it can be modified to include a "tetraloop" structure that increases stability and catalytic activity (see Example 2; see also Yu et al., *Virology 206*:381-386, 1995; Cheong et al., *Nature* 346:680-682, 1990; Anderson et al., *Nucl. Acids Res. 22*:1096-1100, 1994).

The sequence requirement at the cleavage site for the hammerhead ribozyme is any RNA sequence consisting of NUH (where N is any of G, U, C, or A and H represents C, U, or A) can be targeted. Accordingly, the same target within the hairpin leader sequence, GUC, is useful for the hammerhead ribozyme. The additional nucleotides of the hammerhead ribozyme or hairpin ribozyme is determined by the target flanking nucleotides and the hammerhead consensus sequence *(see* Ruffner et al., *Biochemistry* 29:10695-10702, 1990). This information, along with the sequences and disclosure provided herein, enables the production of hairpin ribozymes of this invention.

The ribozymes of this invention, as well as DNA encoding such ribozymes and other suitable nucleic acid molecules, described in more detail below, can be chemically synthesized using methods well known in the art for the synthesis of nucleic acid molecules (*see e.g.,* Heidenreich et al., *J. FASEB 70*(1):90-6, 1993; Sproat, *Curr. Opin. Biotechnol. 4*(1):20-28, 1993). Alternatively, commercial suppliers such as Promega, Madison, Wis., USA, provide a series of protocols suitable for the production of nucleic acid molecules such as ribozymes.

Within one aspect of the present invention, ribozymes are prepared from a DNA molecule or other nucleic acid molecule (which, upon transcription, yields an RNA molecule) operably linked to an RNA polymerase promoter, e.g., the promoter for T7 RNA polymerase or SP6 RNA polymerase. Accordingly, also provided by this invention are nucleic acid molecules, *e.g.,* DNA or cDNA, coding for the ribozymes of this invention. When the vector also contains an RNA polymerase promoter operably linked to the DNA molecule, the ribozyme can be produced *in vitro* upon incubation with the RNA polymerase and appropriate nucleotides. In a separate embodiment, the DNA may be inserted into an expression cassette, such as described in Cotten and Bimstiel, *EMBO J. 8*(12):3861-3866, 1989, and in Hempel et al., *Biochemistry* 28:4929-4933, 1989. A more detailed discussion of molecular biology methodology is disclosed in Sambrook et al., *Molecular Cloning: A Laboratory Manual,* Cold Spring Harbor Press, 1989.

During synthesis, the ribozyme can be modified by ligation to a DNA molecule having the ability to stabilize the ribozyme and make it resistant to RNase (Rossi et al., *Pharmac. Ther. 50*:245-254, 1991). In another embodiment, the ribozyme can be modified to a phosphothio-analog for use in liposome delivery systems. This modification also renders the ribozyme resistant to endonuclease activity. In yet another embodiment, the ribozyme can be modified to contain propanediol linkages or to incorporate 2'-O-methylated nucleotides.

### VECTORS

Use of ribozymes to treat proliferative skin diseases such as psoriasis or eczema involves introduction of functional ribozyme to the infected cell of interest. This can be accomplished by either synthesizing functional ribozyme *in vitro* prior to delivery, or, by delivery of DNA capable of driving ribozyme synthesis *in vivo.*

More specifically, within other aspects of the invention the ribozyme gene may be constructed within a vector which is suitable for introduction to a host cell *(e.g.,* prokaryotic or eukaryotic cells in culture or in the cells of an organism). Appropriate prokaryotic and eukaryotic cells can be transfected with an appropriate transfer vector containing the nucleic acid molecule encoding a ribozyme of this invention.

To produce the ribozymes with a vector *in vivo,* nucleotide sequences coding for ribozymes are preferably placed under the control of a eukaryotic promoter such as pol III (*e*.*g*., tRNA or VA-1 from adenovirus), CMV, SV40 late, or SV40 early promoters. Within certain embodiments, the promoter may be a tissue or cell-specific promoter. Ribozymes may thus be produced directly from the transfer vector *in vivo*.

A wide variety of vectors may be utilized within the context of the present invention, including for example, plasmids, viruses, retrotransposons and cosmids. Representative examples include adenoviral vectors (*e*.*g*., WO 94/26914, WO 93/9191; Yei et al., *Gene Therapy 1*:192-200, 1994; Kolls et al., *PNAS 91*(1):215-219, 1994; Kass-Eisler et al., *PNAS 90*(24):11498-502, 1993; Guzman et al., *Circulation 88*(6):2838-48, 1993; Guzman et al., *Cir. Res. 73*(6):1202-1207, 1993; Zabner et al., *Cell 75*(2):207-216, 1993; Li et al., *Hum Gene Ther. 4*(4):403-409, 1993; Caillaud et al., *Eur. J. Neurosci. 5*(10):1287-1291, 1993), adeno-associated type 1 ("AAV-1") or adeno-associated type 2 ("AAV-2") vectors (*see* WO 95/13365; Flotte et al., *PNAS 90*(22):10613-10617, 1993), hepatitis delta vectors, live, attenuated delta viruses and herpes viral vectors (*e*.*g*., U.S. Patent No. 5,288,641), as well as vectors which are disclosed within U.S. Patent No. 5,166,320. Other representative vectors include retroviral vectors (e.g., EP 0 415 731; WO 90/07936; WO 91/02805; WO 94/03622; WO 93/25698; WO 93/25234; U.S. Patent No. 5,219,740; WO 93/11230; WO 93/10218). General methods of using such vectors in gene therapy are well known in the art (see, for example, Larrick, J.W. and Burck, K.L., *Gene Therapy: Application of Molecular Biology,* Elsevier Science Publishing Co., Inc., New York, New York, 1991 and Kreigler, M., *Gene Transfer and Expression: A Laboratory Manual*, W.H. Freeman and Company, New York, 1990).

Further provided by this invention are vectors having more than one nucleic acid molecule encoding a ribozyme of this invention, each molecule under the control of a separate eukaryotic promoter (or, an Internal Ribosome Entry Site or "IRES") or alternatively, under the control of single eukaryotic promoter. Representative examples of other nucleic acid molecules which may be delivered by the vectors of the present invention include therapeutic molecules such as interferon (e.g., alpha, beta or gamma), as well as a wide variety of other cytokines or growth factors, and facilitators which assist or aid ribozymes in cleaving a target sequence by unwinding or otherwise limiting secondary folding which might otherwise inhibit the ribozyme . These vectors provide the advantage of providing multi-functional therapy against Psoriasis, preferably with the various therapies working together in synergy.

Host prokaryotic and eukaryotic cells stably harboring the vectors described above also are provided by this invention. Suitable host cells include bacterial cells, rat cells, mouse cells, and human cells.

### DELIVERY

Within certain aspects of the invention, ribozyme molecules, or nucleic acid molecules which encode the ribozyme, may be introduced into a host cell or administered to a patient utilizing a vehicle, or by various physical methods. Representative examples of such methods include transformation using calcium phosphate precipitation (Dubensky et al., *PNAS 81*:7529-7533, 1984), direct microinjection of such nucleic acid molecules into intact target cells (Acsadi et al., *Nature 352*:815-818, 1991), and electroporation whereby cells suspended in a conducting solution are subjected to an intense electric field in order to transiently polarize the membrane, allowing entry of the nucleic acid molecules. Other procedures include the use of nucleic acid molecules linked to an inactive adenovirus (Cotton et al., *PNAS 89*:6094, 1990), lipofection (Felgner et al., *Proc. Natl. Acad. Sci. USA 84*:7413-7417,1989), microprojectile bombardment (Williams et al., *PNAS 88*:2726-2730, 1991), polycation compounds such as polylysine, receptor specific ligands, liposomes entrapping the nucleic acid molecules, spheroplast fusion whereby *E. coli* containing the nucleic acid molecules are stripped of their outer cell walls and fused to animal cells using polyethylene glycol, viral transduction, (Cline et al., *Pharmac. Ther. 29*:69, 1985; and Friedmann et al., *Science 244*:1275, 1989), and DNA ligand (Wu et al, *J. of Biol. Chem. 264*:16985-16987, 1989). In one embodiment, the ribozyme is introduced into the host cell using a liposome.

Within further embodiments of the invention, additional therapeutic molecules (e.g., interferon) or facilitators may be delivered utilizing the methods described herein. Such delivery may be either simultaneous to, or before or after the delivery of a ribozyme or vector expressing ribozymes.

### PHARMACEUTICAL COMPOSITIONS

As noted above, pharmaceutical compositions (or "medicaments") also are provided by this invention. These compositions contain any of the above described ribozymes, DNA molecules, vectors or host cells, along with a pharmaceutically or physiologically acceptable carrier, excipient, or, diluent. Generally, such carriers should be nontoxic to recipients at the dosages and concentrations employed. Ordinarily, the preparation of such compositions entails combining the therapeutic agent with buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with nonspecific serum albumin are exemplary appropriate diluents. Particularly preferred carriers include cholesterols such as DOTAP:cholesterol.

Additional ingredients added to the compositions for the express purpose of inhibiting endogenous ribonucleases include reducing agents such as dithiothreitol, detergents such as sodium dodecyl sulfate, and other agents such as vanidyl nucleotides, aurin tricarbocylic acid, hydrogen peroxide and RNA decoys such as tRNA.

Pharmaceutical compositions of the present invention may also be prepared to contain; or express (*e*.*g*., if a vector), one or more additional therapeutic molecules (e.g., interferon) or facilitators.

The pharmaceutical compositions of the present invention may be prepared for administration by a variety of different routes, including for example, topically, intradermally, or intraocullarly. In addition, pharmaceutical compositions of the present invention may be placed within containers, along with packaging material which provides instructions regarding the use of such pharmaceutical compositions. Generally, such instructions will include a tangible expression describing the reagent concentration, as well as within certain embodiments, relative amounts of excipient ingredients or diluents (*e*.*g*., water, saline or PBS) which may be necessary to reconstitute the pharmaceutical composition

Pharmaceutical compositions of the present invention are useful for both diagnostic and therapeutic purposes.

### THERAPEUTIC METHODS

Methods of treating proliferative diseases such as psoriasis or PDR are also provided by this invention. More specifically, within one aspect of the present invention proliferative diseases such as psoriasis or PDR may be treated by administering to a warm-blooded animal (e.g., a human) a therapeutically effective amount of ribozyme, and/or, nucleic acid molecule or vector which encodes the ribozyme.

Briefly, cells in response to injury or other insult typically follow at least one of two possible pathways: normal wound healing or exaggerated growth. For normal wound healing accelerated growth and maturation of cells occurs in order to allow healing of the wound as soon as possible.

Proliferative diseases such as psoriasis, scarring and PDR are similar in many respects to the process of wound healing. Namely, cells are created and migrate in a relatively short period of time. However, if the proliferation is too rapid or the signals to proliferate continue for too long a period of time, the cells build up to form thickened lesions. This growth is supported by new blood vessels, as well as the infiltration of a variety of lymphocytes which produce a wide variety of growth factors (that further increases the proliferation of the cells). These cells can produce tissue degrading enzymes which cause the destruction of surrounding tissue. In some cases, the thickened lesions contract, distorting the surface of the skin or retina. The result of the exaggerated growth is the clinical symptoms associated with the proliferative disease.

The present invention provides the treatment of proliferative skin and eye diseases by contacting desired cells with an effective amount of ribozyme of this invention or, alternatively, by transducing the cell with an effective amount of vector having a nucleic acid molecule encoding the ribozyme. A suitable "therapeutically effective amount" will depend on the nature and extent of diseased tissue being treated, or, if a medical procedure is contemplated in which abnormal proliferation can be expected, prevented. Such "therapeutically effective amounts" can be readily determined by those of skill in the art using well known methodology, and suitable animal models (e.g. a rat, rabbit, or porcine model), or, based upon clinical trials. As utilized herein, a patient is deemed to be "treated" if the proliferative skin or eye disease is reversed or inhibited in a patient in a quantifiable manner. Further, a therapeutically effective amount or regimen of treatment should result in: (1) decrease in the frequency, severity, or, duration of clinical symptoms (e.g., inflammation, thickening of the tissue, contraction, scaling, "burning", or itching); (2) increase of time in the period of remission; (3) a change in pathological symptoms (e.g., inhibition of keratinocyte, fibroblast, glia, or retinal pigment epithelium proliferation); (4) prevention of retinal detachment; and/or (5) prevention of visual impairment.

When exogenously delivering the ribozyme, the RNA molecule can be embedded within a stable RNA molecule or in another form of protective environment, such as a liposome. Alternatively, the RNA can be embedded within RNase-resistant DNA counterparts. Cellular uptake of the exogenous ribozyme can be enhanced by attaching chemical groups to the DNA ends, such as cholesteryl moieties (Letsinger et al., *P.N.A.S., U.S.A.*, 1989).

In another aspect of the invention, the target cell is transduced under conditions favoring insertion of the vector into the target cell and stable expression of the nucleic acid encoding the ribozyme. The target cell can include but is not limited to cells found in the skin basal level, dermis, or epidermis; the vitreous of the eye, and the retinal and pigment epithelium layers of the eye.

In addition, the ribozyme, ribozyme gene or vector may be readily incorporated into a biodegradable polymer, sphere, pleuroinc gel, or the like to aid incorporation into cells.

The ribozyme (or nucleic acid molecule or vector encoding the ribozyme) can be administered in any manner sufficient to achieve the above therapeutic results, but preferred methods include topical and systemic administration. Patients with localized disease can be administered ribozymes (or a nucleic acid molecule or vector) in a topical cream, ointment or emollient applied directly to skin lesions. For example, a topical cream containing 100 milligrams ribozymes by weight can be administered depending upon severity of the disease and the patient's response to treatment. In a preferred embodiment, a topical preparation containing ribozymes 100 milligrams by weight would be administered to psoriatic lesions. Alternatively, direct intracutaneous or intraocular injection of ribozymes in a suitable pharmaceutical vehicle can be used for the management of individual lesions.

The ribozyme may be formulated along with a liquid (e.g., DOTAP:cholesterol). In addition, the ribozyme may be formulated with ribonuclease inhibitors, including, but not limited to, reducing agent (*e.g*., dithiothreitol), detergent (*e*.*g*., sodium dodecyl sulfate), vanidyl nucleotides, aurin tricarboxcylic acid, hydrogen peroxide, and RNA decoys (e.g., tRNAs).

The following examples are offered by way of illustration, and not by way of limitation.

### EXAMPLES

### EXAMPLE 1

### CRITERIA FOR RIBOZYME SITE SELECTION

### A. Selection of Sites for Hairpin Ribozymes

Hairpin ribozymes suitable for use within the present invention preferably recognize the following sequence of RNA: NNNBNGUCNNNNNNNN (SEQ ID NO:4122) wherein the ribozyme is constructed so as to be complementary to the underlined sequences, and wherein B is C, G or U. The sequence GUC must be conserved for all hairpin ribozymes described below. Other nucleotides ("N" as underlined above) preferably have a high degree of sequence conservation in order to limit the need for multiple ribozymes against the same target site.

### B. Selection of Cleavage Sites for Hammerhead Ribozymes

Hammerhead ribozymes suitable for use within the present invention preferably recognize the sequence NUH, wherein N is any of G, U, C, or A and H is C, U, or A. The hairpin recognition sites GUC are a subset of the hammerhead recognition sites NUH. Therefore, all hairpin sites are by definition also hammerhead sites, although the converse is not true. Representative GUC hairpin/hammerhead ribozyme recognition sites for various genes are provided below in Tables 1-16. Hammerhead only sites are shown in Table18.

**Table 1 Hairpin/Hammerhead Ribozyme Recognition Sites for cdc 2 kinase**

| NUCL. POS. | SEQUENCE (5' to 3') | I.D. No. |
|---|---|---|
| 175 | ACTTCGTCATCCAAAT | 4125 |
| 189 | ATATAGTCAGTCTTCA | 4126 |
| 193 | AGTCAGTCTTCAGGAT | 4127 |
| 289 | TCCTGGTCAGTACATG | 4128 |
| 355 | GTTTTGTCACTCTAGA | 4129 |
| 530 | CTGGGGTCAGCTCGTT | 4130 |

**Table 2 Hairpin/Hammerhead Ribozyme Recognition Sites for Cyclin B 1**

| NUCL. POS. | SEQUENCE (5' to 3') | I.D. No. |
|---|---|---|
| 13 | TCCGAGTCACCAGGAA | 4131 |
| 282 | CCAGTGTCTGAGCCAG | 4132 |
| 428 | CCTGTGTCAGGCTTTC | 4133 |
| 559 | AAGCAGTCAGACCAAA | 4134 |
| 581 | ACTGGGTCGGGAAGTC | 4135 |
| 679 | TGACTGTCTCCATTAT | 4136 |
| 745 | TTGGTGTCACTGCCAT | 4137 |
| 887 | CTTTGGTCTGGGTCGG | 4138 |
| 893 | TCTGGGTCGGCCTCTA | 4139 |
| 1107 | TACCTGTCATATACTG | 4140 |
| 1162 | ATGTAGTCATGGTAAA | 4141 |
| 1198 | TGACTGTCAAGAACAA | 4142 |

**Table 3 Hairpin/Hammerhead Ribozyme Recognition Sites for PCNA**

| UCL. POS. | SEOUENCE (5' to 3') | I.D. No. |
|---|---|---|
| -131 | GAGTGGTCGTTGTCTT | 4143 |
| -125 | TCGTTGTCTTTCTAGG | 4144 |
| 19 | GCCTGGTCCAGGGCTC | 4145 |
| 126 | GACTCGTCCCACGTCT | 4146 |
| 159 | CTGCGGTCTGAGGGCT | 4147 |
| 546 | AAATTGTCACAGACAA | 4148 |
| 868 | TTTCTGTCACCAAATT | 4149 |
| 983 | ATCTGGTCTAGTTAAC | 4150 |
| 1008 | TTTTTGTCTCTTAGAA | 4151 |
| 1047 | AAAGGGTCTTGACTCT | 4152 |

**Table 4 Hairpin/Hammerhead Ribozyme Recognition Sites for MMP-1**

| NUCL. POS. | SEQUENCE (5' to 3') | I.D. No. |
|---|---|---|
| 48 | GTGG TGTC TCACAGCT | 4389 |
| 301 | AGTT TGTC CTCACTGA | 4390 |
| 454 | CCAA GGTC TCTGAGGG | 4391 |
| 464 | TGAG GGTC AAGCAGAC | 4392 |
| 799 | ATCC TGTC CAGCCCAT | 4393 |
| 1446 | TGTG AGTC CAAAGAAG- | 4394 |
| 1475 | GAAC TGTC TATTTTCT | 4395 |
| 1488 | TCTC AGTC ATTTTTAA | 4396 |
| 1507 | CTAG AGTC ACTGATAC | 4397 |
| 1766 | CATG AGTC TTTGCCGG | 4398 |

**Table 5 Hairpin/Hammerhead Ribozyme Recognition Sites for IL-1 beta**

| UCL. POS. | SEQUENCE (5' to 3') | I.D. No. |
|---|---|---|
| 13 | caag tgtctgaagcag | 4399 |
| 195 | gccg cgtcagttgttg | 4400 |
| 408 | gtga tgtctggtccat | 4401 |
| 413 | gtct ggtccatatgaa | 4402 |
| 480 | tcca tgtcctttgtac | 4403 |
| 555 | tacc tgtcctgcgtgt | 4404 |
| 646 | gatt tgtcttcaacaa | 4405 |
| 687 | tttg agtctgcccagt | 4406 |
| 782 | tgcc cgtcttcctggg | 4407 |
| 801 | tttg tgtcttcctaaa | 4408 |
| 830 | agag agtcctgtgctg | 4409 |
| 921 | ctgt tgtctacaccaa | 4410 |

**Table 6 Hairpin/Hammerhead Ribozyme Recognition Sites for VEGF**

| UCL. POS. | SEQUENCE (5' to 3') | I.D. No. |
|---|---|---|
| 83 | CTGCTgtcTTGGGTGC | 4411 |
| 136 | AAGTGgtcCCAGGCTG | 4412 |
| 364 | GAGGAgtcCAACATCA | 4413 |

**Table 7 Hairpin/Hammerhead Ribozyme Recognition Sites for IL2**

| UCL. POS. | SEQUENCE (5' to 3') | I.D. No. |
|---|---|---|
| 68 | caactcctgtcttgcattgca | 4414 |
| 96 | ttgcacttgtcacaaacagtg | 4415 |
| 289 | gcttcagtgtctagaagaaga | 4416 |
| 450 | taccttttgtcaaagcatcat | 4417 |

**Table 8 Hairpin/Hammerhead Ribozyme Recognition Sites for IFN gamma**

| UCL. POS. | SEQUENCE (5' to 3') | I.D. No. |
|---|---|---|
| 246 | gccaaattgtctccttttact | 4418 |
| 458 | gctgaactgtcgccagcagct | 4419 |
| 493 | aaaaaggagtcagatgctgtt | 4420 |
| 534 | gtaatggttgtcctgcctgcaa | 4421 |
| 718 | ctgtgactgtctcacttaatc | 4422 |
| 958 | ttgaatgtgtcaggtgaccct | 4423 |

**Table 9 Hairpin/Hammerhead Ribozyme Recognition Sites for IL1 alpha**

| UCL. POS. | SEQUENCE (5' to 3') | I.D. No. |
|---|---|---|
| 19 | GCGTTTGAGTCAGCAAAGAAG | 4424 |
| 120 | GATCATCTGTCTCTGAATCAG | 4425 |
| 192 | CAATCTGTGTCTCTGAGTATC | 4426 |
| 860 | GGTCTGGAGTCTCACTTGTCT | 4427 |
| 869 | TCTCACTTGTCTCACTTGTGC | 4428 |
| 938 | TACTGTTAGTCATTTGCTGAG | 4429 |
| 1624 | GCATTTTGGTCCAAGTTGTGC | 4430 |
| 1729 | AGTAATTGGTCCGATCTTTGA | 4431 |
| 1921 | CCGTGCTGGTCTCGAACTTCT | 4432 |
| 2063 | AATCCTCAGTCAGCCGTGTTT | 4433 |
| 2404 | TTCTTGTCCCAAATAAAA | 4434 |

**Table 10 Hairpin/Hammerhead Ribozyme Recognition Sites for IL6**

| NUCL. POS. | SEQUENCE (5' to 3') | I.D. No. |
|---|---|---|
| 79 | CGCCTTCGGTCCAGTTGCCTT | 4435 |
| 403 | CATCACTGGTCTTTTGGAGTT | 4436 |
| 659 | TTCCTGCAGTCCAGCCTGAGG | 4437 |
| 736 | TTCTTCTGGTCAGAAACCTGT | 4438 |
| 747 | AGAAACCTGTCCACTGGGCAC | 4439 |

**Table 11 Hairpin/Hammerhead Ribozyme Recognition Sites for IL8**

| NUCL. POS. | SEQUENCE (5' to 3') | I.D. No. |
|---|---|---|
| 493 | ttgtgtgggtctgttgtaggg | 4440 |
| 832 | agagctctgtctggaccccaa | 4441 |
| 873 | caaccctagtctgctagccag | 4442 |

**Table 12 Hairpin/Hammerhead Ribozyme Recognition Sites for MMP2**

| UCL. POS. | SEQUENCE (5' to 3') | I.D. No. |
|---|---|---|
| 46 | GCCGCGCCGTCGCCCATCATC | 4443 |
| 614 | GCCAAGTGGTCCGTGTGAAGT | 4444 |
| 774 | CGGCTTCTGTCCCCATGAAGC | 4445 |
| 1001 | CCCCCTGTGTCTTCCCCTTCA | 4446 |
| 1248 | GAACTTCCGTCTGTCCCAGGA | 4447 |
| 1252 | TTCCGTCTGTCCCAGGATGAC | 4448 |
| 1343 | TGGGCCCTGTCACTCCTGAGA | 4449 |
| 1811 | TGGATGCCGTCGTGGACCTGC | 4450 |
| 1832 | GGGCGGCGGTCACAGCTACTT | 4451 |
| 2312 | TGGAGACTGTCTCAAGAGGGC | 4452 |
| 2472 | TTCTTTGGGTCTTGTTTTTTT | 4453 |

**Table 13 Hairpin/Hammerhead Ribozyme Recognition Sites for MMP3**

| UCL. POS. | SEQUENCE (5' to 3') | I.D. No. |
|---|---|---|
| 8 | atgaagagtcttccaatcct | 4454 |
| 173 | ggacagtggtcctgttgttaa | 4455 |
| 293 | tgacgttggtcacttcagaac | 4456 |
| 674 | ctccctgggtctctttcactc | 4457 |
| 753 | ttccgcctgtctcaagatgat | 4458 |
| 780 | ggcattcagtccctctatgga | 4459 |
| 838 | cggaacctgtccctccagaac | 4460 |
| 882 | cctgctttgtcctttgatgct | 4461 |
| 895 | ttgatgctgtcagcactctga | 4462 |

**Table 14 Hairpin/Hammerhead Ribozyme Recognition Sites for MMP9**

| UCL. POS. | SEQUENCE (5' to 3') | I.D. No. |
|---|---|---|
| 41 | agcccctggtcctggtgctcc | 4463 |
| 94 | cagcgccagtccacccttgtg | 4464 |
| 208 | cgtggagagtcgaaatctctg | 4465 |
| 253 | aagcaactgtccctgcccgag | 4466 |
| 320 | ggtgcggggtcccagacctgg | 4467 |
| 518 | cagacatcgtcatccagtttg | 4468 |
| 533 | agtttggtgtcgcggagcacg | 4469 |
| 649 | gagttgtggtccctgggcaag | 4470 |
| 665 | gcaagggcgtcgtggttccaa | 4471 |
| 762 | caccgacggtcgctccgacgg | 4472 |
| 936 | cacggacggtcgctccgacgg | 4473 |
| 1061 | agctgtgcgtcttccccttca | 4474 |
| 1350 | cctctatggtcctcgccctga | 4475 |
| 1418 | ccccgacggtctgccccaccg | 4476 |
| 1442 | cccccactgtccacccctcag | 4477 |
| 1548 | gcctttgagtccggtggacga | 4478 |
| 1727 | tggactcggtctttgaggagc | 4479 |
| 1798 | acaggcgcgtcggtgctgggc | 4480 |
| 1818 | cccgaggcgtctggacaagct | 4481 |
| 2151 | gggctcccgtcctgctttgca | 4482 |

**Table 15 Hairpin/Hammerhead Ribozyme Recognition Sites for PDGF A**

| UCL. POS. | SEQUENCE (5' to 3') | I.D. No. |
|---|---|---|
| 29 | ccggccgggtcgctcctgaag | 4483 |
| 125 | ccccttccgtccccaccccca | 4484 |
| 241 | cgggcgccgtccgccagctcc | 4485 |
| 494 | ggcccgcagtcagatccacag | 4486 |
| 589 | ctcacggggtccatgccacta | 4487 |
| 661 | aggaagctgtccccgctgtct | 4488 |
| 670 | tccccgctgtctgcaagacca | 4489 |
| 688 | ccaggacggtcatttacgaga | 4490 |
| 710 | tcctcggagtcaggtcgaccc | 4491 |
| 799 | cgagcagtgtcaagtgccagc | 4492 |
| 820 | cctcccgcgtccaccaccgca | 4493 |
| 835 | accgcagcgtcaaggtggcca | 4494 |
| 981 | cctagggagtcaggcaaaaaa | 4495 |
| 1140 | agtgtgcggtctttgttctcc | 4496 |
| 1168 | aaaactgtgtccgagaacact | 4497 |
| 1269 | gcttccttgtcaaaaagagag | 4498 |

**Table 16 Hairpin/Hammerhead Ribozyme Recognition Sites for PDGF B**

| UCL. POS. | SEQUENCE (5' to 3') | I.D. No. |
|---|---|---|
| 106 | aggttggagtcccctgggcgc | 4499 |
| 437 | ggcccggagtcggcatgaatc | . 4500 |
| 469 | ctcttcctgtctctctgctgc | 4501 |
| 489 | ctacctgcgtctggtcagcgc | 4502 |
| 494 | tgcgtctggtcagcgccgagg | 4503 |
| 893 | tgcgacctgtccaggtgagaa | 4504 |
| 1085 | cggtgcgagtccgccggcccc | 4505 |
| 1265 | tttccctcgtccgtctgtctc | 4506 |
| 1269 | cctcgtccgtctgtctcgatg | 4507 |
| 1273 | gtccgtctgtctcgatgcctg | 4508 |
| 1325 | ctccacgtgtccgtccaccct | 4509 |
| 1329 | acgtgtccgtccacccttcca | 4510 |
| 1349 | atcagcgggtctcctcccag | 4511 |
| 1476 | ctgatggggtcgctctttggg | 4512 |
| 1545 | tgtggactgtcctgaggagcc | 4513 |

**Table 17 Further Ribozyme Recognition Sites**

| TARGET SITE | I.D. No. |
|---|---|
| cdk1 ribozyme binding sites: | 1-149 |
| cdk2 ribozyme binding sites: | 150-301 |
| cdk3 ribozyme binding sites: | 302-405 |
| cdk4 ribozyme binding sites: | 406-526 |
| cdk6 ribozyme binding sites: | 527-665 |
| cdk7 ribozyme binding sites: | 666-866 |
| cdk8 ribozyme binding sites: | 867-1112 |
| cdk-we-hu ribozyme binding sites: | 1113-1408 |
| cyclin A2 ribozyme binding sites: | 1409-1614 |
| cyclin C ribozyme binding sites: | 1615-1819 |
| cyclin D1 ribozyme binding sites: | 1820-1889 |
| cyclin D2 ribozyme binding sites: | 1890-1975 |
| cyclin D3 ribozyme binding sites: | 1976-2053 |
| cyclin E ribozyme binding sites: | 2054-2318 |
| cyclin F ribozyme binding sites: | 2319-2561 |
| cyclin G1 ribozyme binding sites: | 2562-2787 |
| cyclin H ribozyme binding sites: | 2788-2964 |
| cyclin A1 ribozyme binding sites: | 2965-3257 |
| cyclin B 1 ribozyme binding sites: | 3258-3478 |
| cdc25 hs ribozyme binding sites: | 3479-3854 |
| PCNA HH ribozyme binding sites: | 3855-4115 |
| Example chimeric hairpin ribozymes: | 4116-4119 |

**Table 18 Hammerhead Ribozyme Recognition Sites for VEGF**

| UCL. POS.. | SEQUENCE (5' to 3') | I.D. No. |
|---|---|---|
| 118 | TGCTGCTCtACCTCCACCATG | 4514 |
| 206 | TCATGGATGtCTATCAGCGC | 4515 |
| 254 | GGACATCTtCCAGGAGTAC | 4516 |
| 287 | GTACATCTtCAAGCCATCC | 4517 |
| 386 | CATGCAGATtATGCGGATC | 4518 |
| 402 | ATCAAACCtCACCAAGGCC | 4519 |
| 434 | GATGAGCTtCCTACAGCAC | 4520 |
| 451 | AGAAAAAAAAtCAGTTCGAGG | 4521 |
| 541 | GCAAGAAAtCCCGGTATAA | 4522 |
| 663 | GTACAAGAtCCGCAGACGT | 4523 |

### EXAMPLE 2

### CONSTRUCTION OF HAIRPIN RIBOZYMES

Two single-stranded DNA oligonucleotides are chemically synthesized such that, when combined and converted into double-stranded DNA, they contain the entire hairpin ribozyme, including nucleotides complementary to the target site. In addition, restriction enzyme recognition sites may be placed on either end to facilitate subsequent cloning. More specifically, the oligonucleotides are hybridized together and converted to double-stranded DNA using either Klenow DNA polymerase or Taq DNA polymerase. The resulting DNA is cleaved with restriction enzymes *Bam*HI and *Mlu*I, purified and cloned into vectors for *in vitro* transcription (pGEM, ProMega, Madison, Wis.) or for retrovirus production and mammalian expression (pLNL/MJT backbone). Representative hairpin ribozymes are set forth below (note that the underlined sequences indicate the sites wherein the ribozyme binds the target sequence):

cdc-2 530 (Sequence I.D. No. 4378)

5'
AACGAGCTAGAACCAGACCAGAGAAACACACGTTGTGGTATATTACCTGGTA 3'

Cyclin B1 281 (Sequence I.D. No. 4379)
5'
CTGGCTCAAGAACTGGACCAGAGAAACACACGTTGTGGTATATTACCTGGTA 3'

PCNA 158 (Sequence I.D. No. 4381)
5'
AGCCCTCAAGAAGCAGACCAGAGAAACACACGTTGTGGTATATTACCTGGTA 3'

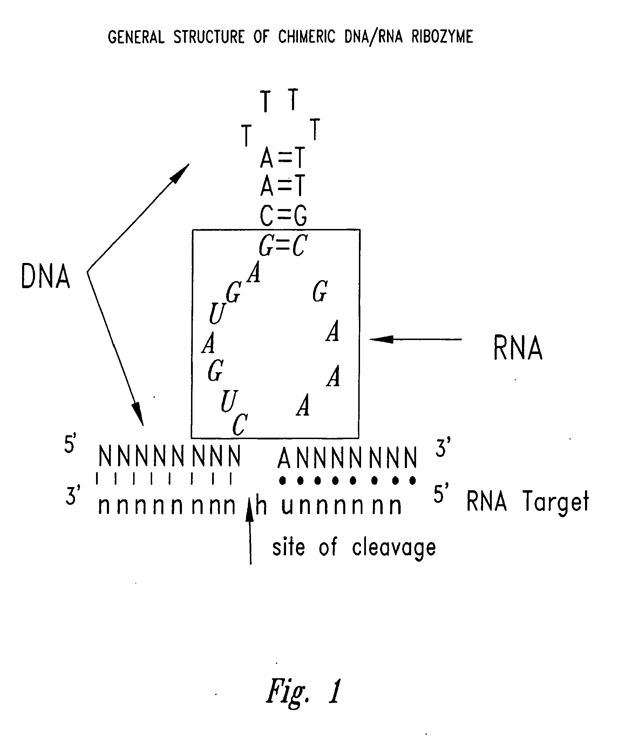

Defective ribozymes for use as controls may be constructed as described above, with the exception that the sequence AAA is changed to a UGC as shown in Figure 1.

### EXAMPLE 3

### CONSTRUCTION OF HAMMERHEAD RIBOZYMES

Chimeric hammerhead ribozymes (i.e., RNA/DNA hybrids) are designed to have an appropriate NUH sequence for ribozyme cleavage. Ribozymes are chemically synthesized with the general structure shown in figure 1. The binding arms bases and stem loop comprise DNA, and the catalytic domain comprises RNA and/or 2'O methyl RNA bases. Specific examples of synthetic human hammerhead ribozymes targeting PCNA are shown below (DNA bases shown in upper case, RNA bases as lower case, 2' O methyl RNA as lower case italics, and propanediol shown as pr pr pr pr):

Sequence ID No. 4382: PN30003 PCN1-HH Length: 40
5' GAGCCCTG cugaugag CAATTTTTTG cgaaa ACCAGGCGC 3'

Sequence ID No. 4383: PN30004 OptPCN1-ome HH Length: 38
5' AGCCC ug cuga u g agg CCGTAAGG cc ga a a cc AGGCGC 3'

Sequence ID No. 4384: PN30005 StabPCN1-ome HH Length: 38
5' AGCCC ugcu ga u g agg CCGTAAGG cc ga a a cc AGGCGC 3'

Sequence ID No. 4385: PN30006 PCN1-ome HH p4 Length: 28
5' AGCCC ug cuga u g ag pr pr pr pr c ga a a cc AGGCGC 3'

Alteration of the base composition at the stem loop and catalytic domain increases the catalytic activity of the chimeric ribozyme as assayed by in vitro cleavage (EXAMPLE 5). The substitution of 2' O methyl bases for RNA bases enhances the stability of the chimeric ribozymes. The assay consists of incubating 10 µg of ribozyme with 100 µl of cell lysate at 37°C for times ranging from 30 seconds to 240 minutes, then separating the intact ribozyme from degradation products on a 15% PAGE, staining with SYBRgreen (Molecular Probes, Eugene, OR), and quantifying by phosphorimager analysis (Molecular Dynamics).

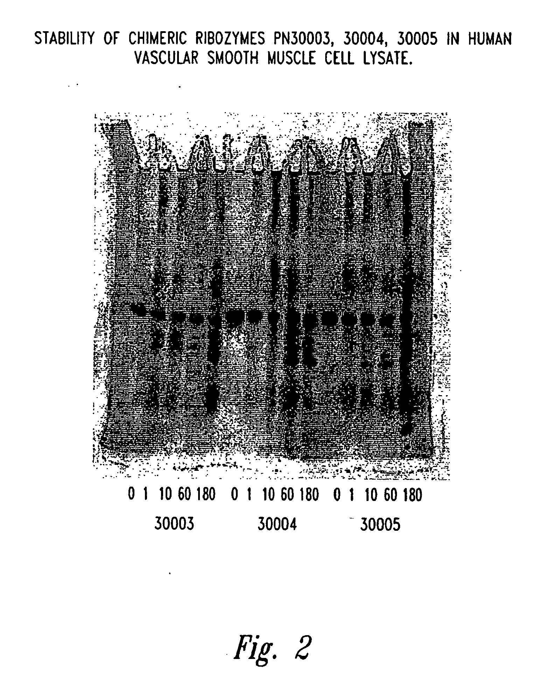

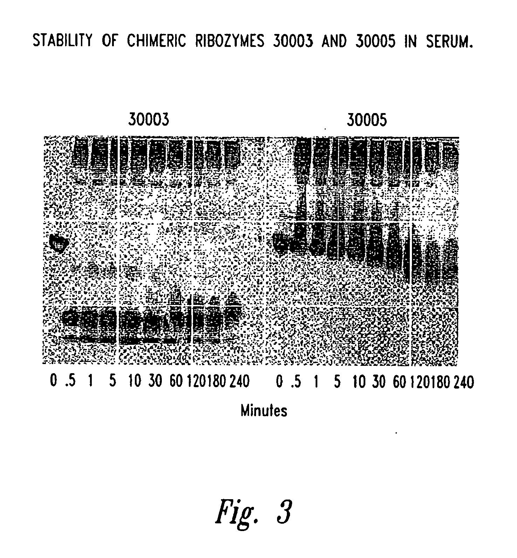

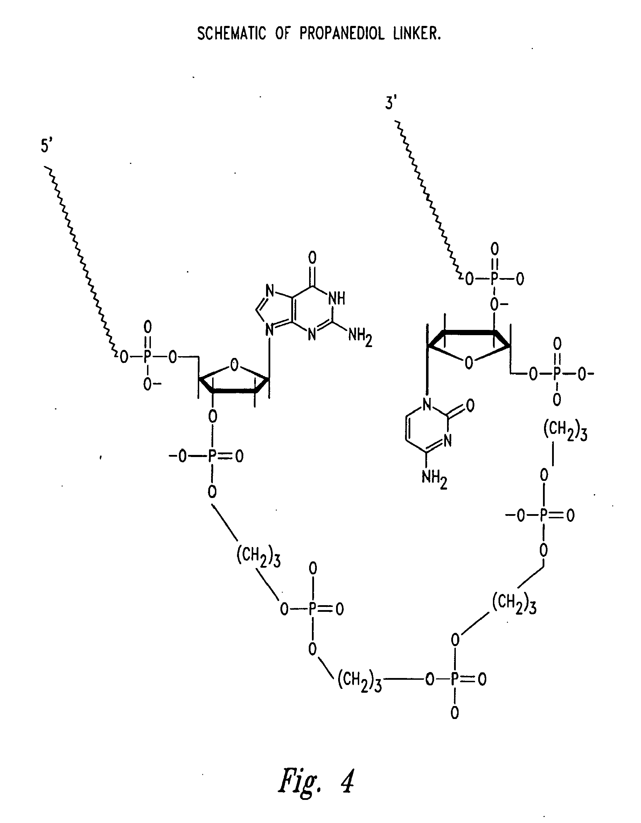

By making specific base modifications to the structure of the ribozymes, the half-life in cell lysate was increased sequentially from approximately 2.5 hours for PN30003, to 3.5 hours for PN30004, and to greater than 10 hours for PN30005 (figure 2). In serum, the half-life of PN30003 is less than 30 seconds. Specific base modifications to ribozyme PN30005 increased the half-life in serum to greater than 4 hours (figure 3). The internal stem loop structure can be substituted with a propanediol linkage to shorten the molecule, facilitating delivery, and enhancing catalytic activity (figure 4).

A scrambled sequence polynucleotide including the same composition of ribonucleotides and deoxyribonucleotides is also synthesized for each ribozyme to serve as a control with no catalytic activity. Lipofectin may be utilized to enhance the uptake of ribozyme into the cells.

### EXAMPLE 4

### CONSTRUCTION OF RIBOZYME MAMMALIAN EXPRESSION VECTORS

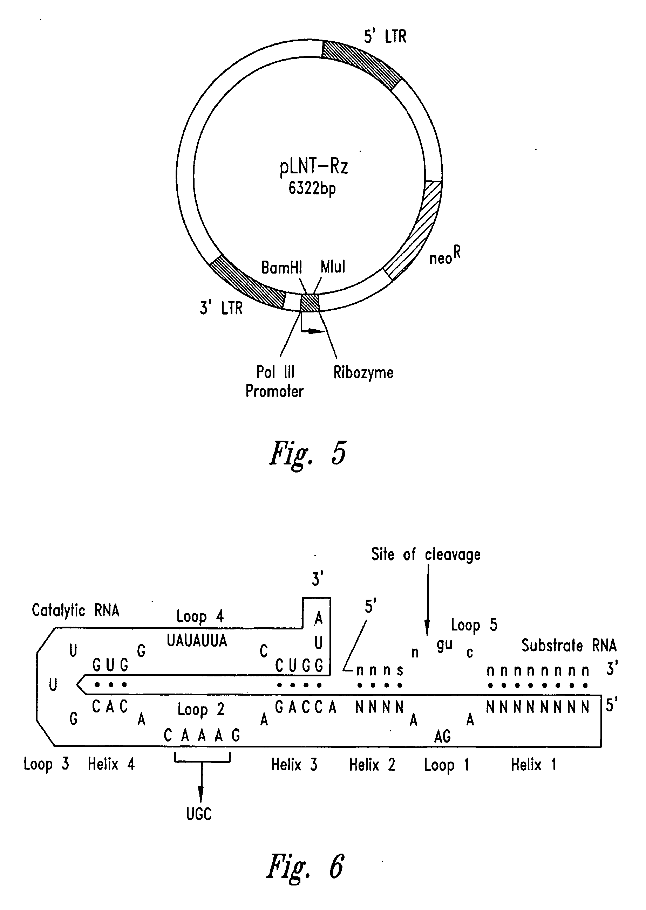

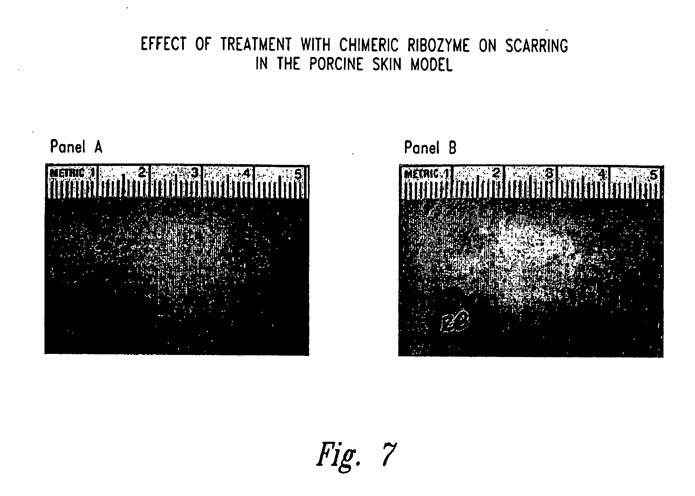

Plasmid pMJT (Yu et al., *Proc. Nat'l Acad. Sci. USA 90*:6340-6344, 1993), which contains the anti-U5 HIV ribozyme driven by the tRNA^{val} RNA pol III promoter, is digested with *Bam*HI and *Mlu*I, and the vector purified from the ribozyme fragment. The hairpin ribozymes, as described above, are excised from the pGem vector with *Bam*HI and *Mlu*I, purified, and ligated into the empty pMJT vector. The resulting vector is designated pLNT-Rz *(see* Figure 5, and contains the Moloney LTR driving the neomycin resistance gene and the tRNA^{val} RNA pol III promoter driving expression of the ribozyme.

### EXAMPLE 5

### IN VITRO CLEAVAGE ASSAYS

Hairpin or hammerhead ribozymes are tested for cleavage activity in an in vitro assay. Ribozyme and substrate synthesis is achieved by a new method of plasmid-independent in vitro transcription (Welch et al 1997). Briefly, oligonucleotides are synthesized (Retrogen, San Diego CA) with the T7 RNA polymerase promoter sequence contiguous with the ribozyme or substrate sequences, to allow for in vitro transcription of annealed oligonucleotides without the need for plasmid cloning. In vitro cleavage is tested in two hour time course reactions in 40 mM Tris pH 7.5, 10 mM MgCl₂, 2 mM spermidine, at 37°C (Welch et al 1997). Reaction products are analyzed by polyacrylamide gel electrophoresis (PAGE) and quantified by phosphorimager analysis (Molecular Dynamics). The Michaelis constant (Kₘ^{app}) and k₂ are determined for each ribozyme by performing single turnover kinetic experiments with ribozyme concentrations of 2-4 nM and substrate concentrations ranging from 2-200 nM, with analysis as above. The Kₘ^{app} and k₂ for the ribozymes is estimated for a Hanes plot with R2 > 0.90. Catalytic efficiency is calculated as k₂/ Kₘ^{app}. In vitro cleavage data for several representative ribozymes targeting specific sites in the CDK4, CDK2, CDC2, cyclin B1, and MMP-1 genes is shown in Table 19.

**Table 19 Summary of kinetics data for additional hairpin (HP) and hammerhead (HH) ribozyme candidates.**

| | HH k₂/Kₘ^{app} | HP k₂/Kₘ^{app} |
|---|---|---|
| CDK4 | | |
| cdk4-4 8.9 | 8.33 | |
| cdk4-4,8g6 | | 7.3 |
| cdk4-1 7.9 | 6.61 | |
| | | |
| CDC2 | | |
| cdc2-6/ 7,8 g7h | 14.4 pig, | |
| | 31.9 human | |
| cdc2-6,8g7h | | 6.25 |
| | | |
| CDK2 | | |
| CDK2-4 /7,9 | 27.37 | |
| CDK2-4,7 | | 10.76 |
| | | |
| CYCB1 | | |
| CycB 8,8 | 9.7 | |
| | | |
| MMP-1 | | |
| ColR3-9,8 | 0.46 pig, | |
| | 0.24 human | |
| ColR9-7,9 | 4615.00 | |
| ColR1086-9,8 | 52.5 | |

### EXAMPLE 6

### IN VIVO USE OF RIBOZYMES

### A. Experimental Protocols

All animals are treated according to the guidelines of the Public Health Service Policy on Humane Care and Use of Laboratory Animals.

### 1. Porcine scar model

Domestic pigs of approximately 35 kg are acclimatized for 14 days to the laboratory environment. Cutaneous lesions are created surgically in five places on the flanks of each pig. Each lesion is 3cm long x 5mm deep, with a 2mm slice removed from each lesion site. The wound edges may be sealed by electrocautery. Alternatively, lesions can be created utilizing a 6 mm punch biopsy.

The wound is treated daily by dermal application to each lesion of the desired test substance (with at least 2 untreated control sites per pig). Sites may be bandaged at the direction of the attending veterinarian. All sites are treated identically. Pigs wear protective 'jackets' to prevent mechanical irritation of the lesion sites.

The healing process is scored subjectively on a daily basis (1 = fresh lesion, 5 = fully healed), and the study terminated when control (untreated) sites score as fully healed.

### 2. Rabbit Disease Model

Dutch belted rabbits of approximately 2 kg are acclimatized for about 14 days in the laboratory environment. 0.06 Units of dispase is injected intravitreally in order to induce disease. Proliferative vitreoretinopathy (PVR) is then scored on days 1, 7, 14, 28 and 48 (1 = 0, 5 = retinal detachment). Typical digital pictures of the process are taken by fundoscopy.

The rabbits are treated on day 21 by injection of either ribozyme or control. The study is terminated when the control (vehicle) sites score as fully detached. Treatment group may be continued if detachment not evident.

### B. In vivo results

An example of the use of the PCNA targeted ribozymes in the porcine scar model is shown in figure 7. Panel A shows the effect of vehicle control on two 6 mm punch biopsies after 14 days. Panel B shows the effect of ribozyme treatment on two 6 mm punch biopsies after 14 days. The wound is healed in the treated sample and the extent of scarring is diminished in this example.

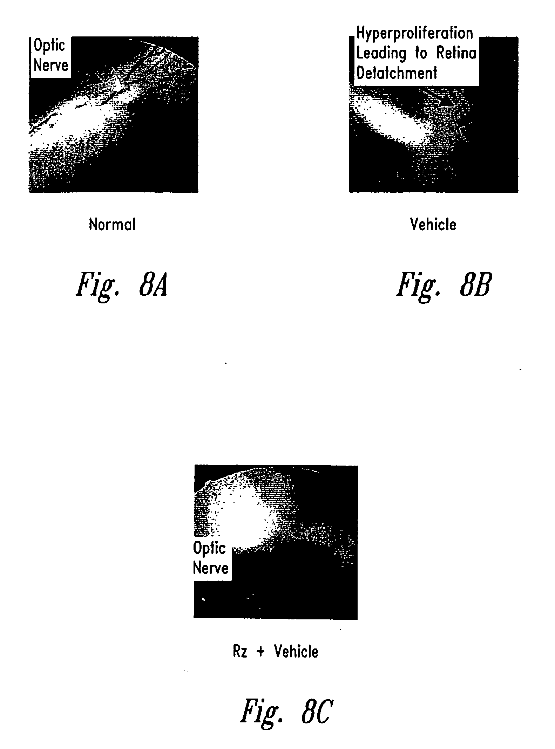

An example of the use of the PCNA targeted ribozymes formulated in DOTAP:cholesterol in the rabbit dispase model is shown in figure 8. Panel A shows the normal eye. Panel B shows the effect of dispase followed by treatment with vehicle after 31 days. Panel C shows the effect of dispase followed by treatment with ribozyme after 31 days. The retina is complete detached in the vehicle treated eye. The extent of proliferative vitreoretinopathy is diminished and no retinal detachment is evident in the ribozyme treated eye.

From the foregoing, it will be appreciated that, although specific embodiments of the invention have been described herein for purposes of illustration, various modifications may be made without deviating from the spirit and scope of the invention. Accordingly, the invention is not limited except as by the appended claims.

### EXAMPLE 7

### IN VITRO PERCUTANEOUS ABSORPTION OF [³²P]-RIBOZYME IN HUMAN CADAVER SKIN

The example was to evaluate the *in vitro* percutaneous absorption of eight test formulations of ³²P-labeled ribozyme (PN30006) in tape-stripped and intact human cadaver skin. Cumulative transdermal absorption of radiolabeled drug was measured at 1 and 24 hours. Tissue compartment (stratum corneum, epidermis, dermis) recovery was measured at 24 hours.

### Experimental Design:

| Group # (n=6) | Skin | Formulation Lot # | Ribozyme (% w/w) | Reservoir Samples (Hrs) | Tissue Recovery (Hrs) |
|---|---|---|---|---|---|
| 1 | stripped | 360-34A | 0.5% | 1, 24 | 24 |
| 2 | stripped | 360-34B | 0.5% | 1, 24 | 24 |
| 3 | stripped | 360-34C | 0.5% | 1, 24 | 24 |
| 4 | stripped | 360-34D | 0.5% | 1,24 | 24 |
| 5 | stripped | 360-35E | 0.5% | 1, 24 | 24 |
| 6 | stripped | 360-35F | 0.5% | 1, 24 | 24 |
| 7 | stripped | 360-35G | 0.5% | 1, 24 | 24 |
| 8 | stripped | 360-35H | 0.5% | 1, 24 | 24 |
| 9 | Intact | 360-35G | 0.5% | 1,24 | 24 |

### Materials

Ribozyme Formulations

| | |
|---|---|
| 360-34A | 5 mg/ml ribozyme in 10 mM DTT, 1.5% HEC |
| 360-34B | 5 mg/ml ribozyme in 0.1% SDS, 1.5% HEC |
| 360-34C | 5 mg/ml ribozyme in 5% DMSO, 1.5% HEC |
| 360-34D | 5 mg/ml ribozyme in 15% PG, 1.5% HEC |
| 360-35E | 5 mg/ml ribozyme in 10 mM DTT, 0.1% SDS, 1.5% HEC |
| 360-35F | 5 mg/ml ribozyme in 5% DMSO, 15% PG, 1.5% HEC |
| 360-35G | 5 mg/ml ribozyme in 10 mM DTT, 0.1% SDS, 5% DMSO, 1.5% HEC |
| 360-35H | 5 mg/ml ribozyme in 10 mM DTT, 5% DMSO, 15% PG, 1.5% HEC |

### Key to abbreviations:

| | |
|---|---|
| DTT | Dithiotheitol (reducing agent) |
| HEC | Hydroxyethylcellulose (gelling agent) |
| SDS | Sodium docedyl sulfate (penetration enhancer) |
| DMSO | Dimethyl sulfoxide (penetration enhancer) |
| PG | Propylene glycol (penetration enhancer) |

### Human Skin:

Human cadaver skin was obtained from a single donor. The skin was dermatomed to approximately 200micron thickness. Skin was excluded if it was damaged, had irregularities (scar tissue, holes, birthmarks, etc.) or was from donors with infectious disease. Skin samples were frozen until use. Skin specimens were thawed overnight in the refrigerator in plastic sealed bags prior to the experiment.

### Spiking Test Formulations:

Test formulations were spiked with [³²P]-ribozyme prior to the experiment. 15 1 of the radiolabeled solution was added to 1.0 gram (ml) of the formulation and spatulated.

To assay the final specific activity of the spiked test formulation, approximately 10-30 mg of the formulation was weighed (n=3), 100 µl samples were placed in 10 ml of Ecoscint® scintillation fluor (National Diagnostics #LS275) and counted in a Beckman Model LS 3801 liquid scintillation counter with a pre-calibrated quench curve for ³²P. Thirty (30) mg of spiked formulation was applied to each chamber. The total DPM of the applied dose was calculated by determining the total DPM applied in the 30 mg dose and subtracting the DPM of the application tips.

### Equipment:

A total of 54 Franz static diffusion glass diffusion chambers (Crown Glass Cat # FDC-100) with a magnetic stirrer mounted on a 9-position Franz diffusion cell drive console with acrylic blocks, magnetic stirrers, and stainless steel manifolds (Crown #FDCD-9-LV) was used for the study. The reservoir volume (6-11 ml) of each cell was pre-calibrated.

### Equipment Set-up:

The diffusion cells were filled with 4% bovine serum album (BSA, Fraction V) (Sigma Cat. #A2153) in isotonic buffered (pH 7.04) saline (PBS Fisher Cat # C5551-20 Celline II isotonic saline solution) with care to avoid bubbles at the skin interface. The diffusion cells were equilibrated for 1-2 hours to a temperature of 37°C by a circulating water pump (Haake) prior to applying skin specimens.

### Application of Drug to Skin:

The intact or prior tape-stripped dermatomed human cadaver skin was placed on the chamber and sealed with an O-ring. The skin surface area exposed to the test formulations was 1.77 cm². The skin was equilibrated for 30 minutes at 37°C on the diffusion cell prior to applying test formulation. A total of 30 mg of the spiked formulation was applied to the skin surface using a Gilson Microman® positive displacement pipette and gently rubbed into the skin using the pipette tip. The dispensing tips were retained and counted. The mean DPM retained by the dispensing tips was calculated and subtracted from the theoretical DPM to determine the mean total DPM applied to each chamber.

### Sample Collection:

### Reservoir:

At 1 and 24 hours a 1.0 ml sample was removed from the reservoir using a calibrated Gilson P1000 Pipetteman® micropipette, and the volume replaced with 1.0 ml BSA-saline solution. The samples were placed in a scintillation vial containing Ecoscint® scintillation fluor (National Diagnostics # LS-275) and equilibrated overnight in the dark before counting.

### Washes and Gauze Wipes:

At 24 hours the skin surface was washed three times with 1.0 ml 2% Oleth-20 (Croda, Inc. # 9004-98-2) in water, followed by 2 washes with 1.0 ml of 5% Span 80 in isopropanol. The wash solutions were collected for recovery counts. The dispensing tips used for the washing procedure were also counted and included in the "wash" compartment. After washing, the skin surface was wiped with 3 sequential cotton gauze cloths, which were saved for recovery counts in the "Gauze" compartment.

### Tape Stripping (Stratum Corneum):

The skin specimens were either tape-stripped prior to drug application or following the gauze wiping. The skin was placed dermis side down onto a flat surface. The stratum corneum was removed by tape-stripping the skin with cellophane tape until "glistening" (approximately 22 strips) or until epidermal separation started to occur. The first two strips that remove the excess drug adhering to the outer surface of the stratum corneum were counted separately. These counts were included in total recovery (SC Surface) but excluded from stratum corneum compartment recovery. Four groups each consisting of five consecutive tape strips were placed in a scintillation vial containing Scintilene® (Fisher # SX2-4).

### Epidermis and Dermis:

After tape-stripping the intact skin or after removing the tape-stripped skin from the chamber, the dermis and epidermis were separated by microwave technique (2-5 sec.). The separated epidermis was placed in a vial containing 1 ml of Soluene 350 (Packard, Inc # 6003038), and dermis in vial containing 2 ml Soluene 350. The tissues were then digested in a 60°C oven for 4 hours. Hionic-Fluor (Packard, Inc # 6013319) was added to the digested tissues, counted in a Beckman LSC and corrected for quenching.

### Recovery:

The percent of ³²P-ribozyme recovered in the reservoir, washes, gauze wipes, and skin compartments (stratum corneum, epidermis, dermis) was determined by calculating the percent of the total applied DPM recovered in the respective compartments.

The total micrograms of drug recovered in each compartment was obtained by multiplying the % recovery by the total amount (micrograms) of drug applied to each chamber.

### Data Entry/Calculations:

The DPM data from the LSC tapes, measurements for cell volume, and quantity of drug applied were entered into a standardized Quattro Pro spreadsheet. The data were calculated after discarding outlier values for each compartment. A value was considered to be an "outlier" if the questionable value differed from the re-calculated average by more than four times the average deviation of the remaining values (Skoog & West, Analytical Chemistry).

### Results

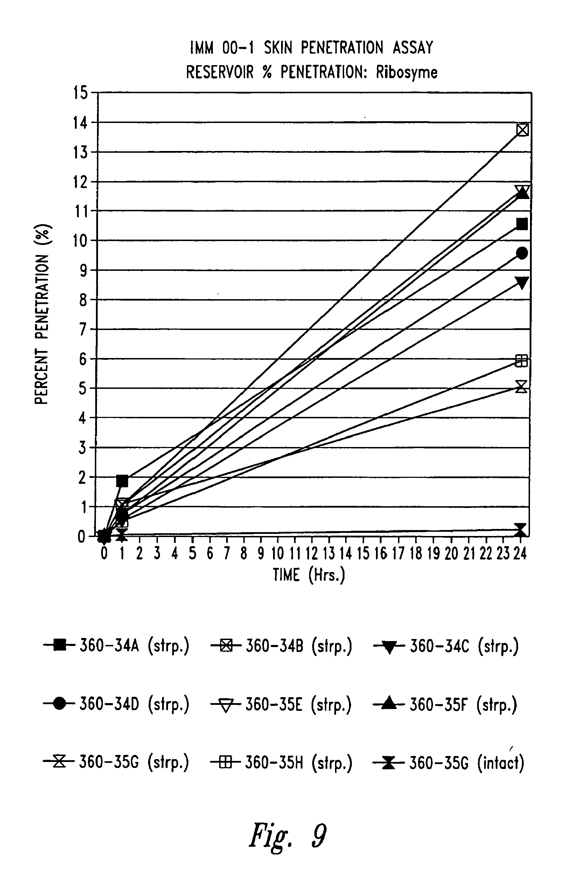

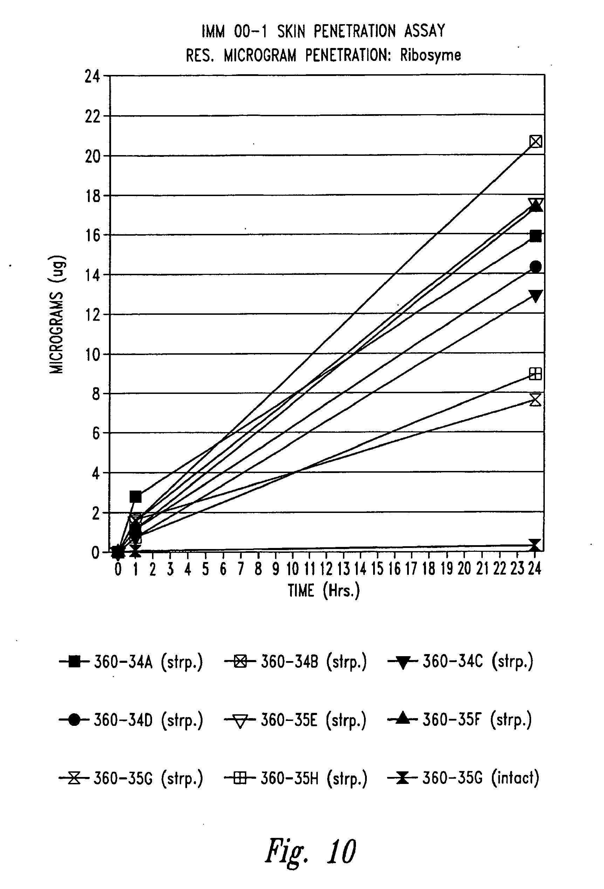

The total DPM applied to each cell ranged from 12-15 x 10⁶. The cumulative transdermal absorption of ³²P-ribozyme is presented as the percent of the applied dose recovered in the reservoir at each time point (Figure 9). The transdermal absorption of ³²P-ribozyme is also presented as total micrograms recovered in the reservoir (Figure 10). The total 24 hr flux in the tape-stripped skin ranged from 5-12% compared to 0.2% in the intact skin.

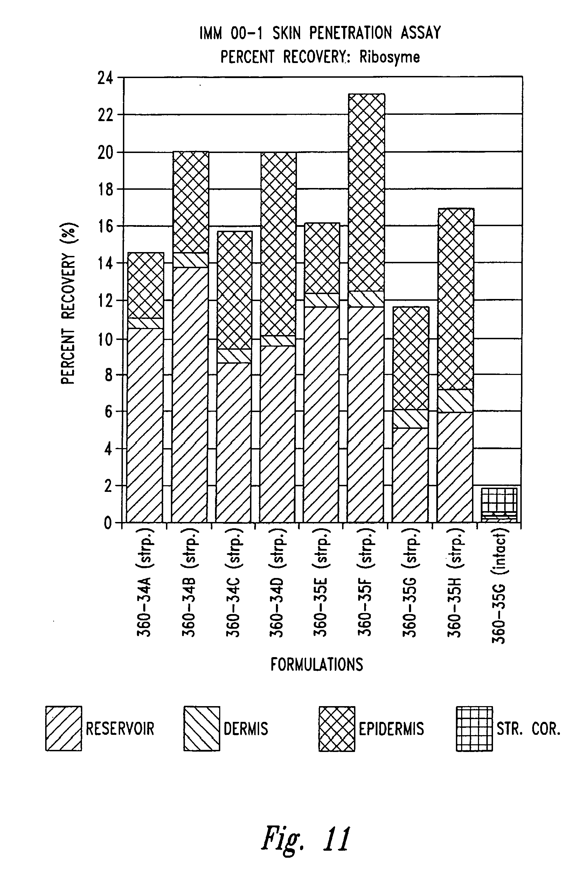

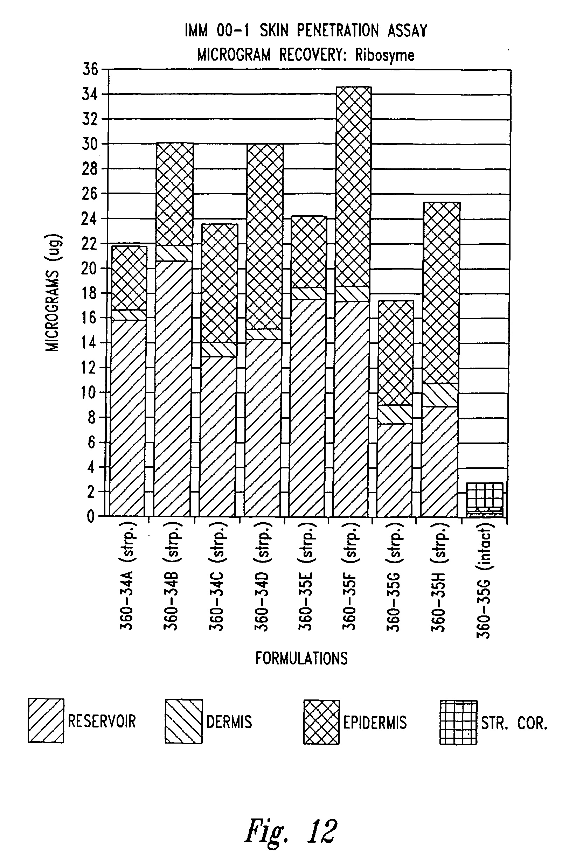

The localization of ³²P-ribozyme in the various skin compartments is presented as both percent of the applied dose (DPM) recovered in each compartment (Figure 11) and total micrograms in each compartment (Figure 12). The recovery of the formulations in the dermal tissues was similar in the tape-stripped skin, ranging from 0.5-1.0% of the applied dose compared to 0.1% in the intact skin. The recovery of all formulations was high in the epidermis in the tape-stripped skin ranging from 3-10% compared to 0.2% in the intact skin.

Tables 20 and 21 rank the absorption (%, g) of the test formulations for the sum of the recoveries in the reservoir, epidermis and dermis.

**Table 20 Overall effectiveness (percent)**

| Relative Effectiveness | Skin | Formulati on Lot # | Ribozyme (% w/w) | Description | SUM (Reservior, Epi. Dermis) % |
|---|---|---|---|---|---|
| 1 | stripped | 360-34A | 0.5 | Cream | 23.1 |
| 2 | stripped | 360-34B | 0.5 | Cream | 20.1 |
| 3 | stripped | 360-34C | 0.5 | Cream | 20.0 |
| 4 | stripped | 360-34D | 0.5 | Cream | 17.0 |
| | | | | | |
| 5 | stripped | 360-35E | 0.5 | Cream | 16.2 |
| 6 | stripped | 360-35F | 0.5 | Cream | 15.7 |
| 7 | stripped | 360-35G | 0.5 | Cream | 14.6 |
| 8 | stripped | 360-35H | 0.5 | Cream | 11.7 |
| 9 | Intact | 360-35G | 0.5 | Cream | 0.5 |

**Table 21 Overall effectiveness (micrograms)**

| Relative Effectiveness | Skin | Formulati on Lot # | Ribozyme (% w/w) | Description | SUM (Reservior, Epi. Dermis) % |
|---|---|---|---|---|---|
| 1 | stripped | 360-34A | 0.5 | Cream | 34.7 |
| 2 | stripped | 360-34B | 0.5 | Cream | 30.1 |
| 3 | stripped | 360-34C | 0.5 | Cream | 29.9 |
| 4 | stripped | 360-34D | 0.5 | Cream | 25.5 |
| | | | | | |
| 5 | stripped | 360-35E | 0.5 | Cream | 24.3 |
| 6 | stripped | 360-35F | 0.5 | Cream | 23.6 |
| 7 | stripped | 360-35G | 0.5 | Cream | 21.8 |
| 8 | stripped | 360-35H | 0.5 | Cream | 17.5 |
| 9 | Intact | 360-35G | 0.5 | Cream | 0.8 |

### EXAMPLE 8

### INHIBITION OF EXPERIMENTAL PVR BY RIBOZYMES TARGETING RABBIT PCNA

### A. Experimental PVR treated with Ribozyme:Lipid Complex

### Experimental design

The rabbit model involves the injection of dispase into the vitreal space without the need to introduce retinal tears. Prior models have relied on exogenous cells such as fibroblasts to induce disease. In the dispase model, no assumptions are made as to the type of endogenous cells involved in the formation of disease. The severity of PVR correlated with the dose of dispase injected. The optimal dose of dispase has been determined to minimize interfering complications such as cataract and hemorrhage. This model uses intravitreal rather than subretinal injection. The timing and complexity of the cellular response correspond to the human disease. These advantages make this model particularly attractive. The single disadvantage of relatively long timeframe is easily overcome by extending the period of observation. This model was used to test the efficacy of the chimeric PCNA targeted ribozyme.

A selected lot of dispase was tested in the rabbit eye to titrate the dose and time frame for disease development and aliquots frozen for all remaining experiments (Frenzel EM, Neely KA, Walsh AW, Cameron JD, Gregerson DS. 1998. A new model of proliferative vitreoretinopathy. IOVS 39:2157-2164). Twelve 1-2 Kg Dutch Belted rabbits received a single intravitreal injection of 0.06, 0.07, or 0.08 U dispase (Boehringer Mannheim, Indianapolis, IN) on day 0 (four rabbits each dose). Rabbits were anesthetized with ketamine/xylazine, their eyes dilated with 2.5% phenylephrine and 1% tropicamide, and a local anesthetic, proparacain HCl applied. 0.1 cc of dispase was injected into the right eye of each rabbit with a 30 gauge needle, delivering the dispase into the vitreous cavity directly in front of the retina without traumatizating the retina, under direct visualization with a dissecting microscope equipped with floating Charles' lens. Left eyes serve as controls. Rabbits were followed for progression of disease by fundoscopy at days 1, 7, 14, 21, 28, and 48. A generous timeframe was allocated to maximize the probability of disease development in the dispase treated groups. Fundoscopic evaluation was by two independent, trained observers for this and the following experiments. In this experiment, a dose of 0.07 U dispase resulted in uniform PVR development by 21 days.

Following the same protocol, a second experiment was performed with eight rabbits given 0.07 U dispase in the right eye on day 0. At 7-14 days post-injection, all eyes had vitreous and pre-retinal hemorrhage. At 21 days 7 of 8 rabbits had early PVR characterized by fibroglial proliferation. Before a retinal detachment was noted in any eyes, the control group of rabbits (n=4) was treated with an intravitreal injection of 0.1 cc DOTAP:cholesterol vehicle. The experimental group (n=4) was given an intravitreal injection of 0.1 cc chimeric PCNA targeted ribozyme (PN30006).

### Fundoscopic analysis

Fundoscopic evaluation of chimeric PCNA targeted ribozyme versus control treated rabbit eyes is the same as shown in Figure 8. By day 35, all rabbits in the control group developed significant retinal detachment. Three of four in the chimeric PCNA targeted ribozyme treated group did not develop a retinal detachment, while one developed a small local detachment, which did not progress over the 60 day follow-up period. These data suggest that inhibiting proliferation of cells causative of PVR with chimeric ribozyme to PCNA may have a therapeutic or preventative role.

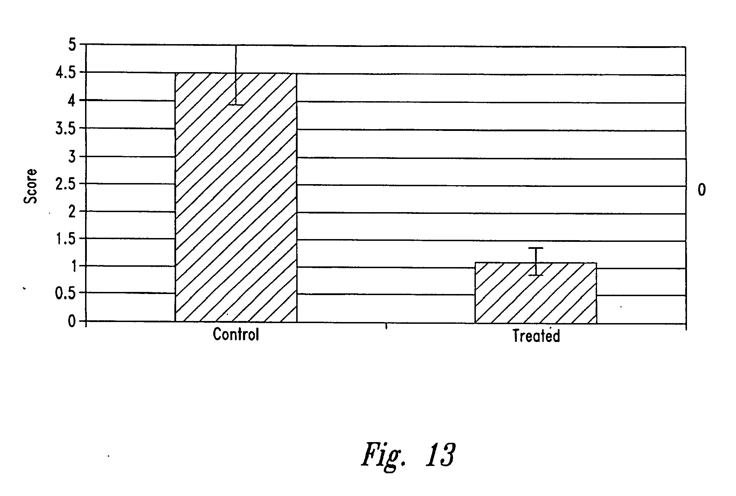

All eyes were scored by two independent trained observers on a scale of 1-6 as per Table 22 (Fastenberg et al. 1982. A comparison of different cellular inocula in an experimental model of massive periretinal proliferation. Am J Ophthalmol. 93:559-56; Sakamoto et al. 1995. Inhibition of experimental proliferative vitreoretinopathy by retroviral vector-mediated transfer of suicide gene. Ophthalmology 102:1417-1424). The scale is based on inflammation, hemorrhage, distortion, intravitreal haze and membranes, and formation of retinal breaks, traction, and detachments. The results (Figure 13) indicate that the chimeric ribozyme is effective in preventing or retarding the development of PVR.

**Table 22 Histopathologic scoring of PVR**

| Score | Histopathologic Evaluation |
|---|---|
| 0 | Normal eye |
| 1 | Hemorrhage/preretinal gliotic changes |
| 2 | Focal Retration |
| 3 | Extensive PVR with focal traction |
| 4 | PVR with focal retinal detachments of medullary ray |
| 5 | Extensive peripapillary retinal detachments |
| 6 | Total retinal detachments |

### Histopathologic analysis

Treated and control eyes were enucleated, fixed in 4% paraformaldehyde, embedded in paraffin, and sectioned for histopathologic evaluation. Selected gross specimens were photographed before sectioning. Histologic examination was performed after hematoxylin and eosin staining. Histopathologic analyses correlated with the gross pathological findings and confirmed the clinical diagnoses of PVR and retinal detachment.

The ability of this chimeric ribozyme complex to prevent or lessen the degree of damage due to aberrant proliferation of cells in the eye could be a significant advancement in the treatment of PVR. This type of therapy may be especially well suited for use in patients facing vision loss due to the failure of traditional surgical treatment (McCormack P, Simcock PR, Charteris D, Lavin MJ. 1994. Is surgery for proliferative vitreoretinopathy justifiable? Eye 8:75-76.).

### B. Experimental PVR treated with Ribozyme Alone

Three additional experiments in the PVR model were carried out. Experiment 2 was a repeat of the first experiment described above (*i*.*e*., Example 8, Section A) with the addition of a control group with scrambled sequence (random specificity) ribozyme complexed to the lipid. The other two experiments were done using the ribozyme alone at 10 mg/ml without the addition of the DOTAP:cholesterol lipid. These were done due to an observed precipitate that took greater than 2 weeks to clear. The precipitate was not observed when lipid was absent. Experiment 3 repeated the timing of the first two experiments, with ribozyme treatment on day 20. Experiment 4 was performed with treatment on day 7 to represent a prophylactic approach. The overall severity of the PVR has decreased over time even in the control group consistent with loss of activity of the dispase. The results are shown in table 23 where two independent assessments were made by trained observers using a modified Fastenberg et al. (1982) scoring system (*i*.*e*., Table 22).

**Table 23 Scoring of ribozyme and control treated rabbit eyes with Dispase induced PVR.**

| Exp # | | Ribozyme | Lipid | N | Treatment Day | Assessment Day | Ave. Score |
|---|---|---|---|---|---|---|---|
| 2 | Treated | PN30004 | + | 4 | 20 | 35 | 3.1±1.3 |
| | Control | Scrambled | | 4 | | | 5.3±1.2 |
| | | Dextrose | | 4 | | | 4.5±1 |
| 3 | Treated | PN30006 | - | 4 | 20 | 49 | 1.8±1.7 |
| | Control | Dextrose | | 3 | | | 3.7±0.6 |
| | | no injection | | 2 | | | 2.5±0.7 |
| 4 | Treated | PN30006 | - | 6 | 7 | 28 | 0.7±0.8 |
| | Control | Dextrose | | 6 | | | 2.2±0.4 |

The results show a consistent decrease in severity of PVR following treatment with active chimeric PCNA targeted ribozyme compared to untreated or scrambled ribozyme controls.

### EXAMPLE 9

### EFFECTS OF PCNA TARGETED RIBOZYME ON SCAR FORMATION IN THE PIG

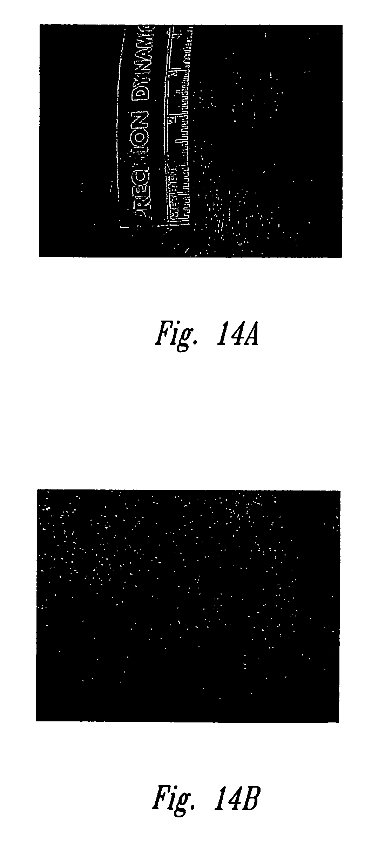

A chimeric PCNA targeted ribozyme (PN30006) has been tested in a pig model of scarring due to surgical incision. Briefly, two female commercial pigs underwent surgical creation of wounds followed by administration of the test or vehicle article into selected wounds for three consecutive days. Crescent shaped wound sites (5 on each side), each measuring approximately 3 cm long x 5 mm deep x 6 mm wide, were made on the back of each animal (Figure14A). Each wound was sutured once (Figure 14B). This type of wound was made to replicate excisional wounding. Wounds were dressed with an Op-site bandage immediately after instillation of the test article on Days 1, 2 and 3. Animals were bandaged and dressed in restraining jackets to prevent mechanical irritation of the surgical sites (as necessary).

Visual observations made in the in-life phase were scored as follows: 1 completely healed/scarred, 2 scab exfoliating, 3 wound scabbing, 4 open wound. Histopathologic evaluation was done at 36 days, and included scoring for: epithelialization, inflammation, scar tissue, and connective tissue.

There were no treatment related clinical signs following instillation of the test or vehicle articles into selected wound sites for 3 consecutive days. All clinical signs that were observed (red skin, exudate, scabbing) were considered to be part of the normal healing process for surgical wounds of the type created on this study.

All wound sites were prepared for histopathological examination by embedding in paraffin wax, sectioning and staining with hematoxylin and eosin/phloxine. Histopathological examination was conducted on all wound sites from both animals to evaluate the following parameters:

| | |
|---|---|
| a. Re-epithelialization | Present, partial, or absent epithelialization of the surface epithelium. |
| b. Inflammation | Degree of severity, cell type and distribution. |
| c. Scar Tissue Formation | Presence or absence of scar tissue, extent, size, depth and severity of scarring. |
| d. Connective Tissue Formation | Presence or absence of collagen and connective tissue associated with normal healing, the extent, size and depth (as opposed to scar tissue formation). |

Scarring in comparison to healing was differentiated by the amount of connective tissue, the amount of neovascularity, inflammation, and amount of normal subcutaneous collagen present. In the scarred lesions, there was more connective tissue and the plug area was clearly differentiated from the adjacent normal tissue, while in the more healed or drug treated lesions, the plug area was almost contiguous with the adjacent tissue and difficult to differentiate from the adjacent tissue.

Histopathological examination failed to detect any negative long term consequences on epithelialization by drug treatment since complete healing occurred at all wound sites. Additionally, there was no correlation between treatment and inflammation. Histopathological examination of wound sites 36 days after wounding revealed that chimeric PCNA targeted ribozyme treated wounds were more completely healed and less scarred than untreated wounds or wounds treated with the vehicle article.

### Statement under Art. 19.1 PCT

1. A method of treating a proliferative skin disease, comprising administering to a patient a therapeutically effective amount of ribozyme which cleaves RNA encoding a cytokine involved in inflammation, a matrix metalloproteinase,a cyclin, a cell-cycle dependent kinase, a growth factor, or a reductase such that said proliferative skin disease is treated.
2. A method of treating a proliferative skin disease, comprising administering to a patient an effective amount of nucleic acid molecule comprising a promoter operably linked to a nucleic acid segment encoding a ribozyme which cleaves RNA encoding a cytokine involved in inflammation, a matrix metalloproteinase, a cyclin, a cell-cycle dependent kinase, a growth factor, or a reductase such that said proliferative skin disease is treated.
3. The method according to 1 or 2 wherein said proliferative skin disease is psoriasis.
4. The method according to 1 or 2 wherein said proliferative skin disease is atopic dermatitis.
5. The method according to 1 or 2 wherein said proliferative skin disease is actinic keratosis.
6. The method according to 1 or 2 wherein said proliferative skin disease is squamous or basal cell carcinoma.
7. The method according to 1 or 2 wherein said proliferative skin disease is viral or seborrheic wart.
8. The method according to 1 or 2 wherein said ribozyme is a hammerhead or hairpin ribozyme.
9. The method according to 1 or 2 wherein said cell-cycle dependent kinase is CDK1, CDK2, or, CDK4.
10. The method according to 1 or 2 wherein said cyclin is PCNA.
11. The method according to 1 or 2 wherein said cyclin is Cyclin B 1 or Cyclin D.
12. The method according to 1 or 2 wherein said cytokine is interleukin 1 alpha and beta, interleukin 2, interleukin 6, interleukin 8, interferon gamma, or tumor necrosis factor.
13. The method according to 1 or 2 wherein said matrix metalloproteinase is MMP 1, MMP 2, MMP 3 or MMP 9.
14. The method according to 1 or 2 wherein said growth factor is vascular endothelial growth factor, or platelet derived growth factor.
15. The method according to 1 wherein said ribozyme is administered topically or intradermally.
16. The method according to 2 wherein said nucleic acid molecule is administered intradermally.
17. The method according to 1 wherein said ribozyme is formulated within a cream, ointment or lotion.
18. The method according to 18 wherein said ribozyme is formulated along with a lipid.
19. The method according to 1 wherein said lipid is DOTAP:cholesterol.
20. The method according to 1 wherein said ribozyme is formulated with ribonuclease inhibitors.
21. The method according to 20 wherein said ribonuclease inhibitor is a reducing agent.
22. The method according to 20 wherein said reducing agent is dithiothreitol.
23. The method according to 20 wherein said ribonuclease inhibitor is a detergent.
24. The method according to 23 wherein the detergent is sodium dodecyl sulfate.
25. The method according to 20 wherein said ribonuclease inhibitor is vanidyl nucleotides.
26. The method according to 20 wherein said ribonuclease inhibitor is aurin tricarboxcylic acid.
27. The method according to 20 wherein said ribonuclease inhibitor is hydrogen peroxide.
28. The method according to 20 wherein said ribonuclease inhibitor is an RNA decoy.
29. The method according to 28 wherein said RNA decoy is a tRNA.
30. The method according to 1 wherein said ribozyme is composed of ribonucleic acids.
31. The method according to 30 wherein one or more of said ribonucleic acids are 2'-O-methyl ribonucleic acids.
32. The method according to 1 wherein said ribozyme is composed of a mixture of deoxyribonucleic acids and ribonucleic acids.
33. The method according to 1 wherein said ribozyme is composed of nucleic acids having phosphothioate linkages.
34. The method according to 1 wherein said ribozyme is composed of nucleic acids having propanediol linkages.
35. The method according to 2 wherein said nucleic acid molecule is contained within a viral vector.
36. The method according to 2 wherein said viral vector is generated from a virus selected from the group consisting of retroviruses, adenoviruses, adeno-associated viruses.
37. A method of treating or preventing scarring, comprising administering to a patient a therapeutically effective amount of ribozyme which cleaves RNA encoding a cytokine involved in inflammation, a matrix metalloproteinase, a cyclin, a cell-cycle dependent kinase, a growth factor, or a reductase such that said scarring is treated or prevented.
38. A method of treating or preventing scarring, comprising administering to a patient an effective amount of nucleic acid molecule comprising a promoter operably linked to a nucleic acid segment encoding a ribozyme which cleaves RNA encoding encoding a cytokine involved in inflammation, a matrix metalloproteinase,a cyclin, a cell-cycle dependent kinase, a growth factor, or a reductase such that said scarring is treated or prevented.
39. The method according to 37 or 38 wherein said scar is a keloid.
40. The method according to 37 or 38 wherein said scar is an adhesion.
41. The method according to 37 or 38 wherein said scar is a hypertrophic or hypertrophic burn scar.
42. The method according to 37 or 38 wherein said ribozyme is a hammerhead or hairpin ribozyme.
43. The method according to 37 or 38 wherein said cell-cycle dependent kinase is CDK1, CDK2, or, CDK4.
44. The method according to 37 or 38 wherein said cyclin is PCNA.
45. The method according to 37 or 38 wherein said cyclin is Cyclin B1 or Cyclin D.
46. The method according to 37 or 38 wherein said cytokine is interleukin 1 alpha or beta, interleukin 2, interleukin 6, interleukin 8, interferon gamma, or tumor necrosis factor.
47. The method according to 37 or 38 wherein said matrix metaloproteinase is MMP 1, MMP2, MMP3, or MMP9.
48. The method according to 37 or 38 wherein said growth factors is VEGF or PDGF.
49. The method according to 37 wherein said ribozyme is administered topically or intradermally.
50. The method according to 38 wherein said nucleic acid molecule is administered intradermally.
51. The method according to 37 wherein said ribozyme is formulated within a cream, ointment or lotion.
52. The method according to 37 wherein said ribozyme is formulated along with a lipid.
53. The method according to 52 wherein said lipid is DOTAP:cholesterol.
54. The method according to 37 wherein said ribozyme is formulated with ribonuclease inhibitors.
55. The method according to 54 wherein said ribonuclease inhibitor is a reducing agent.
56. The method according to 55 wherein said reducing agent is dithiothreitol.
57. The method according to 54 wherein said ribonuclease inhibitor is a detergent.
58. The method according to 57 wherein said detergent is sodium dodecyl sulfate.
59. The method according to 54 wherein said ribonuclease inhibitor is vanidyl nucleotides.
60. The method according to 54 wherein said ribonuclease inhibitor is aurin tricarboxcylic acid.
61. The method according to 54 wherein said ribonuclease inhibitor is hydrogen peroxide.
62. The method according to 54 wherein said ribonuclease inhibitor is an RNA decoy.
63. The method according to 62 wherein said RNA decoy is a tRNA.
64. The method according to 37 wherein said ribozyme is composed of ribonucleic acids.
65. The method according to 64 wherein one or more of said ribonucleic acids are 2'-O-methyl ribonucleic acids.
66. The method according to 37 wherein said ribozyme is composed of a mixture of deoxyribonucleic acids and ribonucleic acids.
67. The method according to 37 wherein said ribozyme is composed of nucleic acids having phosphothioate linkages.
68. The method according to 37 wherein said ribozyme is composed of nucleic acids having propanediol linkages.
69. The method according to 38 wherein said nucleic acid molecule is contained within a viral vector.
70. The method according to 38 wherein said viral vector is generated from a virus selected from the group consisting of retroviruses, adenoviruses, adeno-associated viruses.
71. A method of treating a proliferative eye disease, comprising administering to a patient a therapeutically effective amount of ribozyme which cleaves RNA encoding a cytokine involved in inflammation, a matrix metalloproteinase, a cyclin, a cell-cycle dependent kinase, a growth factor, or a reductase such that said proliferative eye disease is treated.
72. A method of treating a proliferative eye disease, comprising administering to a patient an effective amount of nucleic acid molecule comprising a promoter operably linked to a nucleic acid segment encoding a ribozyme which cleaves RNA encoding encoding a cytokine involved in inflammation, a matrix metalloproteinase, a cyclin, a cell-cycle dependent kinase, a growth factor, or a reductase such that said proliferative eye disease is treated.
73. The method according to 71 or 72 wherein said proliferative eye disease is proliferative diabetic retinopathy.
74. The method according to 71 or 72 wherein said proliferative eye disease is proliferative vitreoretinopathy.
75. The method according to 71 or 72 wherein said proliferative eye disease is proliferative sickle cell retinopathy.
76. The method according to 71 or 72 wherein said proliferative eye disease is retinopathy of prematurity.
77. The method according to 71 or 72 wherein said proliferative eye disease is retinal detachment.
78. The method according to 71 or 72 wherein said ribozyme is a hammerhead or hairpin ribozyme.
79. The method according to 71 or 72 wherein said cell-cycle dependent kinase is CDK1, CDK2, or, CDK4.
80. The method according to 71 or 72 wherein said cyclin is PCNA.
81. The method according to 71 or 72 wherein said cyclin is Cyclin B 1 or Cyclin D.
82. The method according to 71 or 72 wherein said cytokine is interleukin 1 alpha and beta, interleukin 2, interleukin 6, interleukin 8, interleukin 10, interferon gamma, tumor necrosis factor.
83. The method according to 71 or 72 wherein said matrix metaloproteinase is MMP 1, MMP2, MMP3, or MMP9.
84. The method according to 71 or 72 wherein said growth factors is VEGF or PDGF.
85. The method according to 71 or 72 wherein said nucleic acid molecule is administered intraocularly.
86. The method according to 71 or 72 wherein said ribozyme is formulated within a solution.
97. The method according to 89 wherein said ribonuclease inhibitor is an RNA decoy.
98. The method according to 97 wherein said RNA decoy is a tRNA.
99. The method according to 71 wherein said ribozyme is composed of ribonucleic acids.
100. The method according to 99 wherein one or more of said ribonucleic acids are 2'-O-methyl ribonucleic acids.
101. The method according to 71 wherein said ribozyme is composed of a mixture of deoxyribonucleic acids and ribonucleic acids.
102. The method according to 71 wherein said ribozyme is composed of nucleic acids having phosphothioate linkages.
103. The method according to 71 wherein said ribozyme is composed of nucleic acids having propanediol linkages.
104. The method according to 72 wherein said nucleic acid molecule is contained within a viral vector.
105. The method according to 72 wherein said viral vector is generated from a virus selected from the group consisting of retroviruses, adenoviruses, adeno-associated viruses.

SEQUENCE LISTING

<212> DNA<213> Homo sapiens<220> <223> Cdk1 ribozyme binding site
<400> 1
atggacgatc agggttgcc

<212> DNA<213> Homo sapiens<220> <223> Cdk1 ribozyme binding site
<400> 2
gatcagggtt gccctcggt

<212> DNA<213> Homo sapiens<220> <223> Cdk1 ribozyme binding site
<400> 3
ggttgccctc ggtgtaaga

<212> DNA<213> Homo sapiens<220> <223> Cdk1 ribozyme binding site
<400> 4
cctcggtgta agaccacca

<212> DNA<213> Homo sapiens<220> <223> Cdk1 ribozyme binding site
<400> 5
ccaccaaata tcggaaccc

<212> DNA<213> Homo sapiens<220> <223> Cdk1 ribozyme binding site
<400> 6
accaaatatc ggaacccct

<212> DNA<213> Homo sapiens<220> <223> Cdk1 ribozyme binding site
<400> 7
ggaacccctc cttgaagct

<212> DNA<213> Homo sapiens<220> <223> Cdk1 ribozyme binding site
<400> 8
acccctcctt gaagctgat

<212> DNA<213> Homo sapiens<220> <223> Cdk1 ribozyme binding site
<400> 9
ggacacactc tctgtgaaa

<212> DNA<213> Homo sapiens<220> <223> Cdk1 ribozyme binding site
<400> 10
acacactctc tgtgaaagt

<212> DNA<213> Homo sapiens<220> <223> Cdk1 ribozyme binding site
<400> 11
tgtgaaagtt gtgtagatt

<212> DNA<213> Homo sapiens<220> <223> Cdk1 ribozyme binding site
<400> 12
aagttgtgta gatttactg

<212> DNA<213> Homo sapiens<220> <223> Cdk1 ribozyme binding site
<400> 13
tgtgtagatt tactgtttg

<212> DNA<213> Homo sapiens<220> <223> Cdk1 ribozyme binding site
<400> 14
gtgtagattt actgtttgt

<212> DNA<213> Homo sapiens<220> <223> Cdk1 ribozyme binding site
<400> 15
tgtagattta ctgtttgtg

<212> DNA<213> Homo sapiens<220> <223> Cdk1 ribozyme binding site
<400> 16
atttactgtt tgtgagagg

<212> DNA<213> Homo sapiens<220> <223> Cdk1 ribozyme binding site
<400> 17
tttactgttt gtgagagga

<212> DNA<213> Homo sapiens<220> <223> Cdk1 ribozyme binding site
<400> 18
gagtgtggta ctccactca

<212> DNA<213> Homo sapiens<220> <223> Cdk1 ribozyme binding site
<400> 19
tgtggtactc cactcagaa

<212> DNA<213> Homo sapiens<220> <223> Cdk1 ribozyme binding site
<400> 20
tactccactc agaaagagc

<212> DNA<213> Homo sapiens<220> <223> Cdk1 ribozyme binding site
<400> 21
agagcaactt cagggtaca

<212> DNA<213> Homo sapiens<220> <223> Cdk1 ribozyme binding site
<400> 22
gagcaacttc agggtacaa

<212> DNA<213> Homo sapiens<220> <223> Cdk1 ribozyme binding site
<400> 23
cttcagggta caactcttt

<212> DNA<213> Homo sapiens<220> <223> Cdk1 ribozyme binding site
<400> 24
ggtacaactc tttgaagat

<212> DNA<213> Homo sapiens<220> <223> Cdk1 ribozyme binding site
<400> 25
tacaactctt tgaagatcc

<212> DNA<213> Homo sapiens<220> <223> Cdk1 ribozyme binding site
<400> 26
acaactcttt gaagatccc

<212> DNA<213> Homo sapiens<220> <223> Cdk1 ribozyme binding site
<400> 27

tttgaagatc ccactgttg

<212> DNA<213> Homo sapiens<220> <223> Cdk1 ribozyme binding site
<400> 28
tcccactgtt gacaaggag

<212> DNA<213> Homo sapiens<220> <223> Cdk1 ribozyme binding site
<400> 29
caaggaggtt gagatcagg

<212> DNA<213> Homo sapiens<220> <223> Cdk1 ribozyme binding site
<400> 30
ggttgagatc aggaaaaaa

<212> DNA<213> Homo sapiens<220> <223> Cdk1 ribozyme binding site
<400> 31
aaaagtgcta aagatatac

<212> DNA<213> Homo sapiens<220> <223> Cdk1 ribozyme binding site
<400> 32
gctaaagata tacaataaa

<212> DNA<213> Homo sapiens<220> <223> Cdk1 ribozyme binding site
<400> 33
taaagatata caataaaag

<212> DNA<213> Homo sapiens<220> <223> Cdk1 ribozyme binding site
<400> 34
atatacaata aaagggaag

<212> DNA<213> Homo sapiens<220> <223> Cdk1 ribozyme binding site
<400> 35
gaagaagatt ttcctagtc

<212> DNA<213> Homo sapiens<220> <223> Cdk1 ribozyme binding site
<400> 36
aagaagattt tcctagtct

<212> DNA<213> Homo sapiens<220> <223> Cdk1 ribozyme binding site
<400> 37
agaagatttt cctagtcta

<212> DNA<213> Homo sapiens<220> <223> Cdk1 ribozyme binding site
<400> 38
gaagattttc ctagtctaa

<212> DNA<213> Homo sapiens<220> <223> Cdk1 ribozyme binding site
<400> 39
gattttccta gtctaagag

<212> DNA<213> Homo sapiens<220> <223> Cdk1 ribozyme binding site
<400> 40
tttcctagtc taagagaat

<212> DNA<213> Homo sapiens<220> <223> Cdk1 ribozyme binding site

<400> 41
tcctagtcta agagaatac

<212> DNA<213> Homo sapiens<220> <223> Cdk1 ribozyme binding site
<400> 42
taagagaata caatgattt

<212> DNA<213> Homo sapiens<220> <223> Cdk1 ribozyme binding site
<400> 43
tacaatgatt tcttggaag

<212> DNA<213> Homo sapiens<220> <223> Cdk1 ribozyme binding site
<400> 44
acaatgattt cttggaaga

<212> DNA<213> Homo sapiens<220> <223> Cdk1 ribozyme binding site
<400> 45
caatgatttc ttggaagaa

<212> DNA<213> Homo sapiens<220> <223> Cdk1 ribozyme binding site
<400> 46
atgatttctt ggaagaagt

<212> DNA<213> Homo sapiens<220> <223> Cdk1 ribozyme binding site
<400> 47
ggaagaaatt gttttcaac

<212> DNA<213> Homo sapiens<220> <223> Cdk1 ribozyme binding site
<400> 48
agaaattgtt ttcaacttg

<212> DNA<213> Homo sapiens<220> <223> Cdk1 ribozyme binding site
<400> 49
gaaattgttt tcaacttga

<212> DNA<213> Homo sapiens<220> <223> Cdk1 ribozyme binding site
<400> 50
aaattgtttt caacttgac

<212> DNA<213> Homo sapiens<220> <223> Cdk1 ribozyme binding site
<400> 51
aattgttttc aacttgacc

<212> DNA<213> Homo sapiens<220> <223> Cdk1 ribozyme binding site
<400> 52
ttttcaactt gaccaacaa

<212> DNA<213> Homo sapiens<220> <223> Cdk1 ribozyme binding site
<400> 53
aatgtggatt tggacaaca

<212> DNA<213> Homo sapiens<220> <223> Cdk1 ribozyme binding site
<400> 54
atgtggattt ggacaacac

<212> DNA<213> Homo sapiens<220> <223> Cdkl ribozyme binding site
<400> 55
aatggagata taccaaaag

<212> DNA<213> Homo sapiens<220> <223> Cdkl ribozyme binding site
<400> 56
tggagatata ccaaaagga

<212> DNA<213> Homo sapiens<220> <223> Cdkl ribozyme binding site
<400> 57
caaagatgtt attcagaaa

<212> DNA<213> Homo sapiens<220> <223> Cdkl ribozyme binding site
<400> 58
aaagatgtta ttcagaaaa

<212> DNA<213> Homo sapiens<220> <223> Cdkl ribozyme binding site
<400> 59
agatgttatt cagaaaaat

<212> DNA<213> Homo sapiens<220> <223> Cdkl ribozyme binding site
<400> 60
gatgttattc agaaaaata

<212> DNA<213> Homo sapiens<220> <223> Cdkl ribozyme binding site
<400> 61
cagaaaaata aattaaagc

<212> DNA<213> Homo sapiens<220> <223> Cdkl ribozyme binding site
<400> 62
aaaataaatt aaagctgac

<212> DNA<213> Homo sapiens<220> <223> Cdkl ribozyme binding site
<400> 63
aaataaatta aagctgact

<212> DNA<213> Homo sapiens<220> <223> Cdkl ribozyme binding site
<400> 64
aagctgactc gagaacagg

<212> DNA<213> Homo sapiens<220> <223> Cdkl ribozyme binding site
<400> 65
gaagaagctt tagaagtgg

<212> DNA<213> Homo sapiens<220> <223> Cdkl ribozyme binding site
<400> 66
aagaagcttt agaagtgga

<212> DNA<213> Homo sapiens<220> <223> Cdkl ribozyme binding site
<400> 67
agaagcttta gaagtggaa

<212> DNA<213> Homo sapiens<220> <223> Cdkl ribozyme binding site
<400> 68
aaagaagatt atttataca

<212> DNA<213> Homo sapiens<220> <223> Cdk1 ribozyme binding site
<400> 69
aagaagatta tttatacaa

<212> DNA<213> Homo sapiens<220> <223> Cdk1 ribozyme binding site
<400> 70
gaagattatt tatacaaaa

<212> DNA<213> Homo sapiens<220> <223> Cdk1 ribozyme binding site
<400> 71
aagattattt atacaaaaa

<212> DNA<213> Homo sapiens<220> <223> Cdk1 ribozyme binding site
<400> 72
agattattta tacaaaaag

<212> DNA<213> Homo sapiens<220> <223> Cdk1 ribozyme binding site
<400> 73
attatttata caaaaagaa

<212> DNA<213> Homo sapiens<220> <223> Cdk1 ribozyme binding site
<400> 74
gcagcagatt ctaaaaagg

<212> DNA<213> Homo sapiens<220> <223> Cdk1 ribozyme binding site
<400> 75
cagcagattc taaaaagga

<212> DNA<213> Homo sapiens<220> <223> Cdk1 ribozyme binding site
<400> 76
gcagattcta aaaaggaag

<212> DNA<213> Homo sapiens<220> <223> Cdk1 ribozyme binding site
<400> 77
aggaagaata agcaggctt

<212> DNA<213> Homo sapiens<220> <223> Cdk1 ribozyme binding site
<400> 78
aagcaggctt ttttagatg

<212> DNA<213> Homo sapiens<220> <223> Cdk1 ribozyme binding site
<400> 79
agcaggcttt tttagatga

<212> DNA<213> Homo sapiens<220> <223> Cdk1 ribozyme binding site
<400> 80
gcaggctttt ttagatgag

<212> DNA<213> Homo sapiens<220> <223> Cdk1 ribozyme binding site
<400> 81
caggcttttt tagatgagc

<212> DNA<213> Homo sapiens<220> <223> Cdk1 ribozyme binding site
<400> 82

aggcttttt agatgagct

<212> DNA<213> Homo sapiens<220> <223> Cdk1 ribozyme binding site
<400> 83
ggctttttta gatgagctg

<212> DNA<213> Homo sapiens<220> <223> Cdk1 ribozyme binding site
<400> 84
ctggagagtt ctgatctcc

<212> DNA<213> Homo sapiens<220> <223> Cdk1 ribozyme binding site
<400> 85
tggagagttc tgatctccc

<212> DNA<213> Homo sapiens<220> <223> Cdk1 ribozyme binding site
<400> 86
agttctgatc tccctgttg

<212> DNA<213> Homo sapiens<220> <223> Cdk1 ribozyme binding site
<400> 87
ttctgatctc cctgttgct

<212> DNA<213> Homo sapiens<220> <223> Cdk1 ribozyme binding site
<400> 88
tctccctgtt gctctgctt

<212> DNA<213> Homo sapiens<220> <223> Cdk1 ribozyme binding site
<400> 89
cctgttgctc tgcttttgg

<212> DNA<213> Homo sapiens<220> <223> Cdk1 ribozyme binding site
<400> 90
tgctctgctt ttggctcag

<212> DNA<213> Homo sapiens<220> <223> Cdk1 ribozyme binding site
<400> 91
gctctgcttt tggctcagc

<212> DNA<213> Homo sapiens<220> <223> Cdk1 ribozyme binding site
<400> 92
ctctgctttt ggctcagca

<212> DNA<213> Homo sapiens<220> <223> Cdk1 ribozyme binding site
<400> 93
cttttggctc agcataaag

<212> DNA<213> Homo sapiens<220> <223> Cdk1 ribozyme binding site
<400> 94
gctcagcata aagatagat

<212> DNA<213> Homo sapiens<220> <223> Cdk1 ribozyme binding site
<400> 95
cataaagata gatctaccc

<212> DNA<213> Homo sapiens<220> <223> Cdk1 ribozyme binding site

<400> 96
aagatagatc tacccaatt

<212> DNA<213> Homo sapiens<220> <223> Cdk1 ribozyme binding site
<400> 97
gatagatcta cccaattag

<212> DNA<213> Homo sapiens<220> <223> Cdk1 ribozyme binding site
<400> 98
ctacccaatt agaaatgca

<212> DNA<213> Homo sapiens<220> <223> Cdk1 ribozyme binding site
<400> 99
tacccaatta gaaatgcaa

<212> DNA<213> Homo sapiens<220> <223> Cdk1 ribozyme binding site
<400> 100
aatgcaactt gagaaaccc

<212> DNA<213> Homo sapiens<220> <223> Cdk1 ribozyme binding site
<400> 101
caaacctgta aaaccagtg

<212> DNA<213> Homo sapiens<220> <223> Cdk1 ribozyme binding site
<400> 102
cagtgacgtt ttccacagg

<212> DNA<213> Homo sapiens<220> <223> Cdk1 ribozyme binding site
<400> 103
agtgacgttt tccacaggc

<212> DNA<213> Homo sapiens<220> <223> Cdk1 ribozyme binding site
<400> 104
gtgacgtttt ccacaggca

<212> DNA<213> Homo sapiens<220> <223> Cdk1 ribozyme binding site
<400> 105
tgacgttttc cacaggcat

<212> DNA<213> Homo sapiens<220> <223> Cdk1 ribozyme binding site
<400> 106
cacaggcatc aaaatgggt

<212> DNA<213> Homo sapiens<220> <223> Cdk1 ribozyme binding site
<400> 107
aaaatgggtc aacatattt

<212> DNA<213> Homo sapiens<220> <223> Cdk1 ribozyme binding site
<400> 108
ggtcaacata tttcactgg

<212> DNA<213> Homo sapiens<220> <223> Cdk1 ribozyme binding site
<400> 109
tcaacatatt tcactggca

<212> DNA<213> Homo sapiens<220> <223> Cdk1 ribozyme binding site
<400> 110
caacatattt cactggcac

<212> DNA<213> Homo sapiens<220> <223> Cdk1 ribozyme binding site
<400> 111
aacatatttc actggcacc

<212> DNA<213> Homo sapiens<220> <223> Cdk1 ribozyme binding site
<400> 112
ctggcaccta ttcacaagc

<212> DNA<213> Homo sapiens<220> <223> Cdk1 ribozyme binding site
<400> 113
ggcacctatt cacaagctt

<212> DNA<213> Homo sapiens<220> <223> Cdk1 ribozyme binding site
<400> 114
gcacctattc acaagcttg

<212> DNA<213> Homo sapiens<220> <223> Cdk1 ribozyme binding site
<400> 115
tcacaagctt gaagaagct

<212> DNA<213> Homo sapiens<220> <223> Cdk1 ribozyme binding site
<400> 116
gaagaagctc tgtatgaat

<212> DNA<213> Homo sapiens<220> <223> Cdk1 ribozyme binding site
<400> 117
aagctctgta tgaatacca

<212> DNA<213> Homo sapiens<220> <223> Cdk1 ribozyme binding site
<400> 118
tgtatgaata ccagccact

<212> DNA<213> Homo sapiens<220> <223> Cdk1 ribozyme binding site
<400> 119
actgcagata gagacatat

<212> DNA<213> Homo sapiens<220> <223> Cdk1 ribozyme binding site
<400> 120
tagagacata tggaccaca

<212> DNA<213> Homo sapiens<220> <223> Cdk1 ribozyme binding site
<400> 121
accacatgtt cctgagctt

<212> DNA<213> Homo sapiens<220> <223> Cdk1 ribozyme binding site
<400> 122
ccacatgttc ctgagcttg

<212> DNA<213> Homo sapiens<220> <223> Cdk1 ribozyme binding site
<400> 123
tcctgagctt gagatgcta

<212> DNA<213> Homo sapiens<220> <223> Cdk1 ribozyme binding site
<400> 124
tgagatgcta ggaagactt

<212> DNA<213> Homo sapiens<220> <223> Cdk1 ribozyme binding site
<400> 125
aggaagactt gggtattta

<212> DNA<213> Homo sapiens<220> <223> Cdk1 ribozyme binding site
<400> 126
gacttgggta tttaaacca

<212> DNA<213> Homo sapiens<220> <223> Cdk1 ribozyme binding site
<400> 127
cttgggtatt taaaccatg

<212> DNA<213> Homo sapiens<220> <223> Cdk1 ribozyme binding site
<400> 128
ttgggtattt aaaccatgt

<212> DNA<213> Homo sapiens<220> <223> Cdk1 ribozyme binding site
<400> 129
tgggtattta aaccatgtc

<212> DNA<213> Homo sapiens<220> <223> Cdk1 ribozyme binding site
<400> 130
aaaccatgtc agagctgcc

<212> DNA<213> Homo sapiens<220> <223> Cdk1 ribozyme binding site
<400> 131
gagctgcctc accacagga

<212> DNA<213> Homo sapiens<220> <223> Cdk1 ribozyme binding site
<400> 132
acaggacctt gctggaggc

<212> DNA<213> Homo sapiens<220> <223> Cdk1 ribozyme binding site
<400> 133
ctggaggcta tacttcttc

<212> DNA<213> Homo sapiens<220> <223> Cdk1 ribozyme binding site
<400> 134
ggaggctata cttcttctc

<212> DNA<213> Homo sapiens<220> <223> Cdk1 ribozyme binding site
<400> 135
ggctatactt cttctcttg

<212> DNA<213> Homo sapiens<220> <223> Cdk1 ribozyme binding site
<400> 136
gctatacttc ttctcttgc

<212> DNA<213> Homo sapiens<220> <223> Cdk1 ribozyme binding site
<400> 137

tatacttctt ctcttgctt

<212> DNA<213> Homo sapiens<220> <223> Cdk1 ribozyme binding site
<400> 138
atacttcttc tcttgcttg

<212> DNA<213> Homo sapiens<220> <223> Cdk1 ribozyme binding site
<400> 139
acttcttctc ttgcttgtc

<212> DNA<213> Homo sapiens<220> <223> Cdk1 ribozyme binding site
<400> 140
ttcttctctt gcttgtcac

<212> DNA<213> Homo sapiens<220> <223> Cdk1 ribozyme binding site
<400> 141
tctcttgctt gtcacagag

<212> DNA<213> Homo sapiens<220> <223> Cdk1 ribozyme binding site
<400> 142
cttgcttgtc acagagcac

<212> DNA<213> Homo sapiens<220> <223> Cdk1 ribozyme binding site
<400> 143
cagagcacta caggatgca

<212> DNA<213> Homo sapiens<220> <223> Cdk1 ribozyme binding site
<400> 144
aggatgcatt cagtgggct

<212> DNA<213> Homo sapiens<220> <223> Cdk1 ribozyme binding site
<400> 145
ggatgcattc agtgggctt

<212> DNA<213> Homo sapiens<220> <223> Cdk1 ribozyme binding site
<400> 146
cagtgggctt ttctggcag

<212> DNA<213> Homo sapiens<220> <223> Cdk1 ribozyme binding site
<400> 147
agtgggcttt tctggcagc

<212> DNA<213> Homo sapiens<220> <223> Cdk1 ribozyme binding site
<400> 148
gtgggctttt ctggcagcc

<212> DNA<213> Homo sapiens<220> <223> Cdk1 ribozyme binding site
<400> 149
tgggcttttc tggcagccc

<212> DNA<213> Homo sapiens<220> <223> Cdk2 ribozyme binding site
<400> 150
tggagaactt ccaaaaggt

<212> DNA<213> Homo sapiens<220> <223> Cdk2 ribozyme binding site

<400> 151
ggagaacttc caaaaggtg

<212> DNA<213> Homo sapiens<220> <223> Cdk2 ribozyme binding site
<400> 152
ggaaaagatc ggagagggc

<212> DNA<213> Homo sapiens<220> <223> Cdk2 ribozyme binding site
<400> 153
agggcacgta cggcgttgt

<212> DNA<213> Homo sapiens<220> <223> Cdk2 ribozyme binding site
<400> 154
gtacggagtt gtgtacaaa

<212> DNA<213> Homo sapiens<220> <223> Cdk2 ribozyme binding site
<400> 155
gagttgtgta caaagccag

<212> DNA<213> Homo sapiens<220> <223> Cdk2 ribozyme binding site
<400> 156
gaaacaagtt gacgggaga

<212> DNA<213> Homo sapiens<220> <223> Cdk2 ribozyme binding site
<400> 157
ggtggcgctt aagaaaatc

<212> DNA<213> Homo sapiens<220> <223> Cdk2 ribozyme binding site
<400> 158
gtggcgctta agaaaatcc

<212> DNA<213> Homo sapiens<220> <223> Cdk2 ribozyme binding site
<400> 159
taagaaaatc cgtggacac

<212> DNA<213> Homo sapiens<220> <223> Cdk2 ribozyme binding site
<400> 160
gtgcccagta ctgccatcc

<212> DNA<213> Homo sapiens<220> <223> Cdk2 ribozyme binding site
<400> 161
tactgccatc cgagagatc

<212> DNA<213> Homo sapiens<220> <223> Cdk2 ribozyme binding site
<400> 162
ccgagagatc tctctgctt

<212> DNA<213> Homo sapiens<220> <223> Cdk2 ribozyme binding site
<400> 163
gagagatctc tctgcttaa

<212> DNA<213> Homo sapiens<220> <223> Cdk2 ribozyme binding site
<400> 164
gagatctctc tgcttaagg

<212> DNA<213> Homo sapiens<220> <223> Cdk2 ribozyme binding site
<400> 165
ctctctgctt aaggagctt

<212> DNA<213> Homo sapiens<220> <223> Cdk2 ribozyme binding site
<400> 166
tctctgctta aggagctta

<212> DNA<213> Homo sapiens<220> <223> Cdk2 ribozyme binding site
<400> 167
taaggagctt aaccatcct

<212> DNA<213> Homo sapiens<220> <223> Cdk2 ribozyme binding site
<400> 168
aaggagctta accatccta

<212> DNA<213> Homo sapiens<220> <223> Cdk2 ribozyme binding site
<400> 169
cttaaccatc ctaatattg

<212> DNA<213> Homo sapiens<220> <223> Cdk2 ribozyme binding site
<400> 170
aaccatccta atattgtca

<212> DNA<213> Homo sapiens<220> <223> Cdk2 ribozyme binding site
<400> 171
catcctaata ttgtcaagc

<212> DNA<213> Homo sapiens<220> <223> Cdk2 ribozyme binding site
<400> 172
tcctaatatt gtcaagctg

<212> DNA<213> Homo sapiens<220> <223> Cdk2 ribozyme binding site
<400> 173
taatattgtc aagctgctg

<212> DNA<213> Homo sapiens<220> <223> Cdk2 ribozyme binding site
<400> 174
gctggatgtc attcacaca

<212> DNA<213> Homo sapiens<220> <223> Cdk2 ribozyme binding site
<400> 175
ggatgtcatt cacacagaa

<212> DNA<213> Homo sapiens<220> <223> Cdk2 ribozyme binding site
<400> 176
gatgtcattc acacagaaa

<212> DNA<213> Homo sapiens<220> <223> Cdk2 ribozyme binding site
<400> 177
acagaaaata aactctacc

<212> DNA<213> Homo sapiens<220> <223> Cdk2 ribozyme binding site
<400> 178
aaataaactc tacctggtt

<212> DNA<213> Homo sapiens<220> <223> Cdk2 ribozyme binding site
<400> 179
ataaactcta cctggtttt

<212> DNA<213> Homo sapiens<220> <223> Cdk2 ribozyme binding site
<400> 180
ctacctggtt tttgaattt

<212> DNA<213> Homo sapiens<220> <223> Cdk2 ribozyme binding site
<400> 181
tacctggttt ttgaatttc

<212> DNA<213> Homo sapiens<220> <223> Cdk2 ribozyme binding site
<400> 182
acctggtttt tgaatttct

<212> DNA<213> Homo sapiens<220> <223> Cdk2 ribozyme binding site
<400> 183
cctggttttt gaatttctg

<212> DNA<213> Homo sapiens<220> <223> Cdk2 ribozyme binding site
<400> 184
tttttgaatt tctgcacca

<212> DNA<213> Homo sapiens<220> <223> Cdk2 ribozyme binding site
<400> 185
ttttgaattt ctgcaccaa

<212> DNA<213> Homo sapiens<220> <223> Cdk2 ribozyme binding site
<400> 186
tttgaatttc tgcaccaag

<212> DNA<213> Homo sapiens<220> <223> Cdk2 ribozyme binding site
<400> 187
caccaagatc tcaagaaat

<212> DNA<213> Homo sapiens<220> <223> Cdk2 ribozyme binding site
<400> 188
ccaagatctc aagaaattc

<212> DNA<213> Homo sapiens<220> <223> Cdk2 ribozyme binding site
<400> 189
tcaagaaatt catggatgc

<212> DNA<213> Homo sapiens<220> <223> Cdk2 ribozyme binding site
<400> 190
caagaaattc atggatgcc

<212> DNA<213> Homo sapiens<220> <223> Cdk2 ribozyme binding site
<400> 191
tggatgcctc tgctctcac

<212> DNA<213> Homo sapiens<220> <223> Cdk2 ribozyme binding site
<400> 192

gcctctgctc tcactggca

<212> DNA<213> Homo sapiens<220> <223> Cdk2 ribozyme binding site
<400> 193
ctctgctctc actggcatt

<212> DNA<213> Homo sapiens<220> <223> Cdk2 ribozyme binding site
<400> 194
cactggcatt cctcttccc

<212> DNA<213> Homo sapiens<220> <223> Cdk2 ribozyme binding site
<400> 195
actggcattc ctcttcccc

<212> DNA<213> Homo sapiens<220> <223> Cdk2 ribozyme binding site
<400> 196
ggcattcctc ttcccctca

<212> DNA<213> Homo sapiens<220> <223> Cdk2 ribozyme binding site
<400> 197
cattcctctt cccctcatc

<212> DNA<213> Homo sapiens<220> <223> Cdk2 ribozyme binding site
<400> 198
attcctcttc ccctcatca

<212> DNA<213> Homo sapiens<220> <223> Cdk2 ribozyme binding site
<400> 199
tcttcccctc atcaagagc

<212> DNA<213> Homo sapiens<220> <223> Cdk2 ribozyme binding site
<400> 200
tcccctcatc aagagctat

<212> DNA<213> Homo sapiens<220> <223> Cdk2 ribozyme binding site
<400> 201
tcaagagcta tctgttcca

<212> DNA<213> Homo sapiens<220> <223> Cdk2 ribozyme binding site
<400> 202
aagagctatc tgttccagc

<212> DNA<213> Homo sapiens<220> <223> Cdk2 ribozyme binding site
<400> 203
gctatctgtt ccagctgct

<212> DNA<213> Homo sapiens<220> <223> Cdk2 ribozyme binding site
<400> 204
ctatctgttc cagctgctc

<212> DNA<213> Homo sapiens<220> <223> Cdk2 ribozyme binding site
<400> 205
ccagctgctc cagggccta

<212> DNA<213> Homo sapiens<220> <223> Cdk2 ribozyme binding site

<400> 206
ccagggccta gctttctgc

<212> DNA<213> Homo sapiens<220> <223> Cdk2 ribozyme binding site
<400> 207
ggcctagctt tctgccatt

<212> DNA<213> Homo sapiens<220> <223> Cdk2 ribozyme binding site
<400> 208
gcctagcttt ctgccattc

<212> DNA<213> Homo sapiens<220> <223> Cdk2 ribozyme binding site
<400> 209
cctagctttc tgccattct

<212> DNA<213> Homo sapiens<220> <223> Cdk2 ribozyme binding site
<400> 210
ttctgccatt ctcatcggg

<212> DNA<213> Homo sapiens<220> <223> Cdk2 ribozyme binding site
<400> 211
tctgccattc tcatcgggt

<212> DNA<213> Homo sapiens<220> <223> Cdk2 ribozyme binding site
<400> 212
tgccattctc atcgggtcc

<212> DNA<213> Homo sapiens<220> <223> Cdk2 ribozyme binding site
<400> 213
cattctcatc gggtcctcc

<212> DNA<213> Homo sapiens<220> <223> Cdk2 ribozyme binding site
<400> 214
tcatcgggtc ctccaccga

<212> DNA<213> Homo sapiens<220> <223> Cdk2 ribozyme binding site
<400> 215
tcgggtcctc caccgagac

<212> DNA<213> Homo sapiens<220> <223> Cdk2 ribozyme binding site
<400> 216
ccgagacctt aaacctcag

<212> DNA<213> Homo sapiens<220> <223> Cdk2 ribozyme binding site
<400> 217
cgagacctta aacctcaga

<212> DNA<213> Homo sapiens<220> <223> Cdk2 ribozyme binding site
<400> 218
cttaaacctc agaatctgc

<212> DNA<213> Homo sapiens<220> <223> Cdk2 ribozyme binding site
<400> 219
cctcagaatc tgcttatta

<212> DNA<213> Homo sapiens<220> <223> Cdk2 ribozyme binding site
<400> 220
gaatctgctt attaacaca

<212> DNA<213> Homo sapiens<220> <223> Cdk2 ribozyme binding site
<400> 221
aatctgctta ttaacacag

<212> DNA<213> Homo sapiens<220> <223> Cdk2 ribozyme binding site
<400> 222
tctgcttatt aacacagag

<212> DNA<213> Homo sapiens<220> <223> Cdk2 ribozyme binding site
<400> 223
ctgcttatta acacagagg

<212> DNA<213> Homo sapiens<220> <223> Cdk2 ribozyme binding site
<400> 224
gggggccatc aagctagca

<212> DNA<213> Homo sapiens<220> <223> Cdk2 ribozyme binding site
<400> 225
catcaagcta gcagacttt

<212> DNA<213> Homo sapiens<220> <223> Cdk2 ribozyme binding site
<400> 226
tagcagactt tggactagc

<212> DNA<213> Homo sapiens<220> <223> Cdk2 ribozyme binding site
<400> 227
agcagacttt ggactagcc

<212> DNA<213> Homo sapiens<220> <223> Cdk2 ribozyme binding site
<400> 228
ctttggacta gccagagct

<212> DNA<213> Homo sapiens<220> <223> Cdk2 ribozyme binding site
<400> 229
gccagagctt ttggagtcc

<212> DNA<213> Homo sapiens<220> <223> Cdk2 ribozyme binding site
<400> 230
ccagagcttt tggagtccc

<212> DNA<213> Homo sapiens<220> <223> Cdk2 ribozyme binding site
<400> 231
cagagctttt ggagtccct

<212> DNA<213> Homo sapiens<220> <223> Cdk2 ribozyme binding site
<400> 232
ttttggagtc cctgttcgt

<212> DNA<213> Homo sapiens<220> <223> Cdk2 ribozyme binding site
<400> 233
agtccctgtt cgtacttac

<212> DNA<213> Homo sapiens<220> <223> Cdk2 ribozyme binding site
<400> 179
ataaactcta cctggtttt

<212> DNA<213> Homo sapiens<220> <223> Cdk2 ribozyme binding site
<400> 180
ctacctggtt tttgaattt

<212> DNA<213> Homo sapiens<220> <223> Cdk2 ribozyme binding site
<400> 181
tacctggttt ttgaatttc

<212> DNA<213> Homo sapiens<220> <223> Cdk2 ribozyme binding site
<400> 182
acctggtttt tgaatttct

<212> DNA<213> Homo sapiens<220> <223> Cdk2 ribozyme binding site
<400> 183
cctggttttt gaatttctg

<212> DNA<213> Homo sapiens<220> <223> Cdk2 ribozyme binding site
<400> 184
tttttgaatt tctgcacca

<212> DNA<213> Homo sapiens<220> <223> Cdk2 ribozyme binding site
<400> 185
ttttgaattt ctgcaccaa

<212> DNA<213> Homo sapiens<220> <223> Cdk2 ribozyme binding site
<400> 186
tttgaatttc tgcaccaag

<212> DNA<213> Homo sapiens<220> <223> Cdk2 ribozyme binding site
<400> 187
caccaagatc tcaagaaat

<212> DNA<213> Homo sapiens<220> <223> Cdk2 ribozyme binding site
<400> 188
ccaagatctc aagaaattc

<212> DNA<213> Homo sapiens<220> <223> Cdk2 ribozyme binding site
<400> 189
tcaagaaatt catggatgc

<212> DNA<213> Homo sapiens<220> <223> Cdk2 ribozyme binding site
<400> 190
caagaaattc atggatgcc

<212> DNA<213> Homo sapiens<220> <223> Cdk2 ribozyme binding site
<400> 191
tggatgcctc tgctctcac

<212> DNA<213> Homo sapiens<220> <223> Cdk2 ribozyme binding site
<400> 192

gcctctgctc tcactggca

<212> DNA<213> Homo sapiens<220> <223> Cdk2 ribozyme binding site
<400> 193
ctctgctctc actggcatt

<212> DNA<213> Homo sapiens<220> <223> Cdk2 ribozyme binding site
<400> 194
cactggcatt cctcttccc

<212> DNA<213> Homo sapiens<220> <223> Cdk2 ribozyme binding site
<400> 195
actggcattc ctcttcccc

<212> DNA<213> Homo sapiens<220> <223> Cdk2 ribozyme binding site
<400> 196
ggcattcctc ttcccctca

<212> DNA<213> Homo sapiens<220> <223> Cdk2 ribozyme binding site
<400> 197
cattcctctt cccctcatc

<212> DNA<213> Homo sapiens<220> <223> Cdk2 ribozyme binding site
<400> 198
attcctcttc ccctcatca

<212> DNA<213> Homo sapiens<220> <223> Cdk2 ribozyme binding site
<400> 199
tcttcccctc atcaagagc

<212> DNA<213> Homo sapiens<220> <223> Cdk2 ribozyme binding site
<400> 200
tcccctcatc aagagctat

<212> DNA<213> Homo sapiens<220> <223> Cdk2 ribozyme binding site
<400> 201
tcaagagcta tctgttcca

<212> DNA<213> Homo sapiens<220> <223> Cdk2 ribozyme binding site
<400> 202
aagagctatc tgttccagc

<212> DNA<213> Homo sapiens<220> <223> Cdk2 ribozyme binding site
<400> 203
gctatctgtt ccagctgct

<212> DNA<213> Homo sapiens<220> <223> Cdk2 ribozyme binding site
<400> 204
ctatctgttc cagctgctc

<212> DNA<213> Homo sapiens<220> <223> Cdk2 ribozyme binding site
<400> 205
ccagctgctc cagggccta

<212> DNA<213> Homo sapiens<220> <223> Cdk2 ribozyme binding site

<400> 206
ccagggccta gctttctgc

<212> DNA<213> Homo sapiens<220> <223> Cdk2 ribozyme binding site
<400> 207
ggcctagctt tctgccatt

<212> DNA<213> Homo sapiens<220> <223> Cdk2 ribozyme binding site
<400> 208
gcctagcttt ctgccattc

<212> DNA<213> Homo sapiens<220> <223> Cdk2 ribozyme binding site
<400> 209
cctagctttc tgccattct

<212> DNA<213> Homo sapiens<220> <223> Cdk2 ribozyme binding site
<400> 210
ttctgccatt ctcatcggg

<212> DNA<213> Homo sapiens<220> <223> Cdk2 ribozyme binding site
<400> 211
tctgccattc tcatcgggt

<212> DNA<213> Homo sapiens<220> <223> Cdk2 ribozyme binding site
<400> 212
tgccattctc atcgggtcc

<212> DNA<213> Homo sapiens<220> <223> Cdk2 ribozyme binding site
<400> 213
cattctcatc gggtcctcc

<212> DNA<213> Homo sapiens<220> <223> Cdk2 ribozyme binding site
<400> 214
tcatcgggtc ctccaccga

<212> DNA<213> Homo sapiens<220> <223> Cdk2 ribozyme binding site
<400> 215
tcgggtcctc caccgagac

<212> DNA<213> Homo sapiens<220> <223> Cdk2 ribozyme binding site
<400> 216
ccgagacctt aaacctcag

<212> DNA<213> Homo sapiens<220> <223> Cdk2 ribozyme binding site
<400> 217
cgagacctta aacctcaga

<212> DNA<213> Homo sapiens<220> <223> Cdk2 ribozyme binding site
<400> 218
cttaaacctc agaatctgc

<212> DNA<213> Homo sapiens<220> <223> Cdk2 ribozyme binding site
<400> 219
cctcagaatc tgcttatta

<212> DNA<213> Homo sapiens<220> <223> Cdk2 ribozyme binding site
<400> 220
gaatctgctt attaacaca

<212> DNA<213> Homo sapiens<220> <223> Cdk2 ribozyme binding site
<400> 221
aatctgctta ttaacacag

<212> DNA<213> Homo sapiens<220> <223> Cdk2 ribozyme binding site
<400> 222
tctgcttatt aacacagag

<212> DNA<213> Homo sapiens<220> <223> Cdk2 ribozyme binding site
<400> 223
ctgcttatta acacagagg

<212> DNA<213> Homo sapiens<220> <223> Cdk2 ribozyme binding site
<400> 224
gggggccatc aagctagca

<212> DNA<213> Homo sapiens<220> <223> Cdk2 ribozyme binding site
<400> 225
catcaagcta gcagacttt

<212> DNA<213> Homo sapiens<220> <223> Cdk2 ribozyme binding site
<400> 226
tagcagactt tggactagc

<212> DNA<213> Homo sapiens<220> <223> Cdk2 ribozyme binding site
<400> 227
agcagacttt ggactagcc

<212> DNA<213> Homo sapiens<220> <223> Cdk2 ribozyme binding site
<400> 228
ctttggacta gccagagct

<212> DNA<213> Homo sapiens<220> <223> Cdk2 ribozyme binding site
<400> 229
gccagagctt ttggagtcc

<212> DNA<213> Homo sapiens<220> <223> Cdk2 ribozyme binding site
<400> 230
ccagagcttt tggagtccc

<212> DNA<213> Homo sapiens<220> <223> Cdk2 ribozyme binding site
<400> 231
cagagctttt ggagtccct

<212> DNA<213> Homo sapiens<220> <223> Cdk2 ribozyme binding site
<400> 232
ttttggagtc cctgttcgt

<212> DNA<213> Homo sapiens<220> <223> Cdk2 ribozyme binding site
<400> 233
agtccctgtt cgtacttac

<212> DNA<213> Homo sapiens<220> <223> Cdk2 ribozyme binding site
<400> 234
gtccctgttc gtacttaca

<212> DNA<213> Homo sapiens<220> <223> Cdk2 ribozyme binding site
<400> 235
cctgttcgta cttacaccc

<212> DNA<213> Homo sapiens<220> <223> Cdk2 ribozyme binding site
<400> 236
gttcgtactt acacccatg

<212> DNA<213> Homo sapiens<220> <223> Cdk2 ribozyme binding site
<400> 237
ttcgtactta cacccatga

<212> DNA<213> Homo sapiens<220> <223> Cdk2 ribozyme binding site
<400> 238
ccctgtggta ccgagctcc

<212> DNA<213> Homo sapiens<220> <223> Cdk2 ribozyme binding site
<400> 239
taccgagctc ctgaaatcc

<212> DNA<213> Homo sapiens<220> <223> Cdk2 ribozyme binding site
<400> 240
tcctgaaatc ctcctgggc

<212> DNA<213> Homo sapiens<220> <223> Cdk2 ribozyme binding site
<400> 241
tgaaatcctc ctgggctgc

<212> DNA<213> Homo sapiens<220> <223> Cdk2 ribozyme binding site
<400> 242
gctgcaaata ttattccac

<212> DNA<213> Homo sapiens<220> <223> Cdk2 ribozyme binding site
<400> 243
tgcaaatatt attccacag

<212> DNA<213> Homo sapiens<220> <223> Cdk2 ribozyme binding site
<400> 244
gcaaatatta ttccacagc

<212> DNA<213> Homo sapiens<220> <223> Cdk2 ribozyme binding site
<400> 245
aaatattatt ccacagctg

<212> DNA<213> Homo sapiens<220> <223> Cdk2 ribozyme binding site
<400> 246
aatattattc cacagctgt

<212> DNA<213> Homo sapiens<220> <223> Cdk2 ribozyme binding site
<400> 247

tgtggacatc tggagcctg

<212> DNA<213> Homo sapiens<220> <223> Cdk2 ribozyme binding site
<400> 248
gggctgcatc tttgctgag

<212> DNA<213> Homo sapiens<220> <223> Cdk2 ribozyme binding site
<400> 249
gctgcatctt tgctgagat

<212> DNA<213> Homo sapiens<220> <223> Cdk2 ribozyme binding site
<400> 250
ctgcatcttt gctgagatg

<212> DNA<213> Homo sapiens<220> <223> Cdk2 ribozyme binding site
<400> 251
atggtgactc gccgggccc

<212> DNA<213> Homo sapiens<220> <223> Cdk2 ribozyme binding site
<400> 252
ccgggcccta ttccctgga

<212> DNA<213> Homo sapiens<220> <223> Cdk2 ribozyme binding site
<400> 253
gggccctatt ccctggaga

<212> DNA<213> Homo sapiens<220> <223> Cdk2 ribozyme binding site
<400> 254
ggccctattc cctggagat

<212> DNA<213> Homo sapiens<220> <223> Cdk2 ribozyme binding site
<400> 255
cctggagatt ctgagattg

<212> DNA<213> Homo sapiens<220> <223> Cdk2 ribozyme binding site
<400> 256
ctggagattc tgagattga

<212> DNA<213> Homo sapiens<220> <223> Cdk2 ribozyme binding site
<400> 257
ttctgagatt gaccagctc

<212> DNA<213> Homo sapiens<220> <223> Cdk2 ribozyme binding site
<400> 258
tgaccagctc ttccggatc

<212> DNA<213> Homo sapiens<220> <223> Cdk2 ribozyme binding site
<400> 259
accagctctt ccggatctt

<212> DNA<213> Homo sapiens<220> <223> Cdk2 ribozyme binding site
<400> 260
ccagctcttc cggatcttt

<212> DNA<213> Homo sapiens<220> <223> Cdk2 ribozyme binding site

<400> 261
cttccggatc tttcggact

<212> DNA<213> Homo sapiens<220> <223> Cdk2 ribozyme binding site
<400> 262
tccggatctt tcggactct

<212> DNA<213> Homo sapiens<220> <223> Cdk2 ribozyme binding site
<400> 263
ccggatcttt cggactctg

<212> DNA<213> Homo sapiens<220> <223> Cdk2 ribozyme binding site
<400> 264
cggatctttc ggactctgg

<212> DNA<213> Homo sapiens<220> <223> Cdk2 ribozyme binding site
<400> 265
tttcggactc tggggaccc

<212> DNA<213> Homo sapiens<220> <223> Cdk2 ribozyme binding site
<400> 266
gccaggagtt acttctatg

<212> DNA<213> Homo sapiens<220> <223> Cdk2 ribozyme binding site
<400> 267
ccaggagtta cttctatgc

<212> DNA<213> Homo sapiens<220> <223> Cdk2 ribozyme binding site
<400> 268
ggagttactt ctatgcctg

<212> DNA<213> Homo sapiens<220> <223> Cdk2 ribozyme binding site
<400> 269
gagttacttc tatgcctga

<212> DNA<213> Homo sapiens<220> <223> Cdk2 ribozyme binding site
<400> 270
gttacttcta tgcctgatt

<212> DNA<213> Homo sapiens<220> <223> Cdk2 ribozyme binding site
<400> 271
atgcctgatt acaagccaa

<212> DNA<213> Homo sapiens<220> <223> Cdk2 ribozyme binding site
<400> 272
tgcctgatta caagccaag

<212> DNA<213> Homo sapiens<220> <223> Cdk2 ribozyme binding site
<400> 273
aagccaagtt tccccaagt

<212> DNA<213> Homo sapiens<220> <223> Cdk2 ribozyme binding site
<400> 274
agccaagttt ccccaagtg

<212> DNA<213> Homo sapiens<220> <223> Cdk2 ribozyme binding site
<400> 275
gccaagtttc cccaagtgg

<212> DNA<213> Homo sapiens<220> <223> Cdk2 ribozyme binding site
<400> 276
cggcaagatt ttagtaaag

<212> DNA<213> Homo sapiens<220> <223> Cdk2 ribozyme binding site
<400> 277
ggcaagattt tagtaaagt

<212> DNA<213> Homo sapiens<220> <223> Cdk2 ribozyme binding site
<400> 278
gcaagatttt agtaaagtt

<212> DNA<213> Homo sapiens<220> <223> Cdk2 ribozyme binding site
<400> 279
caagatttta gtaaagttg

<212> DNA<213> Homo sapiens<220> <223> Cdk2 ribozyme binding site
<400> 280
gattttagta aagttgtac

<212> DNA<213> Homo sapiens<220> <223> Cdk2 ribozyme binding site
<400> 281
tagtaaagtt gtacctccc

<212> DNA<213> Homo sapiens<220> <223> Cdk2 ribozyme binding site
<400> 282
taaagttgta cctcccctg

<212> DNA<213> Homo sapiens<220> <223> Cdk2 ribozyme binding site
<400> 283
gttgtacctc ccctggatg

<212> DNA<213> Homo sapiens<220> <223> Cdk2 ribozyme binding site
<400> 284
gacggagctt gttatcgca

<212> DNA<213> Homo sapiens<220> <223> Cdk2 ribozyme binding site
<400> 285
ggagcttgtt atcgcaaat

<212> DNA<213> Homo sapiens<220> <223> Cdk2 ribozyme binding site
<400> 286
gagcttgtta tcgcaaatg

<212> DNA<213> Homo sapiens<220> <223> Cdk2 ribozyme binding site
<400> 287
gcttgttatc gcaaatgct

<212> DNA<213> Homo sapiens<220> <223> Cdk2 ribozyme binding site
<400> 288
tgctgcacta cgaccctaa

<212> DNA<213> Homo sapiens<220> <223> Cdk2 ribozyme binding site
<400> 289
tacgacccta acaagcgga

<212> DNA<213> Homo sapiens<220> <223> Cdk2 ribozyme binding site
<400> 290
caagcggatt tcggccaag

<212> DNA<213> Homo sapiens<220> <223> Cdk2 ribozyme binding site
<400> 291
aagcggattt cggccaagg

<212> DNA<213> Homo sapiens<220> <223> Cdk2 ribozyme binding site
<400> 292
agcggatttc ggccaaggc

<212> DNA<213> Homo sapiens<220> <223> Cdk2 ribozyme binding site
<400> 293
gccctggctc accctttct

<212> DNA<213> Homo sapiens<220> <223> Cdk2 ribozyme binding site
<400> 294
gctcaccctt tcttccagg

<212> DNA<213> Homo sapiens<220> <223> Cdk2 ribozyme binding site
<400> 295
ctcacccttt cttccagga

<212> DNA<213> Homo sapiens<220> <223> Cdk2 ribozyme binding site
<400> 296
tcaccctttc ttccaggat

<212> DNA<213> Homo sapiens<220> <223> Cdk2 ribozyme binding site
<400> 297
accctttctt ccaggatgt

<212> DNA<213> Homo sapiens<220> <223> Cdk2 ribozyme binding site
<400> 298
ccctttcttc caggatgtg

<212> DNA<213> Homo sapiens<220> <223> Cdk2 ribozyme binding site
<400> 299
caagccagta ccccatctt

<212> DNA<213> Homo sapiens<220> <223> Cdk2 ribozyme binding site
<400> 300
gtaccccatc ttcgactct

<212> DNA<213> Homo sapiens<220> <223> Cdk2 ribozyme binding site
<400> 301
accccatctt cgactctga

<212> DNA<213> Homo sapiens<220> <223> Cdk3 ribozyme binding site
<400> 302

tggatatgtt ccagaaggt

<212> DNA<213> Homo sapiens<220> <223> Cdk3 ribozyme binding site
<400> 303
ggatatgttc cagaaggta

<212> DNA<213> Homo sapiens<220> <223> Cdk3 ribozyme binding site
<400> 304
ccagaaggta gagaagatc

<212> DNA<213> Homo sapiens<220> <223> Cdk3 ribozyme binding site
<400> 305
agagaagatc ggagagggc

<212> DNA<213> Homo sapiens<220> <223> Cdk3 ribozyme binding site
<400> 306
agggcaccta tggggtggt

<212> DNA<213> Homo sapiens<220> <223> Cdk3 ribozyme binding site
<400> 307
gggtggtgta caaggccaa

<212> DNA<213> Homo sapiens<220> <223> Cdk3 ribozyme binding site
<400> 308
gaagaagatc agactggat

<212> DNA<213> Homo sapiens<220> <223> Cdk3 ribozyme binding site
<400> 309
agactggatt tggagatgg

<212> DNA<213> Homo sapiens<220> <223> Cdk3 ribozyme binding site
<400> 310
gactggattt ggagatgga

<212> DNA<213> Homo sapiens<220> <223> Cdk3 ribozyme binding site
<400> 311
ggagggggtc ccaagcact

<212> DNA<213> Homo sapiens<220> <223> Cdk3 ribozyme binding site
<400> 312
cactgccatc agggagatc

<212> DNA<213> Homo sapiens<220> <223> Cdk3 ribozyme binding site
<400> 313
cagggagatc tcgctgctc

<212> DNA<213> Homo sapiens<220> <223> Cdk3 ribozyme binding site
<400> 314
gggagatctc gctgctcaa

<212> DNA<213> Homo sapiens<220> <223> Cdk3 ribozyme binding site
<400> 315
ctcgctgctc aaggaactg

<212> DNA<213> Homo sapiens<220> <223> Cdk3 ribozyme binding site

<400> 316
ccccaacatc gtccgactg

<212> DNA<213> Homo sapiens<220> <223> Cdk3 ribozyme binding site
<400> 317
caacatcgtc cgactgctg

<212> DNA<213> Homo sapiens<220> <223> Cdk3 ribozyme binding site
<400> 318
gaggaagctc tatctggtg

<212> DNA<213> Homo sapiens<220> <223> Cdk3 ribozyme binding site
<400> 319
ggaagctcta tctggtgtt

<212> DNA<213> Homo sapiens<220> <223> Cdk3 ribozyme binding site
<400> 320
aagctctatc tggtgtttg

<212> DNA<213> Homo sapiens<220> <223> Cdk3 ribozyme binding site
<400> 321
atctggtgtt tgagttcct

<212> DNA<213> Homo sapiens<220> <223> Cdk3 ribozyme binding site
<400> 322
tctggtgttt gagttcctc

<212> DNA<213> Homo sapiens<220> <223> Cdk3 ribozyme binding site
<400> 323
tgtttgagtt cctcagcca

<212> DNA<213> Homo sapiens<220> <223> Cdk3 ribozyme binding site
<400> 324
gtttgagttc ctcagccag

<212> DNA<213> Homo sapiens<220> <223> Cdk3 ribozyme binding site
<400> 325
tgagttcctc agccaggac

<212> DNA<213> Homo sapiens<220> <223> Cdk3 ribozyme binding site
<400> 326
tgaagaagta catggactc

<212> DNA<213> Homo sapiens<220> <223> Cdk3 ribozyme binding site
<400> 327
acatggactc caccccagg

<212> DNA<213> Homo sapiens<220> <223> Cdk3 ribozyme binding site
<400> 328
ccccaggctc agagctccc

<212> DNA<213> Homo sapiens<220> <223> Cdk3 ribozyme binding site
<400> 329
ctcagagctc cccctgcac

<212> DNA<213> Homo sapiens<220> <223> Cdk3 ribozyme binding site
<400> 330
cctgcacctc atcaagagc

<212> DNA<213> Homo sapiens<220> <223> Cdk3 ribozyme binding site
<400> 331
gcacctcatc aagagctac

<212> DNA<213> Homo sapiens<220> <223> Cdk3 ribozyme binding site
<400> 332
tcaagagcta cctcttcca

<212> DNA<213> Homo sapiens<220> <223> Cdk3 ribozyme binding site
<400> 333
gagctacctc ttccagctg

<212> DNA<213> Homo sapiens<220> <223> Cdk3 ribozyme binding site
<400> 334
gctacctctt ccagctgct

<212> DNA<213> Homo sapiens<220> <223> Cdk3 ribozyme binding site
<400> 335
ctacctcttc cagctgctg

<212> DNA<213> Homo sapiens<220> <223> Cdk3 ribozyme binding site
<400> 336
ggggtgagtt tctgccact

<212> DNA<213> Homo sapiens<220> <223> Cdk3 ribozyme binding site
<400> 337
gggtgagttt ctgccactc

<212> DNA<213> Homo sapiens<220> <223> Cdk3 ribozyme binding site
<400> 338
ggtgagtttc tgccactca

<212> DNA<213> Homo sapiens<220> <223> Cdk3 ribozyme binding site
<400> 339
tctgccactc acatcgggt

<212> DNA<213> Homo sapiens<220> <223> Cdk3 ribozyme binding site
<400> 340
cactcacatc gggtcatcc

<212> DNA<213> Homo sapiens<220> <223> Cdk3 ribozyme binding site
<400> 341
acatcgggtc atccaccga

<212> DNA<213> Homo sapiens<220> <223> Cdk3 ribozyme binding site
<400> 342
tcgggtcatc caccgagac

<212> DNA<213> Homo sapiens<220> <223> Cdk3 ribozyme binding site
<400> 343
gaacctgctc atcaatgag

<212> DNA<213> Homo sapiens<220> <223> Cdk3 ribozyme binding site
<400> 344
cctgctcatc aatgagttg

<212> DNA<213> Homo sapiens<220> <223> Cdk3 ribozyme binding site
<400> 345
tcaatgagtt gggtgccat

<212> DNA<213> Homo sapiens<220> <223> Cdk3 ribozyme binding site
<400> 346
gggtgccatc aagctggct

<212> DNA<213> Homo sapiens<220> <223> Cdk3 ribozyme binding site
<400> 347
tggctgactt cggcctggc

<212> DNA<213> Homo sapiens<220> <223> Cdk3 ribozyme binding site
<400> 348
ggctgacttc ggcctggct

<212> DNA<213> Homo sapiens<220> <223> Cdk3 ribozyme binding site
<400> 349
ggcctggctc gcgccttcg

<212> DNA<213> Homo sapiens<220> <223> Cdk3 ribozyme binding site
<400> 350
tcgcgccttc ggggtgccc

<212> DNA<213> Homo sapiens<220> <223> Cdk3 ribozyme binding site
<400> 351
tgcgcaccta cacccatga

<212> DNA<213> Homo sapiens<220> <223> Cdk3 ribozyme binding site
<400> 352
cactgtggta tcgcgcccc

<212> DNA<213> Homo sapiens<220> <223> Cdk3 ribozyme binding site
<400> 353
ctgtggtatc gcgcccccg

<212> DNA<213> Homo sapiens<220> <223> Cdk3 ribozyme binding site
<400> 354
ccccgagatt ctcttgggc

<212> DNA<213> Homo sapiens<220> <223> Cdk3 ribozyme binding site
<400> 355
cccgagattc tcttgggca

<212> DNA<213> Homo sapiens<220> <223> Cdk3 ribozyme binding site
<400> 356
cgagattctc ttgggcagc

<212> DNA<213> Homo sapiens<220> <223> Cdk3 ribozyme binding site
<400> 357

agattctctt gggcagcaa

<212> DNA<213> Homo sapiens<220> <223> Cdk3 ribozyme binding site
<400> 358
gcagcaagtt ctataccac

<212> DNA<213> Homo sapiens<220> <223> Cdk3 ribozyme binding site
<400> 359
cagcaagttc tataccaca

<212> DNA<213> Homo sapiens<220> <223> Cdk3 ribozyme binding site
<400> 360
gcaagttcta taccacagc

<212> DNA<213> Homo sapiens<220> <223> Cdk3 ribozyme binding site
<400> 361
aagttctata ccacagctg

<212> DNA<213> Homo sapiens<220> <223> Cdk3 ribozyme binding site
<400> 362
gctgtggata tctggagca

<212> DNA<213> Homo sapiens<220> <223> Cdk3 ribozyme binding site
<400> 363
tgtggatatc tggagcatt

<212> DNA<213> Homo sapiens<220> <223> Cdk3 ribozyme binding site
<400> 364
ctggagcatt ggttgcatc

<212> DNA<213> Homo sapiens<220> <223> Cdk3 ribozyme binding site
<400> 365
agcattggtt gcatctttg

<212> DNA<213> Homo sapiens<220> <223> Cdk3 ribozyme binding site
<400> 366
tggttgcatc tttgcagag

<212> DNA<213> Homo sapiens<220> <223> Cdk3 ribozyme binding site
<400> 367
gttgcatctt tgcagagat

<212> DNA<213> Homo sapiens<220> <223> Cdk3 ribozyme binding site
<400> 368
ttgcatcttt gcagagatg

<212> DNA<213> Homo sapiens<220> <223> Cdk3 ribozyme binding site
<400> 369
atggtgactc gaaaagccc

<212> DNA<213> Homo sapiens<220> <223> Cdk3 ribozyme binding site
<400> 370
aagccctgtt tcctggtga

<212> DNA<213> Homo sapiens<220> <223> Cdk3 ribozyme binding site

<400> 371
agccctgttt cctggtgac

<212> DNA<213> Homo sapiens<220> <223> Cdk3 ribozyme binding site
<400> 372
gccctgtttc ctggtgact

<212> DNA<213> Homo sapiens<220> <223> Cdk3 ribozyme binding site
<400> 373
ctggtgactc tgagattga

<212> DNA<213> Homo sapiens<220> <223> Cdk3 ribozyme binding site
<400> 374
ctctgagatt gaccagctc

<212> DNA<213> Homo sapiens<220> <223> Cdk3 ribozyme binding site
<400> 375
tgaccagctc tttcgtatc

<212> DNA<213> Homo sapiens<220> <223> Cdk3 ribozyme binding site
<400> 376
accagctctt tcgtatctt

<212> DNA<213> Homo sapiens<220> <223> Cdk3 ribozyme binding site
<400> 377
ccagctcttt cgtatcttt

<212> DNA<213> Homo sapiens<220> <223> Cdk3 ribozyme binding site
<400> 378
cagctctttc gtatctttc

<212> DNA<213> Homo sapiens<220> <223> Cdk3 ribozyme binding site
<400> 379
ctctttcgta tctttcgta

<212> DNA<213> Homo sapiens<220> <223> Cdk3 ribozyme binding site
<400> 380
ctttcgtatc tttcgtatg

<212> DNA<213> Homo sapiens<220> <223> Cdk3 ribozyme binding site
<400> 381
ttcgtatctt tcgtatgct

<212> DNA<213> Homo sapiens<220> <223> Cdk3 ribozyme binding site
<400> 382
tcgtatcttt cgtatgctg

<212> DNA<213> Homo sapiens<220> <223> Cdk3 ribozyme binding site
<400> 383
cgtatctttc gtatgctgg

<212> DNA<213> Homo sapiens<220> <223> Cdk3 ribozyme binding site
<400> 384
atctttcgta tgctgggga

<212> DNA<213> Homo sapiens<220> <223> Cdk3 ribozyme binding site
<400> 385
gcccggggtc acccagctg

<212> DNA<213> Homo sapiens<220> <223> Cdk3 ribozyme binding site
<400> 386
tgcctgacta taagggcag

<212> DNA<213> Homo sapiens<220> <223> Cdk3 ribozyme binding site
<400> 387
cctgactata agggcagct

<212> DNA<213> Homo sapiens<220> <223> Cdk3 ribozyme binding site
<400> 388
agggcagctt ccctaagtg

<212> DNA<213> Homo sapiens<220> <223> Cdk3 ribozyme binding site
<400> 389
gggcagcttc cctaagtgg

<212> DNA<213> Homo sapiens<220> <223> Cdk3 ribozyme binding site
<400> 390
agcttcccta agtggacca

<212> DNA<213> Homo sapiens<220> <223> Cdk3 ribozyme binding site
<400> 391
ggaagagatt gtgcccaat

<212> DNA<213> Homo sapiens<220> <223> Cdk3 ribozyme binding site
<400> 392
gtgcccaatc tggagccag

<212> DNA<213> Homo sapiens<220> <223> Cdk3 ribozyme binding site
<400> 393
ggacctgctc atgcaactc

<212> DNA<213> Homo sapiens<220> <223> Cdk3 ribozyme binding site
<400> 394
catgcaactc ctgcagtat

<212> DNA<213> Homo sapiens<220> <223> Cdk3 ribozyme binding site
<400> 395
tcctgcagta tgaccccag

<212> DNA<213> Homo sapiens<220> <223> Cdk3 ribozyme binding site
<400> 396
ccagcggatc acagccaag

<212> DNA<213> Homo sapiens<220> <223> Cdk3 ribozyme binding site
<400> 397
cccacccgta cttctcatc

<212> DNA<213> Homo sapiens<220> <223> Cdk3 ribozyme binding site
<400> 398
acccgtactt ctcatcccc

<212> DNA<213> Homo sapiens<220> <223> Cdk3 ribozyme binding site
<400> 399
cccgtacttc tcatcccct

<212> DNA<213> Homo sapiens<220> <223> Cdk3 ribozyme binding site
<400> 400
cgtacttctc atcccctga

<212> DNA<213> Homo sapiens<220> <223> Cdk3 ribozyme binding site
<400> 401
acttctcatc ccctgagcc

<212> DNA<213> Homo sapiens<220> <223> Cdk3 ribozyme binding site
<400> 402
ctgagccctc cccagctgc

<212> DNA<213> Homo sapiens<220> <223> Cdk3 ribozyme binding site
<400> 403
cccgccagta tgtgctgca

<212> DNA<213> Homo sapiens<220> <223> Cdk3 ribozyme binding site
<400> 404
tgcagcgatt ccgccattg

<212> DNA<213> Homo sapiens<220> <223> Cdk3 ribozyme binding site
<400> 405
gcagcgattc cgccattga

<212> DNA<213> Homo sapiens<220> <223> Cdk4 ribozyme binding site
<400> 406
tggctacctc tcgatatga

<212> DNA<213> Homo sapiens<220> <223> Cdk4 ribozyme binding site
<400> 407
gctacctctc gatatgagc

<212> DNA<213> Homo sapiens<220> <223> Cdk4 ribozyme binding site
<400> 408
cctctcgata tgagccagt

<212> DNA<213> Homo sapiens<220> <223> Cdk4 ribozyme binding site
<400> 409
ggctgaaatt ggtgtcggt

<212> DNA<213> Homo sapiens<220> <223> Cdk4 ribozyme binding site
<400> 410
aattggtgtc ggtgcctat

<212> DNA<213> Homo sapiens<220> <223> Cdk4 ribozyme binding site
<400> 411
tcggtgccta tgggacagt

<212> DNA<213> Homo sapiens<220> <223> Cdk4 ribozyme binding site
<400> 412

ggacagtgta caaggcccg

<212> DNA<213> Homo sapiens<220> <223> Cdk4 ribozyme binding site
<400> 413
gcccgtgatc cccacagtg

<212> DNA<213> Homo sapiens<220> <223> Cdk4 ribozyme binding site
<400> 414
gtggccactt tgtggccct

<212> DNA<213> Homo sapiens<220> <223> Cdk4 ribozyme binding site
<400> 415
tggccacttt gtggccctc

<212> DNA<213> Homo sapiens<220> <223> Cdk4 ribozyme binding site
<400> 416
tgtggccctc aagagtgtg

<212> DNA<213> Homo sapiens<220> <223> Cdk4 ribozyme binding site
<400> 417
tgtgagagtc cccaatgga

<212> DNA<213> Homo sapiens<220> <223> Cdk4 ribozyme binding site
<400> 418
aggaggcctt cccatcagc

<212> DNA<213> Homo sapiens<220> <223> Cdk4 ribozyme binding site
<400> 419
ggaggccttc ccatcagca

<212> DNA<213> Homo sapiens<220> <223> Cdk4 ribozyme binding site
<400> 420
ccttcccatc agcacagtt

<212> DNA<213> Homo sapiens<220> <223> Cdk4 ribozyme binding site
<400> 421
cagcacagtt cgtgaggtg

<212> DNA<213> Homo sapiens<220> <223> Cdk4 ribozyme binding site
<400> 422
gaggtggctt tactgaggc

<212> DNA<213> Homo sapiens<220> <223> Cdk4 ribozyme binding site
<400> 423
aggtggcttt actgaggcg

<212> DNA<213> Homo sapiens<220> <223> Cdk4 ribozyme binding site
<400> 424
ggtggcttta ctgaggcga

<212> DNA<213> Homo sapiens<220> <223> Cdk4 ribozyme binding site
<400> 425
ctggaggctt ttgagcatc

<212> DNA<213> Homo sapiens<220> <223> Cdk4 ribozyme binding site

<400> 426
tggaggcttt tgagcatcc

<212> DNA<213> Homo sapiens<220> <223> Cdk4 ribozyme binding site
<400> 427
ggaggctttt gagcatccc

<212> DNA<213> Homo sapiens<220> <223> Cdk4 ribozyme binding site
<400> 428
tttgagcatc ccaatgttg

<212> DNA<213> Homo sapiens<220> <223> Cdk4 ribozyme binding site
<400> 429
tcccaatgtt gtccggctg

<212> DNA<213> Homo sapiens<220> <223> Cdk4 ribozyme binding site
<400> 430
caatgttgtc cggctgatg

<212> DNA<213> Homo sapiens<220> <223> Cdk4 ribozyme binding site
<400> 431
gatggacgtc tgtgccaca

<212> DNA<213> Homo sapiens<220> <223> Cdk4 ribozyme binding site
<400> 432
gtgccacatc ccgaactga

<212> DNA<213> Homo sapiens<220> <223> Cdk4 ribozyme binding site
<400> 433
ccgggagatc aaggtaacc

<212> DNA<213> Homo sapiens<220> <223> Cdk4 ribozyme binding site
<400> 434
gatcaaggta accctggtg

<212> DNA<213> Homo sapiens<220> <223> Cdk4 ribozyme binding site
<400> 435
ccctggtgtt tgagcatgt

<212> DNA<213> Homo sapiens<220> <223> Cdk4 ribozyme binding site
<400> 436
cctggtgttt gagcatgta

<212> DNA<213> Homo sapiens<220> <223> Cdk4 ribozyme binding site
<400> 437
tgagcatgta gaccaggac

<212> DNA<213> Homo sapiens<220> <223> Cdk4 ribozyme binding site
<400> 438
ccaggaccta aggacatat

<212> DNA<213> Homo sapiens<220> <223> Cdk4 ribozyme binding site
<400> 439
taaggacata tctggacaa

<212> DNA<213> Homo sapiens<220> <223> Cdk4 ribozyme binding site
<400> 440
aggacatatc tggacaagg

<212> DNA<213> Homo sapiens<220> <223> Cdk4 ribozyme binding site
<400> 441
caccaggctt gccagccga

<212> DNA<213> Homo sapiens<220> <223> Cdk4 ribozyme binding site
<400> 442
cgaaacgatc aaggatctg

<212> DNA<213> Homo sapiens<220> <223> Cdk4 ribozyme binding site
<400> 443
atcaaggatc tgatgcgcc

<212> DNA<213> Homo sapiens<220> <223> Cdk4 ribozyme binding site
<400> 444
tgcgccagtt tctaagagg

<212> DNA<213> Homo sapiens<220> <223> Cdk4 ribozyme binding site
<400> 445
gcgccagttt ctaagaggc

<212> DNA<213> Homo sapiens<220> <223> Cdk4 ribozyme binding site
<400> 446
cgccagtttc taagaggcc

<212> DNA<213> Homo sapiens<220> <223> Cdk4 ribozyme binding site
<400> 447
ccagtttcta agaggccta

<212> DNA<213> Homo sapiens<220> <223> Cdk4 ribozyme binding site
<400> 448
aagaggccta gatttcctt

<212> DNA<213> Homo sapiens<220> <223> Cdk4 ribozyme binding site
<400> 449
aggcctagat ttccttcat

<212> DNA<213> Homo sapiens<220> <223> Cdk4 ribozyme binding site
<400> 450
gggcctagat ttccttcat

<212> DNA<213> Homo sapiens<220> <223> Cdk4 ribozyme binding site
<400> 451
ggcctagatt tccttcatg

<212> DNA<213> Homo sapiens<220> <223> Cdk4 ribozyme binding site
<400> 452
gcctagattt ccttcatgc

<212> DNA<213> Homo sapiens<220> <223> Cdk4 ribozyme binding site
<400> 453
cctagatttc cttcatgcc

<212> DNA<213> Homo sapiens<220> <223> Cdk4 ribozyme binding site
<400> 454
agatttcctt catgccaat

<212> DNA<213> Homo sapiens<220> <223> Cdk4 ribozyme binding site
<400> 455
gatttccttc atgccaatt

<212> DNA<213> Homo sapiens<220> <223> Cdk4 ribozyme binding site
<400> 456
catgccaatt gcatcgttc

<212> DNA<213> Homo sapiens<220> <223> Cdk4 ribozyme binding site
<400> 457
caattgcatc gttcaccga

<212> DNA<213> Homo sapiens<220> <223> Cdk4 ribozyme binding site
<400> 458
ttgcatcgtt caccgagat

<212> DNA<213> Homo sapiens<220> <223> Cdk4 ribozyme binding site
<400> 459
tgcatcgttc accgagatc

<212> DNA<213> Homo sapiens<220> <223> Cdk4 ribozyme binding site
<400> 460
caccgagatc tgaagccag

<212> DNA<213> Homo sapiens<220> <223> Cdk4 ribozyme binding site
<400> 461
agagaacatt ctggtgaca

<212> DNA<213> Homo sapiens<220> <223> Cdk4 ribozyme binding site
<400> 462
gagaacattc tggtgacaa

<212> DNA<213> Homo sapiens<220> <223> Cdk4 ribozyme binding site
<400> 463
tggaacagtc aagctggct

<212> DNA<213> Homo sapiens<220> <223> Cdk4 ribozyme binding site
<400> 464
tggctgactt tggcctggc

<212> DNA<213> Homo sapiens<220> <223> Cdk4 ribozyme binding site
<400> 465
ggctgacttt ggcctggcc

<212> DNA<213> Homo sapiens<220> <223> Cdk4 ribozyme binding site
<400> 466
ggccagaatc tacagctac

<212> DNA<213> Homo sapiens<220> <223> Cdk4 ribozyme binding site
<400> 467

ccagaatcta cagctacca

<212> DNA<213> Homo sapiens<220> <223> Cdk4 ribozyme binding site
<400> 468
tctacagcta ccagatggc

<212> DNA<213> Homo sapiens<220> <223> Cdk4 ribozyme binding site
<400> 469
atggcactta cacccgtgg

<212> DNA<213> Homo sapiens<220> <223> Cdk4 ribozyme binding site
<400> 470
acccgtggtt gttacactc

<212> DNA<213> Homo sapiens<220> <223> Cdk4 ribozyme binding site
<400> 471
cgtggttgtt acactctgg

<212> DNA<213> Homo sapiens<220> <223> Cdk4 ribozyme binding site
<400> 472
gtggttgtta cactctggt

<212> DNA<213> Homo sapiens<220> <223> Cdk4 ribozyme binding site
<400> 473
tgttacactc tggtaccga

<212> DNA<213> Homo sapiens<220> <223> Cdk4 ribozyme binding site
<400> 474
cactctggta ccgagctcc

<212> DNA<213> Homo sapiens<220> <223> Cdk4 ribozyme binding site
<400> 475
taccgagctc ccgaagttc

<212> DNA<213> Homo sapiens<220> <223> Cdk4 ribozyme binding site
<400> 476
tcccgaagtt cttctgcag

<212> DNA<213> Homo sapiens<220> <223> Cdk4 ribozyme binding site
<400> 477
cccgaagttc ttctgcagt

<212> DNA<213> Homo sapiens<220> <223> Cdk4 ribozyme binding site
<400> 478
cgaagttctt ctgcagtcc

<212> DNA<213> Homo sapiens<220> <223> Cdk4 ribozyme binding site
<400> 479
gaagttcttc tgcagtcca

<212> DNA<213> Homo sapiens<220> <223> Cdk4 ribozyme binding site
<400> 480
agtccacata tgcaacacc

<212> DNA<213> Homo sapiens<220> <223> Cdk4 ribozyme binding site

<400> 481
gtggagtgtt ggctgtatc

<212> DNA<213> Homo sapiens<220> <223> Cdk4 ribozyme binding site
<400> 482
gttggctgta tctttgcag

<212> DNA<213> Homo sapiens<220> <223> Cdk4 ribozyme binding site
<400> 483
tggctgtatc tttgcagag

<212> DNA<213> Homo sapiens<220> <223> Cdk4 ribozyme binding site
<400> 484
gctgtatctt tgcagagat

<212> DNA<213> Homo sapiens<220> <223> Cdk4 ribozyme binding site
<400> 485
ctgtatcttt gcagagatg

<212> DNA<213> Homo sapiens<220> <223> Cdk4 ribozyme binding site
<400> 486
cagagatgtt tcgtcgaaa

<212> DNA<213> Homo sapiens<220> <223> Cdk4 ribozyme binding site
<400> 487
agagatgttt cgtcgaaag

<212> DNA<213> Homo sapiens<220> <223> Cdk4 ribozyme binding site
<400> 488
gagatgtttc gtcgaaagc

<212> DNA<213> Homo sapiens<220> <223> Cdk4 ribozyme binding site
<400> 489
atgtttcgtc gaaagcctc

<212> DNA<213> Homo sapiens<220> <223> Cdk4 ribozyme binding site
<400> 490
cgaaagcctc tcttctgtg

<212> DNA<213> Homo sapiens<220> <223> Cdk4 ribozyme binding site
<400> 491
aaagcctctc ttctgtgga

<212> DNA<213> Homo sapiens<220> <223> Cdk4 ribozyme binding site
<400> 492
agcctctctt ctgtggaaa

<212> DNA<213> Homo sapiens<220> <223> Cdk4 ribozyme binding site
<400> 493
gcctctcttc tgtggaaac

<212> DNA<213> Homo sapiens<220> <223> Cdk4 ribozyme binding site
<400> 494
gtggaaactc tgaagccga

<212> DNA<213> Homo sapiens<220> <223> Cdk4 ribozyme binding site
<400> 495
ccgaccagtt gggcaaaat

<212> DNA<213> Homo sapiens<220> <223> Cdk4 ribozyme binding site
<400> 496
gggcaaaatc tttgacctg

<212> DNA<213> Homo sapiens<220> <223> Cdk4 ribozyme binding site
<400> 497
gcaaaatctt tgacctgat

<212> DNA<213> Homo sapiens<220> <223> Cdk4 ribozyme binding site
<400> 498
caaaatcttt gacctgatt

<212> DNA<213> Homo sapiens<220> <223> Cdk4 ribozyme binding site
<400> 499
tgacctgatt gggctgcct

<212> DNA<213> Homo sapiens<220> <223> Cdk4 ribozyme binding site
<400> 500
gggctgcctc cagaggatg

<212> DNA<213> Homo sapiens<220> <223> Cdk4 ribozyme binding site
<400> 501
gactggcctc gagatgtat

<212> DNA<213> Homo sapiens<220> <223> Cdk4 ribozyme binding site
<400> 502
tcgagatgta tccctgccc

<212> DNA<213> Homo sapiens<220> <223> Cdk4 ribozyme binding site
<400> 503
gagatgtatc cctgccccg

<212> DNA<213> Homo sapiens<220> <223> Cdk4 ribozyme binding site
<400> 504
gtggagcctt tcccccccag

<212> DNA<213> Homo sapiens<220> <223> Cdk4 ribozyme binding site
<400> 505
tggagccttt cccccccaga

<212> DNA<213> Homo sapiens<220> <223> Cdk4 ribozyme binding site
<400> 506
ggagcctttc ccccccagag

<212> DNA<213> Homo sapiens<220> <223> Cdk4 ribozyme binding site
<400> 507
cagtgcagtc ggtggtacc

<212> DNA<213> Homo sapiens<220> <223> Cdk4 ribozyme binding site
<400> 508
gtcggtggta cctgagatg

<212> DNA<213> Homo sapiens<220> <223> Cdk4 ribozyme binding site
<400> 509
tggaggagtc gggagcaca

<212> DNA<213> Homo sapiens<220> <223> Cdk4 ribozyme binding site
<400> 510
atgctgactt ttaacccac

<212> DNA<213> Homo sapiens<220> <223> Cdk4 ribozyme binding site
<400> 511
tgctgacttt taacccaca

<212> DNA<213> Homo sapiens<220> <223> Cdk4 ribozyme binding site
<400> 512
gctgactttt aacccacac

<212> DNA<213> Homo sapiens<220> <223> Cdk4 ribozyme binding site
<400> 513
ctgactttta acccacaca

<212> DNA<213> Homo sapiens<220> <223> Cdk4 ribozyme binding site
<400> 514
caagcgaatc tctgccttt

<212> DNA<213> Homo sapiens<220> <223> Cdk4 ribozyme binding site
<400> 515
agcgaatctc tgcctttcg

<212> DNA<213> Homo sapiens<220> <223> Cdk4 ribozyme binding site
<400> 516
tctctgcctt tcgagctct

<212> DNA<213> Homo sapiens<220> <223> Cdk4 ribozyme binding site
<400> 517
ctctgccttt cgagctctg

<212> DNA<213> Homo sapiens<220> <223> Cdk4 ribozyme binding site
<400> 518
tctgcctttc gagctctgc

<212> DNA<213> Homo sapiens<220> <223> Cdk4 ribozyme binding site
<400> 519
tttcgagctc tgcagcact

<212> DNA<213> Homo sapiens<220> <223> Cdk4 ribozyme binding site
<400> 520
tgcagcactc ttatctaca

<212> DNA<213> Homo sapiens<220> <223> Cdk4 ribozyme binding site
<400> 521
cagcactctt atctacata

<212> DNA<213> Homo sapiens<220> <223> Cdk4 ribozyme binding site
<400> 522

agcactctta tctacataa

<212> DNA<213> Homo sapiens<220> <223> Cdk4 ribozyme binding site
<400> 523
cactcttatc tacataagg

<212> DNA<213> Homo sapiens<220> <223> Cdk4 ribozyme binding site
<400> 524
ctcttatcta cataaggat

<212> DNA<213> Homo sapiens<220> <223> Cdk4 ribozyme binding site
<400> 525
tatctacata aggatgaag

<212> DNA<213> Homo sapiens<220> <223> Cdk4 ribozyme binding site
<400> 526
gatgaaggta atccggagt

<212> DNA<213> Homo sapiens<220> <223> Cdk6 ribozyme binding site
<400> 527
accagcagta cgaatgcgt

<212> DNA<213> Homo sapiens<220> <223> Cdk6 ribozyme binding site
<400> 528
ggcggagatc ggggagggc

<212> DNA<213> Homo sapiens<220> <223> Cdk6 ribozyme binding site
<400> 529
agggcgccta tgggaaggt

<212> DNA<213> Homo sapiens<220> <223> Cdk6 ribozyme binding site
<400> 530
ggaaggtgtt caaggcccg

<212> DNA<213> Homo sapiens<220> <223> Cdk6 ribozyme binding site
<400> 531
gaaggtgttc aaggcccgc

<212> DNA<213> Homo sapiens<220> <223> Cdk6 ribozyme binding site
<400> 532
cccgcgactt gaagaacgg

<212> DNA<213> Homo sapiens<220> <223> Cdk6 ribozyme binding site
<400> 533
ggaggccgtt tcgtggcgt

<212> DNA<213> Homo sapiens<220> <223> Cdk6 ribozyme binding site
<400> 534
gaggccgttt cgtggcgtt

<212> DNA<213> Homo sapiens<220> <223> Cdk6 ribozyme binding site
<400> 535
aggccgtttc gtggcgttg

<212> DNA<213> Homo sapiens<220> <223> Cdk6 ribozyme binding site

<400> 536
tcgtggcgtt gaagcgcgt

<212> DNA<213> Homo sapiens<220> <223> Cdk6 ribozyme binding site
<400> 537
catgccgctc tccaccatc

<212> DNA<213> Homo sapiens<220> <223> Cdk6 ribozyme binding site
<400> 538
tgccgctctc caccatccg

<212> DNA<213> Homo sapiens<220> <223> Cdk6 ribozyme binding site
<400> 539
ctccaccatc cgcgaggtg

<212> DNA<213> Homo sapiens<220> <223> Cdk6 ribozyme binding site
<400> 540
tggagacctt cgagcaccc

<212> DNA<213> Homo sapiens<220> <223> Cdk6 ribozyme binding site
<400> 541
ggagaccttc gagcacccc

<212> DNA<213> Homo sapiens<220> <223> Cdk6 ribozyme binding site
<400> 542
caacgtggtc aggttgttt

<212> DNA<213> Homo sapiens<220> <223> Cdk6 ribozyme binding site
<400> 543
tggtcaggtt gtttgatgt

<212> DNA<213> Homo sapiens<220> <223> Cdk6 ribozyme binding site
<400> 544
tcaggttgtt tgatgtgtg

<212> DNA<213> Homo sapiens<220> <223> Cdk6 ribozyme binding site
<400> 545
caggttgttt gatgtgtgc

<212> DNA<213> Homo sapiens<220> <223> Cdk6 ribozyme binding site
<400> 546
gcacagtgtc acgaacaga

<212> DNA<213> Homo sapiens<220> <223> Cdk6 ribozyme binding site
<400> 547
aaactaactt tagtgtttg

<212> DNA<213> Homo sapiens<220> <223> Cdk6 ribozyme binding site
<400> 548
aactaacttt agtgtttga

<212> DNA<213> Homo sapiens<220> <223> Cdk6 ribozyme binding site
<400> 549
actaacttta gtgtttgaa

<212> DNA<213> Homo sapiens<220> <223> Cdk6 ribozyme binding site
<400> 550
ctttagtgtt tgaacatgt

<212> DNA<213> Homo sapiens<220> <223> Cdk6 ribozyme binding site
<400> 551
tttagtgttt gaacatgtc

<212> DNA<213> Homo sapiens<220> <223> Cdk6 ribozyme binding site
<400> 552
tgaacatgtc gatcaagac

<212> DNA<213> Homo sapiens<220> <223> Cdk6 ribozyme binding site
<400> 553
catgtcgatc aagacttga

<212> DNA<213> Homo sapiens<220> <223> Cdk6 ribozyme binding site
<400> 554
atcaagactt gaccactta

<212> DNA<213> Homo sapiens<220> <223> Cdk6 ribozyme binding site
<400> 555
ttgaccactt acttggata

<212> DNA<213> Homo sapiens<220> <223> Cdk6 ribozyme binding site
<400> 556
tgaccactta cttggataa

<212> DNA<213> Homo sapiens<220> <223> Cdk6 ribozyme binding site
<400> 557
ccacttactt ggataaagt

<212> DNA<213> Homo sapiens<220> <223> Cdk6 ribozyme binding site
<400> 558
tacttggata aagttccag

<212> DNA<213> Homo sapiens<220> <223> Cdk6 ribozyme binding site
<400> 559
ggataaagtt ccagagcct

<212> DNA<213> Homo sapiens<220> <223> Cdk6 ribozyme binding site
<400> 560
gataaagttc cagagcctg

<212> DNA<213> Homo sapiens<220> <223> Cdk6 ribozyme binding site
<400> 561
tgaaaccata aaggatatg

<212> DNA<213> Homo sapiens<220> <223> Cdk6 ribozyme binding site
<400> 562
ataaaggata tgatgtttc

<212> DNA<213> Homo sapiens<220> <223> Cdk6 ribozyme binding site
<400> 563
atatgatgtt tcagcttct

<212> DNA<213> Homo sapiens<220> <223> Cdk6 ribozyme binding site
<400> 564
tatgatgttt cagcttctc

<212> DNA<213> Homo sapiens<220> <223> Cdk6 ribozyme binding site
<400> 565
atgatgtttc agcttctcc

<212> DNA<213> Homo sapiens<220> <223> Cdk6 ribozyme binding site
<400> 566
gtttcagctt ctccgaggt

<212> DNA<213> Homo sapiens<220> <223> Cdk6 ribozyme binding site
<400> 567
tttcagcttc tccgaggtc

<212> DNA<213> Homo sapiens<220> <223> Cdk6 ribozyme binding site
<400> 568
tcagcttctc cgaggtctg

<212> DNA<213> Homo sapiens<220> <223> Cdk6 ribozyme binding site
<400> 569
ctccgaggtc tggactttc

<212> DNA<213> Homo sapiens<220> <223> Cdk6 ribozyme binding site
<400> 570
gtctggactt tcttcattc

<212> DNA<213> Homo sapiens<220> <223> Cdk6 ribozyme binding site
<400> 571
tctggacttt cttcattca

<212> DNA<213> Homo sapiens<220> <223> Cdk6 ribozyme binding site
<400> 572
ctggactttc ttcattcac

<212> DNA<213> Homo sapiens<220> <223> Cdk6 ribozyme binding site
<400> 573
ggactttctt cattcacac

<212> DNA<213> Homo sapiens<220> <223> Cdk6 ribozyme binding site
<400> 574
gactttcttc attcacacc

<212> DNA<213> Homo sapiens<220> <223> Cdk6 ribozyme binding site
<400> 575
tttcttcatt cacaccgag

<212> DNA<213> Homo sapiens<220> <223> Cdk6 ribozyme binding site
<400> 576
ttcttcattc acaccgagt

<212> DNA<213> Homo sapiens<220> <223> Cdk6 ribozyme binding site
<400> 577

acaccgagta gtgcatcgc

<212> DNA<213> Homo sapiens<220> <223> Cdk6 ribozyme binding site
<400> 578
gtagtgcatc gcgatctaa

<212> DNA<213> Homo sapiens<220> <223> Cdk6 ribozyme binding site
<400> 579
catcgcgatc taaaaccac

<212> DNA<213> Homo sapiens<220> <223> Cdk6 ribozyme binding site
<400> 580
tcgcgatcta aaaccacag

<212> DNA<213> Homo sapiens<220> <223> Cdk6 ribozyme binding site
<400> 581
acagaacatt ctggtgacc

<212> DNA<213> Homo sapiens<220> <223> Cdk6 ribozyme binding site
<400> 582
cggacaaata aaactcgct

<212> DNA<213> Homo sapiens<220> <223> Cdk6 ribozyme binding site
<400> 583
aataaaactc gctgacttc

<212> DNA<213> Homo sapiens<220> <223> Cdk6 ribozyme binding site
<400> 584
tcgctgactt cggccttgc

<212> DNA<213> Homo sapiens<220> <223> Cdk6 ribozyme binding site
<400> 585
cgctgacttc ggccttgcc

<212> DNA<213> Homo sapiens<220> <223> Cdk6 ribozyme binding site
<400> 586
cttcggcctt gcccgcatc

<212> DNA<213> Homo sapiens<220> <223> Cdk6 ribozyme binding site
<400> 587
tgcccgcatc tatagtttc

<212> DNA<213> Homo sapiens<220> <223> Cdk6 ribozyme binding site
<400> 588
cccgcatcta tagtttcca

<212> DNA<213> Homo sapiens<220> <223> Cdk6 ribozyme binding site
<400> 589
cgcatctata gtttccaga

<212> DNA<213> Homo sapiens<220> <223> Cdk6 ribozyme binding site
<400> 590
atctatagtt tccagatgg

<212> DNA<213> Homo sapiens<220> <223> Cdk6 ribozyme binding site

<400> 591
tctatagttt ccagatggc

<212> DNA<213> Homo sapiens<220> <223> Cdk6 ribozyme binding site
<400> 592
ctatagtttc cagatggct

<212> DNA<213> Homo sapiens<220> <223> Cdk6 ribozyme binding site
<400> 593
cagatggctc taacctcag

<212> DNA<213> Homo sapiens<220> <223> Cdk6 ribozyme binding site
<400> 594
gatggctcta acctcagtg

<212> DNA<213> Homo sapiens<220> <223> Cdk6 ribozyme binding site
<400> 595
ctctaacctc agtggtcgt

<212> DNA<213> Homo sapiens<220> <223> Cdk6 ribozyme binding site
<400> 596
ctcagtggtc gtcacgctg

<212> DNA<213> Homo sapiens<220> <223> Cdk6 ribozyme binding site
<400> 597
agtggtcgtc acgctgtgg

<212> DNA<213> Homo sapiens<220> <223> Cdk6 ribozyme binding site
<400> 598
cgctgtggta cagagcacc

<212> DNA<213> Homo sapiens<220> <223> Cdk6 ribozyme binding site
<400> 599
acccgaagtc ttgctccag

<212> DNA<213> Homo sapiens<220> <223> Cdk6 ribozyme binding site
<400> 600
ccgaagtctt gctccagtc

<212> DNA<213> Homo sapiens<220> <223> Cdk6 ribozyme binding site
<400> 601
agtcttgctc cagtccagc

<212> DNA<213> Homo sapiens<220> <223> Cdk6 ribozyme binding site
<400> 602
tgctccagtc cagctacgc

<212> DNA<213> Homo sapiens<220> <223> Cdk6 ribozyme binding site
<400> 603
agtccagcta cgccacccc

<212> DNA<213> Homo sapiens<220> <223> Cdk6 ribozyme binding site
<400> 604
cccgtggatc tctggagtg

<212> DNA<213> Homo sapiens<220> <223> Cdk6 ribozyme binding site
<400> 605
cgtggatctc tggagtgtt

<212> DNA<213> Homo sapiens<220> <223> Cdk6 ribozyme binding site
<400> 606
ctggagtgtt ggctgcata

<212> DNA<213> Homo sapiens<220> <223> Cdk6 ribozyme binding site
<400> 607
tggctgcata tttgcagaa

<212> DNA<213> Homo sapiens<220> <223> Cdk6 ribozyme binding site
<400> 608
gctgcatatt tgcagaaat

<212> DNA<213> Homo sapiens<220> <223> Cdk6 ribozyme binding site
<400> 609
ctgcatattt gcagaaatg

<212> DNA<213> Homo sapiens<220> <223> Cdk6 ribozyme binding site
<400> 610
cagaaatgtt tcgtagaaa

<212> DNA<213> Homo sapiens<220> <223> Cdk6 ribozyme binding site
<400> 611
agaaatgttt cgtagaaag

<212> DNA<213> Homo sapiens<220> <223> Cdk6 ribozyme binding site
<400> 612
gaaatgtttc gtagaaagc

<212> DNA<213> Homo sapiens<220> <223> Cdk6 ribozyme binding site
<400> 613
atgtttcgta gaaagcctc

<212> DNA<213> Homo sapiens<220> <223> Cdk6 ribozyme binding site
<400> 614
agaaagcctc tttttcgtg

<212> DNA<213> Homo sapiens<220> <223> Cdk6 ribozyme binding site
<400> 615
aaagcctctt tttcgtgga

<212> DNA<213> Homo sapiens<220> <223> Cdk6 ribozyme binding site
<400> 616
aagcctcttt ttcgtggaa

<212> DNA<213> Homo sapiens<220> <223> Cdk6 ribozyme binding site
<400> 617
agcctctttt tcgtggaag

<212> DNA<213> Homo sapiens<220> <223> Cdk6 ribozyme binding site
<400> 618
gcctcttttt cgtggaagt

<212> DNA<213> Homo sapiens<220> <223> Cdk6 ribozyme binding site
<400> 619
cctcttttc gtggaagtt

<212> DNA<213> Homo sapiens<220> <223> Cdk6 ribozyme binding site
<400> 620
cgtggaagtt cagatgttg

<212> DNA<213> Homo sapiens<220> <223> Cdk6 ribozyme binding site
<400> 621
gtggaagttc agatgttga

<212> DNA<213> Homo sapiens<220> <223> Cdk6 ribozyme binding site
<400> 622
ttcagatgtt gatcaacta

<212> DNA<213> Homo sapiens<220> <223> Cdk6 ribozyme binding site
<400> 623
gatgttgatc aactaggaa

<212> DNA<213> Homo sapiens<220> <223> Cdk6 ribozyme binding site
<400> 624
tgatcaacta ggaaaaatc

<212> DNA<213> Homo sapiens<220> <223> Cdk6 ribozyme binding site
<400> 625
aggaaaaatc ttggacgtg

<212> DNA<213> Homo sapiens<220> <223> Cdk6 ribozyme binding site
<400> 626
gaaaaatctt ggacgtgat

<212> DNA<213> Homo sapiens<220> <223> Cdk6 ribozyme binding site
<400> 627
ggacgtgatt ggactccca

<212> DNA<213> Homo sapiens<220> <223> Cdk6 ribozyme binding site
<400> 628
gattggactc ccaggagaa

<212> DNA<213> Homo sapiens<220> <223> Cdk6 ribozyme binding site
<400> 629
gactggccta gagatgttg

<212> DNA<213> Homo sapiens<220> <223> Cdk6 ribozyme binding site
<400> 630
tagagatgtt gcccttccc

<212> DNA<213> Homo sapiens<220> <223> Cdk6 ribozyme binding site
<400> 631
tgttgccctt cccaggcag

<212> DNA<213> Homo sapiens<220> <223> Cdk6 ribozyme binding site
<400> 632

gttgcccttc ccaggcagg

<212> DNA<213> Homo sapiens<220> <223> Cdk6 ribozyme binding site
<400> 633
aggcaggctt ttcattcaa

<212> DNA<213> Homo sapiens<220> <223> Cdk6 ribozyme binding site
<400> 634
ggcaggcttt tcattcaaa

<212> DNA<213> Homo sapiens<220> <223> Cdk6 ribozyme binding site
<400> 635
gcaggctttt cattcaaaa

<212> DNA<213> Homo sapiens<220> <223> Cdk6 ribozyme binding site
<400> 636
caggcttttc attcaaaat

<212> DNA<213> Homo sapiens<220> <223> Cdk6 ribozyme binding site
<400> 637
gcttttcatt caaaatctg

<212> DNA<213> Homo sapiens<220> <223> Cdk6 ribozyme binding site
<400> 638
cttttcattc aaaatctgc

<212> DNA<213> Homo sapiens<220> <223> Cdk6 ribozyme binding site
<400> 639
attcaaaatc tgcccaacc

<212> DNA<213> Homo sapiens<220> <223> Cdk6 ribozyme binding site
<400> 640
ccaaccaatt gagaagttt

<212> DNA<213> Homo sapiens<220> <223> Cdk6 ribozyme binding site
<400> 641
ttgagaagtt tgtaacaga

<212> DNA<213> Homo sapiens<220> <223> Cdk6 ribozyme binding site
<400> 642
tgagaagttt gtaacagat

<212> DNA<213> Homo sapiens<220> <223> Cdk6 ribozyme binding site
<400> 643
gaagtttgta acagatatc

<212> DNA<213> Homo sapiens<220> <223> Cdk6 ribozyme binding site
<400> 644
gtaacagata tcgatgaac

<212> DNA<213> Homo sapiens<220> <223> Cdk6 ribozyme binding site
<400> 645
aacagatatc gatgaacta

<212> DNA<213> Homo sapiens<220> <223> Cdk6 ribozyme binding site

<400> 646
cgatgaacta ggcaaagac

<212> DNA<213> Homo sapiens<220> <223> Cdk6 ribozyme binding site
<400> 647
caaagaccta cttctgaag

<212> DNA<213> Homo sapiens<220> <223> Cdk6 ribozyme binding site
<400> 648
agacctactt ctgaagtgt

<212> DNA<213> Homo sapiens<220> <223> Cdk6 ribozyme binding site
<400> 649
gacctacttc tgaagtgtt

<212> DNA<213> Homo sapiens<220> <223> Cdk6 ribozyme binding site
<400> 650
ctgaagtgtt tgacattta

<212> DNA<213> Homo sapiens<220> <223> Cdk6 ribozyme binding site
<400> 651
tgaagtgttt gacatttaa

<212> DNA<213> Homo sapiens<220> <223> Cdk6 ribozyme binding site
<400> 652
gtttgacatt taacccagc

<212> DNA<213> Homo sapiens<220> <223> Cdk6 ribozyme binding site
<400> 653
tttgacattt aacccagcc

<212> DNA<213> Homo sapiens<220> <223> Cdk6 ribozyme binding site
<400> 654
ttgacattta acccagcca

<212> DNA<213> Homo sapiens<220> <223> Cdk6 ribozyme binding site
<400> 655
caaaagaata tctgcctac

<212> DNA<213> Homo sapiens<220> <223> Cdk6 ribozyme binding site
<400> 656
aaagaatatc tgcctacag

<212> DNA<213> Homo sapiens<220> <223> Cdk6 ribozyme binding site
<400> 657
tatctgccta cagtgccct

<212> DNA<213> Homo sapiens<220> <223> Cdk6 ribozyme binding site
<400> 658
gtgccctgtc tcacccata

<212> DNA<213> Homo sapiens<220> <223> Cdk6 ribozyme binding site
<400> 659
gccctgtctc acccatact

<212> DNA<213> Homo sapiens<220> <223> Cdk6 ribozyme binding site
<400> 660
ctcacccata cttccagga

<212> DNA<213> Homo sapiens<220> <223> Cdk6 ribozyme binding site
<400> 661
acccatactt ccaggacct

<212> DNA<213> Homo sapiens<220> <223> Cdk6 ribozyme binding site
<400> 662
cccatacttc caggacctg

<212> DNA<213> Homo sapiens<220> <223> Cdk6 ribozyme binding site
<400> 663
aacctggatt cccacctgc

<212> DNA<213> Homo sapiens<220> <223> Cdk6 ribozyme binding site
<400> 664
acctggattc ccacctgcc

<212> DNA<213> Homo sapiens<220> <223> Cdk6 ribozyme binding site
<400> 665
agaacacctc ggagctgaa

<212> DNA<213> Homo sapiens<220> <223> Cdk7 ribozyme binding site
<400> 666
acgtgaagtc tcgggcaaa

<212> DNA<213> Homo sapiens<220> <223> Cdk7 ribozyme binding site
<400> 667
gtgaagtctc gggcaaagc

<212> DNA<213> Homo sapiens<220> <223> Cdk7 ribozyme binding site
<400> 668
gcaaagcgtt atgagaagc

<212> DNA<213> Homo sapiens<220> <223> Cdk7 ribozyme binding site
<400> 669
caaagcgtta tgagaagct

<212> DNA<213> Homo sapiens<220> <223> Cdk7 ribozyme binding site
<400> 670
agctggactt ccttgggga

<212> DNA<213> Homo sapiens<220> <223> Cdk7 ribozyme binding site
<400> 671
gctggacttc cttggggag

<212> DNA<213> Homo sapiens<220> <223> Cdk7 ribozyme binding site
<400> 672
ggacttcctt ggggaggga

<212> DNA<213> Homo sapiens<220> <223> Cdk7 ribozyme binding site
<400> 673
agggacagtt tgccaccgt

<212> DNA<213> Homo sapiens<220> <223> Cdk7 ribozyme binding site
<400> 674
gggacagttt gccaccgtt

<212> DNA<213> Homo sapiens<220> <223> Cdk7 ribozyme binding site
<400> 675
tgccaccgtt tacaaggcc

<212> DNA<213> Homo sapiens<220> <223> Cdk7 ribozyme binding site
<400> 676
gccaccgttt acaaggcca

<212> DNA<213> Homo sapiens<220> <223> Cdk7 ribozyme binding site
<400> 677
ccaccgttta caaggccag

<212> DNA<213> Homo sapiens<220> <223> Cdk7 ribozyme binding site
<400> 678
gccagagata agaatacca

<212> DNA<213> Homo sapiens<220> <223> Cdk7 ribozyme binding site
<400> 679
gataagaata ccaaccaaa

<212> DNA<213> Homo sapiens<220> <223> Cdk7 ribozyme binding site
<400> 680
caaccaaatt gtcgccatt

<212> DNA<213> Homo sapiens<220> <223> Cdk7 ribozyme binding site
<400> 681
ccaaattgtc gccattaag

<212> DNA<213> Homo sapiens<220> <223> Cdk7 ribozyme binding site
<400> 682
tgtcgccatt aagaaaatc

<212> DNA<213> Homo sapiens<220> <223> Cdk7 ribozyme binding site
<400> 683
gtcgccatta agaaaatca

<212> DNA<213> Homo sapiens<220> <223> Cdk7 ribozyme binding site
<400> 684
taagaaaatc aaacttgga

<212> DNA<213> Homo sapiens<220> <223> Cdk7 ribozyme binding site
<400> 685
aatcaaactt ggacataga

<212> DNA<213> Homo sapiens<220> <223> Cdk7 ribozyme binding site
<400> 686
cttggacata gatcagaag

<212> DNA<213> Homo sapiens<220> <223> Cdk7 ribozyme binding site
<400> 687

gacatagatc agaagctaa

<212> DNA<213> Homo sapiens<220> <223> Cdk7 ribozyme binding site
<400> 688
tcagaagcta aagatggta

<212> DNA<213> Homo sapiens<220> <223> Cdk7 ribozyme binding site
<400> 689
aaagatggta taaatagaa

<212> DNA<213> Homo sapiens<220> <223> Cdk7 ribozyme binding site
<400> 690
agatggtata aatagaacc

<212> DNA<213> Homo sapiens<220> <223> Cdk7 ribozyme binding site
<400> 691
ggtataaata gaaccgcct

<212> DNA<213> Homo sapiens<220> <223> Cdk7 ribozyme binding site
<400> 692
gaaccgcctt aagagagat

<212> DNA<213> Homo sapiens<220> <223> Cdk7 ribozyme binding site
<400> 693
aaccgcctta agagagata

<212> DNA<213> Homo sapiens<220> <223> Cdk7 ribozyme binding site
<400> 694
aagagagata aaattatta

<212> DNA<213> Homo sapiens<220> <223> Cdk7 ribozyme binding site
<400> 695
agataaaatt attacagga

<212> DNA<213> Homo sapiens<220> <223> Cdk7 ribozyme binding site
<400> 696
gataaaatta ttacaggag

<212> DNA<213> Homo sapiens<220> <223> Cdk7 ribozyme binding site
<400> 697
taaaattatt acaggagct

<212> DNA<213> Homo sapiens<220> <223> Cdk7 ribozyme binding site
<400> 698
aaaattatta caggagcta

<212> DNA<213> Homo sapiens<220> <223> Cdk7 ribozyme binding site
<400> 699
acaggagcta agtcatcca

<212> DNA<213> Homo sapiens<220> <223> Cdk7 ribozyme binding site
<400> 700
gagctaagtc atccaaata

<212> DNA<213> Homo sapiens<220> <223> Cdk7 ribozyme binding site

<400> 701
ctaagtcatc caaatataa

<212> DNA<213> Homo sapiens<220> <223> Cdk7 ribozyme binding site
<400> 702
catccaaata taattggtc

<212> DNA<213> Homo sapiens<220> <223> Cdk7 ribozyme binding site
<400> 703
tccaaatata attggtctc

<212> DNA<213> Homo sapiens<220> <223> Cdk7 ribozyme binding site
<400> 704
aaatataatt ggtctcctt

<212> DNA<213> Homo sapiens<220> <223> Cdk7 ribozyme binding site
<400> 705
ataattggtc tccttgatg

<212> DNA<213> Homo sapiens<220> <223> Cdk7 ribozyme binding site
<400> 706
aattggtctc cttgatgct

<212> DNA<213> Homo sapiens<220> <223> Cdk7 ribozyme binding site
<400> 707
tggtctcctt gatgctttt

<212> DNA<213> Homo sapiens<220> <223> Cdk7 ribozyme binding site
<400> 708
cttgatgctt ttggacata

<212> DNA<213> Homo sapiens<220> <223> Cdk7 ribozyme binding site
<400> 709
ttgatgcttt tggacataa

<212> DNA<213> Homo sapiens<220> <223> Cdk7 ribozyme binding site
<400> 710
tgatgctttt ggacataaa

<212> DNA<213> Homo sapiens<220> <223> Cdk7 ribozyme binding site
<400> 711
tttggacata aatctaata

<212> DNA<213> Homo sapiens<220> <223> Cdk7 ribozyme binding site
<400> 712
gacataaatc taatattag

<212> DNA<213> Homo sapiens<220> <223> Cdk7 ribozyme binding site
<400> 713
cataaatcta atattagcc

<212> DNA<213> Homo sapiens<220> <223> Cdk7 ribozyme binding site
<400> 714
aaatctaata ttagccttg

<212> DNA<213> Homo sapiens<220> <223> Cdk7 ribozyme binding site
<400> 715
atctaatatt agccttgtc

<212> DNA<213> Homo sapiens<220> <223> Cdk7 ribozyme binding site
<400> 716
tctaatatta gccttgtct

<212> DNA<213> Homo sapiens<220> <223> Cdk7 ribozyme binding site
<400> 717
tattagcctt gtctttgat

<212> DNA<213> Homo sapiens<220> <223> Cdk7 ribozyme binding site
<400> 718
tagccttgtc tttgatttt

<212> DNA<213> Homo sapiens<220> <223> Cdk7 ribozyme binding site
<400> 719
gccttgtctt tgattttat

<212> DNA<213> Homo sapiens<220> <223> Cdk7 ribozyme binding site
<400> 720
ccttgtcttt gattttatg

<212> DNA<213> Homo sapiens<220> <223> Cdk7 ribozyme binding site
<400> 721
gtctttgatt ttatggaaa

<212> DNA<213> Homo sapiens<220> <223> Cdk7 ribozyme binding site
<400> 722
tctttgattt tatggaaac

<212> DNA<213> Homo sapiens<220> <223> Cdk7 ribozyme binding site
<400> 723
ctttgatttt atggaaact

<212> DNA<213> Homo sapiens<220> <223> Cdk7 ribozyme binding site
<400> 724
tttgatttta tggaaactg

<212> DNA<213> Homo sapiens<220> <223> Cdk7 ribozyme binding site
<400> 725
gaaactgatc tagaggtta

<212> DNA<213> Homo sapiens<220> <223> Cdk7 ribozyme binding site
<400> 726
aactgatcta gaggttata

<212> DNA<213> Homo sapiens<220> <223> Cdk7 ribozyme binding site
<400> 727
tctagaggtt ataataaag

<212> DNA<213> Homo sapiens<220> <223> Cdk7 ribozyme binding site
<400> 728
ctagaggtta taataaagg

<212> DNA<213> Homo sapiens<220> <223> Cdk7 ribozyme binding site
<400> 729
agaggttata ataaaggat

<212> DNA<213> Homo sapiens<220> <223> Cdk7 ribozyme binding site
<400> 730
ggttataata aaggataat

<212> DNA<213> Homo sapiens<220> <223> Cdk7 ribozyme binding site
<400> 731
ataaaggata atagtcttg

<212> DNA<213> Homo sapiens<220> <223> Cdk7 ribozyme binding site
<400> 732
aaggataata gtcttgtgc

<212> DNA<213> Homo sapiens<220> <223> Cdk7 ribozyme binding site
<400> 733
gataatagtc ttgtgctga

<212> DNA<213> Homo sapiens<220> <223> Cdk7 ribozyme binding site
<400> 734
taatagtctt gtgctgaca

<212> DNA<213> Homo sapiens<220> <223> Cdk7 ribozyme binding site
<400> 735
tgacaccatc acacatcaa

<212> DNA<213> Homo sapiens<220> <223> Cdk7 ribozyme binding site
<400> 736
atcacacatc aaagcctac

<212> DNA<213> Homo sapiens<220> <223> Cdk7 ribozyme binding site
<400> 737
tcaaagccta catgttgat

<212> DNA<213> Homo sapiens<220> <223> Cdk7 ribozyme binding site
<400> 738
cctacatgtt gatgactct

<212> DNA<213> Homo sapiens<220> <223> Cdk7 ribozyme binding site
<400> 739
ttgatgactc ttcaaggat

<212> DNA<213> Homo sapiens<220> <223> Cdk7 ribozyme binding site
<400> 740
gatgactctt caaggatta

<212> DNA<213> Homo sapiens<220> <223> Cdk7 ribozyme binding site
<400> 741
atgactcttc aaggattag

<212> DNA<213> Homo sapiens<220> <223> Cdk7 ribozyme binding site
<400> 742

ttcaaggatt agaatattt

<212> DNA<213> Homo sapiens<220> <223> Cdk7 ribozyme binding site
<400> 743
tcaaggatta gaatattta

<212> DNA<213> Homo sapiens<220> <223> Cdk7 ribozyme binding site
<400> 744
gattagaata tttacatca

<212> DNA<213> Homo sapiens<220> <223> Cdk7 ribozyme binding site
<400> 745
ttagaatatt tacatcaac

<212> DNA<213> Homo sapiens<220> <223> Cdk7 ribozyme binding site
<400> 746
tagaatattt acatcaaca

<212> DNA<213> Homo sapiens<220> <223> Cdk7 ribozyme binding site
<400> 747
agaatattta catcaacat

<212> DNA<213> Homo sapiens<220> <223> Cdk7 ribozyme binding site
<400> 748
tatttacatc aacattgga

<212> DNA<213> Homo sapiens<220> <223> Cdk7 ribozyme binding site
<400> 749
catcaacatt ggatcctac

<212> DNA<213> Homo sapiens<220> <223> Cdk7 ribozyme binding site
<400> 750
acattggatc ctacatagg

<212> DNA<213> Homo sapiens<220> <223> Cdk7 ribozyme binding site
<400> 751
ttggatccta catagggat

<212> DNA<213> Homo sapiens<220> <223> Cdk7 ribozyme binding site
<400> 752
atcctacata gggatctga

<212> DNA<213> Homo sapiens<220> <223> Cdk7 ribozyme binding site
<400> 753
catagggatc tgaaaccaa

<212> DNA<213> Homo sapiens<220> <223> Cdk7 ribozyme binding site
<400> 754
caaacaactt gttgctaga

<212> DNA<213> Homo sapiens<220> <223> Cdk7 ribozyme binding site
<400> 755
acaacttgtt gctagatga

<212> DNA<213> Homo sapiens<220> <223> Cdk7 ribozyme binding site

<400> 756
cttgttgcta gatgaaaat

<212> DNA<213> Homo sapiens<220> <223> Cdk7 ribozyme binding site
<400> 757
aaatggagtt ctaaaactg

<212> DNA<213> Homo sapiens<220> <223> Cdk7 ribozyme binding site
<400> 758
aatggagttc taaaactgg

<212> DNA<213> Homo sapiens<220> <223> Cdk7 ribozyme binding site
<400> 759
tggagttcta aaactggca

<212> DNA<213> Homo sapiens<220> <223> Cdk7 ribozyme binding site
<400> 760
ctggcagatt ttggcctgg

<212> DNA<213> Homo sapiens<220> <223> Cdk7 ribozyme binding site
<400> 761
tggcagattt tggcctggc

<212> DNA<213> Homo sapiens<220> <223> Cdk7 ribozyme binding site
<400> 762
ggcagatttt ggcctggcc

<212> DNA<213> Homo sapiens<220> <223> Cdk7 ribozyme binding site
<400> 763
tggccaaatc ttttgggag

<212> DNA<213> Homo sapiens<220> <223> Cdk7 ribozyme binding site
<400> 764
gccaaatctt ttgggagcc

<212> DNA<213> Homo sapiens<220> <223> Cdk7 ribozyme binding site
<400> 765
ccaaatcttt tgggagccc

<212> DNA<213> Homo sapiens<220> <223> Cdk7 ribozyme binding site
<400> 766
caaatctttt gggagcccc

<212> DNA<213> Homo sapiens<220> <223> Cdk7 ribozyme binding site
<400> 767
agccccaata gagcttata

<212> DNA<213> Homo sapiens<220> <223> Cdk7 ribozyme binding site
<400> 768
aatagagctt atacacatc

<212> DNA<213> Homo sapiens<220> <223> Cdk7 ribozyme binding site
<400> 769
atagagctta tacacatca

<212> DNA<213> Homo sapiens<220> <223> Cdk7 ribozyme binding site
<400> 770
agagcttata cacatcagg

<212> DNA<213> Homo sapiens<220> <223> Cdk7 ribozyme binding site
<400> 771
tatacacatc aggttgtaa

<212> DNA<213> Homo sapiens<220> <223> Cdk7 ribozyme binding site
<400> 772
acatcaggtt gtaaccagg

<212> DNA<213> Homo sapiens<220> <223> Cdk7 ribozyme binding site
<400> 773
tcaggttgta accaggtgg

<212> DNA<213> Homo sapiens<220> <223> Cdk7 ribozyme binding site
<400> 774
ccaggtggta tcgggcccc

<212> DNA<213> Homo sapiens<220> <223> Cdk7 ribozyme binding site
<400> 775
aggtggtatc gggcccccg

<212> DNA<213> Homo sapiens<220> <223> Cdk7 ribozyme binding site
<400> 776
cccccgagtt actatttgg

<212> DNA<213> Homo sapiens<220> <223> Cdk7 ribozyme binding site
<400> 777
ccccgagtta ctatttgga

<212> DNA<213> Homo sapiens<220> <223> Cdk7 ribozyme binding site
<400> 778
cgagttacta tttggagct

<212> DNA<213> Homo sapiens<220> <223> Cdk7 ribozyme binding site
<400> 779
agttactatt tggagctag

<212> DNA<213> Homo sapiens<220> <223> Cdk7 ribozyme binding site
<400> 780
gttactattt ggagctagg

<212> DNA<213> Homo sapiens<220> <223> Cdk7 ribozyme binding site
<400> 781
tttggagcta ggatgtatg

<212> DNA<213> Homo sapiens<220> <223> Cdk7 ribozyme binding site
<400> 782
ctaggatgta tggtgtagg

<212> DNA<213> Homo sapiens<220> <223> Cdk7 ribozyme binding site
<400> 783
gtatggtgta ggtgtggac

<212> DNA<213> Homo sapiens<220> <223> Cdk7 ribozyme binding site
<400> 784
gtgggctgtt ggctgtata

<212> DNA<213> Homo sapiens<220> <223> Cdk7 ribozyme binding site
<400> 785
gttggctgta tattagcag

<212> DNA<213> Homo sapiens<220> <223> Cdk7 ribozyme binding site
<400> 786
tggctgtata ttagcagag

<212> DNA<213> Homo sapiens<220> <223> Cdk7 ribozyme binding site
<400> 787
gctgtatatt agcagagtt

<212> DNA<213> Homo sapiens<220> <223> Cdk7 ribozyme binding site
<400> 788
ctgtatatta gcagagtta

<212> DNA<213> Homo sapiens<220> <223> Cdk7 ribozyme binding site
<400> 789
tagcagagtt acttctaag

<212> DNA<213> Homo sapiens<220> <223> Cdk7 ribozyme binding site
<400> 790
agcagagtta cttctaagg

<212> DNA<213> Homo sapiens<220> <223> Cdk7 ribozyme binding site
<400> 791
agagttactt ctaagggtt

<212> DNA<213> Homo sapiens<220> <223> Cdk7 ribozyme binding site
<400> 792
gagttacttc taagggttc

<212> DNA<213> Homo sapiens<220> <223> Cdk7 ribozyme binding site
<400> 793
gttacttcta agggttcct

<212> DNA<213> Homo sapiens<220> <223> Cdk7 ribozyme binding site
<400> 794
tctaagggtt cctttttg

<212> DNA<213> Homo sapiens<220> <223> Cdk7 ribozyme binding site
<400> 795
ctaagggttc ctttttgc

<212> DNA<213> Homo sapiens<220> <223> Cdk7 ribozyme binding site
<400> 796
agggttcctt ttttgccag

<212> DNA<213> Homo sapiens<220> <223> Cdk7 ribozyme binding site
<400> 797

gggttccttt tttgccagg

<212> DNA<213> Homo sapiens<220> <223> Cdk7 ribozyme binding site
<400> 798
ggttcctttt ttgccagga

<212> DNA<213> Homo sapiens<220> <223> Cdk7 ribozyme binding site
<400> 799
gttccttttt tgccaggag

<212> DNA<213> Homo sapiens<220> <223> Cdk7 ribozyme binding site
<400> 800
ttcctttttt gccaggaga

<212> DNA<213> Homo sapiens<220> <223> Cdk7 ribozyme binding site
<400> 801
ccaggagatt cagaccttg

<212> DNA<213> Homo sapiens<220> <223> Cdk7 ribozyme binding site
<400> 802
caggagattc agaccttga

<212> DNA<213> Homo sapiens<220> <223> Cdk7 ribozyme binding site
<400> 803
ttcagacctt gatcagcta

<212> DNA<213> Homo sapiens<220> <223> Cdk7 ribozyme binding site
<400> 804
gaccttgatc agctaacaa

<212> DNA<213> Homo sapiens<220> <223> Cdk7 ribozyme binding site
<400> 805
tgatcagcta acaagaata

<212> DNA<213> Homo sapiens<220> <223> Cdk7 ribozyme binding site
<400> 806
aacaagaata tttgaaact

<212> DNA<213> Homo sapiens<220> <223> Cdk7 ribozyme binding site
<400> 807
caagaatatt tgaaacttt

<212> DNA<213> Homo sapiens<220> <223> Cdk7 ribozyme binding site
<400> 808
aagaatattt gaaactttg

<212> DNA<213> Homo sapiens<220> <223> Cdk7 ribozyme binding site
<400> 809
tttgaaactt tgggcacac

<212> DNA<213> Homo sapiens<220> <223> Cdk7 ribozyme binding site
<400> 810
ttgaaacttt gggcacacc

<212> DNA<213> Homo sapiens<220> <223> Cdk7 ribozyme binding site

<400> 811
gacatgtgta gtcttccag

<212> DNA<213> Homo sapiens<220> <223> Cdk7 ribozyme binding site
<400> 812
atgtgtagtc ttccagatt

<212> DNA<213> Homo sapiens<220> <223> Cdk7 ribozyme binding site
<400> 813
gtgtagtctt ccagattat

<212> DNA<213> Homo sapiens<220> <223> Cdk7 ribozyme binding site
<400> 814
tgtagtcttc cagattatg

<212> DNA<213> Homo sapiens<220> <223> Cdk7 ribozyme binding site
<400> 815
cttccagatt atgtgacat

<212> DNA<213> Homo sapiens<220> <223> Cdk7 ribozyme binding site
<400> 816
ttccagatta tgtgacatt

<212> DNA<213> Homo sapiens<220> <223> Cdk7 ribozyme binding site
<400> 817
atgtgacatt taagagttt

<212> DNA<213> Homo sapiens<220> <223> Cdk7 ribozyme binding site
<400> 818
tgtgacattt aagagtttc

<212> DNA<213> Homo sapiens<220> <223> Cdk7 ribozyme binding site
<400> 819
gtgacattta agagtttcc

<212> DNA<213> Homo sapiens<220> <223> Cdk7 ribozyme binding site
<400> 820
tttaagagtt tccctggaa

<212> DNA<213> Homo sapiens<220> <223> Cdk7 ribozyme binding site
<400> 821
ttaagagttt ccctggaat

<212> DNA<213> Homo sapiens<220> <223> Cdk7 ribozyme binding site
<400> 822
taagagtttc cctggaata

<212> DNA<213> Homo sapiens<220> <223> Cdk7 ribozyme binding site
<400> 823
ccctggaata cctttgcat

<212> DNA<213> Homo sapiens<220> <223> Cdk7 ribozyme binding site
<400> 824
ggaatacctt tgcatcaca

<212> DNA<213> Homo sapiens<220> <223> Cdk7 ribozyme binding site
<400> 825
gaataccttt gcatcacat

<212> DNA<213> Homo sapiens<220> <223> Cdk7 ribozyme binding site
<400> 826
cctttgcatc acatcttca

<212> DNA<213> Homo sapiens<220> <223> Cdk7 ribozyme binding site
<400> 827
gcatcacatc ttcagtgca

<212> DNA<213> Homo sapiens<220> <223> Cdk7 ribozyme binding site
<400> 828
atcacatctt cagtgcagc

<212> DNA<213> Homo sapiens<220> <223> Cdk7 ribozyme binding site
<400> 829
tcacatcttc agtgcagca

<212> DNA<213> Homo sapiens<220> <223> Cdk7 ribozyme binding site
<400> 830
gagacgactt actagatct

<212> DNA<213> Homo sapiens<220> <223> Cdk7 ribozyme binding site
<400> 831
agacgactta ctagatctc

<212> DNA<213> Homo sapiens<220> <223> Cdk7 ribozyme binding site
<400> 832
cgacttacta gatctcata

<212> DNA<213> Homo sapiens<220> <223> Cdk7 ribozyme binding site
<400> 833
ttactagatc tcatacaag

<212> DNA<213> Homo sapiens<220> <223> Cdk7 ribozyme binding site
<400> 834
actagatctc atacaaggc

<212> DNA<213> Homo sapiens<220> <223> Cdk7 ribozyme binding site
<400> 835
agatctcata caaggctta

<212> DNA<213> Homo sapiens<220> <223> Cdk7 ribozyme binding site
<400> 836
tacaaggctt attcttatt

<212> DNA<213> Homo sapiens<220> <223> Cdk7 ribozyme binding site
<400> 837
acaaggctta ttcttattt

<212> DNA<213> Homo sapiens<220> <223> Cdk7 ribozyme binding site
<400> 838
aaggcttatt cttatttaa

<212> DNA<213> Homo sapiens<220> <223> Cdk7 ribozyme binding site
<400> 839
gcttattctt atttaatcc

<212> DNA<213> Homo sapiens<220> <223> Cdk7 ribozyme binding site
<400> 840
cttattctta tttaatcca

<212> DNA<213> Homo sapiens<220> <223> Cdk7 ribozyme binding site
<400> 841
tattcttatt taatccatg

<212> DNA<213> Homo sapiens<220> <223> Cdk7 ribozyme binding site
<400> 842
attcttattt aatccatgt

<212> DNA<213> Homo sapiens<220> <223> Cdk7 ribozyme binding site
<400> 843
ttcttattta atccatgtg

<212> DNA<213> Homo sapiens<220> <223> Cdk7 ribozyme binding site
<400> 844
ttatttaatc catgtgctc

<212> DNA<213> Homo sapiens<220> <223> Cdk7 ribozyme binding site
<400> 845
ccatgtgctc gaattacgg

<212> DNA<213> Homo sapiens<220> <223> Cdk7 ribozyme binding site
<400> 846
tgctcgaatt acggccaca

<212> DNA<213> Homo sapiens<220> <223> Cdk7 ribozyme binding site
<400> 847
gctcgaatta cggccacac

<212> DNA<213> Homo sapiens<220> <223> Cdk7 ribozyme binding site
<400> 848
aaatgaagta tttcagtaa

<212> DNA<213> Homo sapiens<220> <223> Cdk7 ribozyme binding site
<400> 849
atgaagtatt tcagtaatc

<212> DNA<213> Homo sapiens<220> <223> Cdk7 ribozyme binding site
<400> 850
tgaagtattt cagtaatcg

<212> DNA<213> Homo sapiens<220> <223> Cdk7 ribozyme binding site
<400> 851
gaagtatttc agtaatcgg

<212> DNA<213> Homo sapiens<220> <223> Cdk7 ribozyme binding site
<400> 852

tatttcagta atcggccag

<212> DNA<213> Homo sapiens<220> <223> Cdk7 ribozyme binding site
<400> 853
ttcagtaatc ggccagggc

<212> DNA<213> Homo sapiens<220> <223> Cdk7 ribozyme binding site
<400> 854
cctggatgtc agctgccaa

<212> DNA<213> Homo sapiens<220> <223> Cdk7 ribozyme binding site
<400> 855
ccaaactgtc cagtggaaa

<212> DNA<213> Homo sapiens<220> <223> Cdk7 ribozyme binding site
<400> 856
tggaaacctt aaaggagca

<212> DNA<213> Homo sapiens<220> <223> Cdk7 ribozyme binding site
<400> 857
ggaaacctta aaggagcaa

<212> DNA<213> Homo sapiens<220> <223> Cdk7 ribozyme binding site
<400> 858
aggagcaatc aaatccagc

<212> DNA<213> Homo sapiens<220> <223> Cdk7 ribozyme binding site
<400> 859
caatcaaatc cagctttgg

<212> DNA<213> Homo sapiens<220> <223> Cdk7 ribozyme binding site
<400> 860
aatccagctt tggcaataa

<212> DNA<213> Homo sapiens<220> <223> Cdk7 ribozyme binding site
<400> 861
atccagcttt ggcaataaa

<212> DNA<213> Homo sapiens<220> <223> Cdk7 ribozyme binding site
<400> 862
tttggcaata aaaaggaaa

<212> DNA<213> Homo sapiens<220> <223> Cdk7 ribozyme binding site
<400> 863
cagaggcctt agaacaagg

<212> DNA<213> Homo sapiens<220> <223> Cdk7 ribozyme binding site
<400> 864
agaggcctta gaacaagga

<212> DNA<213> Homo sapiens<220> <223> Cdk7 ribozyme binding site
<400> 865
aaggaggatt gcccaagaa

<212> DNA<213> Homo sapiens<220> <223> Cdk7 ribozyme binding site

<400> 866
caagaaacta attttttaa

<212> DNA<213> Homo sapiens<220> <223> Cdk8 ribozyme binding site
<400> 867
actatgactt taaagtgaa

<212> DNA<213> Homo sapiens<220> <223> Cdk8 ribozyme binding site
<400> 868
ctatgacttt aaagtgaag

<212> DNA<213> Homo sapiens<220> <223> Cdk8 ribozyme binding site
<400> 869
tatgacttta aagtgaagc

<212> DNA<213> Homo sapiens<220> <223> Cdk8 ribozyme binding site
<400> 870
ggagcgggtc gaggacctg

<212> DNA<213> Homo sapiens<220> <223> Cdk8 ribozyme binding site
<400> 871
aggacctgtt tgaatacga

<212> DNA<213> Homo sapiens<220> <223> Cdk8 ribozyme binding site
<400> 872
ggacctgttt gaatacgag

<212> DNA<213> Homo sapiens<220> <223> Cdk8 ribozyme binding site
<400> 873
tgtttgaata cgagggctg

<212> DNA<213> Homo sapiens<220> <223> Cdk8 ribozyme binding site
<400> 874
ctgcaaagtt ggccgaggc

<212> DNA<213> Homo sapiens<220> <223> Cdk8 ribozyme binding site
<400> 875
cgaggcactt atggtcacg

<212> DNA<213> Homo sapiens<220> <223> Cdk8 ribozyme binding site
<400> 876
gaggcactta tggtcacgt

<212> DNA<213> Homo sapiens<220> <223> Cdk8 ribozyme binding site
<400> 877
acttatggtc acgtctaca

<212> DNA<213> Homo sapiens<220> <223> Cdk8 ribozyme binding site
<400> 878
tggtcacgtc tacaaagcc

<212> DNA<213> Homo sapiens<220> <223> Cdk8 ribozyme binding site
<400> 879
gtcacgtcta caaagccaa

<212> DNA<213> Homo sapiens<220> <223> Cdk8 ribozyme binding site
<400> 880
aaggatgata aagactatg

<212> DNA<213> Homo sapiens<220> <223> Cdk8 ribozyme binding site
<400> 881
ataaagacta tgctttaaa

<212> DNA<213> Homo sapiens<220> <223> Cdk8 ribozyme binding site
<400> 882
gactatgctt taaaacaaa

<212> DNA<213> Homo sapiens<220> <223> Cdk8 ribozyme binding site
<400> 883
actatgcttt aaaacaaat

<212> DNA<213> Homo sapiens<220> <223> Cdk8 ribozyme binding site
<400> 884
ctatgcttta aaacaaata

<212> DNA<213> Homo sapiens<220> <223> Cdk8 ribozyme binding site
<400> 885
aaaacaaata gaaggaact

<212> DNA<213> Homo sapiens<220> <223> Cdk8 ribozyme binding site
<400> 886
aactgggatc tctatgtcg

<212> DNA<213> Homo sapiens<220> <223> Cdk8 ribozyme binding site
<400> 887
ctgggatctc tatgtcggc

<212> DNA<213> Homo sapiens<220> <223> Cdk8 ribozyme binding site
<400> 888
gggatctcta tgtcggcat

<212> DNA<213> Homo sapiens<220> <223> Cdk8 ribozyme binding site
<400> 889
tctctatgtc ggcatgtag

<212> DNA<213> Homo sapiens<220> <223> Cdk8 ribozyme binding site
<400> 890
tcggcatgta gagaaatag

<212> DNA<213> Homo sapiens<220> <223> Cdk8 ribozyme binding site
<400> 891
tagagaaata gcattactt

<212> DNA<213> Homo sapiens<220> <223> Cdk8 ribozyme binding site
<400> 892
aaatagcatt acttcgaga

<212> DNA<213> Homo sapiens<220> <223> Cdk8 ribozyme binding site
<400> 893
aatagcatta cttcgagag

<212> DNA<213> Homo sapiens<220> <223> Cdk8 ribozyme binding site
<400> 894
agcattactt cgagagctt

<212> DNA<213> Homo sapiens<220> <223> Cdk8 ribozyme binding site
<400> 895
gcattacttc gagagctta

<212> DNA<213> Homo sapiens<220> <223> Cdk8 ribozyme binding site
<400> 896
tcgagagctt aagcatcca

<212> DNA<213> Homo sapiens<220> <223> Cdk8 ribozyme binding site
<400> 897
cgagagctta agcatccaa

<212> DNA<213> Homo sapiens<220> <223> Cdk8 ribozyme binding site
<400> 898
cttaagcatc caaacgtca

<212> DNA<213> Homo sapiens<220> <223> Cdk8 ribozyme binding site
<400> 899
tccaaacgtc atttctctt

<212> DNA<213> Homo sapiens<220> <223> Cdk8 ribozyme binding site
<400> 900
aaacgtcatt tctcttcaa

<212> DNA<213> Homo sapiens<220> <223> Cdk8 ribozyme binding site
<400> 901
aacgtcattt ctcttcaaa

<212> DNA<213> Homo sapiens<220> <223> Cdk8 ribozyme binding site
<400> 902
acgtcatttc tcttcaaaa

<212> DNA<213> Homo sapiens<220> <223> Cdk8 ribozyme binding site
<400> 903
gtcatttctc ttcaaaagg

<212> DNA<213> Homo sapiens<220> <223> Cdk8 ribozyme binding site
<400> 904
catttctctt caaaaggtg

<212> DNA<213> Homo sapiens<220> <223> Cdk8 ribozyme binding site
<400> 905
atttctcttc aaaaggtgt

<212> DNA<213> Homo sapiens<220> <223> Cdk8 ribozyme binding site
<400> 906
aaaaggtgtt tctgtctca

<212> DNA<213> Homo sapiens<220> <223> Cdk8 ribozyme binding site
<400> 907

aaaggtgttt ctgtctcat

<212> DNA<213> Homo sapiens<220> <223> Cdk8 ribozyme binding site
<400> 908
aaggtgtttc tgtctcatg

<212> DNA<213> Homo sapiens<220> <223> Cdk8 ribozyme binding site
<400> 909
tgtttctgtc tcatgctga

<212> DNA<213> Homo sapiens<220> <223> Cdk8 ribozyme binding site
<400> 910
tttctgtctc atgctgata

<212> DNA<213> Homo sapiens<220> <223> Cdk8 ribozyme binding site
<400> 911
catgctgata ggaaggtgt

<212> DNA<213> Homo sapiens<220> <223> Cdk8 ribozyme binding site
<400> 912
ggtgtggctt ctgtttgac

<212> DNA<213> Homo sapiens<220> <223> Cdk8 ribozyme binding site
<400> 913
gtgtggcttc tgtttgact

<212> DNA<213> Homo sapiens<220> <223> Cdk8 ribozyme binding site
<400> 914
ggcttctgtt tgactatgc

<212> DNA<213> Homo sapiens<220> <223> Cdk8 ribozyme binding site
<400> 915
gcttctgttt gactatgct

<212> DNA<213> Homo sapiens<220> <223> Cdk8 ribozyme binding site
<400> 916
tgtttgacta tgctgaaca

<212> DNA<213> Homo sapiens<220> <223> Cdk8 ribozyme binding site
<400> 917
acatgacctc tggcatata

<212> DNA<213> Homo sapiens<220> <223> Cdk8 ribozyme binding site
<400> 918
ctctggcata taatcaagt

<212> DNA<213> Homo sapiens<220> <223> Cdk8 ribozyme binding site
<400> 919
ctggcatata atcaagttt

<212> DNA<213> Homo sapiens<220> <223> Cdk8 ribozyme binding site
<400> 920
gcatataatc aagtttcac

<212> DNA<213> Homo sapiens<220> <223> Cdk8 ribozyme binding site

<400> 921
taatcaagtt tcacagagc

<212> DNA<213> Homo sapiens<220> <223> Cdk8 ribozyme binding site
<400> 922
aatcaagttt cacagagct

<212> DNA<213> Homo sapiens<220> <223> Cdk8 ribozyme binding site
<400> 923
atcaagtttc acagagctt

<212> DNA<213> Homo sapiens<220> <223> Cdk8 ribozyme binding site
<400> 924
cacagagctt ctaaagcaa

<212> DNA<213> Homo sapiens<220> <223> Cdk8 ribozyme binding site
<400> 925
acagagcttc taaagcaaa

<212> DNA<213> Homo sapiens<220> <223> Cdk8 ribozyme binding site
<400> 926
agagcttcta aagcaaaca

<212> DNA<213> Homo sapiens<220> <223> Cdk8 ribozyme binding site
<400> 927
gaagccagtt cagttacct

<212> DNA<213> Homo sapiens<220> <223> Cdk8 ribozyme binding site
<400> 928
aagccagttc agttacctc

<212> DNA<213> Homo sapiens<220> <223> Cdk8 ribozyme binding site
<400> 929
cagttcagtt acctcgggg

<212> DNA<213> Homo sapiens<220> <223> Cdk8 ribozyme binding site
<400> 930
agttcagtta cctcgggga

<212> DNA<213> Homo sapiens<220> <223> Cdk8 ribozyme binding site
<400> 931
cagttacctc ggggaatgg

<212> DNA<213> Homo sapiens<220> <223> Cdk8 ribozyme binding site
<400> 932
tggtgaagtc actattata

<212> DNA<213> Homo sapiens<220> <223> Cdk8 ribozyme binding site
<400> 933
gaagtcacta ttatatcag

<212> DNA<213> Homo sapiens<220> <223> Cdk8 ribozyme binding site
<400> 934
agtcactatt atatcagat

<212> DNA<213> Homo sapiens<220> <223> Cdk8 ribozyme binding site
<400> 935
gtcactatta tatcagatc

<212> DNA<213> Homo sapiens<220> <223> Cdk8 ribozyme binding site
<400> 936
cactattata tcagatcct

<212> DNA<213> Homo sapiens<220> <223> Cdk8 ribozyme binding site
<400> 937
ctattatatc agatcctag

<212> DNA<213> Homo sapiens<220> <223> Cdk8 ribozyme binding site
<400> 938
atatcagatc ctagatggt

<212> DNA<213> Homo sapiens<220> <223> Cdk8 ribozyme binding site
<400> 939
tcagatccta gatggtatt

<212> DNA<213> Homo sapiens<220> <223> Cdk8 ribozyme binding site
<400> 940
ctagatggta ttcactacc

<212> DNA<213> Homo sapiens<220> <223> Cdk8 ribozyme binding site
<400> 941
agatggtatt cactacctg

<212> DNA<213> Homo sapiens<220> <223> Cdk8 ribozyme binding site
<400> 942
gatggtattc actacctgc

<212> DNA<213> Homo sapiens<220> <223> Cdk8 ribozyme binding site
<400> 943
gtattcacta cctgcatgc

<212> DNA<213> Homo sapiens<220> <223> Cdk8 ribozyme binding site
<400> 944
ctgcatgcta actgggtgt

<212> DNA<213> Homo sapiens<220> <223> Cdk8 ribozyme binding site
<400> 945
actgggtgtt gcacagaga

<212> DNA<213> Homo sapiens<220> <223> Cdk8 ribozyme binding site
<400> 946
cacagagatt tgaaacctg

<212> DNA<213> Homo sapiens<220> <223> Cdk8 ribozyme binding site
<400> 947
acagagattt gaaacctgc

<212> DNA<213> Homo sapiens<220> <223> Cdk8 ribozyme binding site
<400> 948
aaacctgcta arattttag

<212> DNA<213> Homo sapiens<220> <223> Cdk8 ribozyme binding site
<400> 949
cctgctaara ttttagtta

<212> DNA<213> Homo sapiens<220> <223> Cdk8 ribozyme binding site
<400> 950
tgctaatatt ttagttatg

<212> DNA<213> Homo sapiens<220> <223> Cdk8 ribozyme binding site
<400> 951
gctaatattt tagttatgg

<212> DNA<213> Homo sapiens<220> <223> Cdk8 ribozyme binding site
<400> 952
ctaatatttt agttatggg

<212> DNA<213> Homo sapiens<220> <223> Cdk8 ribozyme binding site
<400> 953
taatatttta gttatgggt

<212> DNA<213> Homo sapiens<220> <223> Cdk8 ribozyme binding site
<400> 954
tattttagtt atgggtgaa

<212> DNA<213> Homo sapiens<220> <223> Cdk8 ribozyme binding site
<400> 955
attttagtta tgggtgaag

<212> DNA<213> Homo sapiens<220> <223> Cdk8 ribozyme binding site
<400> 956
ggtgaaggtc ctgagcgag

<212> DNA<213> Homo sapiens<220> <223> Cdk8 ribozyme binding site
<400> 957
aggaagagta aaaattgct

<212> DNA<213> Homo sapiens<220> <223> Cdk8 ribozyme binding site
<400> 958
agtaaaaatt gctgacatg

<212> DNA<213> Homo sapiens<220> <223> Cdk8 ribozyme binding site
<400> 959
acatgggctt tgcccgatt

<212> DNA<213> Homo sapiens<220> <223> Cdk8 ribozyme binding site
<400> 960
catgggcttt gcccgatta

<212> DNA<213> Homo sapiens<220> <223> Cdk8 ribozyme binding site
<400> 961
ttgcccgatt atttaattc

<212> DNA<213> Homo sapiens<220> <223> Cdk8 ribozyme binding site
<400> 962

tgcccgatta tttaattca

<212> DNA<213> Homo sapiens<220> <223> Cdk8 ribozyme binding site
<400> 963
cccgattatt taattcacc

<212> DNA<213> Homo sapiens<220> <223> Cdk8 ribozyme binding site
<400> 964
ccgattattt aattcacct

<212> DNA<213> Homo sapiens<220> <223> Cdk8 ribozyme binding site
<400> 965
cgattattta attcacctt

<212> DNA<213> Homo sapiens<220> <223> Cdk8 ribozyme binding site
<400> 966
ttatttaatt cacctttga

<212> DNA<213> Homo sapiens<220> <223> Cdk8 ribozyme binding site
<400> 967
tatttaattc acctttgaa

<212> DNA<213> Homo sapiens<220> <223> Cdk8 ribozyme binding site
<400> 968
aattcacctt tgaagcctt

<212> DNA<213> Homo sapiens<220> <223> Cdk8 ribozyme binding site
<400> 969
attcaccttt gaagccttt

<212> DNA<213> Homo sapiens<220> <223> Cdk8 ribozyme binding site
<400> 970
ttgaagcctt tagcagatt

<212> DNA<213> Homo sapiens<220> <223> Cdk8 ribozyme binding site
<400> 971
tgaagccttt agcagattt

<212> DNA<213> Homo sapiens<220> <223> Cdk8 ribozyme binding site
<400> 972
gaagccttta gcagatttg

<212> DNA<213> Homo sapiens<220> <223> Cdk8 ribozyme binding site
<400> 973
ttagcagatt tggatccag

<212> DNA<213> Homo sapiens<220> <223> Cdk8 ribozyme binding site
<400> 974
tagcagattt ggatccagt

<212> DNA<213> Homo sapiens<220> <223> Cdk8 ribozyme binding site
<400> 975
gatttggatc cagtggttg

<212> DNA<213> Homo sapiens<220> <223> Cdk8 ribozyme binding site

<400> 976
tccagtggtt gttacattc

<212> DNA<213> Homo sapiens<220> <223> Cdk8 ribozyme binding site
<400> 977
agtggttgtt acattctgg

<212> DNA<213> Homo sapiens<220> <223> Cdk8 ribozyme binding site
<400> 978
gtggttgtta cattctggt

<212> DNA<213> Homo sapiens<220> <223> Cdk8 ribozyme binding site
<400> 979
ttgttacatt ctggtaccg

<212> DNA<213> Homo sapiens<220> <223> Cdk8 ribozyme binding site
<400> 980
tgttacattc tggtaccga

<212> DNA<213> Homo sapiens<220> <223> Cdk8 ribozyme binding site
<400> 981
cattctggta ccgagcccc

<212> DNA<213> Homo sapiens<220> <223> Cdk8 ribozyme binding site
<400> 982
ccctgaacta cttcttgga

<212> DNA<213> Homo sapiens<220> <223> Cdk8 ribozyme binding site
<400> 983
tgaactactt cttggagca

<212> DNA<213> Homo sapiens<220> <223> Cdk8 ribozyme binding site
<400> 984
actacttctt ggagcaagg

<212> DNA<213> Homo sapiens<220> <223> Cdk8 ribozyme binding site
<400> 985
gcaaggcatt ataccaaag

<212> DNA<213> Homo sapiens<220> <223> Cdk8 ribozyme binding site
<400> 986
caaggcatta taccaaagc

<212> DNA<213> Homo sapiens<220> <223> Cdk8 ribozyme binding site
<400> 987
aggcattata ccaaagcta

<212> DNA<213> Homo sapiens<220> <223> Cdk8 ribozyme binding site
<400> 988
accaaagcta ttgatattt

<212> DNA<213> Homo sapiens<220> <223> Cdk8 ribozyme binding site
<400> 989
caaagctatt gatatttgg

<212> DNA<213> Homo sapiens<220> <223> Cdk8 ribozyme binding site
<400> 990
gctattgata tttgggcta

<212> DNA<213> Homo sapiens<220> <223> Cdk8 ribozyme binding site
<400> 991
tattgatatt tgggctata

<212> DNA<213> Homo sapiens<220> <223> Cdk8 ribozyme binding site
<400> 992
attgatattt gggctatag

<212> DNA<213> Homo sapiens<220> <223> Cdk8 ribozyme binding site
<400> 993
atttgggcta tagggtgta

<212> DNA<213> Homo sapiens<220> <223> Cdk8 ribozyme binding site
<400> 994
ttgggctata gggtgtata

<212> DNA<213> Homo sapiens<220> <223> Cdk8 ribozyme binding site
<400> 995
atagggtgta tatttgcag

<212> DNA<213> Homo sapiens<220> <223> Cdk8 ribozyme binding site
<400> 996
agggtgtata tttgcagaa

<212> DNA<213> Homo sapiens<220> <223> Cdk8 ribozyme binding site
<400> 997
ggtgtatatt tgcagaact

<212> DNA<213> Homo sapiens<220> <223> Cdk8 ribozyme binding site
<400> 998
gtgtatattt gcagaacta

<212> DNA<213> Homo sapiens<220> <223> Cdk8 ribozyme binding site
<400> 999
tgcagaacta ctaacgtca

<212> DNA<213> Homo sapiens<220> <223> Cdk8 ribozyme binding site
<400> 1000
agaactacta acgtcagaa

<212> DNA<213> Homo sapiens<220> <223> Cdk8 ribozyme binding site
<400> 1001
tactaacgtc agaaccaat

<212> DNA<213> Homo sapiens<220> <223> Cdk8 ribozyme binding site
<400> 1002
agaaccaata tttcactgt

<212> DNA<213> Homo sapiens<220> <223> Cdk8 ribozyme binding site
<400> 1003
aaccaatatt tcactgtcg

<212> DNA<213> Homo sapiens<220> <223> Cdk8 ribozyme binding site
<400> 1004
accaatattt cactgtcga

<212> DNA<213> Homo sapiens<220> <223> Cdk8 ribozyme binding site
<400> 1005
ccaatatttc actgtcgac

<212> DNA<213> Homo sapiens<220> <223> Cdk8 ribozyme binding site
<400> 1006
tttcactgtc gacaagagg

<212> DNA<213> Homo sapiens<220> <223> Cdk8 ribozyme binding site
<400> 1007
caagaggatc aaaactagt

<212> DNA<213> Homo sapiens<220> <223> Cdk8 ribozyme binding site
<400> 1008
atcaaaacta gtaatcctt

<212> DNA<213> Homo sapiens<220> <223> Cdk8 ribozyme binding site
<400> 1009
aaaactagta atccttatc

<212> DNA<213> Homo sapiens<220> <223> Cdk8 ribozyme binding site
<400> 1010
actagtaatc cttatcacc

<212> DNA<213> Homo sapiens<220> <223> Cdk8 ribozyme binding site
<400> 1011
agtaatcctt atcaccatg

<212> DNA<213> Homo sapiens<220> <223> Cdk8 ribozyme binding site
<400> 1012
gtaatcctta tcaccatga

<212> DNA<213> Homo sapiens<220> <223> Cdk8 ribozyme binding site
<400> 1013
aatccttatc accatgacc

<212> DNA<213> Homo sapiens<220> <223> Cdk8 ribozyme binding site
<400> 1014
ggacagaata ttcaatgta

<212> DNA<213> Homo sapiens<220> <223> Cdk8 ribozyme binding site
<400> 1015
acagaatatt caatgtaat

<212> DNA<213> Homo sapiens<220> <223> Cdk8 ribozyme binding site
<400> 1016
cagaatattc aatgtaatg

<212> DNA<213> Homo sapiens<220> <223> Cdk8 ribozyme binding site
<400> 1017

attcaatgta atgggattt

<212> DNA<213> Homo sapiens<220> <223> Cdk8 ribozyme binding site
<400> 1018
taatgggatt tcctgcaga

<212> DNA<213> Homo sapiens<220> <223> Cdk8 ribozyme binding site
<400> 1019
aatgggattt cctgcagat

<212> DNA<213> Homo sapiens<220> <223> Cdk8 ribozyme binding site
<400> 1020
atgggatttc ctgcagata

<212> DNA<213> Homo sapiens<220> <223> Cdk8 ribozyme binding site
<400> 1021
cctgcagata aagattggg

<212> DNA<213> Homo sapiens<220> <223> Cdk8 ribozyme binding site
<400> 1022
gataaagatt gggaagata

<212> DNA<213> Homo sapiens<220> <223> Cdk8 ribozyme binding site
<400> 1023
tgggaagata taaaaaaga

<212> DNA<213> Homo sapiens<220> <223> Cdk8 ribozyme binding site
<400> 1024
ggaagatata aaaaagatg

<212> DNA<213> Homo sapiens<220> <223> Cdk8 ribozyme binding site
<400> 1025
cctgaacatt caacattaa

<212> DNA<213> Homo sapiens<220> <223> Cdk8 ribozyme binding site
<400> 1026
ctgaacattc aacattaat

<212> DNA<213> Homo sapiens<220> <223> Cdk8 ribozyme binding site
<400> 1027
attcaacatt aatgaaaga

<212> DNA<213> Homo sapiens<220> <223> Cdk8 ribozyme binding site
<400> 1028
ttcaacatta atgaaagat

<212> DNA<213> Homo sapiens<220> <223> Cdk8 ribozyme binding site
<400> 1029
atgaaagatt tcagaagaa

<212> DNA<213> Homo sapiens<220> <223> Cdk8 ribozyme binding site
<400> 1030
tgaaagattt cagaagaaa

<212> DNA<213> Homo sapiens<220> <223> Cdk8 ribozyme binding site

<400> 1031
gaaagatttc agaagaaat

<212> DNA<213> Homo sapiens<220> <223> Cdk8 ribozyme binding site
<400> 1032
agaagaaata cgtatacca

<212> DNA<213> Homo sapiens<220> <223> Cdk8 ribozyme binding site
<400> 1033
gaaatacgta taccaactg

<212> DNA<213> Homo sapiens<220> <223> Cdk8 ribozyme binding site
<400> 1034
aatacgtata ccaactgca

<212> DNA<213> Homo sapiens<220> <223> Cdk8 ribozyme binding site
<400> 1035
ctgcagcctt atcaagtat

<212> DNA<213> Homo sapiens<220> <223> Cdk8 ribozyme binding site
<400> 1036
tgcagcctta tcaagtata

<212> DNA<213> Homo sapiens<220> <223> Cdk8 ribozyme binding site
<400> 1037
cagccttatc aagtatatg

<212> DNA<213> Homo sapiens<220> <223> Cdk8 ribozyme binding site
<400> 1038
ttatcaagta tatggaaaa

<212> DNA<213> Homo sapiens<220> <223> Cdk8 ribozyme binding site
<400> 1039
atcaagtata tggaaaaac

<212> DNA<213> Homo sapiens<220> <223> Cdk8 ribozyme binding site
<400> 1040
gaaaaacata aagttaaac

<212> DNA<213> Homo sapiens<220> <223> Cdk8 ribozyme binding site
<400> 1041
acataaagtt aaaccagat

<212> DNA<213> Homo sapiens<220> <223> Cdk8 ribozyme binding site
<400> 1042
cataaagtta aaccagata

<212> DNA<213> Homo sapiens<220> <223> Cdk8 ribozyme binding site
<400> 1043
aaaccagata gtaaagcat

<212> DNA<213> Homo sapiens<220> <223> Cdk8 ribozyme binding site
<400> 1044
ccagatagta aagcattcc

<212> DNA<213> Homo sapiens<220> <223> Cdk8 ribozyme binding site
<400> 1045
gtaaagcatt ccacttgct

<212> DNA<213> Homo sapiens<220> <223> Cdk8 ribozyme binding site
<400> 1046
taaagcattc cacttgctt

<212> DNA<213> Homo sapiens<220> <223> Cdk8 ribozyme binding site
<400> 1047
cattccactt gcttcagaa

<212> DNA<213> Homo sapiens<220> <223> Cdk8 ribozyme binding site
<400> 1048
ccacttgctt cagaagctg

<212> DNA<213> Homo sapiens<220> <223> Cdk8 ribozyme binding site
<400> 1049
cacttgcttc agaagctgc

<212> DNA<213> Homo sapiens<220> <223> Cdk8 ribozyme binding site
<400> 1050
gaagctgctt accatggac

<212> DNA<213> Homo sapiens<220> <223> Cdk8 ribozyme binding site
<400> 1051
aagctgctta ccatggacc

<212> DNA<213> Homo sapiens<220> <223> Cdk8 ribozyme binding site
<400> 1052
atggaccata aagcgaatt

<212> DNA<213> Homo sapiens<220> <223> Cdk8 ribozyme binding site
<400> 1053
aaagcgaatt acctcagaa

<212> DNA<213> Homo sapiens<220> <223> Cdk8 ribozyme binding site
<400> 1054
aagcgaatta cctcagaac

<212> DNA<213> Homo sapiens<220> <223> Cdk8 ribozyme binding site
<400> 1055
gaattacctc agaacaggc

<212> DNA<213> Homo sapiens<220> <223> Cdk8 ribozyme binding site
<400> 1056
gaacaggcta tgcaggacc

<212> DNA<213> Homo sapiens<220> <223> Cdk8 ribozyme binding site
<400> 1057
aggaccccta tttcttaga

<212> DNA<213> Homo sapiens<220> <223> Cdk8 ribozyme binding site
<400> 1058
gacccctatt tcttagaag

<212> DNA<213> Homo sapiens<220> <223> Cdk8 ribozyme binding site
<400> 1059
acccctattt cttagaaga

<212> DNA<213> Homo sapiens<220> <223> Cdk8 ribozyme binding site
<400> 1060
cccctatttc ttagaagac

<212> DNA<213> Homo sapiens<220> <223> Cdk8 ribozyme binding site
<400> 1061
cctatttctt agaagaccc

<212> DNA<213> Homo sapiens<220> <223> Cdk8 ribozyme binding site
<400> 1062
ctatttctta gaagaccca

<212> DNA<213> Homo sapiens<220> <223> Cdk8 ribozyme binding site
<400> 1063
agacccactt cctacatca

<212> DNA<213> Homo sapiens<220> <223> Cdk8 ribozyme binding site
<400> 1064
gacccacttc ctacatcag

<212> DNA<213> Homo sapiens<220> <223> Cdk8 ribozyme binding site
<400> 1065
ccacttccta catcagacg

<212> DNA<213> Homo sapiens<220> <223> Cdk8 ribozyme binding site
<400> 1066
ttcctacatc agacgtttt

<212> DNA<213> Homo sapiens<220> <223> Cdk8 ribozyme binding site
<400> 1067
atcagacgtt tttgccggt

<212> DNA<213> Homo sapiens<220> <223> Cdk8 ribozyme binding site
<400> 1068
tcagacgttt ttgccggtt

<212> DNA<213> Homo sapiens<220> <223> Cdk8 ribozyme binding site
<400> 1069
cagacgtttt tgccggttg

<212> DNA<213> Homo sapiens<220> <223> Cdk8 ribozyme binding site
<400> 1070
agacgttttt gccggttgt

<212> DNA<213> Homo sapiens<220> <223> Cdk8 ribozyme binding site
<400> 1071
tttgccggtt gtcaaatcc

<212> DNA<213> Homo sapiens<220> <223> Cdk8 ribozyme binding site
<400> 1072

gccggttgtc aaatcccctt

<212> DNA<213> Homo sapiens<220> <223> Cdk8 ribozyme binding site
<400> 1073
ttgtcaaatc ccttaccca

<212> DNA<213> Homo sapiens<220> <223> Cdk8 ribozyme binding site
<400> 1074
caaatccctt acccaaaac

<212> DNA<213> Homo sapiens<220> <223> Cdk8 ribozyme binding site
<400> 1075
aaatccctta cccaaaacg

<212> DNA<213> Homo sapiens<220> <223> Cdk8 ribozyme binding site
<400> 1076
aacgagaatt tttaacgga

<212> DNA<213> Homo sapiens<220> <223> Cdk8 ribozyme binding site
<400> 1077
acgagaattt ttaacggaa

<212> DNA<213> Homo sapiens<220> <223> Cdk8 ribozyme binding site
<400> 1078
cgagaatttt taacggaag

<212> DNA<213> Homo sapiens<220> <223> Cdk8 ribozyme binding site
<400> 1079
gagaattttt aacggaaga

<212> DNA<213> Homo sapiens<220> <223> Cdk8 ribozyme binding site
<400> 1080
agaattttta acggaagaa

<212> DNA<213> Homo sapiens<220> <223> Cdk8 ribozyme binding site
<400> 1081
cagggcaata accacacta

<212> DNA<213> Homo sapiens<220> <223> Cdk8 ribozyme binding site
<400> 1082
aaccacacta atggaactg

<212> DNA<213> Homo sapiens<220> <223> Cdk8 ribozyme binding site
<400> 1083
ccagggaatc aagacagca

<212> DNA<213> Homo sapiens<220> <223> Cdk8 ribozyme binding site
<400> 1084
gacagcagtc acacacagg

<212> DNA<213> Homo sapiens<220> <223> Cdk8 ribozyme binding site
<400> 1085
gacccccgtt gaagaaagt

<212> DNA<213> Homo sapiens<220> <223> Cdk8 ribozyme binding site

<400> 1086
agtgagagtt gttcctcct

<212> DNA<213> Homo sapiens<220> <223> Cdk8 ribozyme binding site
<400> 1087
gagagttgtt cctcctacc

<212> DNA<213> Homo sapiens<220> <223> Cdk8 ribozyme binding site
<400> 1088
agagttgttc ctcctacca

<212> DNA<213> Homo sapiens<220> <223> Cdk8 ribozyme binding site
<400> 1089
gttgttcctc ctaccacta

<212> DNA<213> Homo sapiens<220> <223> Cdk8 ribozyme binding site
<400> 1090
gttcctccta ccactacct

<212> DNA<213> Homo sapiens<220> <223> Cdk8 ribozyme binding site
<400> 1091
cctaccacta cctcaggtg

<212> DNA<213> Homo sapiens<220> <223> Cdk8 ribozyme binding site
<400> 1092
ccactacctc aggtggact

<212> DNA<213> Homo sapiens<220> <223> Cdk8 ribozyme binding site
<400> 1093
aggtggactt atcatgacc

<212> DNA<213> Homo sapiens<220> <223> Cdk8 ribozyme binding site
<400> 1094
ggtggactta tcatgacct

<212> DNA<213> Homo sapiens<220> <223> Cdk8 ribozyme binding site
<400> 1095
tggacttatc atgacctca

<212> DNA<213> Homo sapiens<220> <223> Cdk8 ribozyme binding site
<400> 1096
tcatgacctc agactatca

<212> DNA<213> Homo sapiens<220> <223> Cdk8 ribozyme binding site
<400> 1097
cctcagacta tcagcgttc

<212> DNA<213> Homo sapiens<220> <223> Cdk8 ribozyme binding site
<400> 1098
tcagactatc agcgttcca

<212> DNA<213> Homo sapiens<220> <223> Cdk8 ribozyme binding site
<400> 1099
tatcagcgtt ccaatccac

<212> DNA<213> Homo sapiens<220> <223> Cdk8 ribozyme binding site
<400> 1100
atcagcgttc caatccaca

<212> DNA<213> Homo sapiens<220> <223> Cdk8 ribozyme binding site
<400> 1101
cgttccaatc cacatgctg

<212> DNA<213> Homo sapiens<220> <223> Cdk8 ribozyme binding site
<400> 1102
atgctgccta tcccaaccc

<212> DNA<213> Homo sapiens<220> <223> Cdk8 ribozyme binding site
<400> 1103
gctgcctatc ccaaccctg

<212> DNA<213> Homo sapiens<220> <223> Cdk8 ribozyme binding site
<400> 1104
caagcacatc acagccgca

<212> DNA<213> Homo sapiens<220> <223> Cdk8 ribozyme binding site
<400> 1105
gcatgggata ctcagctac

<212> DNA<213> Homo sapiens<220> <223> Cdk8 ribozyme binding site
<400> 1106
tgggatactc agctacctc

<212> DNA<213> Homo sapiens<220> <223> Cdk8 ribozyme binding site
<400> 1107
tactcagcta cctcccagc

<212> DNA<213> Homo sapiens<220> <223> Cdk8 ribozyme binding site
<400> 1108
cagctacctc ccagcagcc

<212> DNA<213> Homo sapiens<220> <223> Cdk8 ribozyme binding site
<400> 1109
cagcagcctc cacagtact

<212> DNA<213> Homo sapiens<220> <223> Cdk8 ribozyme binding site
<400> 1110
ctccacagta ctcacatca

<212> DNA<213> Homo sapiens<220> <223> Cdk8 ribozyme binding site
<400> 1111
cacagtactc acatcagac

<212> DNA<213> Homo sapiens<220> <223> Cdk8 ribozyme binding site
<400> 1112
tactcacatc agacacatg

<212> DNA<213> Homo sapiens<220> <223> Cdk-we-hu ribozyme binding site
<400> 1113
cctgcacctt gcggcagaa

<212> DNA<213> Homo sapiens<220> <223> Cdk-we-hu ribozyme binding site
<400> 1114
gaagctgatc ttctcgccc

<212> DNA<213> Homo sapiens<220> <223> Cdk-we-hu ribozyme binding site
<400> 1115
agctgatctt ctcgccctg

<212> DNA<213> Homo sapiens<220> <223> Cdk-we-hu ribozyme binding site
<400> 1116
gctgatcttc tcgccctgc

<212> DNA<213> Homo sapiens<220> <223> Cdk-we-hu ribozyme binding site
<400> 1117
tgatcttctc gccctgcag

<212> DNA<213> Homo sapiens<220> <223> Cdk-we-hu ribozyme binding site
<400> 1118
gggaggactc ggcctttca

<212> DNA<213> Homo sapiens<220> <223> Cdk-we-hu ribozyme binding site
<400> 1119
actcggcctt tcaagagcc

<212> DNA<213> Homo sapiens<220> <223> Cdk-we-hu ribozyme binding site
<400> 1120
ctcggccttt caagagccc

<212> DNA<213> Homo sapiens<220> <223> Cdk-we-hu ribozyme binding site
<400> 1121
tcggcctttc aagagcccg

<212> DNA<213> Homo sapiens<220> <223> Cdk-we-hu ribozyme binding site
<400> 1122
agcccgactc gccgctgcc

<212> DNA<213> Homo sapiens<220> <223> Cdk-we-hu ribozyme binding site
<400> 1123
gccgccgctc gcccgggcc

<212> DNA<213> Homo sapiens<220> <223> Cdk-we-hu ribozyme binding site
<400> 1124
aggacctgtt gctgcccgg

<212> DNA<213> Homo sapiens<220> <223> Cdk-we-hu ribozyme binding site
<400> 1125
agggcgactc gtgggagga

<212> DNA<213> Homo sapiens<220> <223> Cdk-we-hu ribozyme binding site
<400> 1126
aggagggctt cggctcctc

<212> DNA<213> Homo sapiens<220> <223> Cdk-we-hu ribozyme binding site
<400> 1127

ggagggcttc ggctcctcg

<212> DNA<213> Homo sapiens<220> <223> Cdk-we-hu ribozyme binding site
<400> 1128
gcttcggctc ctcgtcgcc

<212> DNA<213> Homo sapiens<220> <223> Cdk-we-hu ribozyme binding site
<400> 1129
tcggctcctc gtcgccggt

<212> DNA<213> Homo sapiens<220> <223> Cdk-we-hu ribozyme binding site
<400> 1130
gctcctcgtc gccggtcaa

<212> DNA<213> Homo sapiens<220> <223> Cdk-we-hu ribozyme binding site
<400> 1131
gtcgccggtc aagtcgccg

<212> DNA<213> Homo sapiens<220> <223> Cdk-we-hu ribozyme binding site
<400> 1132
cggtcaagtc gccggcggc

<212> DNA<213> Homo sapiens<220> <223> Cdk-we-hu ribozyme binding site
<400> 1133
cggcccccta cttcctggg

<212> DNA<213> Homo sapiens<220> <223> Cdk-we-hu ribozyme binding site
<400> 1134
ccccctactt cctgggtag

<212> DNA<213> Homo sapiens<220> <223> Cdk-we-hu ribozyme binding site
<400> 1135
cccctacttc ctgggtagc

<212> DNA<213> Homo sapiens<220> <223> Cdk-we-hu ribozyme binding site
<400> 1136
ttcctgggta gctctttct

<212> DNA<213> Homo sapiens<220> <223> Cdk-we-hu ribozyme binding site
<400> 1137
tgggtagctc tttctcgcc

<212> DNA<213> Homo sapiens<220> <223> Cdk-we-hu ribozyme binding site
<400> 1138
ggtagctctt tctcgccgg

<212> DNA<213> Homo sapiens<220> <223> Cdk-we-hu ribozyme binding site
<400> 1139
gtagctcttt ctcgccggt

<212> DNA<213> Homo sapiens<220> <223> Cdk-we-hu ribozyme binding site
<400> 1140
tagctctttc tcgccggtg

<212> DNA<213> Homo sapiens<220> <223> Cdk-we-hu ribozyme binding site

<400> 1141
gct[?]tttctc gccggtgcg

<212> DNA<213> Homo sapiens<220> <223> Cdk-we-hu ribozyme binding site
<400> 1142
gagatgcgtc gccgcgggg

<212> DNA<213> Homo sapiens<220> <223> Cdk-we-hu ribozyme binding site
<400> 1143
ccgcggggtt gcggggcgc

<212> DNA<213> Homo sapiens<220> <223> Cdk-we-hu ribozyme binding site
<400> 1144
gccgccgctc gccgcggcc

<212> DNA<213> Homo sapiens<220> <223> Cdk-we-hu ribozyme binding site
<400> 1145
acaagacctt ccgcaagct

<212> DNA<213> Homo sapiens<220> <223> Cdk-we-hu ribozyme binding site
<400> 1146
caagaccttc cgcaagctg

<212> DNA<213> Homo sapiens<220> <223> Cdk-we-hu ribozyme binding site
<400> 1147
gctgcgactc ttcgacacc

<212> DNA<213> Homo sapiens<220> <223> Cdk-we-hu ribozyme binding site
<400> 1148
tgcgactctt cgacacccc

<212> DNA<213> Homo sapiens<220> <223> Cdk-we-hu ribozyme binding site
<400> 1149
gcgactcttc gacaccccg

<212> DNA<213> Homo sapiens<220> <223> Cdk-we-hu ribozyme binding site
<400> 1150
cccaagagtt tgctctcca

<212> DNA<213> Homo sapiens<220> <223> Cdk-we-hu ribozyme binding site
<400> 1151
ccaagagttt gctctccaa

<212> DNA<213> Homo sapiens<220> <223> Cdk-we-hu ribozyme binding site
<400> 1152
gagtttgctc tccaaagct

<212> DNA<213> Homo sapiens<220> <223> Cdk-we-hu ribozyme binding site
<400> 1153
gtttgctctc caaagctcg

<212> DNA<213> Homo sapiens<220> <223> Cdk-we-hu ribozyme binding site
<400> 1154
tccaaagctc ggggaattg

<212> DNA<213> Homo sapiens<220> <223> Cdk-we-hu ribozyme binding site
<400> 1155
tcggggaatt gattccagc

<212> DNA<213> Homo sapiens<220> <223> Cdk-we-hu ribozyme binding site
<400> 1156
ggaattgatt ccagctctg

<212> DNA<213> Homo sapiens<220> <223> Cdk-we-hu ribozyme binding site
<400> 1157
gaattgattc cagctctgt

<212> DNA<213> Homo sapiens<220> <223> Cdk-we-hu ribozyme binding site
<400> 1158
attccagctc tgttaaact

<212> DNA<213> Homo sapiens<220> <223> Cdk-we-hu ribozyme binding site
<400> 1159
cagctctgtt aaactccgg

<212> DNA<213> Homo sapiens<220> <223> Cdk-we-hu ribozyme binding site
<400> 1160
agctctgtta aactccggg

<212> DNA<213> Homo sapiens<220> <223> Cdk-we-hu ribozyme binding site
<400> 1161
tgttaaactc cggggtagt

<212> DNA<213> Homo sapiens<220> <223> Cdk-we-hu ribozyme binding site
<400> 1162
ctccggggta gttctctct

<212> DNA<213> Homo sapiens<220> <223> Cdk-we-hu ribozyme binding site
<400> 1163
cggggtagtt ctctcttca

<212> DNA<213> Homo sapiens<220> <223> Cdk-we-hu ribozyme binding site
<400> 1164
ggggtagttc tctcttcat

<212> DNA<213> Homo sapiens<220> <223> Cdk-we-hu ribozyme binding site
<400> 1165
ggtagttctc tcttcatgg

<212> DNA<213> Homo sapiens<220> <223> Cdk-we-hu ribozyme binding site
<400> 1166
tagttctctc ttcatggat

<212> DNA<213> Homo sapiens<220> <223> Cdk-we-hu ribozyme binding site
<400> 1167
gttctctctt catggatac

<212> DNA<213> Homo sapiens<220> <223> Cdk-we-hu ribozyme binding site
<400> 1168
ttctctcttc atggataca

<212> DNA<213> Homo sapiens<220> <223> Cdk-we-hu ribozyme binding site
<400> 1169
ttcatggata cagaaaaat

<212> DNA<213> Homo sapiens<220> <223> Cdk-we-hu ribozyme binding site
<400> 1170
cagaaaatc aggaaaaag

<212> DNA<213> Homo sapiens<220> <223> Cdk-we-hu ribozyme binding site
<400> 1171
aaagggaatt tgatgtgcg

<212> DNA<213> Homo sapiens<220> <223> Cdk-we-hu ribozyme binding site
<400> 1172
aagggaattt gatgtgcga

<212> DNA<213> Homo sapiens<220> <223> Cdk-we-hu ribozyme binding site
<400> 1173
cgacagactc ctcaagtga

<212> DNA<213> Homo sapiens<220> <223> Cdk-we-hu ribozyme binding site
<400> 1174
cagactcctc aagtgaata

<212> DNA<213> Homo sapiens<220> <223> Cdk-we-hu ribozyme binding site
<400> 1175
caagtgaata ttaatcctt

<212> DNA<213> Homo sapiens<220> <223> Cdk-we-hu ribozyme binding site
<400> 1176
agtgaatatt aatcctttt

<212> DNA<213> Homo sapiens<220> <223> Cdk-we-hu ribozyme binding site
<400> 1177
gtgaatatta atcctttta

<212> DNA<213> Homo sapiens<220> <223> Cdk-we-hu ribozyme binding site
<400> 1178
aatattaatc cttttactc

<212> DNA<213> Homo sapiens<220> <223> Cdk-we-hu ribozyme binding site
<400> 1179
attaatcctt ttactccgg

<212> DNA<213> Homo sapiens<220> <223> Cdk-we-hu ribozyme binding site
<400> 1180
ttaatccttt tactccgga

<212> DNA<213> Homo sapiens<220> <223> Cdk-we-hu ribozyme binding site
<400> 1181
taatcctttt actccggat

<212> DNA<213> Homo sapiens<220> <223> Cdk-we-hu ribozyme binding site
<400> 1182

aatcctttta ctccggatt

<212> DNA<213> Homo sapiens<220> <223> Cdk-we-hu ribozyme binding site
<400> 1183
ccttttactc cggattctt

<212> DNA<213> Homo sapiens<220> <223> Cdk-we-hu ribozyme binding site
<400> 1184
actccggatt ctttgttgc

<212> DNA<213> Homo sapiens<220> <223> Cdk-we-hu ribozyme binding site
<400> 1185
ctccggattc tttgttgct

<212> DNA<213> Homo sapiens<220> <223> Cdk-we-hu ribozyme binding site
<400> 1186
ccggattctt tgttgcttc

<212> DNA<213> Homo sapiens<220> <223> Cdk-we-hu ribozyme binding site
<400> 1187
cggattcttt gttgcttca

<212> DNA<213> Homo sapiens<220> <223> Cdk-we-hu ribozyme binding site
<400> 1188
attctttgtt gcttcattc

<212> DNA<213> Homo sapiens<220> <223> Cdk-we-hu ribozyme binding site
<400> 1189
tttgttgctt cattcctca

<212> DNA<213> Homo sapiens<220> <223> Cdk-we-hu ribozyme binding site
<400> 1190
ttgttgcttc attcctcag

<212> DNA<213> Homo sapiens<220> <223> Cdk-we-hu ribozyme binding site
<400> 1191
ttgcttcatt cctcaggac

<212> DNA<213> Homo sapiens<220> <223> Cdk-we-hu ribozyme binding site
<400> 1192
tgcttcattc ctcaggaca

<212> DNA<213> Homo sapiens<220> <223> Cdk-we-hu ribozyme binding site
<400> 1193
ttcattcctc aggacagtg

<212> DNA<213> Homo sapiens<220> <223> Cdk-we-hu ribozyme binding site
<400> 1194
ggacagtgtc gtcgtagaa

<212> DNA<213> Homo sapiens<220> <223> Cdk-we-hu ribozyme binding site
<400> 1195
cagtgtcgtc gtagaaaga

<212> DNA<213> Homo sapiens<220> <223> Cdk-we-hu ribozyme binding site

<400> 1196
tgtcgtcgta gaaagagaa

<212> DNA<213> Homo sapiens<220> <223> Cdk-we-hu ribozyme binding site
<400> 1197
agagaacgta ttggaatga

<212> DNA<213> Homo sapiens<220> <223> Cdk-we-hu ribozyme binding site
<400> 1198
agaacgtatt ggaatgatt

<212> DNA<213> Homo sapiens<220> <223> Cdk-we-hu ribozyme binding site
<400> 1199
tggaatgatt cctgtggtg

<212> DNA<213> Homo sapiens<220> <223> Cdk-we-hu ribozyme binding site
<400> 1200
ggaatgattc ctgtggtga

<212> DNA<213> Homo sapiens<220> <223> Cdk-we-hu ribozyme binding site
<400> 1201
gccagtgatt atgagcttg

<212> DNA<213> Homo sapiens<220> <223> Cdk-we-hu ribozyme binding site
<400> 1202
ccagtgatta tgagcttga

<212> DNA<213> Homo sapiens<220> <223> Cdk-we-hu ribozyme binding site
<400> 1203
ttatgagctt gaagatgaa

<212> DNA<213> Homo sapiens<220> <223> Cdk-we-hu ribozyme binding site
<400> 1204
agacctgcta agagaatta

<212> DNA<213> Homo sapiens<220> <223> Cdk-we-hu ribozyme binding site
<400> 1205
taagagaatt acaattact

<212> DNA<213> Homo sapiens<220> <223> Cdk-we-hu ribozyme binding site
<400> 1206
aagagaatta caattactg

<212> DNA<213> Homo sapiens<220> <223> Cdk-we-hu ribozyme binding site
<400> 1207
aattacaatt actgaaagc

<212> DNA<213> Homo sapiens<220> <223> Cdk-we-hu ribozyme binding site
<400> 1208
attacaatta ctgaaagca

<212> DNA<213> Homo sapiens<220> <223> Cdk-we-hu ribozyme binding site
<400> 1209
gaaagcaata tgaagtccc

<212> DNA<213> Homo sapiens<220> <223> Cdk-we-hu ribozyme binding site
<400> 1210
atatgaagtc ccggtatac

<212> DNA<213> Homo sapiens<220> <223> Cdk-we-hu ribozyme binding site
<400> 1211
agtcccggta tacaacaga

<212> DNA<213> Homo sapiens<220> <223> Cdk-we-hu ribozyme binding site
<400> 1212
tcccggtata caacagaat

<212> DNA<213> Homo sapiens<220> <223> Cdk-we-hu ribozyme binding site
<400> 1213
caacagaatt tcatgagct

<212> DNA<213> Homo sapiens<220> <223> Cdk-we-hu ribozyme binding site
<400> 1214
aacagaattt catgagcta

<212> DNA<213> Homo sapiens<220> <223> Cdk-we-hu ribozyme binding site
<400> 1215
acagaatttc atgagctag

<212> DNA<213> Homo sapiens<220> <223> Cdk-we-hu ribozyme binding site
<400> 1216
tcatgagcta gagaaaatc

<212> DNA<213> Homo sapiens<220> <223> Cdk-we-hu ribozyme binding site
<400> 1217
agagaaaatc ggctctgga

<212> DNA<213> Homo sapiens<220> <223> Cdk-we-hu ribozyme binding site
<400> 1218
aaatcggctc tggagaatt

<212> DNA<213> Homo sapiens<220> <223> Cdk-we-hu ribozyme binding site
<400> 1219
ctggagaatt tggttctgt

<212> DNA<213> Homo sapiens<220> <223> Cdk-we-hu ribozyme binding site
<400> 1220
tggagaattt ggttctgta

<212> DNA<213> Homo sapiens<220> <223> Cdk-we-hu ribozyme binding site
<400> 1221
gaatttggtt ctgtattta

<212> DNA<213> Homo sapiens<220> <223> Cdk-we-hu ribozyme binding site
<400> 1222
aatttggttc tgtatttaa

<212> DNA<213> Homo sapiens<220> <223> Cdk-we-hu ribozyme binding site
<400> 1223
tggttctgta tttaagtgt

<212> DNA<213> Homo sapiens<220> <223> Cdk-we-hu ribozyme binding site
<400> 1224
gttctgtatt taagtgtgt

<212> DNA<213> Homo sapiens<220> <223> Cdk-we-hu ribozyme binding site
<400> 1225
ttctgtattt aagtgtgtg

<212> DNA<213> Homo sapiens<220> <223> Cdk-we-hu ribozyme binding site
<400> 1226
tctgtattta agtgtgtga

<212> DNA<213> Homo sapiens<220> <223> Cdk-we-hu ribozyme binding site
<400> 1227
tggatgcatt tatgccatt

<212> DNA<213> Homo sapiens<220> <223> Cdk-we-hu ribozyme binding site
<400> 1228
ggatgcattt atgccatta

<212> DNA<213> Homo sapiens<220> <223> Cdk-we-hu ribozyme binding site
<400> 1229
gatgcattta tgccattaa

<212> DNA<213> Homo sapiens<220> <223> Cdk-we-hu ribozyme binding site
<400> 1230
ttatgccatt aagcgatca

<212> DNA<213> Homo sapiens<220> <223> Cdk-we-hu ribozyme binding site
<400> 1231
tatgccatta agcgatcaa

<212> DNA<213> Homo sapiens<220> <223> Cdk-we-hu ribozyme binding site
<400> 1232
ttaagcgatc aaaaaagcc

<212> DNA<213> Homo sapiens<220> <223> Cdk-we-hu ribozyme binding site
<400> 1233
aaaagccatt ggcgggctc

<212> DNA<213> Homo sapiens<220> <223> Cdk-we-hu ribozyme binding site
<400> 1234
tggcgggctc tgttgatga

<212> DNA<213> Homo sapiens<220> <223> Cdk-we-hu ribozyme binding site
<400> 1235
gggctctgtt gatgagcag

<212> DNA<213> Homo sapiens<220> <223> Cdk-we-hu ribozyme binding site
<400> 1236
cagaacgctt tgagagaag

<212> DNA<213> Homo sapiens<220> <223> Cdk-we-hu ribozyme binding site
<400> 1237

agaacgcttt gagagaagt

<212> DNA<213> Homo sapiens<220> <223> Cdk-we-hu ribozyme binding site
<400> 1238
gagagaagta tatgctcat

<212> DNA<213> Homo sapiens<220> <223> Cdk-we-hu ribozyme binding site
<400> 1239
gagaagtata tgctcatgc

<212> DNA<213> Homo sapiens<220> <223> Cdk-we-hu ribozyme binding site
<400> 1240
gtatatgctc atgcagtgc

<212> DNA<213> Homo sapiens<220> <223> Cdk-we-hu ribozyme binding site
<400> 1241
tgcagtgctt ggacagcat

<212> DNA<213> Homo sapiens<220> <223> Cdk-we-hu ribozyme binding site
<400> 1242
ggacagcatt ctcatgtag

<212> DNA<213> Homo sapiens<220> <223> Cdk-we-hu ribozyme binding site
<400> 1243
gacagcattc tcatgtagt

<212> DNA<213> Homo sapiens<220> <223> Cdk-we-hu ribozyme binding site
<400> 1244
cagcattctc atgtagttc

<212> DNA<213> Homo sapiens<220> <223> Cdk-we-hu ribozyme binding site
<400> 1245
ttctcatgta gttcgatat

<212> DNA<213> Homo sapiens<220> <223> Cdk-we-hu ribozyme binding site
<400> 1246
tcatgtagtt cgatatttc

<212> DNA<213> Homo sapiens<220> <223> Cdk-we-hu ribozyme binding site
<400> 1247
catgtagttc gatatttct

<212> DNA<213> Homo sapiens<220> <223> Cdk-we-hu ribozyme binding site
<400> 1248
tagttcgata tttctctgc

<212> DNA<213> Homo sapiens<220> <223> Cdk-we-hu ribozyme binding site
<400> 1249
gttcgatatt tctctgcgt

<212> DNA<213> Homo sapiens<220> <223> Cdk-we-hu ribozyme binding site
<400> 1250
ttcgatattt ctctgcgtg

<212> DNA<213> Homo sapiens<220> <223> Cdk-we-hu ribozyme binding site

<400> 1251
tcgatatttc tctgcgtgg

<212> DNA<213> Homo sapiens<220> <223> Cdk-we-hu ribozyme binding site
<400> 1252
gatatttctc tgcgtgggc

<212> DNA<213> Homo sapiens<220> <223> Cdk-we-hu ribozyme binding site
<400> 1253
gaagatgatc atatgctta

<212> DNA<213> Homo sapiens<220> <223> Cdk-we-hu ribozyme binding site
<400> 1254
gatgatcata tgcttatac

<212> DNA<213> Homo sapiens<220> <223> Cdk-we-hu ribozyme binding site
<400> 1255
tcatatgctt atacagaat

<212> DNA<213> Homo sapiens<220> <223> Cdk-we-hu ribozyme binding site
<400> 1256
catatgctta tacagaatg

<212> DNA<213> Homo sapiens<220> <223> Cdk-we-hu ribozyme binding site
<400> 1257
tatgcttata cagaatgaa

<212> DNA<213> Homo sapiens<220> <223> Cdk-we-hu ribozyme binding site
<400> 1258
agaatgaata ttgtaatgg

<212> DNA<213> Homo sapiens<220> <223> Cdk-we-hu ribozyme binding site
<400> 1259
aatgaatatt gtaatggtg

<212> DNA<213> Homo sapiens<220> <223> Cdk-we-hu ribozyme binding site
<400> 1260
gaatattgta atggtggaa

<212> DNA<213> Homo sapiens<220> <223> Cdk-we-hu ribozyme binding site
<400> 1261
ggtggaagtt tagctgatg

<212> DNA<213> Homo sapiens<220> <223> Cdk-we-hu ribozyme binding site
<400> 1262
gtggaagttt agctgatgc

<212> DNA<213> Homo sapiens<220> <223> Cdk-we-hu ribozyme binding site
<400> 1263
tggaagttta gctgatgct

<212> DNA<213> Homo sapiens<220> <223> Cdk-we-hu ribozyme binding site
<400> 1264
gctgatgcta taagtgaaa

<212> DNA<213> Homo sapiens<220> <223> Cdk-we-hu ribozyme binding site
<400> 1265
tgatgctata agtgaaaac

<212> DNA<213> Homo sapiens<220> <223> Cdk-we-hu ribozyme binding site
<400> 1266
gtgaaaacta cagaatcat

<212> DNA<213> Homo sapiens<220> <223> Cdk-we-hu ribozyme binding site
<400> 1267
ctacagaatc atgagttac

<212> DNA<213> Homo sapiens<220> <223> Cdk-we-hu ribozyme binding site
<400> 1268
atcatgagtt actttaaag

<212> DNA<213> Homo sapiens<220> <223> Cdk-we-hu ribozyme binding site
<400> 1269
tcatgagtta ctttaaaga

<212> DNA<213> Homo sapiens<220> <223> Cdk-we-hu ribozyme binding site
<400> 1270
tgagttactt taaagaagc

<212> DNA<213> Homo sapiens<220> <223> Cdk-we-hu ribozyme binding site
<400> 1271
gagttacttt aaagaagca

<212> DNA<213> Homo sapiens<220> <223> Cdk-we-hu ribozyme binding site
<400> 1272
agttacttta aagaagcag

<212> DNA<213> Homo sapiens<220> <223> Cdk-we-hu ribozyme binding site
<400> 1273
aagcagagtt gaaggatct

<212> DNA<213> Homo sapiens<220> <223> Cdk-we-hu ribozyme binding site
<400> 1274
ttgaaggatc tccttttgc

<212> DNA<213> Homo sapiens<220> <223> Cdk-we-hu ribozyme binding site
<400> 1275
gaaggatctc cttttgcaa

<212> DNA<213> Homo sapiens<220> <223> Cdk-we-hu ribozyme binding site
<400> 1276
ggatctcctt ttgcaagtt

<212> DNA<213> Homo sapiens<220> <223> Cdk-we-hu ribozyme binding site
<400> 1277
gatctccttt tgcaagttg

<212> DNA<213> Homo sapiens<220> <223> Cdk-we-hu ribozyme binding site
<400> 1278
atctcctttt gcaagttgg

<212> DNA<213> Homo sapiens<220> <223> Cdk-we-hu ribozyme binding site
<400> 1279
tttgcaagtt ggccgaggc

<212> DNA<213> Homo sapiens<220> <223> Cdk-we-hu ribozyme binding site
<400> 1280
gccgaggctt gaggtatat

<212> DNA<213> Homo sapiens<220> <223> Cdk-we-hu ribozyme binding site
<400> 1281
gcttgaggta tattcattc

<212> DNA<213> Homo sapiens<220> <223> Cdk-we-hu ribozyme binding site
<400> 1282
ttgaggtata ttcattcaa

<212> DNA<213> Homo sapiens<220> <223> Cdk-we-hu ribozyme binding site
<400> 1283
gaggtatatt cattcaatg

<212> DNA<213> Homo sapiens<220> <223> Cdk-we-hu ribozyme binding site
<400> 1284
aggtatattc attcaatgt

<212> DNA<213> Homo sapiens<220> <223> Cdk-we-hu ribozyme binding site
<400> 1285
tatattcatt caatgtctt

<212> DNA<213> Homo sapiens<220> <223> Cdk-we-hu ribozyme binding site
<400> 1286
atattcattc aatgtcttt

<212> DNA<213> Homo sapiens<220> <223> Cdk-we-hu ribozyme binding site
<400> 1287
attcaatgtc tttggttca

<212> DNA<213> Homo sapiens<220> <223> Cdk-we-hu ribozyme binding site
<400> 1288
tcaatgtctt tggttcaca

<212> DNA<213> Homo sapiens<220> <223> Cdk-we-hu ribozyme binding site
<400> 1289
caatgtcttt ggttcacat

<212> DNA<213> Homo sapiens<220> <223> Cdk-we-hu ribozyme binding site
<400> 1290
gtctttggtt cacatggat

<212> DNA<213> Homo sapiens<220> <223> Cdk-we-hu ribozyme binding site
<400> 1291
tctttggttc acatggata

<212> DNA<213> Homo sapiens<220> <223> Cdk-we-hu ribozyme binding site
<400> 1292

cacatggata taaaaccta

<212> DNA<213> Homo sapiens<220> <223> Cdk-we-hu ribozyme binding site
<400> 1293
catggatata aaacctagt

<212> DNA<213> Homo sapiens<220> <223> Cdk-we-hu ribozyme binding site
<400> 1294
ataaaaccta gtaatattt

<212> DNA<213> Homo sapiens<220> <223> Cdk-we-hu ribozyme binding site
<400> 1295
aaacctagta atattttca

<212> DNA<213> Homo sapiens<220> <223> Cdk-we-hu ribozyme binding site
<400> 1296
cctagtaata ttttcatat

<212> DNA<213> Homo sapiens<220> <223> Cdk-we-hu ribozyme binding site
<400> 1297
tagtaatatt ttcatatct

<212> DNA<213> Homo sapiens<220> <223> Cdk-we-hu ribozyme binding site
<400> 1298
agtaatattt tcatatctc

<212> DNA<213> Homo sapiens<220> <223> Cdk-we-hu ribozyme binding site
<400> 1299
gtaatatttt catatctcg

<212> DNA<213> Homo sapiens<220> <223> Cdk-we-hu ribozyme binding site
<400> 1300
taatattttc atatctcga

<212> DNA<213> Homo sapiens<220> <223> Cdk-we-hu ribozyme binding site
<400> 1301
tattttcata tctcgaacc

<212> DNA<213> Homo sapiens<220> <223> Cdk-we-hu ribozyme binding site
<400> 1302
ttttcatatc tcgaacctc

<212> DNA<213> Homo sapiens<220> <223> Cdk-we-hu ribozyme binding site
<400> 1303
ttcatatctc gaacctcaa

<212> DNA<213> Homo sapiens<220> <223> Cdk-we-hu ribozyme binding site
<400> 1304
ctcgaacctc aatcccaaa

<212> DNA<213> Homo sapiens<220> <223> Cdk-we-hu ribozyme binding site
<400> 1305
aacctcaatc ccaaatgct

<212> DNA<213> Homo sapiens<220> <223> Cdk-we-hu ribozyme binding site

<400> 1306
atgctgcctc [?]gaagaagg

<212> DNA<213> Homo sapiens<220> <223> Cdk-we-hu ribozyme binding site
<400> 1307
gaagatgatt gggcatcca

<212> DNA<213> Homo sapiens<220> <223> Cdk-we-hu ribozyme binding site
<400> 1308
attgggcatc caacaaagt

<212> DNA<213> Homo sapiens<220> <223> Cdk-we-hu ribozyme binding site
<400> 1309
caacaaagtt atgtttaaa

<212> DNA<213> Homo sapiens<220> <223> Cdk-we-hu ribozyme binding site
<400> 1310
aacaaagtta tgtttaaaa

<212> DNA<213> Homo sapiens<220> <223> Cdk-we-hu ribozyme binding site
<400> 1311
aagttatgtt taaaatagg

<212> DNA<213> Homo sapiens<220> <223> Cdk-we-hu ribozyme binding site
<400> 1312
agttatgttt aaaataggt

<212> DNA<213> Homo sapiens<220> <223> Cdk-we-hu ribozyme binding site
<400> 1313
gttatgttta aaataggtg

<212> DNA<213> Homo sapiens<220> <223> Cdk-we-hu ribozyme binding site
<400> 1314
gtttaaaata ggtgatctt

<212> DNA<213> Homo sapiens<220> <223> Cdk-we-hu ribozyme binding site
<400> 1315
ataggtgatc ttgggcatg

<212> DNA<213> Homo sapiens<220> <223> Cdk-we-hu ribozyme binding site
<400> 1316
aggtgatctt gggcatgta

<212> DNA<213> Homo sapiens<220> <223> Cdk-we-hu ribozyme binding site
<400> 1317
tgggcatgta acaaggatc

<212> DNA<213> Homo sapiens<220> <223> Cdk-we-hu ribozyme binding site
<400> 1318
aacaaggatc tccagtcca

<212> DNA<213> Homo sapiens<220> <223> Cdk-we-hu ribozyme binding site
<400> 1319
caaggatctc cagtccaca

<212> DNA<213> Homo sapiens<220> <223> Cdk-we-hu ribozyme binding site
<400> 1320
atctccagtc cacaagttg

<212> DNA<213> Homo sapiens<220> <223> Cdk-we-hu ribozyme binding site
<400> 1321
tccacaagtt gaagagggc

<212> DNA<213> Homo sapiens<220> <223> Cdk-we-hu ribozyme binding site
<400> 1322
gagggcgata gtcgttttc

<212> DNA<213> Homo sapiens<220> <223> Cdk-we-hu ribozyme binding site
<400> 1323
ggcgatagtc gttttcttg

<212> DNA<213> Homo sapiens<220> <223> Cdk-we-hu ribozyme binding site
<400> 1324
gatagtcgtt ttcttgcaa

<212> DNA<213> Homo sapiens<220> <223> Cdk-we-hu ribozyme binding site
<400> 1325
atagtcgttt tcttgcaaa

<212> DNA<213> Homo sapiens<220> <223> Cdk-we-hu ribozyme binding site
<400> 1326
tagtcgtttt cttgcaaat

<212> DNA<213> Homo sapiens<220> <223> Cdk-we-hu ribozyme binding site
<400> 1327
agtcgttttc ttgcaaatg

<212> DNA<213> Homo sapiens<220> <223> Cdk-we-hu ribozyme binding site
<400> 1328
tcgttttctt gcaaatgaa

<212> DNA<213> Homo sapiens<220> <223> Cdk-we-hu ribozyme binding site
<400> 1329
aaatgaagtt ttacaggag

<212> DNA<213> Homo sapiens<220> <223> Cdk-we-hu ribozyme binding site
<400> 1330
aatgaagttt tacaggaga

<212> DNA<213> Homo sapiens<220> <223> Cdk-we-hu ribozyme binding site
<400> 1331
atgaagtttt acaggagaa

<212> DNA<213> Homo sapiens<220> <223> Cdk-we-hu ribozyme binding site
<400> 1332
tgaagtttta caggagaat

<212> DNA<213> Homo sapiens<220> <223> Cdk-we-hu ribozyme binding site
<400> 1333
caggagaatt atacccatc

<212> DNA<213> Homo sapiens<220> <223> Cdk-we-hu ribozyme binding site
<400> 1334
aggagaatta tacccatct

<212> DNA<213> Homo sapiens<220> <223> Cdk-we-hu ribozyme binding site
<400> 1335
gagaattata cccatctac

<212> DNA<213> Homo sapiens<220> <223> Cdk-we-hu ribozyme binding site
<400> 1336
tatacccatc taccaaaag

<212> DNA<213> Homo sapiens<220> <223> Cdk-we-hu ribozyme binding site
<400> 1337
tacccatcta ccaaaagca

<212> DNA<213> Homo sapiens<220> <223> Cdk-we-hu ribozyme binding site
<400> 1338
aaagcagata tttttgcgc

<212> DNA<213> Homo sapiens<220> <223> Cdk-we-hu ribozyme binding site
<400> 1339
agcagatatt tttgcgctt

<212> DNA<213> Homo sapiens<220> <223> Cdk-we-hu ribozyme binding site
<400> 1340
gcagatattt ttgcgcttg

<212> DNA<213> Homo sapiens<220> <223> Cdk-we-hu ribozyme binding site
<400> 1341
cagatatttt tgcgcttgc

<212> DNA<213> Homo sapiens<220> <223> Cdk-we-hu ribozyme binding site
<400> 1342
agatattttt gcgcttgcc

<212> DNA<213> Homo sapiens<220> <223> Cdk-we-hu ribozyme binding site
<400> 1343
ttttgcgctt gccctcaca

<212> DNA<213> Homo sapiens<220> <223> Cdk-we-hu ribozyme binding site
<400> 1344
gcttgccctc acagtggta

<212> DNA<213> Homo sapiens<220> <223> Cdk-we-hu ribozyme binding site
<400> 1345
cacagtggta tgtgctgct

<212> DNA<213> Homo sapiens<220> <223> Cdk-we-hu ribozyme binding site
<400> 1346
gctgaacctc ttccgagaa

<212> DNA<213> Homo sapiens<220> <223> Cdk-we-hu ribozyme binding site
<400> 1347

tgaacctctt ccgagaaat

<212> DNA<213> Homo sapiens<220> <223> Cdk-we-hu ribozyme binding site
<400> 1348
gaacctcttc cgagaaatg

<212> DNA<213> Homo sapiens<220> <223> Cdk-we-hu ribozyme binding site
<400> 1349
aatggagatc aatggcatg

<212> DNA<213> Homo sapiens<220> <223> Cdk-we-hu ribozyme binding site
<400> 1350
gcatgaaatc agacagggt

<212> DNA<213> Homo sapiens<220> <223> Cdk-we-hu ribozyme binding site
<400> 1351
agacagggta gattacctc

<212> DNA<213> Homo sapiens<220> <223> Cdk-we-hu ribozyme binding site
<400> 1352
agggtagatt acctcggat

<212> DNA<213> Homo sapiens<220> <223> Cdk-we-hu ribozyme binding site
<400> 1353
gggtagatta cctcggata

<212> DNA<213> Homo sapiens<220> <223> Cdk-we-hu ribozyme binding site
<400> 1354
agattacctc ggataccac

<212> DNA<213> Homo sapiens<220> <223> Cdk-we-hu ribozyme binding site
<400> 1355
acctcggata ccacaagtg

<212> DNA<213> Homo sapiens<220> <223> Cdk-we-hu ribozyme binding site
<400> 1356
acaagtgctt tcccaagaa

<212> DNA<213> Homo sapiens<220> <223> Cdk-we-hu ribozyme binding site
<400> 1357
caagtgcttt cccaagaat

<212> DNA<213> Homo sapiens<220> <223> Cdk-we-hu ribozyme binding site
<400> 1358
aagtgctttc ccaagaatt

<212> DNA<213> Homo sapiens<220> <223> Cdk-we-hu ribozyme binding site
<400> 1359
cccaagaatt tacagagtt

<212> DNA<213> Homo sapiens<220> <223> Cdk-we-hu ribozyme binding site
<400> 1360
ccaagaattt acagagttg

<212> DNA<213> Homo sapiens<220> <223> Cdk-we-hu ribozyme binding site

<400> 1361
caagaattta cagagttgc

<212> DNA<213> Homo sapiens<220> <223> Cdk-we-hu ribozyme binding site
<400> 1362
ttacagagtt gctaaaagt

<212> DNA<213> Homo sapiens<220> <223> Cdk-we-hu ribozyme binding site
<400> 1363
agagttgcta aaagttatg

<212> DNA<213> Homo sapiens<220> <223> Cdk-we-hu ribozyme binding site
<400> 1364
gctaaaagtt atgattcat

<212> DNA<213> Homo sapiens<220> <223> Cdk-we-hu ribozyme binding site
<400> 1365
ctaaaagtta tgattcatc

<212> DNA<213> Homo sapiens<220> <223> Cdk-we-hu ribozyme binding site
<400> 1366
agttatgatt catccagat

<212> DNA<213> Homo sapiens<220> <223> Cdk-we-hu ribozyme binding site
<400> 1367
gttatgattc atccagatc

<212> DNA<213> Homo sapiens<220> <223> Cdk-we-hu ribozyme binding site
<400> 1368
atgattcatc cagatccag

<212> DNA<213> Homo sapiens<220> <223> Cdk-we-hu ribozyme binding site
<400> 1369
catccagatc cagagagaa

<212> DNA<213> Homo sapiens<220> <223> Cdk-we-hu ribozyme binding site
<400> 1370
agaagacctt cagcaatgg

<212> DNA<213> Homo sapiens<220> <223> Cdk-we-hu ribozyme binding site
<400> 1371
gaagaccttc agcaatggc

<212> DNA<213> Homo sapiens<220> <223> Cdk-we-hu ribozyme binding site
<400> 1372
ggcactggta aagcattca

<212> DNA<213> Homo sapiens<220> <223> Cdk-we-hu ribozyme binding site
<400> 1373
gtaaagcatt cagtattgc

<212> DNA<213> Homo sapiens<220> <223> Cdk-we-hu ribozyme binding site
<400> 1374
taaagcattc agtattgct

<212> DNA<213> Homo sapiens<220> <223> Cdk-we-hu ribozyme binding site
<400> 1375
gcattcagta ttgctgtcc

<212> DNA<213> Homo sapiens<220> <223> Cdk-we-hu ribozyme binding site
<400> 1376
attcagtatt gctgtccgc

<212> DNA<213> Homo sapiens<220> <223> Cdk-we-hu ribozyme binding site
<400> 1377
tattgctgtc cgcttctag

<212> DNA<213> Homo sapiens<220> <223> Cdk-we-hu ribozyme binding site
<400> 1378
ctgtccgctt ctagaaaga

<212> DNA<213> Homo sapiens<220> <223> Cdk-we-hu ribozyme binding site
<400> 1379
tgtccgcttc tagaaagag

<212> DNA<213> Homo sapiens<220> <223> Cdk-we-hu ribozyme binding site
<400> 1380
tccgcttcta gaaagagtg

<212> DNA<213> Homo sapiens<220> <223> Cdk-we-hu ribozyme binding site
<400> 1381
cagaacaatt acgaataga

<212> DNA<213> Homo sapiens<220> <223> Cdk-we-hu ribozyme binding site
<400> 1382
agaacaatta cgaatagaa

<212> DNA<213> Homo sapiens<220> <223> Cdk-we-hu ribozyme binding site
<400> 1383
attacgaata gaattgaat

<212> DNA<213> Homo sapiens<220> <223> Cdk-we-hu ribozyme binding site
<400> 1384
gaatagaatt gaatgccga

<212> DNA<213> Homo sapiens<220> <223> Cdk-we-hu ribozyme binding site
<400> 1385
ccgaaaagtt caaaaattc

<212> DNA<213> Homo sapiens<220> <223> Cdk-we-hu ribozyme binding site
<400> 1386
cgaaaagttc aaaaattca

<212> DNA<213> Homo sapiens<220> <223> Cdk-we-hu ribozyme binding site
<400> 1387
ttcaaaaatt cacttttac

<212> DNA<213> Homo sapiens<220> <223> Cdk-we-hu ribozyme binding site
<400> 1388
tcaaaaattc acttttaca

<212> DNA<213> Homo sapiens<220> <223> Cdk-we-hu ribozyme binding site
<400> 1389
aaattcactt ttacaaaaa

<212> DNA<213> Homo sapiens<220> <223> Cdk-we-hu ribozyme binding site
<400> 1390
aattcacttt tacaaaaag

<212> DNA<213> Homo sapiens<220> <223> Cdk-we-hu ribozyme binding site
<400> 1391
attcactttt acaaaaaga

<212> DNA<213> Homo sapiens<220> <223> Cdk-we-hu ribozyme binding site
<400> 1392
ttcactttta caaaaagaa

<212> DNA<213> Homo sapiens<220> <223> Cdk-we-hu ribozyme binding site
<400> 1393
aaaagaactc aagaaagca

<212> DNA<213> Homo sapiens<220> <223> Cdk-we-hu ribozyme binding site
<400> 1394
aagagcactc ttcactgac

<212> DNA<213> Homo sapiens<220> <223> Cdk-we-hu ribozyme binding site
<400> 1395
gagcactctt cactgaccg

<212> DNA<213> Homo sapiens<220> <223> Cdk-we-hu ribozyme binding site
<400> 1396
agcactcttc actgaccgg

<212> DNA<213> Homo sapiens<220> <223> Cdk-we-hu ribozyme binding site
<400> 1397
atggccacta ggtccacca

<212> DNA<213> Homo sapiens<220> <223> Cdk-we-hu ribozyme binding site
<400> 1398
acccagagta atagaacat

<212> DNA<213> Homo sapiens<220> <223> Cdk-we-hu ribozyme binding site
<400> 1399
cagagtaata gaacatctc

<212> DNA<213> Homo sapiens<220> <223> Cdk-we-hu ribozyme binding site
<400> 1400
atagaacatc tcgacttat

<212> DNA<213> Homo sapiens<220> <223> Cdk-we-hu ribozyme binding site
<400> 1401
agaacatctc gacttattg

<212> DNA<213> Homo sapiens<220> <223> Cdk-we-hu ribozyme binding site
<400> 1402

atctcgactt attggaaag

<212> DNA<213> Homo sapiens<220> <223> Cdk-we-hu ribozyme binding site
<400> 1403
tctcgactta ttggaaaga

<212> DNA<213> Homo sapiens<220> <223> Cdk-we-hu ribozyme binding site
<400> 1404
tcgacttatt ggaaagaaa

<212> DNA<213> Homo sapiens<220> <223> Cdk-we-hu ribozyme binding site
<400> 1405
tgaaccgctc tgtcagcct

<212> DNA<213> Homo sapiens<220> <223> Cdk-we-hu ribozyme binding site
<400> 1406
ccgctctgtc agccttact

<212> DNA<213> Homo sapiens<220> <223> Cdk-we-hu ribozyme binding site
<400> 1407
tgtcagcctt actatatac

<212> DNA<213> Homo sapiens<220> <223> Cdk-we-hu ribozyme binding site
<400> 1408
gtcagcctta ctatatact

<212> DNA<213> Homo sapiens<220> <223> Cyclin A2 ribozyme binding site
<400> 1409
tgggcaactc tgcgccggg

<212> DNA<213> Homo sapiens<220> <223> Cyclin A2 ribozyme binding site
<400> 1410
aggcgggctc ggcgctgct

<212> DNA<213> Homo sapiens<220> <223> Cyclin A2 ribozyme binding site
<400> 1411
ggcgctgcta gcattgcag

<212> DNA<213> Homo sapiens<220> <223> Cyclin A2 ribozyme binding site
<400> 1412
tgctagcatt gcagcagac

<212> DNA<213> Homo sapiens<220> <223> Cyclin A2 ribozyme binding site
<400> 1413
gacggcgctc caagaggac

<212> DNA<213> Homo sapiens<220> <223> Cyclin A2 ribozyme binding site
<400> 1414
caggagaata tcaacccgg

<212> DNA<213> Homo sapiens<220> <223> Cyclin A2 ribozyme binding site
<400> 1415
ggagaatatc aacccggaa

<212> DNA<213> Homo sapiens<220> <223> Cyclin A2 ribozyme binding site

<400> 1416
agcgcccgtc caacaaccg

<212> DNA<213> Homo sapiens<220> <223> Cyclin A2 ribozyme binding site
<400> 1417
gctggcggta ctgaagtcc

<212> DNA<213> Homo sapiens<220> <223> Cyclin A2 ribozyme binding site
<400> 1418
tactgaagtc cgggaaccc

<212> DNA<213> Homo sapiens<220> <223> Cyclin A2 ribozyme binding site
<400> 1419
ccgcggggtc tagcgcagc

<212> DNA<213> Homo sapiens<220> <223> Cyclin A2 ribozyme binding site
<400> 1420
gcggggtcta gcgcagcag

<212> DNA<213> Homo sapiens<220> <223> Cyclin A2 ribozyme binding site
<400> 1421
gagacgggtt gcaccccctt

<212> DNA<213> Homo sapiens<220> <223> Cyclin A2 ribozyme binding site
<400> 1422
tgcacccctt aaggatctt

<212> DNA<213> Homo sapiens<220> <223> Cyclin A2 ribozyme binding site
<400> 1423
gcacccctta aggatcttc

<212> DNA<213> Homo sapiens<220> <223> Cyclin A2 ribozyme binding site
<400> 1424
cttaaggatc ttcctgtaa

<212> DNA<213> Homo sapiens<220> <223> Cyclin A2 ribozyme binding site
<400> 1425
taaggatctt cctgtaaat

<212> DNA<213> Homo sapiens<220> <223> Cyclin A2 ribozyme binding site
<400> 1426
aaggatcttc ctgtaaatg

<212> DNA<213> Homo sapiens<220> <223> Cyclin A2 ribozyme binding site
<400> 1427
tcttcctgta aatgatgag

<212> DNA<213> Homo sapiens<220> <223> Cyclin A2 ribozyme binding site
<400> 1428
tgagcatgtc accgttcct

<212> DNA<213> Homo sapiens<220> <223> Cyclin A2 ribozyme binding site
<400> 1429
tgtcaccgtt cctccttgg

<212> DNA<213> Homo sapiens<220> <223> Cyclin A2 ribozyme binding site
<400> 1430
gtcaccgttc ctccttgga

<212> DNA<213> Homo sapiens<220> <223> Cyclin A2 ribozyme binding site
<400> 1431
accgttcctc cttggaaag

<212> DNA<213> Homo sapiens<220> <223> Cyclin A2 ribozyme binding site
<400> 1432
gttcctcctt ggaaagcaa

<212> DNA<213> Homo sapiens<220> <223> Cyclin A2 ribozyme binding site
<400> 1433
gcaaacagta aacagcctg

<212> DNA<213> Homo sapiens<220> <223> Cyclin A2 ribozyme binding site
<400> 1434
agcctgcgtt caccattca

<212> DNA<213> Homo sapiens<220> <223> Cyclin A2 ribozyme binding site
<400> 1435
gcctgcgttc accattcat

<212> DNA<213> Homo sapiens<220> <223> Cyclin A2 ribozyme binding site
<400> 1436
gttcaccatt catgtggat

<212> DNA<213> Homo sapiens<220> <223> Cyclin A2 ribozyme binding site
<400> 1437
ttcaccattc atgtggatg

<212> DNA<213> Homo sapiens<220> <223> Cyclin A2 ribozyme binding site
<400> 1438
aaagaagctc agaagaagc

<212> DNA<213> Homo sapiens<220> <223> Cyclin A2 ribozyme binding site
<400> 1439
cagctgaatc tcaaaaaat

<212> DNA<213> Homo sapiens<220> <223> Cyclin A2 ribozyme binding site
<400> 1440
gctgaatctc aaaaaatag

<212> DNA<213> Homo sapiens<220> <223> Cyclin A2 ribozyme binding site
<400> 1441
tcaaaaaata gagcgtgaa

<212> DNA<213> Homo sapiens<220> <223> Cyclin A2 ribozyme binding site
<400> 1442
gccctggctt ttaattcag

<212> DNA<213> Homo sapiens<220> <223> Cyclin A2 ribozyme binding site
<400> 1443
ccctggcttt taattcagc

<212> DNA<213> Homo sapiens<220> <223> Cyclin A2 ribozyme binding site
<400> 1444
cctggctttt aattcagcc

<212> DNA<213> Homo sapiens<220> <223> Cyclin A2 ribozyme binding site
<400> 1445
ctggctttta attcagcca

<212> DNA<213> Homo sapiens<220> <223> Cyclin A2 ribozyme binding site
<400> 1446
gcttttaatt cagccatta

<212> DNA<213> Homo sapiens<220> <223> Cyclin A2 ribozyme binding site
<400> 1447
cttttaattc agccattag

<212> DNA<213> Homo sapiens<220> <223> Cyclin A2 ribozyme binding site
<400> 1448
ttcagccatt agtttacct

<212> DNA<213> Homo sapiens<220> <223> Cyclin A2 ribozyme binding site
<400> 1449
tcagccatta gtttacctg

<212> DNA<213> Homo sapiens<220> <223> Cyclin A2 ribozyme binding site
<400> 1450
gccattagtt tacctggac

<212> DNA<213> Homo sapiens<220> <223> Cyclin A2 ribozyme binding site
<400> 1451
ccattagttt acctggacc

<212> DNA<213> Homo sapiens<220> <223> Cyclin A2 ribozyme binding site
<400> 1452
cattagttta cctggaccc

<212> DNA<213> Homo sapiens<220> <223> Cyclin A2 ribozyme binding site
<400> 1453
gaaaaccatt ggtccctct

<212> DNA<213> Homo sapiens<220> <223> Cyclin A2 ribozyme binding site
<400> 1454
accattggtc cctcttgat

<212> DNA<213> Homo sapiens<220> <223> Cyclin A2 ribozyme binding site
<400> 1455
ttggtccctc ttgattatc

<212> DNA<213> Homo sapiens<220> <223> Cyclin A2 ribozyme binding site
<400> 1456
ggtccctctt gattatcca

<212> DNA<213> Homo sapiens<220> <223> Cyclin A2 ribozyme binding site
<400> 1457

cctcttgatt atccaatgg

<212> DNA<213> Homo sapiens<220> <223> Cyclin A2 ribozyme binding site
<400> 1458
ctcttgatta tccaatgga

<212> DNA<213> Homo sapiens<220> <223> Cyclin A2 ribozyme binding site
<400> 1459
cttgattatc caatggatg

<212> DNA<213> Homo sapiens<220> <223> Cyclin A2 ribozyme binding site
<400> 1460
atggatggta gttttgagt

<212> DNA<213> Homo sapiens<220> <223> Cyclin A2 ribozyme binding site
<400> 1461
gatggtagtt ttgagtcac

<212> DNA<213> Homo sapiens<220> <223> Cyclin A2 ribozyme binding site
<400> 1462
atggtagttt tgagtcacc

<212> DNA<213> Homo sapiens<220> <223> Cyclin A2 ribozyme binding site
<400> 1463
tggtagtttt gagtcacca

<212> DNA<213> Homo sapiens<220> <223> Cyclin A2 ribozyme binding site
<400> 1464
gttttgagtc accacatac

<212> DNA<213> Homo sapiens<220> <223> Cyclin A2 ribozyme binding site
<400> 1465
tcaccacata ctatggaca

<212> DNA<213> Homo sapiens<220> <223> Cyclin A2 ribozyme binding site
<400> 1466
ccacatacta tggacatgt

<212> DNA<213> Homo sapiens<220> <223> Cyclin A2 ribozyme binding site
<400> 1467
tggacatgtc aattgtatt

<212> DNA<213> Homo sapiens<220> <223> Cyclin A2 ribozyme binding site
<400> 1468
catgtcaatt gtattagaa

<212> DNA<213> Homo sapiens<220> <223> Cyclin A2 ribozyme binding site
<400> 1469
caattgtatt agaagatga

<212> DNA<213> Homo sapiens<220> <223> Cyclin A2 ribozyme binding site
<400> 1470
aattgtatta gaagatgaa

<212> DNA<213> Homo sapiens<220> <223> Cyclin A2 ribozyme binding site

<400> 1471
agtgagtgtt aatgaagta

<212> DNA<213> Homo sapiens<220> <223> Cyclin A2 ribozyme binding site
<400> 1472
gtgagtgtta atgaagtac

<212> DNA<213> Homo sapiens<220> <223> Cyclin A2 ribozyme binding site
<400> 1473
taatgaagta ccagactac

<212> DNA<213> Homo sapiens<220> <223> Cyclin A2 ribozyme binding site
<400> 1474
taccagacta ccatgagga

<212> DNA<213> Homo sapiens<220> <223> Cyclin A2 ribozyme binding site
<400> 1475
catgaggata ttcacacat

<212> DNA<213> Homo sapiens<220> <223> Cyclin A2 ribozyme binding site
<400> 1476
tgaggatatt cacacatac

<212> DNA<213> Homo sapiens<220> <223> Cyclin A2 ribozyme binding site
<400> 1477
gaggatattc acacatacc

<212> DNA<213> Homo sapiens<220> <223> Cyclin A2 ribozyme binding site
<400> 1478
ttcacacata ccttaggga

<212> DNA<213> Homo sapiens<220> <223> Cyclin A2 ribozyme binding site
<400> 1479
cacatacctt agggaaatg

<212> DNA<213> Homo sapiens<220> <223> Cyclin A2 ribozyme binding site
<400> 1480
acatacctta gggaaatgg

<212> DNA<213> Homo sapiens<220> <223> Cyclin A2 ribozyme binding site
<400> 1481
aatggaggtt aaatgtaaa

<212> DNA<213> Homo sapiens<220> <223> Cyclin A2 ribozyme binding site
<400> 1482
atggaggtta aatgtaaac

<212> DNA<213> Homo sapiens<220> <223> Cyclin A2 ribozyme binding site
<400> 1483
gttaaatgta aacctaaag

<212> DNA<213> Homo sapiens<220> <223> Cyclin A2 ribozyme binding site
<400> 1484
tgtaaaccta aagtgggtt

<212> DNA<213> Homo sapiens<220> <223> Cyclin A2 ribozyme binding site
<400> 1485
aaagtgggtt acatgaaga

<212> DNA<213> Homo sapiens<220> <223> Cyclin A2 ribozyme binding site
<400> 1486
aagtgggtta catgaagaa

<212> DNA<213> Homo sapiens<220> <223> Cyclin A2 ribozyme binding site
<400> 1487
gccagacatc actaacagt

<212> DNA<213> Homo sapiens<220> <223> Cyclin A2 ribozyme binding site
<400> 1488
gacatcacta acagtatga

<212> DNA<213> Homo sapiens<220> <223> Cyclin A2 ribozyme binding site
<400> 1489
actaacagta tgagagcta

<212> DNA<213> Homo sapiens<220> <223> Cyclin A2 ribozyme binding site
<400> 1490
atgagagcta tcctcgtgg

<212> DNA<213> Homo sapiens<220> <223> Cyclin A2 ribozyme binding site
<400> 1491
gagagctatc ctcgtggac

<212> DNA<213> Homo sapiens<220> <223> Cyclin A2 ribozyme binding site
<400> 1492
agctatcctc gtggactgg

<212> DNA<213> Homo sapiens<220> <223> Cyclin A2 ribozyme binding site
<400> 1493
tggactggtt agttgaagt

<212> DNA<213> Homo sapiens<220> <223> Cyclin A2 ribozyme binding site
<400> 1494
ggactggtta gttgaagta

<212> DNA<213> Homo sapiens<220> <223> Cyclin A2 ribozyme binding site
<400> 1495
ctggttagtt gaagtagga

<212> DNA<213> Homo sapiens<220> <223> Cyclin A2 ribozyme binding site
<400> 1496
agttgaagta ggagaagaa

<212> DNA<213> Homo sapiens<220> <223> Cyclin A2 ribozyme binding site
<400> 1497
gagaagaata taaactaca

<212> DNA<213> Homo sapiens<220> <223> Cyclin A2 ribozyme binding site
<400> 1498
gaagaatata aactacaga

<212> DNA<213> Homo sapiens<220> <223> Cyclin A2 ribozyme binding site
<400> 1499
atataaacta cagaatgag

<212> DNA<213> Homo sapiens<220> <223> Cyclin A2 ribozyme binding site
<400> 1500
accctgcatt tggctgtga

<212> DNA<213> Homo sapiens<220> <223> Cyclin A2 ribozyme binding site
<400> 1501
ccctgcattt ggctgtgaa

<212> DNA<213> Homo sapiens<220> <223> Cyclin A2 ribozyme binding site
<400> 1502
ctgtgaacta cattgatag

<212> DNA<213> Homo sapiens<220> <223> Cyclin A2 ribozyme binding site
<400> 1503
gaactacatt gataggttc

<212> DNA<213> Homo sapiens<220> <223> Cyclin A2 ribozyme binding site
<400> 1504
tacattgata ggttcctgt

<212> DNA<213> Homo sapiens<220> <223> Cyclin A2 ribozyme binding site
<400> 1505
ttgataggtt cctgtcttc

<212> DNA<213> Homo sapiens<220> <223> Cyclin A2 ribozyme binding site
<400> 1506
tgataggttc ctgtcttcc

<212> DNA<213> Homo sapiens<220> <223> Cyclin A2 ribozyme binding site
<400> 1507
ggttcctgtc ttccatgtc

<212> DNA<213> Homo sapiens<220> <223> Cyclin A2 ribozyme binding site
<400> 1508
ttcctgtctt ccatgtcag

<212> DNA<213> Homo sapiens<220> <223> Cyclin A2 ribozyme binding site
<400> 1509
tcctgtcttc catgtcagt

<212> DNA<213> Homo sapiens<220> <223> Cyclin A2 ribozyme binding site
<400> 1510
cttccatgtc agtgctgag

<212> DNA<213> Homo sapiens<220> <223> Cyclin A2 ribozyme binding site
<400> 1511
aggaaaactt cagcttgtg

<212> DNA<213> Homo sapiens<220> <223> Cyclin A2 ribozyme binding site
<400> 1512

ggaaaacttc agcttgtgg

<212> DNA<213> Homo sapiens<220> <223> Cyclin A2 ribozyme binding site
<400> 1513
acttcagctt gtgggcact

<212> DNA<213> Homo sapiens<220> <223> Cyclin A2 ribozyme binding site
<400> 1514
actgctgcta tgctgttag

<212> DNA<213> Homo sapiens<220> <223> Cyclin A2 ribozyme binding site
<400> 1515
ctatgctgtt agcctcaaa

<212> DNA<213> Homo sapiens<220> <223> Cyclin A2 ribozyme binding site
<400> 1516
tatgctgtta gcctcaaag

<212> DNA<213> Homo sapiens<220> <223> Cyclin A2 ribozyme binding site
<400> 1517
tgttagcctc aaagtttga

<212> DNA<213> Homo sapiens<220> <223> Cyclin A2 ribozyme binding site
<400> 1518
cctcaaagtt tgaagaaat

<212> DNA<213> Homo sapiens<220> <223> Cyclin A2 ribozyme binding site
<400> 1519
ctcaaagttt gaagaaata

<212> DNA<213> Homo sapiens<220> <223> Cyclin A2 ribozyme binding site
<400> 1520
tgaagaaata tacccccca

<212> DNA<213> Homo sapiens<220> <223> Cyclin A2 ribozyme binding site
<400> 1521
aagaaatata cccccccaga

<212> DNA<213> Homo sapiens<220> <223> Cyclin A2 ribozyme binding site
<400> 1522
cccagaagta gcagagttt

<212> DNA<213> Homo sapiens<220> <223> Cyclin A2 ribozyme binding site
<400> 1523
tagcagagtt tgtgtacat

<212> DNA<213> Homo sapiens<220> <223> Cyclin A2 ribozyme binding site
<400> 1524
agcagagttt gtgtacatt

<212> DNA<213> Homo sapiens<220> <223> Cyclin A2 ribozyme binding site
<400> 1525
agtttgtgta cattacaga

<212> DNA<213> Homo sapiens<220> <223> Cyclin A2 ribozyme binding site

<400> 1526
tgtgtacatt acagatgat

<212> DNA<213> Homo sapiens<220> <223> Cyclin A2 ribozyme binding site
<400> 1527
gtgtacatta cagatgata

<212> DNA<213> Homo sapiens<220> <223> Cyclin A2 ribozyme binding site
<400> 1528
acagatgata cctacacca

<212> DNA<213> Homo sapiens<220> <223> Cyclin A2 ribozyme binding site
<400> 1529
atgataccta caccaagaa

<212> DNA<213> Homo sapiens<220> <223> Cyclin A2 ribozyme binding site
<400> 1530
gaaacaagtt ctgagaatg

<212> DNA<213> Homo sapiens<220> <223> Cyclin A2 ribozyme binding site
<400> 1531
atggagcatc tagttttga

<212> DNA<213> Homo sapiens<220> <223> Cyclin A2 ribozyme binding site
<400> 1532
ggagcatcta gttttgaaa

<212> DNA<213> Homo sapiens<220> <223> Cyclin A2 ribozyme binding site
<400> 1533
gcatctagtt ttgaaagtc

<212> DNA<213> Homo sapiens<220> <223> Cyclin A2 ribozyme binding site
<400> 1534
catctagttt tgaaagtcc

<212> DNA<213> Homo sapiens<220> <223> Cyclin A2 ribozyme binding site
<400> 1535
atctagtttt gaaagtcct

<212> DNA<213> Homo sapiens<220> <223> Cyclin A2 ribozyme binding site
<400> 1536
tttgaaagtc cttactttt

<212> DNA<213> Homo sapiens<220> <223> Cyclin A2 ribozyme binding site
<400> 1537
gaaagtcctt acttttgac

<212> DNA<213> Homo sapiens<220> <223> Cyclin A2 ribozyme binding site
<400> 1538
aaagtcctta cttttgact

<212> DNA<213> Homo sapiens<220> <223> Cyclin A2 ribozyme binding site
<400> 1539
gtccttactt ttgacttag

<212> DNA<213> Homo sapiens<220> <223> Cyclin A2 ribozyme binding site
<400> 1540
tccttacttt tgacttagc

<212> DNA<213> Homo sapiens<220> <223> Cyclin A2 ribozyme binding site
<400> 1541
ccttactttt gacttagct

<212> DNA<213> Homo sapiens<220> <223> Cyclin A2 ribozyme binding site
<400> 1542
cttttgactt agctgctcc

<212> DNA<213> Homo sapiens<220> <223> Cyclin A2 ribozyme binding site
<400> 1543
ttttgactta gctgctcca

<212> DNA<213> Homo sapiens<220> <223> Cyclin A2 ribozyme binding site
<400> 1544
ttagctgctc caacagtaa

<212> DNA<213> Homo sapiens<220> <223> Cyclin A2 ribozyme binding site
<400> 1545
tccaacagta aatcagttt

<212> DNA<213> Homo sapiens<220> <223> Cyclin A2 ribozyme binding site
<400> 1546
acagtaaatc agtttctta

<212> DNA<213> Homo sapiens<220> <223> Cyclin A2 ribozyme binding site
<400> 1547
taaatcagtt tcttaccca

<212> DNA<213> Homo sapiens<220> <223> Cyclin A2 ribozyme binding site
<400> 1548
aaatcagttt cttacccaa

<212> DNA<213> Homo sapiens<220> <223> Cyclin A2 ribozyme binding site
<400> 1549
aatcagtttc ttacccaat

<212> DNA<213> Homo sapiens<220> <223> Cyclin A2 ribozyme binding site
<400> 1550
tcagtttctt acccaatac

<212> DNA<213> Homo sapiens<220> <223> Cyclin A2 ribozyme binding site
<400> 1551
cagtttctta cccaatact

<212> DNA<213> Homo sapiens<220> <223> Cyclin A2 ribozyme binding site
<400> 1552
ttacccaata ctttctgca

<212> DNA<213> Homo sapiens<220> <223> Cyclin A2 ribozyme binding site
<400> 1553
cccaatactt tctgcatca

<212> DNA<213> Homo sapiens<220> <223> Cyclin A2 ribozyme binding site
<400> 1554
ccaatacttt ctgcatcag

<212> DNA<213> Homo sapiens<220> <223> Cyclin A2 ribozyme binding site
<400> 1555
caatactttc tgcatcagc

<212> DNA<213> Homo sapiens<220> <223> Cyclin A2 ribozyme binding site
<400> 1556
tttctgcatc agcagcctg

<212> DNA<213> Homo sapiens<220> <223> Cyclin A2 ribozyme binding site
<400> 1557
ctgcaaagtt gaaagttta

<212> DNA<213> Homo sapiens<220> <223> Cyclin A2 ribozyme binding site
<400> 1558
gttgaaagtt tagcaatgt

<212> DNA<213> Homo sapiens<220> <223> Cyclin A2 ribozyme binding site
<400> 1559
ttgaaagttt agcaatgtt

<212> DNA<213> Homo sapiens<220> <223> Cyclin A2 ribozyme binding site
<400> 1560
tgaaagttta gcaatgttt

<212> DNA<213> Homo sapiens<220> <223> Cyclin A2 ribozyme binding site
<400> 1561
tagcaatgtt tttgggaga

<212> DNA<213> Homo sapiens<220> <223> Cyclin A2 ribozyme binding site
<400> 1562
agcaatgttt ttgggagaa

<212> DNA<213> Homo sapiens<220> <223> Cyclin A2 ribozyme binding site
<400> 1563
gcaatgtttt tgggagaat

<212> DNA<213> Homo sapiens<220> <223> Cyclin A2 ribozyme binding site
<400> 1564
caatgttttt gggagaatt

<212> DNA<213> Homo sapiens<220> <223> Cyclin A2 ribozyme binding site
<400> 1565
tgggagaatt aagtttgat

<212> DNA<213> Homo sapiens<220> <223> Cyclin A2 ribozyme binding site
<400> 1566
gggagaatta agtttgata

<212> DNA<213> Homo sapiens<220> <223> Cyclin A2 ribozyme binding site
<400> 1567

gaattaagtt tgatagatg

<212> DNA<213> Homo sapiens<220> <223> Cyclin A2 ribozyme binding site
<400> 1568
aattaagttt gatagatgc

<212> DNA<213> Homo sapiens<220> <223> Cyclin A2 ribozyme binding site
<400> 1569
aagtttgata gatgctgac

<212> DNA<213> Homo sapiens<220> <223> Cyclin A2 ribozyme binding site
<400> 1570
ctgacccata cctcaagta

<212> DNA<213> Homo sapiens<220> <223> Cyclin A2 ribozyme binding site
<400> 1571
cccatacctc aagtatttg

<212> DNA<213> Homo sapiens<220> <223> Cyclin A2 ribozyme binding site
<400> 1572
acctcaagta tttgccatc

<212> DNA<213> Homo sapiens<220> <223> Cyclin A2 ribozyme binding site
<400> 1573
ctcaagtatt tgccatcag

<212> DNA<213> Homo sapiens<220> <223> Cyclin A2 ribozyme binding site
<400> 1574
tcaagtattt gccatcagt

<212> DNA<213> Homo sapiens<220> <223> Cyclin A2 ribozyme binding site
<400> 1575
atttgccatc agttattgc

<212> DNA<213> Homo sapiens<220> <223> Cyclin A2 ribozyme binding site
<400> 1576
gccatcagtt attgctgga

<212> DNA<213> Homo sapiens<220> <223> Cyclin A2 ribozyme binding site
<400> 1577
ccatcagtta ttgctggag

<212> DNA<213> Homo sapiens<220> <223> Cyclin A2 ribozyme binding site
<400> 1578
atcagttatt gctggagct

<212> DNA<213> Homo sapiens<220> <223> Cyclin A2 ribozyme binding site
<400> 1579
gagctgcctt tcatttagc

<212> DNA<213> Homo sapiens<220> <223> Cyclin A2 ribozyme binding site
<400> 1580
agctgccttt catttagca

<212> DNA<213> Homo sapiens<220> <223> Cyclin A2 ribozyme binding site

<400> 1581
gctgcctttc atttagcac

<212> DNA<213> Homo sapiens<220> <223> Cyclin A2 ribozyme binding site
<400> 1582
gcctttcatt tagcactct

<212> DNA<213> Homo sapiens<220> <223> Cyclin A2 ribozyme binding site
<400> 1583
cctttcattt agcactcta

<212> DNA<213> Homo sapiens<220> <223> Cyclin A2 ribozyme binding site
<400> 1584
ctttcattta gcactctac

<212> DNA<213> Homo sapiens<220> <223> Cyclin A2 ribozyme binding site
<400> 1585
tttagcactc tacacagtc

<212> DNA<213> Homo sapiens<220> <223> Cyclin A2 ribozyme binding site
<400> 1586
tagcactcta cacagtcac

<212> DNA<213> Homo sapiens<220> <223> Cyclin A2 ribozyme binding site
<400> 1587
ctacacagtc acgggacaa

<212> DNA<213> Homo sapiens<220> <223> Cyclin A2 ribozyme binding site
<400> 1588
ggcctgaatc attaatacg

<212> DNA<213> Homo sapiens<220> <223> Cyclin A2 ribozyme binding site
<400> 1589
ctgaatcatt aatacgaaa

<212> DNA<213> Homo sapiens<220> <223> Cyclin A2 ribozyme binding site
<400> 1590
atcattaata cgaaagact

<212> DNA<213> Homo sapiens<220> <223> Cyclin A2 ribozyme binding site
<400> 1591
agactggata taccctgga

<212> DNA<213> Homo sapiens<220> <223> Cyclin A2 ribozyme binding site
<400> 1592
actggatata ccctggaaa

<212> DNA<213> Homo sapiens<220> <223> Cyclin A2 ribozyme binding site
<400> 1593
ctggaaagtc ttaagcctt

<212> DNA<213> Homo sapiens<220> <223> Cyclin A2 ribozyme binding site
<400> 1594
ggaaagtctt aagccttgt

<212> DNA<213> Homo sapiens<220> <223> Cyclin A2 ribozyme binding site
<400> 1595
gaaagtctta agccttgtc

<212> DNA<213> Homo sapiens<220> <223> Cyclin A2 ribozyme binding site
<400> 1596
cttaagcctt gtctcatgg

<212> DNA<213> Homo sapiens<220> <223> Cyclin A2 ribozyme binding site
<400> 1597
aagccttgtc tcatggacc

<212> DNA<213> Homo sapiens<220> <223> Cyclin A2 ribozyme binding site
<400> 1598
gccttgtctc atggacctt

<212> DNA<213> Homo sapiens<220> <223> Cyclin A2 ribozyme binding site
<400> 1599
catggacctt caccagacc

<212> DNA<213> Homo sapiens<220> <223> Cyclin A2 ribozyme binding site
<400> 1600
atggaccttc accagacct

<212> DNA<213> Homo sapiens<220> <223> Cyclin A2 ribozyme binding site
<400> 1601
accagaccta cctcaaagc

<212> DNA<213> Homo sapiens<220> <223> Cyclin A2 ribozyme binding site
<400> 1602
gacctacctc aaagcacca

<212> DNA<213> Homo sapiens<220> <223> Cyclin A2 ribozyme binding site
<400> 1603
cacaacagtc aataagaga

<212> DNA<213> Homo sapiens<220> <223> Cyclin A2 ribozyme binding site
<400> 1604
acagtcaata agagaaaag

<212> DNA<213> Homo sapiens<220> <223> Cyclin A2 ribozyme binding site
<400> 1605
gagaaaagta caaaaattc

<212> DNA<213> Homo sapiens<220> <223> Cyclin A2 ribozyme binding site
<400> 1606
tacaaaaatt caaagtatc

<212> DNA<213> Homo sapiens<220> <223> Cyclin A2 ribozyme binding site
<400> 1607
acaaaaattc aaagtatca

<212> DNA<213> Homo sapiens<220> <223> Cyclin A2 ribozyme binding site
<400> 1608
attcaaagta tcatggtgt

<212> DNA<213> Homo sapiens<220> <223> Cyclin A2 ribozyme binding site
<400> 1609
tcaaagtatc atggtgttt

<212> DNA<213> Homo sapiens<220> <223> Cyclin A2 ribozyme binding site
<400> 1610
tcatggtgtt tctctcctc

<212> DNA<213> Homo sapiens<220> <223> Cyclin A2 ribozyme binding site
<400> 1611
catggtgttt ctctcctca

<212> DNA<213> Homo sapiens<220> <223> Cyclin A2 ribozyme binding site
<400> 1612
atggtgtttc tctcctcaa

<212> DNA<213> Homo sapiens<220> <223> Cyclin A2 ribozyme binding site
<400> 1613
gtgtttctct cctcaaccc

<212> DNA<213> Homo sapiens<220> <223> Cyclin A2 ribozyme binding site
<400> 1614
ttctctcctc aacccacca

<212> DNA<213> Homo sapiens<220> <223> Cyclin C ribozyme binding site
<400> 1615
atggtcgctc cgcggccgc

<212> DNA<213> Homo sapiens<220> <223> Cyclin C ribozyme binding site
<400> 1616
gcggccgctc cgccgcgtg

<212> DNA<213> Homo sapiens<220> <223> Cyclin C ribozyme binding site
<400> 1617
cgtggtgctt ttttatcag

<212> DNA<213> Homo sapiens<220> <223> Cyclin C ribozyme binding site
<400> 1618
gtggtgcttt tttatcagg

<212> DNA<213> Homo sapiens<220> <223> Cyclin C ribozyme binding site
<400> 1619
tggtgctttt ttatcaggg

<212> DNA<213> Homo sapiens<220> <223> Cyclin C ribozyme binding site
<400> 1620
ggtgcttttt tatcagggc

<212> DNA<213> Homo sapiens<220> <223> Cyclin C ribozyme binding site
<400> 1621
gtgctttttt atcagggca

<212> DNA<213> Homo sapiens<220> <223> Cyclin C ribozyme binding site
<400> 1622

tgcttttta tcagggcaa

<212> DNA<213> Homo sapiens<220> <223> Cyclin C ribozyme binding site
<400> 1623
ctttttatc agggcaagc

<212> DNA<213> Homo sapiens<220> <223> Cyclin C ribozyme binding site
<400> 1624
aagctgtgtt ccatggcag

<212> DNA<213> Homo sapiens<220> <223> Cyclin C ribozyme binding site
<400> 1625
agctgtgttc catggcagg

<212> DNA<213> Homo sapiens<220> <223> Cyclin C ribozyme binding site
<400> 1626
cagggaactt ttggcagag

<212> DNA<213> Homo sapiens<220> <223> Cyclin C ribozyme binding site
<400> 1627
agggaacttt tggcagagc

<212> DNA<213> Homo sapiens<220> <223> Cyclin C ribozyme binding site
<400> 1628
gggaactttt ggcagagct

<212> DNA<213> Homo sapiens<220> <223> Cyclin C ribozyme binding site
<400> 1629
ggcagagctc ccactattt

<212> DNA<213> Homo sapiens<220> <223> Cyclin C ribozyme binding site
<400> 1630
gctcccacta tttgcaatg

<212> DNA<213> Homo sapiens<220> <223> Cyclin C ribozyme binding site
<400> 1631
tcccactatt tgcaatgga

<212> DNA<213> Homo sapiens<220> <223> Cyclin C ribozyme binding site
<400> 1632
cccactattt gcaatggat

<212> DNA<213> Homo sapiens<220> <223> Cyclin C ribozyme binding site
<400> 1633
gcaatggatt ttggataaa

<212> DNA<213> Homo sapiens<220> <223> Cyclin C ribozyme binding site
<400> 1634
caatggattt tggataaac

<212> DNA<213> Homo sapiens<220> <223> Cyclin C ribozyme binding site
<400> 1635
aatggatttt ggataaaca

<212> DNA<213> Homo sapiens<220> <223> Cyclin C ribozyme binding site

<400> 1636
atttggata aacaagatc

<212> DNA<213> Homo sapiens<220> <223> Cyclin C ribozyme binding site
<400> 1637
aaacaagatc tgttgaagg

<212> DNA<213> Homo sapiens<220> <223> Cyclin C ribozyme binding site
<400> 1638
aagatctgtg gaaggagcg

<212> DNA<213> Homo sapiens<220> <223> Cyclin C ribozyme binding site
<400> 1639
caaaaggatt taaagtttc

<212> DNA<213> Homo sapiens<220> <223> Cyclin C ribozyme binding site
<400> 1640
aaaaggattt aaagtttct

<212> DNA<213> Homo sapiens<220> <223> Cyclin C ribozyme binding site
<400> 1641
aaaggattta aagtttctc

<212> DNA<213> Homo sapiens<220> <223> Cyclin C ribozyme binding site
<400> 1642
atttaaagtt tctctcaga

<212> DNA<213> Homo sapiens<220> <223> Cyclin C ribozyme binding site
<400> 1643
tttaaagttt ctctcagag

<212> DNA<213> Homo sapiens<220> <223> Cyclin C ribozyme binding site
<400> 1644
ttaaagtttc tctcagagg

<212> DNA<213> Homo sapiens<220> <223> Cyclin C ribozyme binding site
<400> 1645
aaagtttctc tcagaggaa

<212> DNA<213> Homo sapiens<220> <223> Cyclin C ribozyme binding site
<400> 1646
agtttctctc agaggaaga

<212> DNA<213> Homo sapiens<220> <223> Cyclin C ribozyme binding site
<400> 1647
aggaagaata ttggaagtt

<212> DNA<213> Homo sapiens<220> <223> Cyclin C ribozyme binding site
<400> 1648
gaagaatatt ggaagttac

<212> DNA<213> Homo sapiens<220> <223> Cyclin C ribozyme binding site
<400> 1649
attggaagtt acaaatatt

<212> DNA<213> Homo sapiens<220> <223> Cyclin C ribozyme binding site
<400> 1650
ttggaagtta caaatattt

<212> DNA<213> Homo sapiens<220> <223> Cyclin C ribozyme binding site
<400> 1651
gttacaaata tttttaca

<212> DNA<213> Homo sapiens<220> <223> Cyclin C ribozyme binding site
<400> 1652
tacaaatatt ttttacaaa

<212> DNA<213> Homo sapiens<220> <223> Cyclin C ribozyme binding site
<400> 1653
acaaatattt tttacaaat

<212> DNA<213> Homo sapiens<220> <223> Cyclin C ribozyme binding site
<400> 1654
caaatatttt ttacaaatg

<212> DNA<213> Homo sapiens<220> <223> Cyclin C ribozyme binding site
<400> 1655
aaatattttt tacaaatgt

<212> DNA<213> Homo sapiens<220> <223> Cyclin C ribozyme binding site
<400> 1656
aatattttt acaaatgtt

<212> DNA<213> Homo sapiens<220> <223> Cyclin C ribozyme binding site
<400> 1657
atattttta caaatgtta

<212> DNA<213> Homo sapiens<220> <223> Cyclin C ribozyme binding site
<400> 1658
tacaaatgtt atccaagca

<212> DNA<213> Homo sapiens<220> <223> Cyclin C ribozyme binding site
<400> 1659
acaaatgtta tccaagcat

<212> DNA<213> Homo sapiens<220> <223> Cyclin C ribozyme binding site
<400> 1660
aaatgttatc caagcatta

<212> DNA<213> Homo sapiens<220> <223> Cyclin C ribozyme binding site
<400> 1661
tccaagcatt aggtgaaca

<212> DNA<213> Homo sapiens<220> <223> Cyclin C ribozyme binding site
<400> 1662
ccaagcatta ggtgaacat

<212> DNA<213> Homo sapiens<220> <223> Cyclin C ribozyme binding site
<400> 1663
ggtgaacatc ttaaattaa

<212> DNA<213> Homo sapiens<220> <223> Cyclin C ribozyme binding site
<400> 1664
tgaacatctt aaattaaga

<212> DNA<213> Homo sapiens<220> <223> Cyclin C ribozyme binding site
<400> 1665
gaacatctta aattaagac

<212> DNA<213> Homo sapiens<220> <223> Cyclin C ribozyme binding site
<400> 1666
atcttaaatt aagacaaca

<212> DNA<213> Homo sapiens<220> <223> Cyclin C ribozyme binding site
<400> 1667
tcttaaatta agacaacaa

<212> DNA<213> Homo sapiens<220> <223> Cyclin C ribozyme binding site
<400> 1668
acaacaagtt attgccact

<212> DNA<213> Homo sapiens<220> <223> Cyclin C ribozyme binding site
<400> 1669
caacaagtta ttgccactg

<212> DNA<213> Homo sapiens<220> <223> Cyclin C ribozyme binding site
<400> 1670
acaagttatt gccactgct

<212> DNA<213> Homo sapiens<220> <223> Cyclin C ribozyme binding site
<400> 1671
gccactgcta cggtatatt

<212> DNA<213> Homo sapiens<220> <223> Cyclin C ribozyme binding site
<400> 1672
tgctacggta tatttcaag

<212> DNA<213> Homo sapiens<220> <223> Cyclin C ribozyme binding site
<400> 1673
ctacggtata tttcaagag

<212> DNA<213> Homo sapiens<220> <223> Cyclin C ribozyme binding site
<400> 1674
acggtatatt tcaagagat

<212> DNA<213> Homo sapiens<220> <223> Cyclin C ribozyme binding site
<400> 1675
cggtatattt caagagatt

<212> DNA<213> Homo sapiens<220> <223> Cyclin C ribozyme binding site
<400> 1676
tcaagagatt ctatgccag

<212> DNA<213> Homo sapiens<220> <223> Cyclin C ribozyme binding site
<400> 1677

caagagattc tatgccagg

<212> DNA<213> Homo sapiens<220> <223> Cyclin C ribozyme binding site
<400> 1678
agagattcta tgccaggta

<212> DNA<213> Homo sapiens<220> <223> Cyclin C ribozyme binding site
<400> 1679
atgccaggta ttctctgaa

<212> DNA<213> Homo sapiens<220> <223> Cyclin C ribozyme binding site
<400> 1680
gccaggtatt ctctgaaaa

<212> DNA<213> Homo sapiens<220> <223> Cyclin C ribozyme binding site
<400> 1681
ccaggtattc tctgaaaag

<212> DNA<213> Homo sapiens<220> <223> Cyclin C ribozyme binding site
<400> 1682
aggtattctc tgaaaagta

<212> DNA<213> Homo sapiens<220> <223> Cyclin C ribozyme binding site
<400> 1683
ctgaaaagta tagatcctg

<212> DNA<213> Homo sapiens<220> <223> Cyclin C ribozyme binding site
<400> 1684
gaaaagtata gatcctgta

<212> DNA<213> Homo sapiens<220> <223> Cyclin C ribozyme binding site
<400> 1685
agtatagatc ctgtattaa

<212> DNA<213> Homo sapiens<220> <223> Cyclin C ribozyme binding site
<400> 1686
agatcctgta ttaatggct

<212> DNA<213> Homo sapiens<220> <223> Cyclin C ribozyme binding site
<400> 1687
atcctgtatt aatggctcc

<212> DNA<213> Homo sapiens<220> <223> Cyclin C ribozyme binding site
<400> 1688
tcctgtatta atggctcct

<212> DNA<213> Homo sapiens<220> <223> Cyclin C ribozyme binding site
<400> 1689
ttaatggctc ctacatgtg

<212> DNA<213> Homo sapiens<220> <223> Cyclin C ribozyme binding site
<400> 1690
atggctccta catgtgtgt

<212> DNA<213> Homo sapiens<220> <223> Cyclin C ribozyme binding site

<400> 1691
catgtgtgtt tttggcatc

<212> DNA<213> Homo sapiens<220> <223> Cyclin C ribozyme binding site
<400> 1692
atgtgtgttt ttggcatcc

<212> DNA<213> Homo sapiens<220> <223> Cyclin C ribozyme binding site
<400> 1693
tgtgtgtttt tggcatcca

<212> DNA<213> Homo sapiens<220> <223> Cyclin C ribozyme binding site
<400> 1694
gtgtgttttt ggcatccaa

<212> DNA<213> Homo sapiens<220> <223> Cyclin C ribozyme binding site
<400> 1695
ttttggcatc caaagtaga

<212> DNA<213> Homo sapiens<220> <223> Cyclin C ribozyme binding site
<400> 1696
atccaaagta gaggaattt

<212> DNA<213> Homo sapiens<220> <223> Cyclin C ribozyme binding site
<400> 1697
tagaggaatt tggagtagt

<212> DNA<213> Homo sapiens<220> <223> Cyclin C ribozyme binding site
<400> 1698
agaggaattt ggagtagtt

<212> DNA<213> Homo sapiens<220> <223> Cyclin C ribozyme binding site
<400> 1699
atttggagta gtttcaaat

<212> DNA<213> Homo sapiens<220> <223> Cyclin C ribozyme binding site
<400> 1700
tggagtagtt tcaaataca

<212> DNA<213> Homo sapiens<220> <223> Cyclin C ribozyme binding site
<400> 1701
ggagtagttt caaatacaa

<212> DNA<213> Homo sapiens<220> <223> Cyclin C ribozyme binding site
<400> 1702
gagtagtttc aaatacaag

<212> DNA<213> Homo sapiens<220> <223> Cyclin C ribozyme binding site
<400> 1703
gtttcaaata caagattga

<212> DNA<213> Homo sapiens<220> <223> Cyclin C ribozyme binding site
<400> 1704
atacaagatt gattgctgc

<212> DNA<213> Homo sapiens<220> <223> Cyclin C ribozyme binding site
<400> 1705
aagattgatt gctgctgct

<212> DNA<213> Homo sapiens<220> <223> Cyclin C ribozyme binding site
<400> 1706
gctgctgcta cttctgtat

<212> DNA<213> Homo sapiens<220> <223> Cyclin C ribozyme binding site
<400> 1707
gctgctactt ctgtattaa

<212> DNA<213> Homo sapiens<220> <223> Cyclin C ribozyme binding site
<400> 1708
ctgctacttc tgtattaaa

<212> DNA<213> Homo sapiens<220> <223> Cyclin C ribozyme binding site
<400> 1709
tacttctgta ttaaaaact

<212> DNA<213> Homo sapiens<220> <223> Cyclin C ribozyme binding site
<400> 1710
cttctgtatt aaaaactag

<212> DNA<213> Homo sapiens<220> <223> Cyclin C ribozyme binding site
<400> 1711
ttctgtatta aaaactaga

<212> DNA<213> Homo sapiens<220> <223> Cyclin C ribozyme binding site
<400> 1712
ttaaaaacta gattttcat

<212> DNA<213> Homo sapiens<220> <223> Cyclin C ribozyme binding site
<400> 1713
aaactagatt ttcatatgc

<212> DNA<213> Homo sapiens<220> <223> Cyclin C ribozyme binding site
<400> 1714
aactagattt tcatatgcc

<212> DNA<213> Homo sapiens<220> <223> Cyclin C ribozyme binding site
<400> 1715
actagatttt catatgcct

<212> DNA<213> Homo sapiens<220> <223> Cyclin C ribozyme binding site
<400> 1716
ctagattttc atatgcctt

<212> DNA<213> Homo sapiens<220> <223> Cyclin C ribozyme binding site
<400> 1717
gattttcata tgcctttcc

<212> DNA<213> Homo sapiens<220> <223> Cyclin C ribozyme binding site
<400> 1718
catatgcctt tccaaagga

<212> DNA<213> Homo sapiens<220> <223> Cyclin C ribozyme binding site
<400> 1719
atatgccttt ccaaaggaa

<212> DNA<213> Homo sapiens<220> <223> Cyclin C ribozyme binding site
<400> 1720
tatgcctttc caaaggaat

<212> DNA<213> Homo sapiens<220> <223> Cyclin C ribozyme binding site
<400> 1721
caaaggaatt tccttatag

<212> DNA<213> Homo sapiens<220> <223> Cyclin C ribozyme binding site
<400> 1722
aaaggaattt ccttatagg

<212> DNA<213> Homo sapiens<220> <223> Cyclin C ribozyme binding site
<400> 1723
aaggaatttc cttatagga

<212> DNA<213> Homo sapiens<220> <223> Cyclin C ribozyme binding site
<400> 1724
gaatttcctt ataggatga

<212> DNA<213> Homo sapiens<220> <223> Cyclin C ribozyme binding site
<400> 1725
aatttcctta taggatgaa

<212> DNA<213> Homo sapiens<220> <223> Cyclin C ribozyme binding site
<400> 1726
tttccttata ggatgaatc

<212> DNA<213> Homo sapiens<220> <223> Cyclin C ribozyme binding site
<400> 1727
aggatgaatc atatattag

<212> DNA<213> Homo sapiens<220> <223> Cyclin C ribozyme binding site
<400> 1728
atgaatcata tattagaat

<212> DNA<213> Homo sapiens<220> <223> Cyclin C ribozyme binding site
<400> 1729
gaatcatata ttagaatgt

<212> DNA<213> Homo sapiens<220> <223> Cyclin C ribozyme binding site
<400> 1730
atcatatatt agaatgtga

<212> DNA<213> Homo sapiens<220> <223> Cyclin C ribozyme binding site
<400> 1731
tcatatatta gaatgtgaa

<212> DNA<213> Homo sapiens<220> <223> Cyclin C ribozyme binding site
<400> 1732

aatgtgaatt ctatctgtt

<212> DNA<213> Homo sapiens<220> <223> Cyclin C ribozyme binding site
<400> 1733
atgtgaattc tatctgtta

<212> DNA<213> Homo sapiens<220> <223> Cyclin C ribozyme binding site
<400> 1734
gtgaattcta tctgttaga

<212> DNA<213> Homo sapiens<220> <223> Cyclin C ribozyme binding site
<400> 1735
gaattctatc tgttagaac

<212> DNA<213> Homo sapiens<220> <223> Cyclin C ribozyme binding site
<400> 1736
tctatctgtt agaactaat

<212> DNA<213> Homo sapiens<220> <223> Cyclin C ribozyme binding site
<400> 1737
ctatctgtta gaactaatg

<212> DNA<213> Homo sapiens<220> <223> Cyclin C ribozyme binding site
<400> 1738
gttagaacta atggattgt

<212> DNA<213> Homo sapiens<220> <223> Cyclin C ribozyme binding site
<400> 1739
ctaatggatt gttgcttga

<212> DNA<213> Homo sapiens<220> <223> Cyclin C ribozyme binding site
<400> 1740
atggattgtt gcttgatag

<212> DNA<213> Homo sapiens<220> <223> Cyclin C ribozyme binding site
<400> 1741
attgttgctt gatagtgta

<212> DNA<213> Homo sapiens<220> <223> Cyclin C ribozyme binding site
<400> 1742
ttgcttgata gtgtatcat

<212> DNA<213> Homo sapiens<220> <223> Cyclin C ribozyme binding site
<400> 1743
tgatagtgta tcatcctta

<212> DNA<213> Homo sapiens<220> <223> Cyclin C ribozyme binding site
<400> 1744
atagtgtatc atccttata

<212> DNA<213> Homo sapiens<220> <223> Cyclin C ribozyme binding site
<400> 1745
gtgtatcatc cttatagac

<212> DNA<213> Homo sapiens<220> <223> Cyclin C ribozyme binding site

<400> 1746
tatcatcctt atagacctt

<212> DNA<213> Homo sapiens<220> <223> Cyclin C ribozyme binding site
<400> 1747
atcatcctta tagaccttt

<212> DNA<213> Homo sapiens<220> <223> Cyclin C ribozyme binding site
<400> 1748
catccttata gacctttgc

<212> DNA<213> Homo sapiens<220> <223> Cyclin C ribozyme binding site
<400> 1749
tatagacctt tgctccagt

<212> DNA<213> Homo sapiens<220> <223> Cyclin C ribozyme binding site
<400> 1750
atagaccttt gctccagta

<212> DNA<213> Homo sapiens<220> <223> Cyclin C ribozyme binding site
<400> 1751
acctttgctc cagtatgtg

<212> DNA<213> Homo sapiens<220> <223> Cyclin C ribozyme binding site
<400> 1752
tgctccagta tgtgcagga

<212> DNA<213> Homo sapiens<220> <223> Cyclin C ribozyme binding site
<400> 1753
aagacatgtt gcttcccct

<212> DNA<213> Homo sapiens<220> <223> Cyclin C ribozyme binding site
<400> 1754
catgttgctt ccccttgca

<212> DNA<213> Homo sapiens<220> <223> Cyclin C ribozyme binding site
<400> 1755
atgttgcttc cccttgcat

<212> DNA<213> Homo sapiens<220> <223> Cyclin C ribozyme binding site
<400> 1756
gcttcccctt gcatggagg

<212> DNA<213> Homo sapiens<220> <223> Cyclin C ribozyme binding site
<400> 1757
atggaggata gtgaatgat

<212> DNA<213> Homo sapiens<220> <223> Cyclin C ribozyme binding site
<400> 1758
gtgaatgata cctacagaa

<212> DNA<213> Homo sapiens<220> <223> Cyclin C ribozyme binding site
<400> 1759
atgataccta cagaacgga

<212> DNA<213> Homo sapiens<220> <223> Cyclin C ribozyme binding site
<400> 1760
agaacggatc tttgcctac

<212> DNA<213> Homo sapiens<220> <223> Cyclin C ribozyme binding site
<400> 1761
aacggatctt tgcctactg

<212> DNA<213> Homo sapiens<220> <223> Cyclin C ribozyme binding site
<400> 1762
acggatcttt gcctactgt

<212> DNA<213> Homo sapiens<220> <223> Cyclin C ribozyme binding site
<400> 1763
tctttgccta ctgtatcct

<212> DNA<213> Homo sapiens<220> <223> Cyclin C ribozyme binding site
<400> 1764
gcctactgta tcctccttt

<212> DNA<213> Homo sapiens<220> <223> Cyclin C ribozyme binding site
<400> 1765
ctactgtatc ctcctttca

<212> DNA<213> Homo sapiens<220> <223> Cyclin C ribozyme binding site
<400> 1766
ctgtatcctc ctttcatga

<212> DNA<213> Homo sapiens<220> <223> Cyclin C ribozyme binding site
<400> 1767
tatcctcctt tcatgatag

<212> DNA<213> Homo sapiens<220> <223> Cyclin C ribozyme binding site
<400> 1768
atcctccttt catgatagc

<212> DNA<213> Homo sapiens<220> <223> Cyclin C ribozyme binding site
<400> 1769
tcctcctttc atgatagct

<212> DNA<213> Homo sapiens<220> <223> Cyclin C ribozyme binding site
<400> 1770
tttcatgata gctttagct

<212> DNA<213> Homo sapiens<220> <223> Cyclin C ribozyme binding site
<400> 1771
atgatagctt tagcttgcc

<212> DNA<213> Homo sapiens<220> <223> Cyclin C ribozyme binding site
<400> 1772
tgatagcttt agcttgcct

<212> DNA<213> Homo sapiens<220> <223> Cyclin C ribozyme binding site
<400> 1773
gatagcttta gcttgccta

<212> DNA<213> Homo sapiens<220> <223> Cyclin C ribozyme binding site
<400> 1774
gctttagctt gcctacatg

<212> DNA<213> Homo sapiens<220> <223> Cyclin C ribozyme binding site
<400> 1775
agcttgccta catgtagcc

<212> DNA<213> Homo sapiens<220> <223> Cyclin C ribozyme binding site
<400> 1776
cctacatgta gcctgtgtt

<212> DNA<213> Homo sapiens<220> <223> Cyclin C ribozyme binding site
<400> 1777
agcctgtgtt gtacagcag

<212> DNA<213> Homo sapiens<220> <223> Cyclin C ribozyme binding site
<400> 1778
ctgtgttgta cagcagaaa

<212> DNA<213> Homo sapiens<220> <223> Cyclin C ribozyme binding site
<400> 1779
ggcaatggtt tgctgagct

<212> DNA<213> Homo sapiens<220> <223> Cyclin C ribozyme binding site
<400> 1780
gcaatggttt gctgagctt

<212> DNA<213> Homo sapiens<220> <223> Cyclin C ribozyme binding site
<400> 1781
tgctgagctt tctgtggat

<212> DNA<213> Homo sapiens<220> <223> Cyclin C ribozyme binding site
<400> 1782
gctgagcttt ctgtggata

<212> DNA<213> Homo sapiens<220> <223> Cyclin C ribozyme binding site
<400> 1783
tctgtggata tggaaaaga

<212> DNA<213> Homo sapiens<220> <223> Cyclin C ribozyme binding site
<400> 1784
ggaaaagatt ttggaaata

<212> DNA<213> Homo sapiens<220> <223> Cyclin C ribozyme binding site
<400> 1785
gaaaagattt tggaaataa

<212> DNA<213> Homo sapiens<220> <223> Cyclin C ribozyme binding site
<400> 1786
aaaagatttt ggaaataat

<212> DNA<213> Homo sapiens<220> <223> Cyclin C ribozyme binding site
<400> 1787

tttggaaata atcagggtt

<212> DNA<213> Homo sapiens<220> <223> Cyclin C ribozyme binding site
<400> 1788
ggaaataatc agggttatt

<212> DNA<213> Homo sapiens<220> <223> Cyclin C ribozyme binding site
<400> 1789
aatcagggtt attttaaaa

<212> DNA<213> Homo sapiens<220> <223> Cyclin C ribozyme binding site
<400> 1790
atcagggtta ttttaaaac

<212> DNA<213> Homo sapiens<220> <223> Cyclin C ribozyme binding site
<400> 1791
cagggttatt ttaaaacta

<212> DNA<213> Homo sapiens<220> <223> Cyclin C ribozyme binding site
<400> 1792
agggttattt taaaactat

<212> DNA<213> Homo sapiens<220> <223> Cyclin C ribozyme binding site
<400> 1793
gggttatttt aaaactata

<212> DNA<213> Homo sapiens<220> <223> Cyclin C ribozyme binding site
<400> 1794
ggttatttta aaactatat

<212> DNA<213> Homo sapiens<220> <223> Cyclin C ribozyme binding site
<400> 1795
tttaaaacta tatgagcag

<212> DNA<213> Homo sapiens<220> <223> Cyclin C ribozyme binding site
<400> 1796
taaaactata tgagcagtg

<212> DNA<213> Homo sapiens<220> <223> Cyclin C ribozyme binding site
<400> 1797
tggaagaatt tcgatgaga

<212> DNA<213> Homo sapiens<220> <223> Cyclin C ribozyme binding site
<400> 1798
ggaagaattt cgatgagag

<212> DNA<213> Homo sapiens<220> <223> Cyclin C ribozyme binding site
<400> 1799
gaagaatttc gatgagaga

<212> DNA<213> Homo sapiens<220> <223> Cyclin C ribozyme binding site
<400> 1800
ggcaaccatt cttagtaag

<212> DNA<213> Homo sapiens<220> <223> Cyclin C ribozyme binding site

<400> 1801
gcaaccattc ttagtaaga

<212> DNA<213> Homo sapiens<220> <223> Cyclin C ribozyme binding site
<400> 1802
aaccattctt agtaagatg

<212> DNA<213> Homo sapiens<220> <223> Cyclin C ribozyme binding site
<400> 1803
accattctta gtaagatgc

<212> DNA<213> Homo sapiens<220> <223> Cyclin C ribozyme binding site
<400> 1804
attcttagta agatgccaa

<212> DNA<213> Homo sapiens<220> <223> Cyclin C ribozyme binding site
<400> 1805
aaaccacctc caaacagtg

<212> DNA<213> Homo sapiens<220> <223> Cyclin C ribozyme binding site
<400> 1806
gagcagggtc caaatggaa

<212> DNA<213> Homo sapiens<220> <223> Cyclin C ribozyme binding site
<400> 1807
aatggaagtc agaactcta

<212> DNA<213> Homo sapiens<220> <223> Cyclin C ribozyme binding site
<400> 1808
gtcagaactc tagctacag

<212> DNA<213> Homo sapiens<220> <223> Cyclin C ribozyme binding site
<400> 1809
cagaactcta gctacagcc

<212> DNA<213> Homo sapiens<220> <223> Cyclin C ribozyme binding site
<400> 1810
actctagcta cagccaatc

<212> DNA<213> Homo sapiens<220> <223> Cyclin C ribozyme binding site
<400> 1811
acagccaatc ttaaaacat

<212> DNA<213> Homo sapiens<220> <223> Cyclin C ribozyme binding site
<400> 1812
agccaatctt aaaacattc

<212> DNA<213> Homo sapiens<220> <223> Cyclin C ribozyme binding site
<400> 1813
gccaatctta aaacattcc

<212> DNA<213> Homo sapiens<220> <223> Cyclin C ribozyme binding site
<400> 1814
ttaaaacatt ccgaagaat

<212> DNA<213> Homo sapiens<220> <223> Cyclin C ribozyme binding site
<400> 1815
taaaacattc cgaagaatt

<212> DNA<213> Homo sapiens<220> <223> Cyclin C ribozyme binding site
<400> 1816
ccgaagaatt ccatagtgg

<212> DNA<213> Homo sapiens<220> <223> Cyclin C ribozyme binding site
<400> 1817
cgaagaattc catagtgga

<212> DNA<213> Homo sapiens<220> <223> Cyclin C ribozyme binding site
<400> 1818
gaattccata gtggaccac

<212> DNA<213> Homo sapiens<220> <223> Cyclin C ribozyme binding site
<400> 1819
tggaccactt ggaaataaa

<212> DNA<213> Homo sapiens<220> <223> Cyclin D1 ribozyme binding site
<400> 1820
acaccagctc ctgtgctgc

<212> DNA<213> Homo sapiens<220> <223> Cyclin D1 ribozyme binding site
<400> 1821
ggaaaccatc cgccgcgcg

<212> DNA<213> Homo sapiens<220> <223> Cyclin D1 ribozyme binding site
<400> 1822
gccgcgcgta ccccgatgc

<212> DNA<213> Homo sapiens<220> <223> Cyclin D1 ribozyme binding site
<400> 1823
tgccaacctc ctcaacgac

<212> DNA<213> Homo sapiens<220> <223> Cyclin D1 ribozyme binding site
<400> 1824
caacctcctc aacgaccgg

<212> DNA<213> Homo sapiens<220> <223> Cyclin D1 ribozyme binding site
<400> 1825
gcgcgccctc ggtgtccta

<212> DNA<213> Homo sapiens<220> <223> Cyclin D1 ribozyme binding site
<400> 1826
cctcggtgtc ctacttcaa

<212> DNA<213> Homo sapiens<220> <223> Cyclin D1 ribozyme binding site
<400> 1827
cggtgtccta cttcaaatg

<212> DNA<213> Homo sapiens<220> <223> Cyclin D1 ribozyme binding site
<400> 1828
tgtcctactt caaatgtgt

<212> DNA<213> Homo sapiens<220> <223> Cyclin D1 ribozyme binding site
<400> 1829
gtcctacttc aaatgtgtg

<212> DNA<213> Homo sapiens<220> <223> Cyclin D1 ribozyme binding site
<400> 1830
gaaggaggtc ctgccgtcc

<212> DNA<213> Homo sapiens<220> <223> Cyclin D1 ribozyme binding site
<400> 1831
tcctgccgtc catgcggaa

<212> DNA<213> Homo sapiens<220> <223> Cyclin D1 ribozyme binding site
<400> 1832
gcggaagatc gtcgccacc

<212> DNA<213> Homo sapiens<220> <223> Cyclin D1 ribozyme binding site
<400> 1833
gaagatcgtc gccacctgg

<212> DNA<213> Homo sapiens<220> <223> Cyclin D1 ribozyme binding site
<400> 1834
gctggaggtc tgcgaggaa

<212> DNA<213> Homo sapiens<220> <223> Cyclin D1 ribozyme binding site
<400> 1835
ggaggaggtc ttcccgctg

<212> DNA<213> Homo sapiens<220> <223> Cyclin D1 ribozyme binding site
<400> 1836
aggaggtctt cccgctggc

<212> DNA<213> Homo sapiens<220> <223> Cyclin D1 ribozyme binding site
<400> 1837
ggaggtcttc ccgctggcc

<212> DNA<213> Homo sapiens<220> <223> Cyclin D1 ribozyme binding site
<400> 1838
ccatgaacta cctggaccg

<212> DNA<213> Homo sapiens<220> <223> Cyclin D1 ribozyme binding site
<400> 1839
tggaccgctt cctgtcgct

<212> DNA<213> Homo sapiens<220> <223> Cyclin D1 ribozyme binding site
<400> 1840
ggaccgcttc ctgtcgctg

<212> DNA<213> Homo sapiens<220> <223> Cyclin D1 ribozyme binding site
<400> 1841
gcttcctgtc gctggagcc

<212> DNA<213> Homo sapiens<220> <223> Cyclin D1 ribozyme binding site
<400> 1842

ggggccactt gcatgttcg

<212> DNA<213> Homo sapiens<220> <223> Cyclin D1 ribozyme binding site
<400> 1843
cttgcatgtt cgtggcctc

<212> DNA<213> Homo sapiens<220> <223> Cyclin D1 ribozyme binding site
<400> 1844
ttgcatgttc gtggcctct

<212> DNA<213> Homo sapiens<220> <223> Cyclin D1 ribozyme binding site
<400> 1845
tcgtggcctc taagatgaa

<212> DNA<213> Homo sapiens<220> <223> Cyclin D1 ribozyme binding site
<400> 1846
gtggcctcta agatgaagg

<212> DNA<213> Homo sapiens<220> <223> Cyclin D1 ribozyme binding site
<400> 1847
ggagaccatc cccctgacg

<212> DNA<213> Homo sapiens<220> <223> Cyclin D1 ribozyme binding site
<400> 1848
gctgtgcatc tacaccgac

<212> DNA<213> Homo sapiens<220> <223> Cyclin D1 ribozyme binding site
<400> 1849
tgtgcatcta caccgacaa

<212> DNA<213> Homo sapiens<220> <223> Cyclin D1 ribozyme binding site
<400> 1850
ccgacaactc catccggcc

<212> DNA<213> Homo sapiens<220> <223> Cyclin D1 ribozyme binding site
<400> 1851
caactccatc cggcccgag

<212> DNA<213> Homo sapiens<220> <223> Cyclin D1 ribozyme binding site
<400> 1852
ggagctgctc ctggtgaac

<212> DNA<213> Homo sapiens<220> <223> Cyclin D1 ribozyme binding site
<400> 1853
gaacaagctc aagtggaac

<212> DNA<213> Homo sapiens<220> <223> Cyclin D1 ribozyme binding site
<400> 1854
ccgcacgatt tcattgaac

<212> DNA<213> Homo sapiens<220> <223> Cyclin D1 ribozyme binding site
<400> 1855
cgcacgattt cattgaaca

<212> DNA<213> Homo sapiens<220> <223> Cyclin D1 ribozyme binding site

<400> 1856
gcacgatttc attgaacac

<212> DNA<213> Homo sapiens<220> <223> Cyclin D1 ribozyme binding site
<400> 1857
cgatttcatt gaacacttc

<212> DNA<213> Homo sapiens<220> <223> Cyclin D1 ribozyme binding site
<400> 1858
ttgaacactt cctctccaa

<212> DNA<213> Homo sapiens<220> <223> Cyclin D1 ribozyme binding site
<400> 1859
tgaacacttc ctctccaaa

<212> DNA<213> Homo sapiens<220> <223> Cyclin D1 ribozyme binding site
<400> 1860
acacttcctc tccaaaatg

<212> DNA<213> Homo sapiens<220> <223> Cyclin D1 ribozyme binding site
<400> 1861
acttcctctc caaaatgcc

<212> DNA<213> Homo sapiens<220> <223> Cyclin D1 ribozyme binding site
<400> 1862
caaacagatc atccgcaaa

<212> DNA<213> Homo sapiens<220> <223> Cyclin D1 ribozyme binding site
<400> 1863
acagatcatc cgcaaacac

<212> DNA<213> Homo sapiens<220> <223> Cyclin D1 ribozyme binding site
<400> 1864
cgcagacctt cgttgccct

<212> DNA<213> Homo sapiens<220> <223> Cyclin D1 ribozyme binding site
<400> 1865
gcagaccttc gttgccctc

<212> DNA<213> Homo sapiens<220> <223> Cyclin D1 ribozyme binding site
<400> 1866
gaccttcgtt gccctctgt

<212> DNA<213> Homo sapiens<220> <223> Cyclin D1 ribozyme binding site
<400> 1867
cgttgccctc tgtgccaca

<212> DNA<213> Homo sapiens<220> <223> Cyclin D1 ribozyme binding site
<400> 1868
atgtgaagtt catttccaa

<212> DNA<213> Homo sapiens<220> <223> Cyclin D1 ribozyme binding site
<400> 1869
tgtgaagttc atttccaat

<212> DNA<213> Homo sapiens<220> <223> Cyclin D1 ribozyme binding site
<400> 1870
gaagttcatt tccaatccg

<212> DNA<213> Homo sapiens<220> <223> Cyclin D1 ribozyme binding site
<400> 1871
aagttcattt ccaatccgc

<212> DNA<213> Homo sapiens<220> <223> Cyclin D1 ribozyme binding site
<400> 1872
agttcatttc caatccgcc

<212> DNA<213> Homo sapiens<220> <223> Cyclin D1 ribozyme binding site
<400> 1873
atttccaatc cgccctcca

<212> DNA<213> Homo sapiens<220> <223> Cyclin D1 ribozyme binding site
<400> 1874
atccgccctc catggtggc

<212> DNA<213> Homo sapiens<220> <223> Cyclin D1 ribozyme binding site
<400> 1875
ccaacaactt cctgtccta

<212> DNA<213> Homo sapiens<220> <223> Cyclin D1 ribozyme binding site
<400> 1876
caacaacttc ctgtcctac

<212> DNA<213> Homo sapiens<220> <223> Cyclin D1 ribozyme binding site
<400> 1877
acttcctgtc ctactaccg

<212> DNA<213> Homo sapiens<220> <223> Cyclin D1 ribozyme binding site
<400> 1878
tcctgtccta ctaccgcct

<212> DNA<213> Homo sapiens<220> <223> Cyclin D1 ribozyme binding site
<400> 1879
tgtcctacta ccgcctcac

<212> DNA<213> Homo sapiens<220> <223> Cyclin D1 ribozyme binding site
<400> 1880
ctaccgcctc acacgcttc

<212> DNA<213> Homo sapiens<220> <223> Cyclin D1 ribozyme binding site
<400> 1881
tcacacgctt cctctccag

<212> DNA<213> Homo sapiens<220> <223> Cyclin D1 ribozyme binding site
<400> 1882
cacacgcttc ctctccaga

<212> DNA<213> Homo sapiens<220> <223> Cyclin D1 ribozyme binding site
<400> 1883
acgcttcctc tccagagtg

<212> DNA<213> Homo sapiens<220> <223> Cyclin D1 ribozyme binding site
<400> 1884
gcttcctctc cagagtgat

<212> DNA<213> Homo sapiens<220> <223> Cyclin D1 ribozyme binding site
<400> 1885
cagagtgatc aagtgtgac

<212> DNA<213> Homo sapiens<220> <223> Cyclin D1 ribozyme binding site
<400> 1886
ggactgcctc cgggcctgc

<212> DNA<213> Homo sapiens<220> <223> Cyclin D1 ribozyme binding site
<400> 1887
ggagcagatc gaagccctg

<212> DNA<213> Homo sapiens<220> <223> Cyclin D1 ribozyme binding site
<400> 1888
tgctggagtc aagcctgcg

<212> DNA<213> Homo sapiens<220> <223> Cyclin D1 ribozyme binding site
<400> 1889
gacctggctt gcacaccca

<212> DNA<213> Homo sapiens<220> <223> Cyclin D2 ribozyme binding site
<400> 1890
ggacccggtc cgcagggcc

<212> DNA<213> Homo sapiens<220> <223> Cyclin D2 ribozyme binding site
<400> 1891
caacctgctc cgagacgac

<212> DNA<213> Homo sapiens<220> <223> Cyclin D2 ribozyme binding site
<400> 1892
cgaccgcgtc ctgcagaac

<212> DNA<213> Homo sapiens<220> <223> Cyclin D2 ribozyme binding site
<400> 1893
gaacctgctc accatcgag

<212> DNA<213> Homo sapiens<220> <223> Cyclin D2 ribozyme binding site
<400> 1894
gctcaccatc gaggagcgc

<212> DNA<213> Homo sapiens<220> <223> Cyclin D2 ribozyme binding site
<400> 1895
aggagcgcta ccttccgca

<212> DNA<213> Homo sapiens<220> <223> Cyclin D2 ribozyme binding site
<400> 1896
gcgctacctt ccgcagtgc

<212> DNA<213> Homo sapiens<220> <223> Cyclin D2 ribozyme binding site
<400> 1897

cgctaccttc cgcagtgct

<212> DNA<213> Homo sapiens<220> <223> Cyclin D2 ribozyme binding site
<400> 1898
cgcagtgctc ctacttcaa

<212> DNA<213> Homo sapiens<220> <223> Cyclin D2 ribozyme binding site
<400> 1899
agtgctccta cttcaagtg

<212> DNA<213> Homo sapiens<220> <223> Cyclin D2 ribozyme binding site
<400> 1900
gctcctactt caagtgcgt

<212> DNA<213> Homo sapiens<220> <223> Cyclin D2 ribozyme binding site
<400> 1901
ctcctacttc aagtgcgtg

<212> DNA<213> Homo sapiens<220> <223> Cyclin D2 ribozyme binding site
<400> 1902
gaaggacatc caaccctac

<212> DNA<213> Homo sapiens<220> <223> Cyclin D2 ribozyme binding site
<400> 1903
tccaacccta catgcgcag

<212> DNA<213> Homo sapiens<220> <223> Cyclin D2 ribozyme binding site
<400> 1904
gctggaggtc tgtgaggaa

<212> DNA<213> Homo sapiens<220> <223> Cyclin D2 ribozyme binding site
<400> 1905
agaagaggtc ttccctctg

<212> DNA<213> Homo sapiens<220> <223> Cyclin D2 ribozyme binding site
<400> 1906
aagaggtctt ccctctggc

<212> DNA<213> Homo sapiens<220> <223> Cyclin D2 ribozyme binding site
<400> 1907
agaggtcttc cctctggcc

<212> DNA<213> Homo sapiens<220> <223> Cyclin D2 ribozyme binding site
<400> 1908
gtcttccctc tggccatga

<212> DNA<213> Homo sapiens<220> <223> Cyclin D2 ribozyme binding site
<400> 1909
gccatgaatt acctggacc

<212> DNA<213> Homo sapiens<220> <223> Cyclin D2 ribozyme binding site
<400> 1910
ccatgaatta cctggaccg

<212> DNA<213> Homo sapiens<220> <223> Cyclin D2 ribozyme binding site

<400> 1911
ctggaccgtt tcttggctg

<212> DNA<213> Homo sapiens<220> <223> Cyclin D2 ribozyme binding site
<400> 1912
tggaccgttt cttggctgg

<212> DNA<213> Homo sapiens<220> <223> Cyclin D2 ribozyme binding site
<400> 1913
ggaccgtttc ttggctggg

<212> DNA<213> Homo sapiens<220> <223> Cyclin D2 ribozyme binding site
<400> 1914
accgtttctt ggctggggt

<212> DNA<213> Homo sapiens<220> <223> Cyclin D2 ribozyme binding site
<400> 1915
ggctggggtc ccgactccg

<212> DNA<213> Homo sapiens<220> <223> Cyclin D2 ribozyme binding site
<400> 1916
gtcccgactc cgaagtccc

<212> DNA<213> Homo sapiens<220> <223> Cyclin D2 ribozyme binding site
<400> 1917
ctccgaagtc ccatctgca

<212> DNA<213> Homo sapiens<220> <223> Cyclin D2 ribozyme binding site
<400> 1918
aagtcccatc tgcaactcc

<212> DNA<213> Homo sapiens<220> <223> Cyclin D2 ribozyme binding site
<400> 1919
tctgcaactc ctgggtgct

<212> DNA<213> Homo sapiens<220> <223> Cyclin D2 ribozyme binding site
<400> 1920
gggtgctgtc tgcatgttc

<212> DNA<213> Homo sapiens<220> <223> Cyclin D2 ribozyme binding site
<400> 1921
tctgcatgtt cctggcctc

<212> DNA<213> Homo sapiens<220> <223> Cyclin D2 ribozyme binding site
<400> 1922
ctgcatgttc ctggcctcc

<212> DNA<213> Homo sapiens<220> <223> Cyclin D2 ribozyme binding site
<400> 1923
tcctggcctc caaactcaa

<212> DNA<213> Homo sapiens<220> <223> Cyclin D2 ribozyme binding site
<400> 1924
ctccaaactc aaagagacc

<212> DNA<213> Homo sapiens<220> <223> Cyclin D2 ribozyme binding site
<400> 1925
gctgtgcatt tacaccgac

<212> DNA<213> Homo sapiens<220> <223> Cyclin D2 ribozyme binding site
<400> 1926
ctgtgcattt acaccgaca

<212> DNA<213> Homo sapiens<220> <223> Cyclin D2 ribozyme binding site
<400> 1927
tgtgcattta caccgacaa

<212> DNA<213> Homo sapiens<220> <223> Cyclin D2 ribozyme binding site
<400> 1928
ccgacaactc catcaagcc

<212> DNA<213> Homo sapiens<220> <223> Cyclin D2 ribozyme binding site
<400> 1929
caactccatc aagcctcag

<212> DNA<213> Homo sapiens<220> <223> Cyclin D2 ribozyme binding site
<400> 1930
atcaagcctc aggagctgc

<212> DNA<213> Homo sapiens<220> <223> Cyclin D2 ribozyme binding site
<400> 1931
tggggaagtt gaagtggaa

<212> DNA<213> Homo sapiens<220> <223> Cyclin D2 ribozyme binding site
<400> 1932
ggcagctgtc actcctcat

<212> DNA<213> Homo sapiens<220> <223> Cyclin D2 ribozyme binding site
<400> 1933
gctgtcactc ctcatgact

<212> DNA<213> Homo sapiens<220> <223> Cyclin D2 ribozyme binding site
<400> 1934
gtcactcctc atgacttca

<212> DNA<213> Homo sapiens<220> <223> Cyclin D2 ribozyme binding site
<400> 1935
ctcatgactt cattgagca

<212> DNA<213> Homo sapiens<220> <223> Cyclin D2 ribozyme binding site
<400> 1936
tcatgacttc attgagcac

<212> DNA<213> Homo sapiens<220> <223> Cyclin D2 ribozyme binding site
<400> 1937
tgacttcatt gagcacatc

<212> DNA<213> Homo sapiens<220> <223> Cyclin D2 ribozyme binding site
<400> 1938
tgagcacatc ttgcgcaag

<212> DNA<213> Homo sapiens<220> <223> Cyclin D2 ribozyme binding site
<400> 1939
agcacatctt gcgcaagct

<212> DNA<213> Homo sapiens<220> <223> Cyclin D2 ribozyme binding site
<400> 1940
agaagctgtc tctgatccg

<212> DNA<213> Homo sapiens<220> <223> Cyclin D2 ribozyme binding site
<400> 1941
aagctgtctc tgatccgca

<212> DNA<213> Homo sapiens<220> <223> Cyclin D2 ribozyme binding site
<400> 1942
gtctctgatc cgcaagcat

<212> DNA<213> Homo sapiens<220> <223> Cyclin D2 ribozyme binding site
<400> 1943
aagcatgctc agaccttca

<212> DNA<213> Homo sapiens<220> <223> Cyclin D2 ribozyme binding site
<400> 1944
ctcagacctt cattgctct

<212> DNA<213> Homo sapiens<220> <223> Cyclin D2 ribozyme binding site
<400> 1945
tcagaccttc attgctctg

<212> DNA<213> Homo sapiens<220> <223> Cyclin D2 ribozyme binding site
<400> 1946
gaccttcatt gctctgtgt

<212> DNA<213> Homo sapiens<220> <223> Cyclin D2 ribozyme binding site
<400> 1947
ttcattgctc tgtgtgcca

<212> DNA<213> Homo sapiens<220> <223> Cyclin D2 ribozyme binding site
<400> 1948
ccaccgactt taagtttgc

<212> DNA<213> Homo sapiens<220> <223> Cyclin D2 ribozyme binding site
<400> 1949
caccgacttt aagtttgcc

<212> DNA<213> Homo sapiens<220> <223> Cyclin D2 ribozyme binding site
<400> 1950
accgacttta agtttgcca

<212> DNA<213> Homo sapiens<220> <223> Cyclin D2 ribozyme binding site
<400> 1951
actttaagtt tgccatgta

<212> DNA<213> Homo sapiens<220> <223> Cyclin D2 ribozyme binding site
<400> 1952

ctttaagttt gccatgtac

<212> DNA<213> Homo sapiens<220> <223> Cyclin D2 ribozyme binding site
<400> 1953
ttgccatgta cccaccgtc

<212> DNA<213> Homo sapiens<220> <223> Cyclin D2 ribozyme binding site
<400> 1954
acccaccgtc gatgatcgc

<212> DNA<213> Homo sapiens<220> <223> Cyclin D2 ribozyme binding site
<400> 1955
gtcgatgatc gcaactgga

<212> DNA<213> Homo sapiens<220> <223> Cyclin D2 ribozyme binding site
<400> 1956
agcagccatc tgtgggctc

<212> DNA<213> Homo sapiens<220> <223> Cyclin D2 ribozyme binding site
<400> 1957
ctgtgggctc cagcaggat

<212> DNA<213> Homo sapiens<220> <223> Cyclin D2 ribozyme binding site
<400> 1958
aagtgagctc gctcacttg

<212> DNA<213> Homo sapiens<220> <223> Cyclin D2 ribozyme binding site
<400> 1959
gagctcgctc acttgtgat

<212> DNA<213> Homo sapiens<220> <223> Cyclin D2 ribozyme binding site
<400> 1960
tcgctcactt gtgatgccc

<212> DNA<213> Homo sapiens<220> <223> Cyclin D2 ribozyme binding site
<400> 1961
ctgctggcta agatcacca

<212> DNA<213> Homo sapiens<220> <223> Cyclin D2 ribozyme binding site
<400> 1962
ggctaagatc accaacaca

<212> DNA<213> Homo sapiens<220> <223> Cyclin D2 ribozyme binding site
<400> 1963
gacgtggatt gtctcaaag

<212> DNA<213> Homo sapiens<220> <223> Cyclin D2 ribozyme binding site
<400> 1964
gtggattgtc tcaaagctt

<212> DNA<213> Homo sapiens<220> <223> Cyclin D2 ribozyme binding site
<400> 1965
ggattgtctc aaagcttgc

<212> DNA<213> Homo sapiens<220> <223> Cyclin D2 ribozyme binding site

<400> 1966
ctcaaagctt gccaggagc

<212> DNA<213> Homo sapiens<220> <223> Cyclin D2 ribozyme binding site
<400> 1967
ggagcagatt gaggcggtg

<212> DNA<213> Homo sapiens<220> <223> Cyclin D2 ribozyme binding site
<400> 1968
ggcggtgctc ctcaatagc

<212> DNA<213> Homo sapiens<220> <223> Cyclin D2 ribozyme binding site
<400> 1969
ctcctcaata gcctgcagc

<212> DNA<213> Homo sapiens<220> <223> Cyclin D2 ribozyme binding site
<400> 1970
tgcagcagta ccgtcagga

<212> DNA<213> Homo sapiens<220> <223> Cyclin D2 ribozyme binding site
<400> 1971
cagtaccgtc aggaccaac

<212> DNA<213> Homo sapiens<220> <223> Cyclin D2 ribozyme binding site
<400> 1972
gtgacggatc caagtcgga

<212> DNA<213> Homo sapiens<220> <223> Cyclin D2 ribozyme binding site
<400> 1973
gatccaagtc ggaggatga

<212> DNA<213> Homo sapiens<220> <223> Cyclin D2 ribozyme binding site
<400> 1974
agcaccccta cagacgtgc

<212> DNA<213> Homo sapiens<220> <223> Cyclin D2 ribozyme binding site
<400> 1975
gtgcgggata tcgacctgt

<212> DNA<213> Homo sapiens<220> <223> Cyclin D3 ribozyme binding site
<400> 1976
ctgctgtgtt gcgaaggca

<212> DNA<213> Homo sapiens<220> <223> Cyclin D3 ribozyme binding site
<400> 1977
ccagcgtgtc ctgcagagc

<212> DNA<213> Homo sapiens<220> <223> Cyclin D3 ribozyme binding site
<400> 1978
gagcctgctc cgcctggag

<212> DNA<213> Homo sapiens<220> <223> Cyclin D3 ribozyme binding site
<400> 1979
aggagcgcta cgtaccccg

<212> DNA<213> Homo sapiens<220> <223> Cyclin D3 ribozyme binding site
<400> 1980
gcgctacgta ccccgcgcc

<212> DNA<213> Homo sapiens<220> <223> Cyclin D3 ribozyme binding site
<400> 1981
cccgcgcctc ctacttcca

<212> DNA<213> Homo sapiens<220> <223> Cyclin D3 ribozyme binding site
<400> 1982
gcgcctccta cttccagtg

<212> DNA<213> Homo sapiens<220> <223> Cyclin D3 ribozyme binding site
<400> 1983
cctcctactt ccagtgcgt

<212> DNA<213> Homo sapiens<220> <223> Cyclin D3 ribozyme binding site
<400> 1984
ctcctacttc cagtgcgtg

<212> DNA<213> Homo sapiens<220> <223> Cyclin D3 ribozyme binding site
<400> 1985
gcgggagatc aagccgcac

<212> DNA<213> Homo sapiens<220> <223> Cyclin D3 ribozyme binding site
<400> 1986
atgctggctt actggatgc

<212> DNA<213> Homo sapiens<220> <223> Cyclin D3 ribozyme binding site
<400> 1987
tgctggctta ctggatgct

<212> DNA<213> Homo sapiens<220> <223> Cyclin D3 ribozyme binding site
<400> 1988
gctggaggta tgtgaggag

<212> DNA<213> Homo sapiens<220> <223> Cyclin D3 ribozyme binding site
<400> 1989
ggaggaagtc ttcccctg

<212> DNA<213> Homo sapiens<220> <223> Cyclin D3 ribozyme binding site
<400> 1990
aggaagtctt cccctggc

<212> DNA<213> Homo sapiens<220> <223> Cyclin D3 ribozyme binding site
<400> 1991
ggaagtcttc cccctggcc

<212> DNA<213> Homo sapiens<220> <223> Cyclin D3 ribozyme binding site
<400> 1992
ccatgaacta cctggatcg

<212> DNA<213> Homo sapiens<220> <223> Cyclin D3 ribozyme binding site
<400> 1993
tacctggatc gctacctgt

<212> DNA<213> Homo sapiens<220> <223> Cyclin D3 ribozyme binding site
<400> 1994
tggatcgcta cctgtcttg

<212> DNA<213> Homo sapiens<220> <223> Cyclin D3 ribozyme binding site
<400> 1995
gctacctgtc ttgcgtccc

<212> DNA<213> Homo sapiens<220> <223> Cyclin D3 ribozyme binding site
<400> 1996
tacctgtctt gcgtcccca

<212> DNA<213> Homo sapiens<220> <223> Cyclin D3 ribozyme binding site
<400> 1997
gtcttgcgtc cccacccga

<212> DNA<213> Homo sapiens<220> <223> Cyclin D3 ribozyme binding site
<400> 1998
aggcgcagtt gcagctcct

<212> DNA<213> Homo sapiens<220> <223> Cyclin D3 ribozyme binding site
<400> 1999
gttgcagctc ctgggtgcg

<212> DNA<213> Homo sapiens<220> <223> Cyclin D3 ribozyme binding site
<400> 2000
gggtgcggtc tgcatgctg

<212> DNA<213> Homo sapiens<220> <223> Cyclin D3 ribozyme binding site
<400> 2001
tgctggcctc caagctgcg

<212> DNA<213> Homo sapiens<220> <223> Cyclin D3 ribozyme binding site
<400> 2002
cctgaccatc gaaaaactg

<212> DNA<213> Homo sapiens<220> <223> Cyclin D3 ribozyme binding site
<400> 2003
actgtgcatc tacaccgac

<212> DNA<213> Homo sapiens<220> <223> Cyclin D3 ribozyme binding site
<400> 2004
tgtgcatcta caccgacca

<212> DNA<213> Homo sapiens<220> <223> Cyclin D3 ribozyme binding site
<400> 2005
ccacgctgtc tctccccgc

<212> DNA<213> Homo sapiens<220> <223> Cyclin D3 ribozyme binding site
<400> 2006
acgctgtctc tccccgcca

<212> DNA<213> Homo sapiens<220> <223> Cyclin D3 ribozyme binding site
<400> 2007

gctgtctctc cccgccagt

<212> DNA<213> Homo sapiens<220> <223> Cyclin D3 ribozyme binding site
<400> 2008
cccgccagtt gcgggactg

<212> DNA<213> Homo sapiens<220> <223> Cyclin D3 ribozyme binding site
<400> 2009
ggtgctggtc ctagggaag

<212> DNA<213> Homo sapiens<220> <223> Cyclin D3 ribozyme binding site
<400> 2010
gctggtccta gggaagctc

<212> DNA<213> Homo sapiens<220> <223> Cyclin D3 ribozyme binding site
<400> 2011
agggaagctc aagtgggac

<212> DNA<213> Homo sapiens<220> <223> Cyclin D3 ribozyme binding site
<400> 2012
tgctgtgatt gcacatgat

<212> DNA<213> Homo sapiens<220> <223> Cyclin D3 ribozyme binding site
<400> 2013
gcacatgatt tcctggcct

<212> DNA<213> Homo sapiens<220> <223> Cyclin D3 ribozyme binding site
<400> 2014
cacatgattt cctggcctt

<212> DNA<213> Homo sapiens<220> <223> Cyclin D3 ribozyme binding site
<400> 2015
acatgatttc ctggccttc

<212> DNA<213> Homo sapiens<220> <223> Cyclin D3 ribozyme binding site
<400> 2016
tcctggcctt cattctgca

<212> DNA<213> Homo sapiens<220> <223> Cyclin D3 ribozyme binding site
<400> 2017
cctggccttc attctgcac

<212> DNA<213> Homo sapiens<220> <223> Cyclin D3 ribozyme binding site
<400> 2018
ggccttcatt ctgcaccgg

<212> DNA<213> Homo sapiens<220> <223> Cyclin D3 ribozyme binding site
<400> 2019
gccttcattc tgcaccggc

<212> DNA<213> Homo sapiens<220> <223> Cyclin D3 ribozyme binding site
<400> 2020
gcaccggctc tctctgccc

<212> DNA<213> Homo sapiens<220> <223> Cyclin D3 ribozyme binding site

<400> 2021
accggctctc tctgccccg

<212> DNA<213> Homo sapiens<220> <223> Cyclin D3 ribozyme binding site
<400> 2022
cggctctctc tgccccgtg

<212> DNA<213> Homo sapiens<220> <223> Cyclin D3 ribozyme binding site
<400> 2023
gacaggcctt ggtcaaaaa

<212> DNA<213> Homo sapiens<220> <223> Cyclin D3 ribozyme binding site
<400> 2024
ggccttggtc aaaaagcat

<212> DNA<213> Homo sapiens<220> <223> Cyclin D3 ribozyme binding site
<400> 2025
cccagacctt tttggccct

<212> DNA<213> Homo sapiens<220> <223> Cyclin D3 ribozyme binding site
<400> 2026
ccagaccttt ttggccctc

<212> DNA<213> Homo sapiens<220> <223> Cyclin D3 ribozyme binding site
<400> 2027
cagacctttt tggccctct

<212> DNA<213> Homo sapiens<220> <223> Cyclin D3 ribozyme binding site
<400> 2028
agaccttttt ggccctctg

<212> DNA<213> Homo sapiens<220> <223> Cyclin D3 ribozyme binding site
<400> 2029
tttggccctc tgtgctaca

<212> DNA<213> Homo sapiens<220> <223> Cyclin D3 ribozyme binding site
<400> 2030
ctctgtgcta cagattata

<212> DNA<213> Homo sapiens<220> <223> Cyclin D3 ribozyme binding site
<400> 2031
gctacagatt atacctttg

<212> DNA<213> Homo sapiens<220> <223> Cyclin D3 ribozyme binding site
<400> 2032
ctacagatta tacctttgc

<212> DNA<213> Homo sapiens<220> <223> Cyclin D3 ribozyme binding site
<400> 2033
acagattata cctttgcca

<212> DNA<213> Homo sapiens<220> <223> Cyclin D3 ribozyme binding site
<400> 2034
attatacctt tgccatgta

<212> DNA<213> Homo sapiens<220> <223> Cyclin D3 ribozyme binding site
<400> 2035
ttataccttt gccatgtac

<212> DNA<213> Homo sapiens<220> <223> Cyclin D3 ribozyme binding site
<400> 2036
ttgccatgta cccgccatc

<212> DNA<213> Homo sapiens<220> <223> Cyclin D3 ribozyme binding site
<400> 2037
acccgccatc catgatcgc

<212> DNA<213> Homo sapiens<220> <223> Cyclin D3 ribozyme binding site
<400> 2038
atccatgatc gccacgggc

<212> DNA<213> Homo sapiens<220> <223> Cyclin D3 ribozyme binding site
<400> 2039
gggcagcatt ggggctgca

<212> DNA<213> Homo sapiens<220> <223> Cyclin D3 ribozyme binding site
<400> 2040
gtgcctgctc catgtccgg

<212> DNA<213> Homo sapiens<220> <223> Cyclin D3 ribozyme binding site
<400> 2041
gctccatgtc cggggatga

<212> DNA<213> Homo sapiens<220> <223> Cyclin D3 ribozyme binding site
<400> 2042
ggatgagctc acagagctg

<212> DNA<213> Homo sapiens<220> <223> Cyclin D3 ribozyme binding site
<400> 2043
ggcagggatc actggcact

<212> DNA<213> Homo sapiens<220> <223> Cyclin D3 ribozyme binding site
<400> 2044
cgggcctgtc aggagcaga

<212> DNA<213> Homo sapiens<220> <223> Cyclin D3 ribozyme binding site
<400> 2045
ggagcagatc gaagctgca

<212> DNA<213> Homo sapiens<220> <223> Cyclin D3 ribozyme binding site
<400> 2046
agctgcactc agggagagc

<212> DNA<213> Homo sapiens<220> <223> Cyclin D3 ribozyme binding site
<400> 2047
ggagagcctc agggaagcc

<212> DNA<213> Homo sapiens<220> <223> Cyclin D3 ribozyme binding site
<400> 2048
gaagccgctc agaccagct

<212> DNA<213> Homo sapiens<220> <223> Cyclin D3 ribozyme binding site
<400> 2049
agaccagctc cagcccagc

<212> DNA<213> Homo sapiens<220> <223> Cyclin D3 ribozyme binding site
<400> 2050
cccggggctc cagcagcca

<212> DNA<213> Homo sapiens<220> <223> Cyclin D3 ribozyme binding site
<400> 2051
accagcactc ctacagatg

<212> DNA<213> Homo sapiens<220> <223> Cyclin D3 ribozyme binding site
<400> 2052
agcactccta cagatgtca

<212> DNA<213> Homo sapiens<220> <223> Cyclin D3 ribozyme binding site
<400> 2053
tacagatgtc acagccata

<212> DNA<213> Homo sapiens<220> <223> Cyclin E ribozyme binding site
<400> 2054
gcgcggagtt ctcggctcg

<212> DNA<213> Homo sapiens<220> <223> Cyclin E ribozyme binding site
<400> 2055
cgcggagttc tcggctcgc

<212> DNA<213> Homo sapiens<220> <223> Cyclin E ribozyme binding site
<400> 2056
cggagttctc ggctcgctc

<212> DNA<213> Homo sapiens<220> <223> Cyclin E ribozyme binding site
<400> 2057
ttctcggctc gctccagga

<212> DNA<213> Homo sapiens<220> <223> Cyclin E ribozyme binding site
<400> 2058
cggctcgctc caggaagag

<212> DNA<213> Homo sapiens<220> <223> Cyclin E ribozyme binding site
<400> 2059
cgtgaccgtt tttttccag

<212> DNA<213> Homo sapiens<220> <223> Cyclin E ribozyme binding site
<400> 2060
gtgaccgttt ttttgcagg

<212> DNA<213> Homo sapiens<220> <223> Cyclin E ribozyme binding site
<400> 2061
tgaccgtttt tttgcagga

<212> DNA<213> Homo sapiens<220> <223> Cyclin E ribozyme binding site
<400> 2062

gaccgttttt ttgcaggat

<212> DNA<213> Homo sapiens<220> <223> Cyclin E ribozyme binding site
<400> 2063
accgtttttt tgcaggatc

<212> DNA<213> Homo sapiens<220> <223> Cyclin E ribozyme binding site
<400> 2064
ccgttttttt gcaggatcc

<212> DNA<213> Homo sapiens<220> <223> Cyclin E ribozyme binding site
<400> 2065
ttgcaggatc cagatgaag

<212> DNA<213> Homo sapiens<220> <223> Cyclin E ribozyme binding site
<400> 2066
ggccaaaatc gacaggacg

<212> DNA<213> Homo sapiens<220> <223> Cyclin E ribozyme binding site
<400> 2067
agccagcctt gggacaata

<212> DNA<213> Homo sapiens<220> <223> Cyclin E ribozyme binding site
<400> 2068
tgggacaata atgcagtct

<212> DNA<213> Homo sapiens<220> <223> Cyclin E ribozyme binding site
<400> 2069
taatgcagtc tgtgcagac

<212> DNA<213> Homo sapiens<220> <223> Cyclin E ribozyme binding site
<400> 2070
acccctgctc cctgatccc

<212> DNA<213> Homo sapiens<220> <223> Cyclin E ribozyme binding site
<400> 2071
ctccctgatc cccacacct

<212> DNA<213> Homo sapiens<220> <223> Cyclin E ribozyme binding site
<400> 2072
tgaccgggtt tacccaaac

<212> DNA<213> Homo sapiens<220> <223> Cyclin E ribozyme binding site
<400> 2073
gaccgggttt acccaaact

<212> DNA<213> Homo sapiens<220> <223> Cyclin E ribozyme binding site
<400> 2074
accgggttta cccaaactc

<212> DNA<213> Homo sapiens<220> <223> Cyclin E ribozyme binding site
<400> 2075
acccaaactc aacgtgcaa

<212> DNA<213> Homo sapiens<220> <223> Cyclin E ribozyme binding site

<400> 2076
tgcaagcctc ggattattg

<212> DNA<213> Homo sapiens<220> <223> Cyclin E ribozyme binding site
<400> 2077
gcctcggatt attgcacca

<212> DNA<213> Homo sapiens<220> <223> Cyclin E ribozyme binding site
<400> 2078
cctcggatta ttgcaccat

<212> DNA<213> Homo sapiens<220> <223> Cyclin E ribozyme binding site
<400> 2079
tcggattatt gcaccatcc

<212> DNA<213> Homo sapiens<220> <223> Cyclin E ribozyme binding site
<400> 2080
ttgcaccatc cagaggctc

<212> DNA<213> Homo sapiens<220> <223> Cyclin E ribozyme binding site
<400> 2081
ccagaggctc cccgctgcc

<212> DNA<213> Homo sapiens<220> <223> Cyclin E ribozyme binding site
<400> 2082
gctgcctgta ctgagctgg

<212> DNA<213> Homo sapiens<220> <223> Cyclin E ribozyme binding site
<400> 2083
tgggcaaata gagaggaag

<212> DNA<213> Homo sapiens<220> <223> Cyclin E ribozyme binding site
<400> 2084
agaggaagtc tggaaaatc

<212> DNA<213> Homo sapiens<220> <223> Cyclin E ribozyme binding site
<400> 2085
ctggaaaatc atgttaaac

<212> DNA<213> Homo sapiens<220> <223> Cyclin E ribozyme binding site
<400> 2086
aaatcatgtt aaacaagga

<212> DNA<213> Homo sapiens<220> <223> Cyclin E ribozyme binding site
<400> 2087
aatcatgtta aacaaggaa

<212> DNA<213> Homo sapiens<220> <223> Cyclin E ribozyme binding site
<400> 2088
aaaagacata cttaaggga

<212> DNA<213> Homo sapiens<220> <223> Cyclin E ribozyme binding site
<400> 2089
agacatactt aagggatca

<212> DNA<213> Homo sapiens<220> <223> Cyclin E ribozyme binding site
<400> 2090
gacatactta agggatcag

<212> DNA<213> Homo sapiens<220> <223> Cyclin E ribozyme binding site
<400> 2091
ttaagggatc agcactttc

<212> DNA<213> Homo sapiens<220> <223> Cyclin E ribozyme binding site
<400> 2092
atcagcactt tcttgagca

<212> DNA<213> Homo sapiens<220> <223> Cyclin E ribozyme binding site
<400> 2093
tcagcacttt cttgagcaa

<212> DNA<213> Homo sapiens<220> <223> Cyclin E ribozyme binding site
<400> 2094
cagcactttc ttgagcaac

<212> DNA<213> Homo sapiens<220> <223> Cyclin E ribozyme binding site
<400> 2095
gcactttctt gagcaacac

<212> DNA<213> Homo sapiens<220> <223> Cyclin E ribozyme binding site
<400> 2096
caacaccctc ttctgcagc

<212> DNA<213> Homo sapiens<220> <223> Cyclin E ribozyme binding site
<400> 2097
acaccctctt ctgcagcca

<212> DNA<213> Homo sapiens<220> <223> Cyclin E ribozyme binding site
<400> 2098
caccctcttc tgcagccaa

<212> DNA<213> Homo sapiens<220> <223> Cyclin E ribozyme binding site
<400> 2099
gcgagcaatt cttctggat

<212> DNA<213> Homo sapiens<220> <223> Cyclin E ribozyme binding site
<400> 2100
cgagcaattc ttctggatt

<212> DNA<213> Homo sapiens<220> <223> Cyclin E ribozyme binding site
<400> 2101
agcaattctt ctggattgg

<212> DNA<213> Homo sapiens<220> <223> Cyclin E ribozyme binding site
<400> 2102
gcaattcttc tggattggt

<212> DNA<213> Homo sapiens<220> <223> Cyclin E ribozyme binding site
<400> 2103
cttctggatt ggttaatgg

<212> DNA<213> Homo sapiens<220> <223> Cyclin E ribozyme binding site
<400> 2104
tggattggtt aatggaggt

<212> DNA<213> Homo sapiens<220> <223> Cyclin E ribozyme binding site
<400> 2105
ggattggtta atggaggtg

<212> DNA<213> Homo sapiens<220> <223> Cyclin E ribozyme binding site
<400> 2106
gtgtgaagtc tataaactt

<212> DNA<213> Homo sapiens<220> <223> Cyclin E ribozyme binding site
<400> 2107
gtgaagtcta taaacttca

<212> DNA<213> Homo sapiens<220> <223> Cyclin E ribozyme binding site
<400> 2108
gaagtctata aacttcaca

<212> DNA<213> Homo sapiens<220> <223> Cyclin E ribozyme binding site
<400> 2109
ctataaactt cacagggag

<212> DNA<213> Homo sapiens<220> <223> Cyclin E ribozyme binding site
<400> 2110
tataaacttc acagggaga

<212> DNA<213> Homo sapiens<220> <223> Cyclin E ribozyme binding site
<400> 2111
gggagacctt ttacttggc

<212> DNA<213> Homo sapiens<220> <223> Cyclin E ribozyme binding site
<400> 2112
ggagaccttt tacttggca

<212> DNA<213> Homo sapiens<220> <223> Cyclin E ribozyme binding site
<400> 2113
gagacctttt acttggcac

<212> DNA<213> Homo sapiens<220> <223> Cyclin E ribozyme binding site
<400> 2114
agacctttta cttggcaca

<212> DNA<213> Homo sapiens<220> <223> Cyclin E ribozyme binding site
<400> 2115
ccttttactt ggcacaaga

<212> DNA<213> Homo sapiens<220> <223> Cyclin E ribozyme binding site
<400> 2116
gcacaagatt tctttgacc

<212> DNA<213> Homo sapiens<220> <223> Cyclin E ribozyme binding site
<400> 2117

cacaagattt ctttgaccg

<212> DNA<213> Homo sapiens<220> <223> Cyclin E ribozyme binding site
<400> 2118
acaagatttc tttgaccgg

<212> DNA<213> Homo sapiens<220> <223> Cyclin E ribozyme binding site
<400> 2119
aagatttctt tgaccggta

<212> DNA<213> Homo sapiens<220> <223> Cyclin E ribozyme binding site
<400> 2120
agatttcttt gaccggtat

<212> DNA<213> Homo sapiens<220> <223> Cyclin E ribozyme binding site
<400> 2121
ttgaccggta tatggcgac

<212> DNA<213> Homo sapiens<220> <223> Cyclin E ribozyme binding site
<400> 2122
gaccggtata tggcgacac

<212> DNA<213> Homo sapiens<220> <223> Cyclin E ribozyme binding site
<400> 2123
agaaaatgtt gtaaaaact

<212> DNA<213> Homo sapiens<220> <223> Cyclin E ribozyme binding site
<400> 2124
aaatgttgta aaaactctt

<212> DNA<213> Homo sapiens<220> <223> Cyclin E ribozyme binding site
<400> 2125
gtaaaaactc ttttacagc

<212> DNA<213> Homo sapiens<220> <223> Cyclin E ribozyme binding site
<400> 2126
aaaaactctt ttacagctt

<212> DNA<213> Homo sapiens<220> <223> Cyclin E ribozyme binding site
<400> 2127
aaaactcttt tacagctta

<212> DNA<213> Homo sapiens<220> <223> Cyclin E ribozyme binding site
<400> 2128
aaactctttt acagcttat

<212> DNA<213> Homo sapiens<220> <223> Cyclin E ribozyme binding site
<400> 2129
aactctttta cagcttatt

<212> DNA<213> Homo sapiens<220> <223> Cyclin E ribozyme binding site
<400> 2130
tttacagctt attgggatt

<212> DNA<213> Homo sapiens<220> <223> Cyclin E ribozyme binding site

<400> 2131
ttacagctta ttgggattt

<212> DNA<213> Homo sapiens<220> <223> Cyclin E ribozyme binding site
<400> 2132
acagcttatt gggatttca

<212> DNA<213> Homo sapiens<220> <223> Cyclin E ribozyme binding site
<400> 2133
tattgggatt tcatcttta

<212> DNA<213> Homo sapiens<220> <223> Cyclin E ribozyme binding site
<400> 2134
attgggattt catctttat

<212> DNA<213> Homo sapiens<220> <223> Cyclin E ribozyme binding site
<400> 2135
ttgggatttc atctttatt

<212> DNA<213> Homo sapiens<220> <223> Cyclin E ribozyme binding site
<400> 2136
ggatttcatc tttatttat

<212> DNA<213> Homo sapiens<220> <223> Cyclin E ribozyme binding site
<400> 2137
atttcatctt tatttattg

<212> DNA<213> Homo sapiens<220> <223> Cyclin E ribozyme binding site
<400> 2138
tttcatcttt atttattgc

<212> DNA<213> Homo sapiens<220> <223> Cyclin E ribozyme binding site
<400> 2139
ttcatcttta tttattgca

<212> DNA<213> Homo sapiens<220> <223> Cyclin E ribozyme binding site
<400> 2140
catctttatt tattgcagc

<212> DNA<213> Homo sapiens<220> <223> Cyclin E ribozyme binding site
<400> 2141
atctttattt attgcagcc

<212> DNA<213> Homo sapiens<220> <223> Cyclin E ribozyme binding site
<400> 2142
tctttattta ttgcagcca

<212> DNA<213> Homo sapiens<220> <223> Cyclin E ribozyme binding site
<400> 2143
tttatttatt gcagccaaa

<212> DNA<213> Homo sapiens<220> <223> Cyclin E ribozyme binding site
<400> 2144
agccaaactt gaggaaatc

<212> DNA<213> Homo sapiens<220> <223> Cyclin E ribozyme binding site
<400> 2145
tgaggaaatc tatcctcca

<212> DNA<213> Homo sapiens<220> <223> Cyclin E ribozyme binding site
<400> 2146
aggaaatcta tcctccaaa

<212> DNA<213> Homo sapiens<220> <223> Cyclin E ribozyme binding site
<400> 2147
gaaatctatc ctccaaagt

<212> DNA<213> Homo sapiens<220> <223> Cyclin E ribozyme binding site
<400> 2148
atctatcctc caaagttgc

<212> DNA<213> Homo sapiens<220> <223> Cyclin E ribozyme binding site
<400> 2149
ctccaaagtt gcaccagtt

<212> DNA<213> Homo sapiens<220> <223> Cyclin E ribozyme binding site
<400> 2150
tgcaccagtt tgcgtatgt

<212> DNA<213> Homo sapiens<220> <223> Cyclin E ribozyme binding site
<400> 2151
gcaccagttt gcgtatgtg

<212> DNA<213> Homo sapiens<220> <223> Cyclin E ribozyme binding site
<400> 2152
agtttgcgta tgtgacaga

<212> DNA<213> Homo sapiens<220> <223> Cyclin E ribozyme binding site
<400> 2153
gatggagctt gttcaggag

<212> DNA<213> Homo sapiens<220> <223> Cyclin E ribozyme binding site
<400> 2154
ggagcttgtt caggagatg

<212> DNA<213> Homo sapiens<220> <223> Cyclin E ribozyme binding site
<400> 2155
gagcttgttc aggagatga

<212> DNA<213> Homo sapiens<220> <223> Cyclin E ribozyme binding site
<400> 2156
agatgaaatt ctcaccatg

<212> DNA<213> Homo sapiens<220> <223> Cyclin E ribozyme binding site
<400> 2157
gatgaaattc tcaccatgg

<212> DNA<213> Homo sapiens<220> <223> Cyclin E ribozyme binding site
<400> 2158
tgaaattctc accatggaa

<212> DNA<213> Homo sapiens<220> <223> Cyclin E ribozyme binding site
<400> 2159
ccatggaatt aatgattat

<212> DNA<213> Homo sapiens<220> <223> Cyclin E ribozyme binding site
<400> 2160
catggaatta atgattatg

<212> DNA<213> Homo sapiens<220> <223> Cyclin E ribozyme binding site
<400> 2161
attaatgatt atgaaggcc

<212> DNA<213> Homo sapiens<220> <223> Cyclin E ribozyme binding site
<400> 2162
ttaatgatta tgaaggccc

<212> DNA<213> Homo sapiens<220> <223> Cyclin E ribozyme binding site
<400> 2163
gaaggccctt aagtggcgt

<212> DNA<213> Homo sapiens<220> <223> Cyclin E ribozyme binding site
<400> 2164
aaggccctta agtggcgtt

<212> DNA<213> Homo sapiens<220> <223> Cyclin E ribozyme binding site
<400> 2165
aagtggcgtt taagtcccc

<212> DNA<213> Homo sapiens<220> <223> Cyclin E ribozyme binding site
<400> 2166
agtggcgttt aagtcccct

<212> DNA<213> Homo sapiens<220> <223> Cyclin E ribozyme binding site
<400> 2167
gtggcgttta agtcccctg

<212> DNA<213> Homo sapiens<220> <223> Cyclin E ribozyme binding site
<400> 2168
cgtttaagtc ccctgacta

<212> DNA<213> Homo sapiens<220> <223> Cyclin E ribozyme binding site
<400> 2169
cccctgacta ttgtgtcct

<212> DNA<213> Homo sapiens<220> <223> Cyclin E ribozyme binding site
<400> 2170
cctgactatt gtgtcctgg

<212> DNA<213> Homo sapiens<220> <223> Cyclin E ribozyme binding site
<400> 2171
ctattgtgtc ctggctgaa

<212> DNA<213> Homo sapiens<220> <223> Cyclin E ribozyme binding site
<400> 2172

gctgaatgta tacatgcag

<212> DNA<213> Homo sapiens<220> <223> Cyclin E ribozyme binding site
<400> 2173
tgaatgtata catgcaggt

<212> DNA<213> Homo sapiens<220> <223> Cyclin E ribozyme binding site
<400> 2174
catgcaggtt gcatatcta

<212> DNA<213> Homo sapiens<220> <223> Cyclin E ribozyme binding site
<400> 2175
aggttgcata tctaaatga

<212> DNA<213> Homo sapiens<220> <223> Cyclin E ribozyme binding site
<400> 2176
gttgcatatc taaatgact

<212> DNA<213> Homo sapiens<220> <223> Cyclin E ribozyme binding site
<400> 2177
tgcatatcta aatgactta

<212> DNA<213> Homo sapiens<220> <223> Cyclin E ribozyme binding site
<400> 2178
taaatgactt acatgaagt

<212> DNA<213> Homo sapiens<220> <223> Cyclin E ribozyme binding site
<400> 2179
aaatgactta catgaagtg

<212> DNA<213> Homo sapiens<220> <223> Cyclin E ribozyme binding site
<400> 2180
tgaagtgcta ctgccgcag

<212> DNA<213> Homo sapiens<220> <223> Cyclin E ribozyme binding site
<400> 2181
tgccgcagta tccccagca

<212> DNA<213> Homo sapiens<220> <223> Cyclin E ribozyme binding site
<400> 2182
ccgcagtatc cccagcaaa

<212> DNA<213> Homo sapiens<220> <223> Cyclin E ribozyme binding site
<400> 2183
ccagcaaatc tttatacag

<212> DNA<213> Homo sapiens<220> <223> Cyclin E ribozyme binding site
<400> 2184
agcaaatctt tatacagat

<212> DNA<213> Homo sapiens<220> <223> Cyclin E ribozyme binding site
<400> 2185
gcaaatcttt atacagatt

<212> DNA<213> Homo sapiens<220> <223> Cyclin E ribozyme binding site

<400> 2186
caaatcttta tacagattg

<212> DNA<213> Homo sapiens<220> <223> Cyclin E ribozyme binding site
<400> 2187
aatctttata cagattgca

<212> DNA<213> Homo sapiens<220> <223> Cyclin E ribozyme binding site
<400> 2188
tatacagatt gcagagctg

<212> DNA<213> Homo sapiens<220> <223> Cyclin E ribozyme binding site
<400> 2189
cagagctgtt ggatctctg

<212> DNA<213> Homo sapiens<220> <223> Cyclin E ribozyme binding site
<400> 2190
ctgttggatc tctgtgtcc

<212> DNA<213> Homo sapiens<220> <223> Cyclin E ribozyme binding site
<400> 2191
gttggatctc tgtgtcctg

<212> DNA<213> Homo sapiens<220> <223> Cyclin E ribozyme binding site
<400> 2192
tctctgtgtc ctggatgtt

<212> DNA<213> Homo sapiens<220> <223> Cyclin E ribozyme binding site
<400> 2193
cctggatgtt gactgcctt

<212> DNA<213> Homo sapiens<220> <223> Cyclin E ribozyme binding site
<400> 2194
tgactgcctt gaatttcct

<212> DNA<213> Homo sapiens<220> <223> Cyclin E ribozyme binding site
<400> 2195
gccttgaatt tccttatgg

<212> DNA<213> Homo sapiens<220> <223> Cyclin E ribozyme binding site
<400> 2196
ccttgaattt ccttatggt

<212> DNA<213> Homo sapiens<220> <223> Cyclin E ribozyme binding site
<400> 2197
cttgaatttc cttatggta

<212> DNA<213> Homo sapiens<220> <223> Cyclin E ribozyme binding site
<400> 2198
gaatttcctt atggtatac

<212> DNA<213> Homo sapiens<220> <223> Cyclin E ribozyme binding site
<400> 2199
aatttcctta tggtatact

<212> DNA<213> Homo sapiens<220> <223> Cyclin E ribozyme binding site
<400> 2200
ccttatggta tacttgctg

<212> DNA<213> Homo sapiens<220> <223> Cyclin E ribozyme binding site
<400> 2201
ttatggtata cttgctgct

<212> DNA<213> Homo sapiens<220> <223> Cyclin E ribozyme binding site
<400> 2202
tggtatactt gctgcttcg

<212> DNA<213> Homo sapiens<220> <223> Cyclin E ribozyme binding site
<400> 2203
cttgctgctt cggccttgt

<212> DNA<213> Homo sapiens<220> <223> Cyclin E ribozyme binding site
<400> 2204
ttgctgcttc ggccttgta

<212> DNA<213> Homo sapiens<220> <223> Cyclin E ribozyme binding site
<400> 2205
cttcggcctt gtatcattt

<212> DNA<213> Homo sapiens<220> <223> Cyclin E ribozyme binding site
<400> 2206
cggccttgta tcatttctc

<212> DNA<213> Homo sapiens<220> <223> Cyclin E ribozyme binding site
<400> 2207
gccttgtatc atttctcgt

<212> DNA<213> Homo sapiens<220> <223> Cyclin E ribozyme binding site
<400> 2208
ttgtatcatt tctcgtcat

<212> DNA<213> Homo sapiens<220> <223> Cyclin E ribozyme binding site
<400> 2209
tgtatcattt ctcgtcatc

<212> DNA<213> Homo sapiens<220> <223> Cyclin E ribozyme binding site
<400> 2210
gtatcatttc tcgtcatct

<212> DNA<213> Homo sapiens<220> <223> Cyclin E ribozyme binding site
<400> 2211
atcatttctc gtcatctga

<212> DNA<213> Homo sapiens<220> <223> Cyclin E ribozyme binding site
<400> 2212
atttctcgtc atctgaatt

<212> DNA<213> Homo sapiens<220> <223> Cyclin E ribozyme binding site
<400> 2213
tctcgtcatc tgaattgat

<212> DNA<213> Homo sapiens<220> <223> Cyclin E ribozyme binding site
<400> 2214
catctgaatt gatgcaaaa

<212> DNA<213> Homo sapiens<220> <223> Cyclin E ribozyme binding site
<400> 2215
gcaaaaggtt tcagggtat

<212> DNA<213> Homo sapiens<220> <223> Cyclin E ribozyme binding site
<400> 2216
caaaaggttt cagggtatc

<212> DNA<213> Homo sapiens<220> <223> Cyclin E ribozyme binding site
<400> 2217
aaaaggtttc agggtatca

<212> DNA<213> Homo sapiens<220> <223> Cyclin E ribozyme binding site
<400> 2218
tttcagggta tcagtggtg

<212> DNA<213> Homo sapiens<220> <223> Cyclin E ribozyme binding site
<400> 2219
tcagggtatc agtggtgcg

<212> DNA<213> Homo sapiens<220> <223> Cyclin E ribozyme binding site
<400> 2220
gtgcgacata gagaactgt

<212> DNA<213> Homo sapiens<220> <223> Cyclin E ribozyme binding site
<400> 2221
gaactgtgtc aagtggatg

<212> DNA<213> Homo sapiens<220> <223> Cyclin E ribozyme binding site
<400> 2222
gtggatggtt ccatttgcc

<212> DNA<213> Homo sapiens<220> <223> Cyclin E ribozyme binding site
<400> 2223
tggatggttc catttgcca

<212> DNA<213> Homo sapiens<220> <223> Cyclin E ribozyme binding site
<400> 2224
tggttccatt tgccatggt

<212> DNA<213> Homo sapiens<220> <223> Cyclin E ribozyme binding site
<400> 2225
ggttccattt gccatggtt

<212> DNA<213> Homo sapiens<220> <223> Cyclin E ribozyme binding site
<400> 2226
tgccatggtt ataagggag

<212> DNA<213> Homo sapiens<220> <223> Cyclin E ribozyme binding site
<400> 2227

gccatggtta taagggaga

<212> DNA<213> Homo sapiens<220> <223> Cyclin E ribozyme binding site
<400> 2228
catggttata agggagacg

<212> DNA<213> Homo sapiens<220> <223> Cyclin E ribozyme binding site
<400> 2229
cggggagctc aaaactgaa

<212> DNA<213> Homo sapiens<220> <223> Cyclin E ribozyme binding site
<400> 2230
tgaagcactt caggggcgt

<212> DNA<213> Homo sapiens<220> <223> Cyclin E ribozyme binding site
<400> 2231
gaagcacttc aggggcgtc

<212> DNA<213> Homo sapiens<220> <223> Cyclin E ribozyme binding site
<400> 2232
caggggcgtc gctgatgaa

<212> DNA<213> Homo sapiens<220> <223> Cyclin E ribozyme binding site
<400> 2233
acacaacata cagacccac

<212> DNA<213> Homo sapiens<220> <223> Cyclin E ribozyme binding site
<400> 2234
gagacagctt ggatttgct

<212> DNA<213> Homo sapiens<220> <223> Cyclin E ribozyme binding site
<400> 2235
agcttggatt tgctggaca

<212> DNA<213> Homo sapiens<220> <223> Cyclin E ribozyme binding site
<400> 2236
gcttggattt gctggacaa

<212> DNA<213> Homo sapiens<220> <223> Cyclin E ribozyme binding site
<400> 2237
aagccatgtt gtctgaaca

<212> DNA<213> Homo sapiens<220> <223> Cyclin E ribozyme binding site
<400> 2238
ccatgttgtc tgaacaaaa

<212> DNA<213> Homo sapiens<220> <223> Cyclin E ribozyme binding site
<400> 2239
gaacaaaata gggcttctc

<212> DNA<213> Homo sapiens<220> <223> Cyclin E ribozyme binding site
<400> 2240
aatagggctt ctcctctcc

<212> DNA<213> Homo sapiens<220> <223> Cyclin E ribozyme binding site

<400> 2241
atagggcttc tcctctccc

<212> DNA<213> Homo sapiens<220> <223> Cyclin E ribozyme binding site
<400> 2242
agggcttctc ctctcccca

<212> DNA<213> Homo sapiens<220> <223> Cyclin E ribozyme binding site
<400> 2243
gcttctcctc tccccagtg

<212> DNA<213> Homo sapiens<220> <223> Cyclin E ribozyme binding site
<400> 2244
ttctcctctc cccagtggg

<212> DNA<213> Homo sapiens<220> <223> Cyclin E ribozyme binding site
<400> 2245
cagtgggctc ctcaccccg

<212> DNA<213> Homo sapiens<220> <223> Cyclin E ribozyme binding site
<400> 2246
tgggctcctc accccgcca

<212> DNA<213> Homo sapiens<220> <223> Cyclin E ribozyme binding site
<400> 2247
cagagcggta agaagcaga

<212> DNA<213> Homo sapiens<220> <223> Cyclin E ribozyme binding site
<400> 2248
ccaccccatc cttctccac

<212> DNA<213> Homo sapiens<220> <223> Cyclin E ribozyme binding site
<400> 2249
ccccatcctt ctccaccaa

<212> DNA<213> Homo sapiens<220> <223> Cyclin E ribozyme binding site
<400> 2250
cccatccttc tccaccaaa

<212> DNA<213> Homo sapiens<220> <223> Cyclin E ribozyme binding site
<400> 2251
catccttctc caccaaaga

<212> DNA<213> Homo sapiens<220> <223> Cyclin E ribozyme binding site
<400> 2252
aaagacagtt gcgcgcctg

<212> DNA<213> Homo sapiens<220> <223> Cyclin E ribozyme binding site
<400> 2253
gcgcctgctc cacgttctc

<212> DNA<213> Homo sapiens<220> <223> Cyclin E ribozyme binding site
<400> 2254
gctccacgtt ctcttctgt

<212> DNA<213> Homo sapiens<220> <223> Cyclin E ribozyme binding site
<400> 2255
ctccacgttc tcttctgtc

<212> DNA<213> Homo sapiens<220> <223> Cyclin E ribozyme binding site
<400> 2256
ccacgttctc ttctgtctg

<212> DNA<213> Homo sapiens<220> <223> Cyclin E ribozyme binding site
<400> 2257
acgttctctt ctgtctgtt

<212> DNA<213> Homo sapiens<220> <223> Cyclin E ribozyme binding site
<400> 2258
cgttctcttc tgtctgttg

<212> DNA<213> Homo sapiens<220> <223> Cyclin E ribozyme binding site
<400> 2259
ctcttctgtc tgttgcagc

<212> DNA<213> Homo sapiens<220> <223> Cyclin E ribozyme binding site
<400> 2260
tctgtctgtt gcagcggag

<212> DNA<213> Homo sapiens<220> <223> Cyclin E ribozyme binding site
<400> 2261
ggcgtgcgtt tgctttaca

<212> DNA<213> Homo sapiens<220> <223> Cyclin E ribozyme binding site
<400> 2262
gcgtgcgttt gctttaca

<212> DNA<213> Homo sapiens<220> <223> Cyclin E ribozyme binding site
<400> 2263
gcgtttgctt ttacagata

<212> DNA<213> Homo sapiens<220> <223> Cyclin E ribozyme binding site
<400> 2264
cgtttgcttt tacagatat

<212> DNA<213> Homo sapiens<220> <223> Cyclin E ribozyme binding site
<400> 2265
gtttgctttt acagatatc

<212> DNA<213> Homo sapiens<220> <223> Cyclin E ribozyme binding site
<400> 2266
tttgctttta cagatatct

<212> DNA<213> Homo sapiens<220> <223> Cyclin E ribozyme binding site
<400> 2267
tttacagata tctgaatgg

<212> DNA<213> Homo sapiens<220> <223> Cyclin E ribozyme binding site
<400> 2268
tacagatatc tgaatggaa

<212> DNA<213> Homo sapiens<220> <223> Cyclin E ribozyme binding site
<400> 2269
gaagagtgtt tcttccaca

<212> DNA<213> Homo sapiens<220> <223> Cyclin E ribozyme binding site
<400> 2270
aagagtgttt cttccacaa

<212> DNA<213> Homo sapiens<220> <223> Cyclin E ribozyme binding site
<400> 2271
agagtgtttc ttccacaac

<212> DNA<213> Homo sapiens<220> <223> Cyclin E ribozyme binding site
<400> 2272
agtgtttctt ccacaacag

<212> DNA<213> Homo sapiens<220> <223> Cyclin E ribozyme binding site
<400> 2273
gtgtttcttc cacaacaga

<212> DNA<213> Homo sapiens<220> <223> Cyclin E ribozyme binding site
<400> 2274
aacagaagta tttctgtgg

<212> DNA<213> Homo sapiens<220> <223> Cyclin E ribozyme binding site
<400> 2275
cagaagtatt tctgtggat

<212> DNA<213> Homo sapiens<220> <223> Cyclin E ribozyme binding site
<400> 2276
agaagtattt ctgtggatg

<212> DNA<213> Homo sapiens<220> <223> Cyclin E ribozyme binding site
<400> 2277
gaagtatttc tgtggatgg

<212> DNA<213> Homo sapiens<220> <223> Cyclin E ribozyme binding site
<400> 2278
ggatggcatc aaacagggc

<212> DNA<213> Homo sapiens<220> <223> Cyclin E ribozyme binding site
<400> 2279
gcaaagtgtt ttttattga

<212> DNA<213> Homo sapiens<220> <223> Cyclin E ribozyme binding site
<400> 2280
caaagtgttt tttattgaa

<212> DNA<213> Homo sapiens<220> <223> Cyclin E ribozyme binding site
<400> 2281
aaagtgtttt ttattgaat

<212> DNA<213> Homo sapiens<220> <223> Cyclin E ribozyme binding site
<400> 2282

aagtgttttt tattgaatg

<212> DNA<213> Homo sapiens<220> <223> Cyclin E ribozyme binding site
<400> 2283
agtgtttttt attgaatgc

<212> DNA<213> Homo sapiens<220> <223> Cyclin E ribozyme binding site
<400> 2284
gtgtttttta ttgaatgct

<212> DNA<213> Homo sapiens<220> <223> Cyclin E ribozyme binding site
<400> 2285
gtttttttatt gaatgctta

<212> DNA<213> Homo sapiens<220> <223> Cyclin E ribozyme binding site
<400> 2286
ttgaatgctt ataggtttt

<212> DNA<213> Homo sapiens<220> <223> Cyclin E ribozyme binding site
<400> 2287
tgaatgctta taggttttt

<212> DNA<213> Homo sapiens<220> <223> Cyclin E ribozyme binding site
<400> 2288
aatgcttata ggttttttt

<212> DNA<213> Homo sapiens<220> <223> Cyclin E ribozyme binding site
<400> 2289
cttataggtt ttttttaaa

<212> DNA<213> Homo sapiens<220> <223> Cyclin E ribozyme binding site
<400> 2290
ttataggttt tttttaaat

<212> DNA<213> Homo sapiens<220> <223> Cyclin E ribozyme binding site
<400> 2291
tataggtttt ttttaaata

<212> DNA<213> Homo sapiens<220> <223> Cyclin E ribozyme binding site
<400> 2292
ataggttttt tttaaataa

<212> DNA<213> Homo sapiens<220> <223> Cyclin E ribozyme binding site
<400> 2293
taggtttttt ttaaataag

<212> DNA<213> Homo sapiens<220> <223> Cyclin E ribozyme binding site
<400> 2294
aggttttttt taaataagt

<212> DNA<213> Homo sapiens<220> <223> Cyclin E ribozyme binding site
<400> 2295
ggtttttttt aaataagtg

<212> DNA<213> Homo sapiens<220> <223> Cyclin E ribozyme binding site

<400> 2296
gtttttttta aataattgg

<212> DNA<213> Homo sapiens<220> <223> Cyclin E ribozyme binding site
<400> 2297
tttttaaata agtgggtca

<212> DNA<213> Homo sapiens<220> <223> Cyclin E ribozyme binding site
<400> 2298
taagtgggtc aagtacacc

<212> DNA<213> Homo sapiens<220> <223> Cyclin E ribozyme binding site
<400> 2299
gggtcaagta caccagcca

<212> DNA<213> Homo sapiens<220> <223> Cyclin E ribozyme binding site
<400> 2300
cagccacctc cagacacca

<212> DNA<213> Homo sapiens<220> <223> Cyclin E ribozyme binding site
<400> 2301
gtgcgtgctc ccgatgctg

<212> DNA<213> Homo sapiens<220> <223> Cyclin E ribozyme binding site
<400> 2302
gatgctgcta tggaaggtg

<212> DNA<213> Homo sapiens<220> <223> Cyclin E ribozyme binding site
<400> 2303
gaaggtgcta cttgaccta

<212> DNA<213> Homo sapiens<220> <223> Cyclin E ribozyme binding site
<400> 2304
ggtgctactt gacctaaag

<212> DNA<213> Homo sapiens<220> <223> Cyclin E ribozyme binding site
<400> 2305
acttgaccta aaggactcc

<212> DNA<213> Homo sapiens<220> <223> Cyclin E ribozyme binding site
<400> 2306
taaaggactc ccacaacaa

<212> DNA<213> Homo sapiens<220> <223> Cyclin E ribozyme binding site
<400> 2307
acaaaagctt gaagctgtg

<212> DNA<213> Homo sapiens<220> <223> Cyclin E ribozyme binding site
<400> 2308
ggcgtggctc tcctcgcag

<212> DNA<213> Homo sapiens<220> <223> Cyclin E ribozyme binding site
<400> 2309
cgtggctctc ctcgcaggt

<212> DNA<213> Homo sapiens<220> <223> Cyclin E ribozyme binding site
<400> 2310
ggctctcctc gcaggtgtt

<212> DNA<213> Homo sapiens<220> <223> Cyclin E ribozyme binding site
<400> 2311
cgcaggtgtt ctgggctcc

<212> DNA<213> Homo sapiens<220> <223> Cyclin E ribozyme binding site
<400> 2312
gcaggtgttc tgggctccg

<212> DNA<213> Homo sapiens<220> <223> Cyclin E ribozyme binding site
<400> 2313
ttctgggctc cgttgtacc

<212> DNA<213> Homo sapiens<220> <223> Cyclin E ribozyme binding site
<400> 2314
gggctccgtt gtaccaagt

<212> DNA<213> Homo sapiens<220> <223> Cyclin E ribozyme binding site
<400> 2315
ctccgttgta ccaagtgga

<212> DNA<213> Homo sapiens<220> <223> Cyclin E ribozyme binding site
<400> 2316
gcaggtggtt gcgggcaag

<212> DNA<213> Homo sapiens<220> <223> Cyclin E ribozyme binding site
<400> 2317
ggcaagcgtt gtgcagagc

<212> DNA<213> Homo sapiens<220> <223> Cyclin E ribozyme binding site
<400> 2318
agagcccata gccagctgg

<212> DNA<213> Homo sapiens<220> <223> Cyclin F ribozyme binding site
<400> 2319
cggcgtggtc cactgtagg

<212> DNA<213> Homo sapiens<220> <223> Cyclin F ribozyme binding site
<400> 2320
gtccactgta ggtgtgcca

<212> DNA<213> Homo sapiens<220> <223> Cyclin F ribozyme binding site
<400> 2321
gccaagtgtt tctgttatc

<212> DNA<213> Homo sapiens<220> <223> Cyclin F ribozyme binding site
<400> 2322
ccaagtgttt ctgttatcc

<212> DNA<213> Homo sapiens<220> <223> Cyclin F ribozyme binding site
<400> 2323
caagtgtttc tgttatcct

<212> DNA<213> Homo sapiens<220> <223> Cyclin F ribozyme binding site
<400> 2324
tgtttctgtt atcctacaa

<212> DNA<213> Homo sapiens<220> <223> Cyclin F ribozyme binding site
<400> 2325
gtttctgtta tcctacaaa

<212> DNA<213> Homo sapiens<220> <223> Cyclin F ribozyme binding site
<400> 2326
ttctgttatc ctacaaagc

<212> DNA<213> Homo sapiens<220> <223> Cyclin F ribozyme binding site
<400> 2327
tgttatccta caaagcgaa

<212> DNA<213> Homo sapiens<220> <223> Cyclin F ribozyme binding site
<400> 2328
gcgaagaata aggaggagg

<212> DNA<213> Homo sapiens<220> <223> Cyclin F ribozyme binding site
<400> 2329
cctgaccatc ttgagtctc

<212> DNA<213> Homo sapiens<220> <223> Cyclin F ribozyme binding site
<400> 2330
tgaccatctt gagtctccc

<212> DNA<213> Homo sapiens<220> <223> Cyclin F ribozyme binding site
<400> 2331
atcttgagtc tccccgaag

<212> DNA<213> Homo sapiens<220> <223> Cyclin F ribozyme binding site
<400> 2332
cttgagtctc cccgaagat

<212> DNA<213> Homo sapiens<220> <223> Cyclin F ribozyme binding site
<400> 2333
agatgtgctc tttcacatc

<212> DNA<213> Homo sapiens<220> <223> Cyclin F ribozyme binding site
<400> 2334
atgtgctctt tcacatcct

<212> DNA<213> Homo sapiens<220> <223> Cyclin F ribozyme binding site
<400> 2335
tgtgctcttt cacatcctg

<212> DNA<213> Homo sapiens<220> <223> Cyclin F ribozyme binding site
<400> 2336
gtgctctttc acatcctga

<212> DNA<213> Homo sapiens<220> <223> Cyclin F ribozyme binding site
<400> 2337

ctttcacatc ctgaaatgg

<212> DNA<213> Homo sapiens<220> <223> Cyclin F ribozyme binding site
<400> 2338
gaaatggctt tctgtagag

<212> DNA<213> Homo sapiens<220> <223> Cyclin F ribozyme binding site
<400> 2339
aaatggcttt ctgtagagg

<212> DNA<213> Homo sapiens<220> <223> Cyclin F ribozyme binding site
<400> 2340
aatggctttc tgtagagga

<212> DNA<213> Homo sapiens<220> <223> Cyclin F ribozyme binding site
<400> 2341
gctttctgta gaggacatc

<212> DNA<213> Homo sapiens<220> <223> Cyclin F ribozyme binding site
<400> 2342
agaggacatc ctggccgtc

<212> DNA<213> Homo sapiens<220> <223> Cyclin F ribozyme binding site
<400> 2343
cctggccgtc cgagctgta

<212> DNA<213> Homo sapiens<220> <223> Cyclin F ribozyme binding site
<400> 2344
ccgagctgta cactcccag

<212> DNA<213> Homo sapiens<220> <223> Cyclin F ribozyme binding site
<400> 2345
ctgtacactc ccagctgaa

<212> DNA<213> Homo sapiens<220> <223> Cyclin F ribozyme binding site
<400> 2346
gtgccagctt ccaggagct

<212> DNA<213> Homo sapiens<220> <223> Cyclin F ribozyme binding site
<400> 2347
tgccagcttc caggagctg

<212> DNA<213> Homo sapiens<220> <223> Cyclin F ribozyme binding site
<400> 2348
tgtggccgtc tccagggaa

<212> DNA<213> Homo sapiens<220> <223> Cyclin F ribozyme binding site
<400> 2349
tggccgtctc cagggaacc

<212> DNA<213> Homo sapiens<220> <223> Cyclin F ribozyme binding site
<400> 2350
cctgaagctc tttgaaagg

<212> DNA<213> Homo sapiens<220> <223> Cyclin F ribozyme binding site

<400> 2351
tgaagctctt tgaaagggc

<212> DNA<213> Homo sapiens<220> <223> Cyclin F ribozyme binding site
<400> 2352
gaagctcttt gaaagggct

<212> DNA<213> Homo sapiens<220> <223> Cyclin F ribozyme binding site
<400> 2353
aaggggaatt tcgaagctg

<212> DNA<213> Homo sapiens<220> <223> Cyclin F ribozyme binding site
<400> 2354
aggggaattt cgaagctgc

<212> DNA<213> Homo sapiens<220> <223> Cyclin F ribozyme binding site
<400> 2355
ggggaatttc gaagctgct

<212> DNA<213> Homo sapiens<220> <223> Cyclin F ribozyme binding site
<400> 2356
gctgggcata gcctacctc

<212> DNA<213> Homo sapiens<220> <223> Cyclin F ribozyme binding site
<400> 2357
gcatagccta cctctacaa

<212> DNA<213> Homo sapiens<220> <223> Cyclin F ribozyme binding site
<400> 2358
agcctacctc tacaatgaa

<212> DNA<213> Homo sapiens<220> <223> Cyclin F ribozyme binding site
<400> 2359
cctacctcta caatgaagg

<212> DNA<213> Homo sapiens<220> <223> Cyclin F ribozyme binding site
<400> 2360
aaggcctgtc tgtgtctga

<212> DNA<213> Homo sapiens<220> <223> Cyclin F ribozyme binding site
<400> 2361
tgtctgtgtc tgatgaggc

<212> DNA<213> Homo sapiens<220> <223> Cyclin F ribozyme binding site
<400> 2362
tgaaggcctc tcgcttctt

<212> DNA<213> Homo sapiens<220> <223> Cyclin F ribozyme binding site
<400> 2363
aaggcctctc gcttcttca

<212> DNA<213> Homo sapiens<220> <223> Cyclin F ribozyme binding site
<400> 2364
cctctcgctt cttcagtct

<212> DNA<213> Homo sapiens<220> <223> Cyclin F ribozyme binding site
<400> 2365
ctcgcttctt cagtctcgc

<212> DNA<213> Homo sapiens<220> <223> Cyclin F ribozyme binding site
<400> 2366
tcgcttcttc agtctcgct

<212> DNA<213> Homo sapiens<220> <223> Cyclin F ribozyme binding site
<400> 2367
ttcttcagtc tcgctgagc

<212> DNA<213> Homo sapiens<220> <223> Cyclin F ribozyme binding site
<400> 2368
cttcagtctc gctgagcgg

<212> DNA<213> Homo sapiens<220> <223> Cyclin F ribozyme binding site
<400> 2369
gccgcacctt tcatctggc

<212> DNA<213> Homo sapiens<220> <223> Cyclin F ribozyme binding site
<400> 2370
ccgcaccttt catctggct

<212> DNA<213> Homo sapiens<220> <223> Cyclin F ribozyme binding site
<400> 2371
cgcacctttc atctggctc

<212> DNA<213> Homo sapiens<220> <223> Cyclin F ribozyme binding site
<400> 2372
acctttcatc tggctcttc

<212> DNA<213> Homo sapiens<220> <223> Cyclin F ribozyme binding site
<400> 2373
catctggctc ttcatccgc

<212> DNA<213> Homo sapiens<220> <223> Cyclin F ribozyme binding site
<400> 2374
tctggctctt catccgccc

<212> DNA<213> Homo sapiens<220> <223> Cyclin F ribozyme binding site
<400> 2375
ctggctcttc atccgccct

<212> DNA<213> Homo sapiens<220> <223> Cyclin F ribozyme binding site
<400> 2376
gctcttcatc cgccctccg

<212> DNA<213> Homo sapiens<220> <223> Cyclin F ribozyme binding site
<400> 2377
atccgccctc cgtggtcgg

<212> DNA<213> Homo sapiens<220> <223> Cyclin F ribozyme binding site
<400> 2378
ctccgtggtc ggtgagcgg

<212> DNA<213> Homo sapiens<220> <223> Cyclin F ribozyme binding site
<400> 2379
ggccgtggtt cacgagagc

<212> DNA<213> Homo sapiens<220> <223> Cyclin F ribozyme binding site
<400> 2380
gccgtggttc acgagagcc

<212> DNA<213> Homo sapiens<220> <223> Cyclin F ribozyme binding site
<400> 2381
cgagagcctc agggcagag

<212> DNA<213> Homo sapiens<220> <223> Cyclin F ribozyme binding site
<400> 2382
cagaggactc acaaagcat

<212> DNA<213> Homo sapiens<220> <223> Cyclin F ribozyme binding site
<400> 2383
acaaagcatc catattgca

<212> DNA<213> Homo sapiens<220> <223> Cyclin F ribozyme binding site
<400> 2384
agcatccata ttgcactgc

<212> DNA<213> Homo sapiens<220> <223> Cyclin F ribozyme binding site
<400> 2385
catccatatt gcactgctt

<212> DNA<213> Homo sapiens<220> <223> Cyclin F ribozyme binding site
<400> 2386
tgcactgctt gggcagagt

<212> DNA<213> Homo sapiens<220> <223> Cyclin F ribozyme binding site
<400> 2387
gtgctgagtc tgttcgagg

<212> DNA<213> Homo sapiens<220> <223> Cyclin F ribozyme binding site
<400> 2388
tgagtctgtt cgaggatga

<212> DNA<213> Homo sapiens<220> <223> Cyclin F ribozyme binding site
<400> 2389
gagtctgttc gaggatgag

<212> DNA<213> Homo sapiens<220> <223> Cyclin F ribozyme binding site
<400> 2390
atgacctgtt tgaggaggc

<212> DNA<213> Homo sapiens<220> <223> Cyclin F ribozyme binding site
<400> 2391
tgacctgttt gaggaggct

<212> DNA<213> Homo sapiens<220> <223> Cyclin F ribozyme binding site
<400> 2392

gaggctgctc atcagggat

<212> DNA<213> Homo sapiens<220> <223> Cyclin F ribozyme binding site
<400> 2393
gctgctcatc agggatgtc

<212> DNA<213> Homo sapiens<220> <223> Cyclin F ribozyme binding site
<400> 2394
cagggatgtc tgaccagct

<212> DNA<213> Homo sapiens<220> <223> Cyclin F ribozyme binding site
<400> 2395
tgaccagctc ctacctcct

<212> DNA<213> Homo sapiens<220> <223> Cyclin F ribozyme binding site
<400> 2396
ccagctccta cctcctctg

<212> DNA<213> Homo sapiens<220> <223> Cyclin F ribozyme binding site
<400> 2397
ctcctacctc ctctgggaa

<212> DNA<213> Homo sapiens<220> <223> Cyclin F ribozyme binding site
<400> 2398
ctacctcctc tgggaaagc

<212> DNA<213> Homo sapiens<220> <223> Cyclin F ribozyme binding site
<400> 2399
cagatgtgtc agatcctgg

<212> DNA<213> Homo sapiens<220> <223> Cyclin F ribozyme binding site
<400> 2400
gtgtcagatc ctgggcgat

<212> DNA<213> Homo sapiens<220> <223> Cyclin F ribozyme binding site
<400> 2401
gcgatgcctc cacagcttc

<212> DNA<213> Homo sapiens<220> <223> Cyclin F ribozyme binding site
<400> 2402
tccacagctt ccgaaaact

<212> DNA<213> Homo sapiens<220> <223> Cyclin F ribozyme binding site
<400> 2403
ccacagcttc cgaaaactc

<212> DNA<213> Homo sapiens<220> <223> Cyclin F ribozyme binding site
<400> 2404
ccgaaaactc agggactac

<212> DNA<213> Homo sapiens<220> <223> Cyclin F ribozyme binding site
<400> 2405
tcagggacta cgctcgcaa

<212> DNA<213> Homo sapiens<220> <223> Cyclin F ribozyme binding site

<400> 2406
gactacgctc gcaaaggct

<212> DNA<213> Homo sapiens<220> <223> Cyclin F ribozyme binding site
<400> 2407
cgcagctgtc tttagccaa

<212> DNA<213> Homo sapiens<220> <223> Cyclin F ribozyme binding site
<400> 2408
cagctgtctt tagccaaag

<212> DNA<213> Homo sapiens<220> <223> Cyclin F ribozyme binding site
<400> 2409
agctgtcttt agccaaagc

<212> DNA<213> Homo sapiens<220> <223> Cyclin F ribozyme binding site
<400> 2410
gctgtcttta gccaaagcc

<212> DNA<213> Homo sapiens<220> <223> Cyclin F ribozyme binding site
<400> 2411
aaaccagctt ggactggag

<212> DNA<213> Homo sapiens<220> <223> Cyclin F ribozyme binding site
<400> 2412
gtgagagctt ccagtgaga

<212> DNA<213> Homo sapiens<220> <223> Cyclin F ribozyme binding site
<400> 2413
tgagagcttc cagtgagat

<212> DNA<213> Homo sapiens<220> <223> Cyclin F ribozyme binding site
<400> 2414
cagtgagatc gtctgccag

<212> DNA<213> Homo sapiens<220> <223> Cyclin F ribozyme binding site
<400> 2415
tgagatcgtc tgccagcta

<212> DNA<213> Homo sapiens<220> <223> Cyclin F ribozyme binding site
<400> 2416
ctgccagcta tttcaggct

<212> DNA<213> Homo sapiens<220> <223> Cyclin F ribozyme binding site
<400> 2417
gccagctatt tcaggcttc

<212> DNA<213> Homo sapiens<220> <223> Cyclin F ribozyme binding site
<400> 2418
ccagctattt caggcttcc

<212> DNA<213> Homo sapiens<220> <223> Cyclin F ribozyme binding site
<400> 2419
cagctatttc aggcttccc

<212> DNA<213> Homo sapiens<220> <223> Cyclin F ribozyme binding site
<400> 2420
tttcaggctt cccaggctg

<212> DNA<213> Homo sapiens<220> <223> Cyclin F ribozyme binding site
<400> 2421
ttcaggcttc ccaggctgt

<212> DNA<213> Homo sapiens<220> <223> Cyclin F ribozyme binding site
<400> 2422
ccaggctgtc agtaaacaa

<212> DNA<213> Homo sapiens<220> <223> Cyclin F ribozyme binding site
<400> 2423
gctgtcagta aacaacaag

<212> DNA<213> Homo sapiens<220> <223> Cyclin F ribozyme binding site
<400> 2424
acaacaagtc ttctccgtg

<212> DNA<213> Homo sapiens<220> <223> Cyclin F ribozyme binding site
<400> 2425
aacaagtctt ctccgtgca

<212> DNA<213> Homo sapiens<220> <223> Cyclin F ribozyme binding site
<400> 2426
acaagtcttc tccgtgcag

<212> DNA<213> Homo sapiens<220> <223> Cyclin F ribozyme binding site
<400> 2427
aagtcttctc cgtgcagaa

<212> DNA<213> Homo sapiens<220> <223> Cyclin F ribozyme binding site
<400> 2428
gaagggactc aatgacaca

<212> DNA<213> Homo sapiens<220> <223> Cyclin F ribozyme binding site
<400> 2429
caatgaggta cattctgat

<212> DNA<213> Homo sapiens<220> <223> Cyclin F ribozyme binding site
<400> 2430
gaggtacatt ctgatcgac

<212> DNA<213> Homo sapiens<220> <223> Cyclin F ribozyme binding site
<400> 2431
aggtacattc tgatcgact

<212> DNA<213> Homo sapiens<220> <223> Cyclin F ribozyme binding site
<400> 2432
cattctgatc gactggctg

<212> DNA<213> Homo sapiens<220> <223> Cyclin F ribozyme binding site
<400> 2433
ggtggaagtt gccaccatg

<212> DNA<213> Homo sapiens<220> <223> Cyclin F ribozyme binding site
<400> 2434
tgaatgactt cacaagcct

<212> DNA<213> Homo sapiens<220> <223> Cyclin F ribozyme binding site
<400> 2435
gaatgacttc acaagcctg

<212> DNA<213> Homo sapiens<220> <223> Cyclin F ribozyme binding site
<400> 2436
tggaccggta cctgcggag

<212> DNA<213> Homo sapiens<220> <223> Cyclin F ribozyme binding site
<400> 2437
tgccgcggta caggctcca

<212> DNA<213> Homo sapiens<220> <223> Cyclin F ribozyme binding site
<400> 2438
gtacaggctc cagctgctg

<212> DNA<213> Homo sapiens<220> <223> Cyclin F ribozyme binding site
<400> 2439
gctgggcatc gcctgcatg

<212> DNA<213> Homo sapiens<220> <223> Cyclin F ribozyme binding site
<400> 2440
ctgcatggtc atctgcacc

<212> DNA<213> Homo sapiens<220> <223> Cyclin F ribozyme binding site
<400> 2441
catggtcatc tgcacccgg

<212> DNA<213> Homo sapiens<220> <223> Cyclin F ribozyme binding site
<400> 2442
gcacccggtt tatcagtaa

<212> DNA<213> Homo sapiens<220> <223> Cyclin F ribozyme binding site
<400> 2443
cacccggttt atcagtaaa

<212> DNA<213> Homo sapiens<220> <223> Cyclin F ribozyme binding site
<400> 2444
acccggttta tcagtaaag

<212> DNA<213> Homo sapiens<220> <223> Cyclin F ribozyme binding site
<400> 2445
ccggtttatc agtaaagag

<212> DNA<213> Homo sapiens<220> <223> Cyclin F ribozyme binding site
<400> 2446
tttatcagta aagagatcc

<212> DNA<213> Homo sapiens<220> <223> Cyclin F ribozyme binding site
<400> 2447

taaagagatc ctgaccatc

<212> DNA<213> Homo sapiens<220> <223> Cyclin F ribozyme binding site
<400> 2448
cctgaccatc cgggaggcc

<212> DNA<213> Homo sapiens<220> <223> Cyclin F ribozyme binding site
<400> 2449
ggaggccgta tggctcacg

<212> DNA<213> Homo sapiens<220> <223> Cyclin F ribozyme binding site
<400> 2450
cgtatggctc acggacaac

<212> DNA<213> Homo sapiens<220> <223> Cyclin F ribozyme binding site
<400> 2451
gacaacactt acaagtacg

<212> DNA<213> Homo sapiens<220> <223> Cyclin F ribozyme binding site
<400> 2452
acaacactta caagtacga

<212> DNA<213> Homo sapiens<220> <223> Cyclin F ribozyme binding site
<400> 2453
cttacaagta cgaggacct

<212> DNA<213> Homo sapiens<220> <223> Cyclin F ribozyme binding site
<400> 2454
gggcgagatc gtctccgcc

<212> DNA<213> Homo sapiens<220> <223> Cyclin F ribozyme binding site
<400> 2455
cgagatcgtc tccgccttg

<212> DNA<213> Homo sapiens<220> <223> Cyclin F ribozyme binding site
<400> 2456
agatcgtctc cgccttgga

<212> DNA<213> Homo sapiens<220> <223> Cyclin F ribozyme binding site
<400> 2457
tctccgcctt ggaagggaa

<212> DNA<213> Homo sapiens<220> <223> Cyclin F ribozyme binding site
<400> 2458
agggaagatt cgagtcccc

<212> DNA<213> Homo sapiens<220> <223> Cyclin F ribozyme binding site
<400> 2459
gggaagattc gagtcccca

<212> DNA<213> Homo sapiens<220> <223> Cyclin F ribozyme binding site
<400> 2460
gattcgagtc cccactgtg

<212> DNA<213> Homo sapiens<220> <223> Cyclin F ribozyme binding site

<400> 2461
gtggtggatt acaaggagg

<212> DNA<213> Homo sapiens<220> <223> Cyclin F ribozyme binding site
<400> 2462
tggtggatta caaggaggt

<212> DNA<213> Homo sapiens<220> <223> Cyclin F ribozyme binding site
<400> 2463
caaggaggtc ctgctgacg

<212> DNA<213> Homo sapiens<220> <223> Cyclin F ribozyme binding site
<400> 2464
gctgacgcta gtccctgtg

<212> DNA<213> Homo sapiens<220> <223> Cyclin F ribozyme binding site
<400> 2465
gacgctagtc cctgtggag

<212> DNA<213> Homo sapiens<220> <223> Cyclin F ribozyme binding site
<400> 2466
tgtgcagctt cctctgcga

<212> DNA<213> Homo sapiens<220> <223> Cyclin F ribozyme binding site
<400> 2467
gtgcagcttc ctctgcgag

<212> DNA<213> Homo sapiens<220> <223> Cyclin F ribozyme binding site
<400> 2468
cagcttcctc tgcgagctc

<212> DNA<213> Homo sapiens<220> <223> Cyclin F ribozyme binding site
<400> 2469
ctgcgagctc tccctgctg

<212> DNA<213> Homo sapiens<220> <223> Cyclin F ribozyme binding site
<400> 2470
gcgagctctc cctgctgca

<212> DNA<213> Homo sapiens<220> <223> Cyclin F ribozyme binding site
<400> 2471
ccagcctgtc cgcctacgc

<212> DNA<213> Homo sapiens<220> <223> Cyclin F ribozyme binding site
<400> 2472
tgtccgccta cgccccagc

<212> DNA<213> Homo sapiens<220> <223> Cyclin F ribozyme binding site
<400> 2473
agccctgctc ctggccaga

<212> DNA<213> Homo sapiens<220> <223> Cyclin F ribozyme binding site
<400> 2474
tggaccactc agctgtggg

<212> DNA<213> Homo sapiens<220> <223> Cyclin F ribozyme binding site
<400> 2475
gtgggacctc accggattc

<212> DNA<213> Homo sapiens<220> <223> Cyclin F ribozyme binding site
<400> 2476
tcaccggatt ctcctatga

<212> DNA<213> Homo sapiens<220> <223> Cyclin F ribozyme binding site
<400> 2477
caccggattc tcctatgaa

<212> DNA<213> Homo sapiens<220> <223> Cyclin F ribozyme binding site
<400> 2478
ccggattctc ctatgaaga

<212> DNA<213> Homo sapiens<220> <223> Cyclin F ribozyme binding site
<400> 2479
gattctccta tgaagacct

<212> DNA<213> Homo sapiens<220> <223> Cyclin F ribozyme binding site
<400> 2480
tgaagacctc attccctgc

<212> DNA<213> Homo sapiens<220> <223> Cyclin F ribozyme binding site
<400> 2481
agacctcatt ccctgcgtc

<212> DNA<213> Homo sapiens<220> <223> Cyclin F ribozyme binding site
<400> 2482
gacctcattc cctgcgtct

<212> DNA<213> Homo sapiens<220> <223> Cyclin F ribozyme binding site
<400> 2483
tccctgcgtc ttgagcctc

<212> DNA<213> Homo sapiens<220> <223> Cyclin F ribozyme binding site
<400> 2484
cctgcgtctt gagcctcca

<212> DNA<213> Homo sapiens<220> <223> Cyclin F ribozyme binding site
<400> 2485
cttgagcctc cataagaag

<212> DNA<213> Homo sapiens<220> <223> Cyclin F ribozyme binding site
<400> 2486
agcctccata agaagtgct

<212> DNA<213> Homo sapiens<220> <223> Cyclin F ribozyme binding site
<400> 2487
agaagtgctt ccatgatga

<212> DNA<213> Homo sapiens<220> <223> Cyclin F ribozyme binding site
<400> 2488
gaagtgcttc catgatgac

<212> DNA<213> Homo sapiens<220> <223> Cyclin F ribozyme binding site
<400> 2489
ccaaggacta caggcaagt

<212> DNA<213> Homo sapiens<220> <223> Cyclin F ribozyme binding site
<400> 2490
caggcaagtc tctctgacc

<212> DNA<213> Homo sapiens<220> <223> Cyclin F ribozyme binding site
<400> 2491
ggcaagtctc tctgaccgc

<212> DNA<213> Homo sapiens<220> <223> Cyclin F ribozyme binding site
<400> 2492
caagtctctc tgaccgccg

<212> DNA<213> Homo sapiens<220> <223> Cyclin F ribozyme binding site
<400> 2493
agcagcggtt tgaggacaa

<212> DNA<213> Homo sapiens<220> <223> Cyclin F ribozyme binding site
<400> 2494
gcagcggttt gaggacaag

<212> DNA<213> Homo sapiens<220> <223> Cyclin F ribozyme binding site
<400> 2495
acaagcgcta tggagaaat

<212> DNA<213> Homo sapiens<220> <223> Cyclin F ribozyme binding site
<400> 2496
tggagaaatc agccaggaa

<212> DNA<213> Homo sapiens<220> <223> Cyclin F ribozyme binding site
<400> 2497
tgctgagcta cagccagtt

<212> DNA<213> Homo sapiens<220> <223> Cyclin F ribozyme binding site
<400> 2498
acagccagtt gtgtgctgc

<212> DNA<213> Homo sapiens<220> <223> Cyclin F ribozyme binding site
<400> 2499
gtgctgcatt aggagtgac

<212> DNA<213> Homo sapiens<220> <223> Cyclin F ribozyme binding site
<400> 2500
tgctgcatta ggagtgaca

<212> DNA<213> Homo sapiens<220> <223> Cyclin F ribozyme binding site
<400> 2501
cccccgactt tcctcagca

<212> DNA<213> Homo sapiens<220> <223> Cyclin F ribozyme binding site
<400> 2502

ccccgacttt cctcagcac

<212> DNA<213> Homo sapiens<220> <223> Cyclin F ribozyme binding site
<400> 2503
cccgactttc ctcagcaca

<212> DNA<213> Homo sapiens<220> <223> Cyclin F ribozyme binding site
<400> 2504
gactttcctc agcacaggg

<212> DNA<213> Homo sapiens<220> <223> Cyclin F ribozyme binding site
<400> 2505
aggggagatc cacgccttc

<212> DNA<213> Homo sapiens<220> <223> Cyclin F ribozyme binding site
<400> 2506
tccacgcctt cctcagctc

<212> DNA<213> Homo sapiens<220> <223> Cyclin F ribozyme binding site
<400> 2507
ccacgccttc ctcagctct

<212> DNA<213> Homo sapiens<220> <223> Cyclin F ribozyme binding site
<400> 2508
cgccttcctc agctctccc

<212> DNA<213> Homo sapiens<220> <223> Cyclin F ribozyme binding site
<400> 2509
tcctcagctc tccctcggg

<212> DNA<213> Homo sapiens<220> <223> Cyclin F ribozyme binding site
<400> 2510
ctcagctctc cctcggggc

<212> DNA<213> Homo sapiens<220> <223> Cyclin F ribozyme binding site
<400> 2511
gctctccctc ggggcggag

<212> DNA<213> Homo sapiens<220> <223> Cyclin F ribozyme binding site
<400> 2512
gaacagcctc caggaagac

<212> DNA<213> Homo sapiens<220> <223> Cyclin F ribozyme binding site
<400> 2513
gaggcagctt cgttaccac

<212> DNA<213> Homo sapiens<220> <223> Cyclin F ribozyme binding site
<400> 2514
aggcagcttc gttaccacc

<212> DNA<213> Homo sapiens<220> <223> Cyclin F ribozyme binding site
<400> 2515
cagcttcgtt accaccccc

<212> DNA<213> Homo sapiens<220> <223> Cyclin F ribozyme binding site

<400> 2516
agcttcgtta ccacccccca

<212> DNA<213> Homo sapiens<220> <223> Cyclin F ribozyme binding site
<400> 2517
cggagctgtc cagccagga

<212> DNA<213> Homo sapiens<220> <223> Cyclin F ribozyme binding site
<400> 2518
tgggcagctt cctcgactg

<212> DNA<213> Homo sapiens<220> <223> Cyclin F ribozyme binding site
<400> 2519
gggcagcttc ctcgactgg

<212> DNA<213> Homo sapiens<220> <223> Cyclin F ribozyme binding site
<400> 2520
cagcttcctc gactggagc

<212> DNA<213> Homo sapiens<220> <223> Cyclin F ribozyme binding site
<400> 2521
actgctgctc tggctatga

<212> DNA<213> Homo sapiens<220> <223> Cyclin F ribozyme binding site
<400> 2522
gctctggcta tgaaggcga

<212> DNA<213> Homo sapiens<220> <223> Cyclin F ribozyme binding site
<400> 2523
gtgacagctc ccagcggca

<212> DNA<213> Homo sapiens<220> <223> Cyclin F ribozyme binding site
<400> 2524
cagcggcatc ctcgatgtc

<212> DNA<213> Homo sapiens<220> <223> Cyclin F ribozyme binding site
<400> 2525
cggcatcctc gatgtcacc

<212> DNA<213> Homo sapiens<220> <223> Cyclin F ribozyme binding site
<400> 2526
cctcgatgtc accgtggtc

<212> DNA<213> Homo sapiens<220> <223> Cyclin F ribozyme binding site
<400> 2527
caccgtggtc tacctgaac

<212> DNA<213> Homo sapiens<220> <223> Cyclin F ribozyme binding site
<400> 2528
ccgtggtcta cctgaaccc

<212> DNA<213> Homo sapiens<220> <223> Cyclin F ribozyme binding site
<400> 2529
gaacagcatt gctgccagg

<212> DNA<213> Homo sapiens<220> <223> Cyclin F ribozyme binding site
<400> 2530
gccaggaatc cagtgatga

<212> DNA<213> Homo sapiens<220> <223> Cyclin F ribozyme binding site
<400> 2531
gaggaggctt gtccagagg

<212> DNA<213> Homo sapiens<220> <223> Cyclin F ribozyme binding site
<400> 2532
gaggcttgtc cagaggcaa

<212> DNA<213> Homo sapiens<220> <223> Cyclin F ribozyme binding site
<400> 2533
cacccagatc cctgcaacc

<212> DNA<213> Homo sapiens<220> <223> Cyclin F ribozyme binding site
<400> 2534
acccctggtc cgcaccagc

<212> DNA<213> Homo sapiens<220> <223> Cyclin F ribozyme binding site
<400> 2535
gaaggacgtc acgacctca

<212> DNA<213> Homo sapiens<220> <223> Cyclin F ribozyme binding site
<400> 2536
tcacgacctc agggtactc

<212> DNA<213> Homo sapiens<220> <223> Cyclin F ribozyme binding site
<400> 2537
cctcagggta ctcctccgt

<212> DNA<213> Homo sapiens<220> <223> Cyclin F ribozyme binding site
<400> 2538
cagggtactc ctccgtcag

<212> DNA<213> Homo sapiens<220> <223> Cyclin F ribozyme binding site
<400> 2539
ggtactcctc cgtcagcac

<212> DNA<213> Homo sapiens<220> <223> Cyclin F ribozyme binding site
<400> 2540
ctcctccgtc agcaccgca

<212> DNA<213> Homo sapiens<220> <223> Cyclin F ribozyme binding site
<400> 2541
accgcaagtc ccacaagct

<212> DNA<213> Homo sapiens<220> <223> Cyclin F ribozyme binding site
<400> 2542
ccacaagctc cgtggacgg

<212> DNA<213> Homo sapiens<220> <223> Cyclin F ribozyme binding site
<400> 2543
acggtggctt gggggccct

<212> DNA<213> Homo sapiens<220> <223> Cyclin F ribozyme binding site
<400> 2544
ccccaaccta cctcagtgc

<212> DNA<213> Homo sapiens<220> <223> Cyclin F ribozyme binding site
<400> 2545
aacctacctc agtgctgtc

<212> DNA<213> Homo sapiens<220> <223> Cyclin F ribozyme binding site
<400> 2546
cagtgctgtc cctgcacag

<212> DNA<213> Homo sapiens<220> <223> Cyclin F ribozyme binding site
<400> 2547
acagtgactc gcacacaca

<212> DNA<213> Homo sapiens<220> <223> Cyclin F ribozyme binding site
<400> 2548
tgccaccatc aggccagga

<212> DNA<213> Homo sapiens<220> <223> Cyclin F ribozyme binding site
<400> 2549
ccaggaagtc atgtttaca

<212> DNA<213> Homo sapiens<220> <223> Cyclin F ribozyme binding site
<400> 2550
aagtcatgtt tacagtgtc

<212> DNA<213> Homo sapiens<220> <223> Cyclin F ribozyme binding site
<400> 2551
agtcatgttt acagtgtcg

<212> DNA<213> Homo sapiens<220> <223> Cyclin F ribozyme binding site
<400> 2552
gtcatgttta cagtgtcgt

<212> DNA<213> Homo sapiens<220> <223> Cyclin F ribozyme binding site
<400> 2553
ttacagtgtc gtcccccaa

<212> DNA<213> Homo sapiens<220> <223> Cyclin F ribozyme binding site
<400> 2554
cagtgtcgtc ccccaagtc

<212> DNA<213> Homo sapiens<220> <223> Cyclin F ribozyme binding site
<400> 2555
cccccaagtc ccccggaga

<212> DNA<213> Homo sapiens<220> <223> Cyclin F ribozyme binding site
<400> 2556
gagcagtgtt ccccagcaa

<212> DNA<213> Homo sapiens<220> <223> Cyclin F ribozyme binding site
<400> 2557

agcagtgttc cccagcaac

<212> DNA<213> Homo sapiens<220> <223> Cyclin F ribozyme binding site
<400> 2558
gaagcggata aacctatgc

<212> DNA<213> Homo sapiens<220> <223> Cyclin F ribozyme binding site
<400> 2559
gataaaccta tgcatacac

<212> DNA<213> Homo sapiens<220> <223> Cyclin F ribozyme binding site
<400> 2560
cctatgcata cacagtgag

<212> DNA<213> Homo sapiens<220> <223> Cyclin F ribozyme binding site
<400> 2561
cctgggcctt gtgaggctg

<212> DNA<213> Homo sapiens<220> <223> Cyclin G1 ribozyme binding site
<400> 2562
gggccgagtt gtctcggcg

<212> DNA<213> Homo sapiens<220> <223> Cyclin G1 ribozyme binding site
<400> 2563
ccgagttgtc tcggcggcg

<212> DNA<213> Homo sapiens<220> <223> Cyclin G1 ribozyme binding site
<400> 2564
ccgaggcctc cacccagga

<212> DNA<213> Homo sapiens<220> <223> Cyclin G1 ribozyme binding site
<400> 2565
caggacagtc cccctcccc

<212> DNA<213> Homo sapiens<220> <223> Cyclin G1 ribozyme binding site
<400> 2566
agtccccctc cccgggcct

<212> DNA<213> Homo sapiens<220> <223> Cyclin G1 ribozyme binding site
<400> 2567
cccgggcctc tctcctctt

<212> DNA<213> Homo sapiens<220> <223> Cyclin G1 ribozyme binding site
<400> 2568
cgggcctctc tcctcttgc

<212> DNA<213> Homo sapiens<220> <223> Cyclin G1 ribozyme binding site
<400> 2569
ggcctctctc ctcttgcct

<212> DNA<213> Homo sapiens<220> <223> Cyclin G1 ribozyme binding site
<400> 2570
ctctctcctc ttgcctacg

<212> DNA<213> Homo sapiens<220> <223> Cyclin G1 ribozyme binding site

<400> 2571
ctctcctctt gcctacgag

<212> DNA<213> Homo sapiens<220> <223> Cyclin G1 ribozyme binding site
<400> 2572
ctcttgccta cgagtcccc

<212> DNA<213> Homo sapiens<220> <223> Cyclin G1 ribozyme binding site
<400> 2573
cctacgagtc cccctctcc

<212> DNA<213> Homo sapiens<220> <223> Cyclin G1 ribozyme binding site
<400> 2574
agtcccccctc tcctcgtag

<212> DNA<213> Homo sapiens<220> <223> Cyclin G1 ribozyme binding site
<400> 2575
tccccctctc ctcgtaggc

<212> DNA<213> Homo sapiens<220> <223> Cyclin G1 ribozyme binding site
<400> 2576
ccctctcctc gtaggcctc

<212> DNA<213> Homo sapiens<220> <223> Cyclin G1 ribozyme binding site
<400> 2577
tctcctcgta ggcctctcg

<212> DNA<213> Homo sapiens<220> <223> Cyclin G1 ribozyme binding site
<400> 2578
cgtaggcctc tcggatctg

<212> DNA<213> Homo sapiens<220> <223> Cyclin G1 ribozyme binding site
<400> 2579
taggcctctc ggatctgat

<212> DNA<213> Homo sapiens<220> <223> Cyclin G1 ribozyme binding site
<400> 2580
ctctcggatc tgatatcgt

<212> DNA<213> Homo sapiens<220> <223> Cyclin G1 ribozyme binding site
<400> 2581
ggatctgata tcgtggggt

<212> DNA<213> Homo sapiens<220> <223> Cyclin G1 ribozyme binding site
<400> 2582
atctgatatc gtggggtga

<212> DNA<213> Homo sapiens<220> <223> Cyclin G1 ribozyme binding site
<400> 2583
gagggtggtt accgtgagg

<212> DNA<213> Homo sapiens<220> <223> Cyclin G1 ribozyme binding site
<400> 2584
agggtggtta ccgtgagga

<212> DNA<213> Homo sapiens<220> <223> Cyclin G1 ribozyme binding site
<400> 2585
agctgcagtc tctgtcaag

<212> DNA<213> Homo sapiens<220> <223> Cyclin G1 ribozyme binding site
<400> 2586
ctgcagtctc tgtcaagat

<212> DNA<213> Homo sapiens<220> <223> Cyclin G1 ribozyme binding site
<400> 2587
agtctctgtc aagatgata

<212> DNA<213> Homo sapiens<220> <223> Cyclin G1 ribozyme binding site
<400> 2588
caagatgata gaggtactg

<212> DNA<213> Homo sapiens<220> <223> Cyclin G1 ribozyme binding site
<400> 2589
gatagaggta ctgacaaca

<212> DNA<213> Homo sapiens<220> <223> Cyclin G1 ribozyme binding site
<400> 2590
caactgactc tcagaaact

<212> DNA<213> Homo sapiens<220> <223> Cyclin G1 ribozyme binding site
<400> 2591
actgactctc agaaactgc

<212> DNA<213> Homo sapiens<220> <223> Cyclin G1 ribozyme binding site
<400> 2592
gaaactgcta caccagctg

<212> DNA<213> Homo sapiens<220> <223> Cyclin G1 ribozyme binding site
<400> 2593
atgccctgtt ggaacagga

<212> DNA<213> Homo sapiens<220> <223> Cyclin G1 ribozyme binding site
<400> 2594
aacaggagtc tagatgtca

<212> DNA<213> Homo sapiens<220> <223> Cyclin G1 ribozyme binding site
<400> 2595
caggagtcta gatgtcagc

<212> DNA<213> Homo sapiens<220> <223> Cyclin G1 ribozyme binding site
<400> 2596
tctagatgtc agccaaagg

<212> DNA<213> Homo sapiens<220> <223> Cyclin G1 ribozyme binding site
<400> 2597
gccaaaggtc tgtggtttg

<212> DNA<213> Homo sapiens<220> <223> Cyclin G1 ribozyme binding site
<400> 2598
gtctgtggtt tgagactaa

<212> DNA<213> Homo sapiens<220> <223> Cyclin G1 ribozyme binding site
<400> 2599
tctgtggttt gagactaat

<212> DNA<213> Homo sapiens<220> <223> Cyclin G1 ribozyme binding site
<400> 2600
tttgagacta attgagtct

<212> DNA<213> Homo sapiens<220> <223> Cyclin G1 ribozyme binding site
<400> 2601
gagactaatt gagtctgca

<212> DNA<213> Homo sapiens<220> <223> Cyclin G1 ribozyme binding site
<400> 2602
taattgagtc tgcacacga

<212> DNA<213> Homo sapiens<220> <223> Cyclin G1 ribozyme binding site
<400> 2603
gcacacgata atggcctca

<212> DNA<213> Homo sapiens<220> <223> Cyclin G1 ribozyme binding site
<400> 2604
taatggcctc agaatgact

<212> DNA<213> Homo sapiens<220> <223> Cyclin G1 ribozyme binding site
<400> 2605
tgcaagacta agggacttt

<212> DNA<213> Homo sapiens<220> <223> Cyclin G1 ribozyme binding site
<400> 2606
taagggactt tgaagtaaa

<212> DNA<213> Homo sapiens<220> <223> Cyclin G1 ribozyme binding site
<400> 2607
aagggacttt gaagtaaaa

<212> DNA<213> Homo sapiens<220> <223> Cyclin G1 ribozyme binding site
<400> 2608
ctttgaagta aaagatctt

<212> DNA<213> Homo sapiens<220> <223> Cyclin G1 ribozyme binding site
<400> 2609
gtaaaagatc ttcttagtc

<212> DNA<213> Homo sapiens<220> <223> Cyclin G1 ribozyme binding site
<400> 2610
aaaagatctt cttagtcta

<212> DNA<213> Homo sapiens<220> <223> Cyclin G1 ribozyme binding site
<400> 2611
aaagatcttc ttagtctaa

<212> DNA<213> Homo sapiens<220> <223> Cyclin G1 ribozyme binding site
<400> 2612

agatcttctt agtctaact

<212> DNA<213> Homo sapiens<220> <223> Cyclin G1 ribozyme binding site
<400> 2613
gatcttctta gtctaactc

<212> DNA<213> Homo sapiens<220> <223> Cyclin G1 ribozyme binding site
<400> 2614
cttcttagtc taactcagt

<212> DNA<213> Homo sapiens<220> <223> Cyclin G1 ribozyme binding site
<400> 2615
tcttagtcta actcagttc

<212> DNA<213> Homo sapiens<220> <223> Cyclin G1 ribozyme binding site
<400> 2616
agtctaactc agttctttg

<212> DNA<213> Homo sapiens<220> <223> Cyclin G1 ribozyme binding site
<400> 2617
taactcagtt ctttggctt

<212> DNA<213> Homo sapiens<220> <223> Cyclin G1 ribozyme binding site
<400> 2618
aactcagttc tttggcttt

<212> DNA<213> Homo sapiens<220> <223> Cyclin G1 ribozyme binding site
<400> 2619
ctcagttctt tggctttga

<212> DNA<213> Homo sapiens<220> <223> Cyclin G1 ribozyme binding site
<400> 2620
tcagttcttt ggctttgac

<212> DNA<213> Homo sapiens<220> <223> Cyclin G1 ribozyme binding site
<400> 2621
tctttggctt tgacacaga

<212> DNA<213> Homo sapiens<220> <223> Cyclin G1 ribozyme binding site
<400> 2622
ctttggcttt gacacagag

<212> DNA<213> Homo sapiens<220> <223> Cyclin G1 ribozyme binding site
<400> 2623
cagagacatt ttctctagc

<212> DNA<213> Homo sapiens<220> <223> Cyclin G1 ribozyme binding site
<400> 2624
agagacattt tctctagct

<212> DNA<213> Homo sapiens<220> <223> Cyclin G1 ribozyme binding site
<400> 2625
gagacatttt ctctagctg

<212> DNA<213> Homo sapiens<220> <223> Cyclin G1 ribozyme binding site

<400> 2626
agacatttc tctagctgt

<212> DNA<213> Homo sapiens<220> <223> Cyclin G1 ribozyme binding site
<400> 2627
acatttctc tagctgtga

<212> DNA<213> Homo sapiens<220> <223> Cyclin G1 ribozyme binding site
<400> 2628
atttctcta gctgtgaat

<212> DNA<213> Homo sapiens<220> <223> Cyclin G1 ribozyme binding site
<400> 2629
gctgtgaatt tactggaca

<212> DNA<213> Homo sapiens<220> <223> Cyclin G1 ribozyme binding site
<400> 2630
ctgtgaattt actggacag

<212> DNA<213> Homo sapiens<220> <223> Cyclin G1 ribozyme binding site
<400> 2631
tgtgaattta ctggacaga

<212> DNA<213> Homo sapiens<220> <223> Cyclin G1 ribozyme binding site
<400> 2632
tggacagatt cctgtctaa

<212> DNA<213> Homo sapiens<220> <223> Cyclin G1 ribozyme binding site
<400> 2633
ggacagattc ctgtctaaa

<212> DNA<213> Homo sapiens<220> <223> Cyclin G1 ribozyme binding site
<400> 2634
gattcctgtc taaaatgaa

<212> DNA<213> Homo sapiens<220> <223> Cyclin G1 ribozyme binding site
<400> 2635
ttcctgtcta aaatgaagg

<212> DNA<213> Homo sapiens<220> <223> Cyclin G1 ribozyme binding site
<400> 2636
aatgaaggta cagcccaag

<212> DNA<213> Homo sapiens<220> <223> Cyclin G1 ribozyme binding site
<400> 2637
caagcacctt gggtgtgtt

<212> DNA<213> Homo sapiens<220> <223> Cyclin G1 ribozyme binding site
<400> 2638
tgggtgtgtt ggactgagc

<212> DNA<213> Homo sapiens<220> <223> Cyclin G1 ribozyme binding site
<400> 2639
tgagctgctt ttatttggc

<212> DNA<213> Homo sapiens<220> <223> Cyclin G1 ribozyme binding site
<400> 2640
gagctgcttt tatttggct

<212> DNA<213> Homo sapiens<220> <223> Cyclin G1 ribozyme binding site
<400> 2641
agctgctttt atttggctg

<212> DNA<213> Homo sapiens<220> <223> Cyclin G1 ribozyme binding site
<400> 2642
gctgctttta tttggctgt

<212> DNA<213> Homo sapiens<220> <223> Cyclin G1 ribozyme binding site
<400> 2643
tgcttttatt tggctgtaa

<212> DNA<213> Homo sapiens<220> <223> Cyclin G1 ribozyme binding site
<400> 2644
gcttttattt ggctgtaaa

<212> DNA<213> Homo sapiens<220> <223> Cyclin G1 ribozyme binding site
<400> 2645
tttggctgta aaatcaata

<212> DNA<213> Homo sapiens<220> <223> Cyclin G1 ribozyme binding site
<400> 2646
ctgtaaaatc aatagaaga

<212> DNA<213> Homo sapiens<220> <223> Cyclin G1 ribozyme binding site
<400> 2647
aaaatcaata gaagaggaa

<212> DNA<213> Homo sapiens<220> <223> Cyclin G1 ribozyme binding site
<400> 2648
aaggaatgtc ccattggca

<212> DNA<213> Homo sapiens<220> <223> Cyclin G1 ribozyme binding site
<400> 2649
atgtcccatt ggcaactga

<212> DNA<213> Homo sapiens<220> <223> Cyclin G1 ribozyme binding site
<400> 2650
caactgactt gatccgaat

<212> DNA<213> Homo sapiens<220> <223> Cyclin G1 ribozyme binding site
<400> 2651
tgacttgatc cgaataagt

<212> DNA<213> Homo sapiens<220> <223> Cyclin G1 ribozyme binding site
<400> 2652
gatccgaata agtcaatat

<212> DNA<213> Homo sapiens<220> <223> Cyclin G1 ribozyme binding site
<400> 2653
cgaataagtc aatataggt

<212> DNA<213> Homo sapiens<220> <223> Cyclin G1 ribozyme binding site
<400> 2654
taagtcaata taggtttac

<212> DNA<213> Homo sapiens<220> <223> Cyclin G1 ribozyme binding site
<400> 2655
agtcaatata ggtttacgg

<212> DNA<213> Homo sapiens<220> <223> Cyclin G1 ribozyme binding site
<400> 2656
aatataggtt tacggtttc

<212> DNA<213> Homo sapiens<220> <223> Cyclin G1 ribozyme binding site
<400> 2657
atataggttt acggtttca

<212> DNA<213> Homo sapiens<220> <223> Cyclin G1 ribozyme binding site
<400> 2658
tataggttta cggtttcag

<212> DNA<213> Homo sapiens<220> <223> Cyclin G1 ribozyme binding site
<400> 2659
gtttacggtt tcagacttg

<212> DNA<213> Homo sapiens<220> <223> Cyclin G1 ribozyme binding site
<400> 2660
tttacggttt cagacttga

<212> DNA<213> Homo sapiens<220> <223> Cyclin G1 ribozyme binding site
<400> 2661
ttacggtttc agacttgat

<212> DNA<213> Homo sapiens<220> <223> Cyclin G1 ribozyme binding site
<400> 2662
tttcagactt gatgagaat

<212> DNA<213> Homo sapiens<220> <223> Cyclin G1 ribozyme binding site
<400> 2663
ggaaaagatt gtattggag

<212> DNA<213> Homo sapiens<220> <223> Cyclin G1 ribozyme binding site
<400> 2664
aaagattgta ttggagaag

<212> DNA<213> Homo sapiens<220> <223> Cyclin G1 ribozyme binding site
<400> 2665
agattgtatt ggagaaggt

<212> DNA<213> Homo sapiens<220> <223> Cyclin G1 ribozyme binding site
<400> 2666
aaggtgtgtt ggaaagtca

<212> DNA<213> Homo sapiens<220> <223> Cyclin G1 ribozyme binding site
<400> 2667

ttggaaagtc aaagctact

<212> DNA<213> Homo sapiens<220> <223> Cyclin G1 ribozyme binding site
<400> 2668
gtcaaagcta ctactgcct

<212> DNA<213> Homo sapiens<220> <223> Cyclin G1 ribozyme binding site
<400> 2669
aaagctacta ctgcctttc

<212> DNA<213> Homo sapiens<220> <223> Cyclin G1 ribozyme binding site
<400> 2670
ctactgcctt tcaatttct

<212> DNA<213> Homo sapiens<220> <223> Cyclin G1 ribozyme binding site
<400> 2671
tactgccttt caatttctg

<212> DNA<213> Homo sapiens<220> <223> Cyclin G1 ribozyme binding site
<400> 2672
actgcctttc aatttctgc

<212> DNA<213> Homo sapiens<220> <223> Cyclin G1 ribozyme binding site
<400> 2673
cctttcaatt tctgcaact

<212> DNA<213> Homo sapiens<220> <223> Cyclin G1 ribozyme binding site
<400> 2674
ctttcaattt ctgcaactg

<212> DNA<213> Homo sapiens<220> <223> Cyclin G1 ribozyme binding site
<400> 2675
tttcaatttc tgcaactgt

<212> DNA<213> Homo sapiens<220> <223> Cyclin G1 ribozyme binding site
<400> 2676
tgcaactgta ttattcact

<212> DNA<213> Homo sapiens<220> <223> Cyclin G1 ribozyme binding site
<400> 2677
caactgtatt attcactcc

<212> DNA<213> Homo sapiens<220> <223> Cyclin G1 ribozyme binding site
<400> 2678
aactgtatta ttcactcct

<212> DNA<213> Homo sapiens<220> <223> Cyclin G1 ribozyme binding site
<400> 2679
ctgtattatt cactccttc

<212> DNA<213> Homo sapiens<220> <223> Cyclin G1 ribozyme binding site
<400> 2680
tgtattattc actccttca

<212> DNA<213> Homo sapiens<220> <223> Cyclin G1 ribozyme binding site

<400> 2681
ttattcactc cttcaagag

<212> DNA<213> Homo sapiens<220> <223> Cyclin G1 ribozyme binding site
<400> 2682
ttcactcctt caagagaac

<212> DNA<213> Homo sapiens<220> <223> Cyclin G1 ribozyme binding site
<400> 2683
tcactccttc aagagaact

<212> DNA<213> Homo sapiens<220> <223> Cyclin G1 ribozyme binding site
<400> 2684
aagagaactt gccacttga

<212> DNA<213> Homo sapiens<220> <223> Cyclin G1 ribozyme binding site
<400> 2685
cttgccactt gaaaggaga

<212> DNA<213> Homo sapiens<220> <223> Cyclin G1 ribozyme binding site
<400> 2686
aggagaaata gcattaatt

<212> DNA<213> Homo sapiens<220> <223> Cyclin G1 ribozyme binding site
<400> 2687
aaatagcatt aattttgaa

<212> DNA<213> Homo sapiens<220> <223> Cyclin G1 ribozyme binding site
<400> 2688
agcattaatt ttgaaagac

<212> DNA<213> Homo sapiens<220> <223> Cyclin G1 ribozyme binding site
<400> 2689
gcattaattt tgaaagact

<212> DNA<213> Homo sapiens<220> <223> Cyclin G1 ribozyme binding site
<400> 2690
cattaatttt gaaagacta

<212> DNA<213> Homo sapiens<220> <223> Cyclin G1 ribozyme binding site
<400> 2691
tgaaagacta gaagctcaa

<212> DNA<213> Homo sapiens<220> <223> Cyclin G1 ribozyme binding site
<400> 2692
ctagaagctc aactgaagg

<212> DNA<213> Homo sapiens<220> <223> Cyclin G1 ribozyme binding site
<400> 2693
aaggcatgtc attgcagga

<212> DNA<213> Homo sapiens<220> <223> Cyclin G1 ribozyme binding site
<400> 2694
gcatgtcatt gcaggatca

<212> DNA<213> Homo sapiens<220> <223> Cyclin G1 ribozyme binding site
<400> 2695
ttgcaggatc atattttct

<212> DNA<213> Homo sapiens<220> <223> Cyclin G1 ribozyme binding site
<400> 2696
caggatcata ttttctaaa

<212> DNA<213> Homo sapiens<220> <223> Cyclin G1 ribozyme binding site
<400> 2697
ggatcatatt ttctaaagc

<212> DNA<213> Homo sapiens<220> <223> Cyclin G1 ribozyme binding site
<400> 2698
gatcatattt tctaaagca

<212> DNA<213> Homo sapiens<220> <223> Cyclin G1 ribozyme binding site
<400> 2699
atcatatttt ctaaagcaa

<212> DNA<213> Homo sapiens<220> <223> Cyclin G1 ribozyme binding site
<400> 2700
tcatattttc taaagcaaa

<212> DNA<213> Homo sapiens<220> <223> Cyclin G1 ribozyme binding site
<400> 2701
atattttcta aagcaaagc

<212> DNA<213> Homo sapiens<220> <223> Cyclin G1 ribozyme binding site
<400> 2702
gcaaagcctt ctgtgttgg

<212> DNA<213> Homo sapiens<220> <223> Cyclin G1 ribozyme binding site
<400> 2703
caaagccttc tgtgttggc

<212> DNA<213> Homo sapiens<220> <223> Cyclin G1 ribozyme binding site
<400> 2704
cttctgtgtt ggcattgtc

<212> DNA<213> Homo sapiens<220> <223> Cyclin G1 ribozyme binding site
<400> 2705
tgttggcatt gtctatcat

<212> DNA<213> Homo sapiens<220> <223> Cyclin G1 ribozyme binding site
<400> 2706
tggcattgtc tatcattgc

<212> DNA<213> Homo sapiens<220> <223> Cyclin G1 ribozyme binding site
<400> 2707
gcattgtcta tcattgcat

<212> DNA<213> Homo sapiens<220> <223> Cyclin G1 ribozyme binding site
<400> 2708
attgtctatc attgcatta

<212> DNA<213> Homo sapiens<220> <223> Cyclin G1 ribozyme binding site
<400> 2709
gtctatcatt gcattagag

<212> DNA<213> Homo sapiens<220> <223> Cyclin G1 ribozyme binding site
<400> 2710
tcattgcatt agagatcca

<212> DNA<213> Homo sapiens<220> <223> Cyclin G1 ribozyme binding site
<400> 2711
cattgcatta gagatccaa

<212> DNA<213> Homo sapiens<220> <223> Cyclin G1 ribozyme binding site
<400> 2712
attagagatc caagcacag

<212> DNA<213> Homo sapiens<220> <223> Cyclin G1 ribozyme binding site
<400> 2713
gaagtgtgta gagttaaca

<212> DNA<213> Homo sapiens<220> <223> Cyclin G1 ribozyme binding site
<400> 2714
gtgtagagtt aacagaagg

<212> DNA<213> Homo sapiens<220> <223> Cyclin G1 ribozyme binding site
<400> 2715
tgtagagtta acagaagga

<212> DNA<213> Homo sapiens<220> <223> Cyclin G1 ribozyme binding site
<400> 2716
agaaggaata gaatgtctt

<212> DNA<213> Homo sapiens<220> <223> Cyclin G1 ribozyme binding site
<400> 2717
atagaatgtc ttcagaaac

<212> DNA<213> Homo sapiens<220> <223> Cyclin G1 ribozyme binding site
<400> 2718
agaatgtctt cagaaacat

<212> DNA<213> Homo sapiens<220> <223> Cyclin G1 ribozyme binding site
<400> 2719
gaatgtcttc agaaacatt

<212> DNA<213> Homo sapiens<220> <223> Cyclin G1 ribozyme binding site
<400> 2720
cagaaacatt ccaagataa

<212> DNA<213> Homo sapiens<220> <223> Cyclin G1 ribozyme binding site
<400> 2721
agaaacattc caagataaa

<212> DNA<213> Homo sapiens<220> <223> Cyclin G1 ribozyme binding site
<400> 2722

ttccaagata aatggcaga

<212> DNA<213> Homo sapiens<220> <223> Cyclin G1 ribozyme binding site
<400> 2723
ggcagagatc tgaccttct

<212> DNA<213> Homo sapiens<220> <223> Cyclin G1 ribozyme binding site
<400> 2724
atctgacctt ctggcaaga

<212> DNA<213> Homo sapiens<220> <223> Cyclin G1 ribozyme binding site
<400> 2725
tctgaccttc tggcaagag

<212> DNA<213> Homo sapiens<220> <223> Cyclin G1 ribozyme binding site
<400> 2726
gcaagagctt gtatccaaa

<212> DNA<213> Homo sapiens<220> <223> Cyclin G1 ribozyme binding site
<400> 2727
agagcttgta tccaaatgt

<212> DNA<213> Homo sapiens<220> <223> Cyclin G1 ribozyme binding site
<400> 2728
agcttgtatc caaatgttt

<212> DNA<213> Homo sapiens<220> <223> Cyclin G1 ribozyme binding site
<400> 2729
tccaaatgtt taactgaat

<212> DNA<213> Homo sapiens<220> <223> Cyclin G1 ribozyme binding site
<400> 2730
ccaaatgttt aactgaata

<212> DNA<213> Homo sapiens<220> <223> Cyclin G1 ribozyme binding site
<400> 2731
caaatgttta actgaatat

<212> DNA<213> Homo sapiens<220> <223> Cyclin G1 ribozyme binding site
<400> 2732
taactgaata ttcatcaaa

<212> DNA<213> Homo sapiens<220> <223> Cyclin G1 ribozyme binding site
<400> 2733
actgaatatt catcaaata

<212> DNA<213> Homo sapiens<220> <223> Cyclin G1 ribozyme binding site
<400> 2734
ctgaatattc atcaaataa

<212> DNA<213> Homo sapiens<220> <223> Cyclin G1 ribozyme binding site
<400> 2735
aatattcatc aaataagtg

<212> DNA<213> Homo sapiens<220> <223> Cyclin G1 ribozyme binding site

<400> 2736
tcatcaaata agtgttcca

<212> DNA<213> Homo sapiens<220> <223> Cyclin G1 ribozyme binding site
<400> 2737
aataagtgtt ccaaaccaa

<212> DNA<213> Homo sapiens<220> <223> Cyclin G1 ribozyme binding site
<400> 2738
ataagtgttc caaaccaaa

<212> DNA<213> Homo sapiens<220> <223> Cyclin G1 ribozyme binding site
<400> 2739
accaaatgtt cagaagttg

<212> DNA<213> Homo sapiens<220> <223> Cyclin G1 ribozyme binding site
<400> 2740
ccaaatgttc agaagttga

<212> DNA<213> Homo sapiens<220> <223> Cyclin G1 ribozyme binding site
<400> 2741
ttcagaagtt gaaatggat

<212> DNA<213> Homo sapiens<220> <223> Cyclin G1 ribozyme binding site
<400> 2742
gaaatggatt gtttctggg

<212> DNA<213> Homo sapiens<220> <223> Cyclin G1 ribozyme binding site
<400> 2743
atggattgtt tctgggcgt

<212> DNA<213> Homo sapiens<220> <223> Cyclin G1 ribozyme binding site
<400> 2744
tggattgttt ctgggcgta

<212> DNA<213> Homo sapiens<220> <223> Cyclin G1 ribozyme binding site
<400> 2745
ggattgtttc tgggcgtac

<212> DNA<213> Homo sapiens<220> <223> Cyclin G1 ribozyme binding site
<400> 2746
tctgggcgta ctgcacggc

<212> DNA<213> Homo sapiens<220> <223> Cyclin G1 ribozyme binding site
<400> 2747
cacggcaatt gaagcatag

<212> DNA<213> Homo sapiens<220> <223> Cyclin G1 ribozyme binding site
<400> 2748
ttgaagcata gctactaca

<212> DNA<213> Homo sapiens<220> <223> Cyclin G1 ribozyme binding site
<400> 2749
agcatagcta ctacagaat

<212> DNA<213> Homo sapiens<220> <223> Cyclin G1 ribozyme binding site
<400> 2750
atagctacta cagaataac

<212> DNA<213> Homo sapiens<220> <223> Cyclin G1 ribozyme binding site
<400> 2751
ctacagaata actcacctt

<212> DNA<213> Homo sapiens<220> <223> Cyclin G1 ribozyme binding site
<400> 2752
agaataactc accttccaa

<212> DNA<213> Homo sapiens<220> <223> Cyclin G1 ribozyme binding site
<400> 2753
aactcacctt ccaacaatt

<212> DNA<213> Homo sapiens<220> <223> Cyclin G1 ribozyme binding site
<400> 2754
actcaccttc caacaattc

<212> DNA<213> Homo sapiens<220> <223> Cyclin G1 ribozyme binding site
<400> 2755
tccaacaatt cctgaaatg

<212> DNA<213> Homo sapiens<220> <223> Cyclin G1 ribozyme binding site
<400> 2756
ccaacaattc ctgaaatgg

<212> DNA<213> Homo sapiens<220> <223> Cyclin G1 ribozyme binding site
<400> 2757
tgaaatggtc ccttaactg

<212> DNA<213> Homo sapiens<220> <223> Cyclin G1 ribozyme binding site
<400> 2758
atggtccctt aactggatt

<212> DNA<213> Homo sapiens<220> <223> Cyclin G1 ribozyme binding site
<400> 2759
tggtccctta actggatta

<212> DNA<213> Homo sapiens<220> <223> Cyclin G1 ribozyme binding site
<400> 2760
taactggatt attacagca

<212> DNA<213> Homo sapiens<220> <223> Cyclin G1 ribozyme binding site
<400> 2761
aactggatta ttacagcac

<212> DNA<213> Homo sapiens<220> <223> Cyclin G1 ribozyme binding site
<400> 2762
ctggattatt acagcacca

<212> DNA<213> Homo sapiens<220> <223> Cyclin G1 ribozyme binding site
<400> 2763
tggattatta cagcaccaa

<212> DNA<213> Homo sapiens<220> <223> Cyclin G1 ribozyme binding site
<400> 2764
caaaaaactt ctctgaagc

<212> DNA<213> Homo sapiens<220> <223> Cyclin G1 ribozyme binding site
<400> 2765
aaaaaacttc tctgaagcc

<212> DNA<213> Homo sapiens<220> <223> Cyclin G1 ribozyme binding site
<400> 2766
aaaacttctc tgaagcctt

<212> DNA<213> Homo sapiens<220> <223> Cyclin G1 ribozyme binding site
<400> 2767
ctgaagcctt tctccacaa

<212> DNA<213> Homo sapiens<220> <223> Cyclin G1 ribozyme binding site
<400> 2768
tgaagccttt ctccacaac

<212> DNA<213> Homo sapiens<220> <223> Cyclin G1 ribozyme binding site
<400> 2769
gaagcctttc tccacaacc

<212> DNA<213> Homo sapiens<220> <223> Cyclin G1 ribozyme binding site
<400> 2770
agcctttctc cacaacctt

<212> DNA<213> Homo sapiens<220> <223> Cyclin G1 ribozyme binding site
<400> 2771
ccacaacctt gttctatgg

<212> DNA<213> Homo sapiens<220> <223> Cyclin G1 ribozyme binding site
<400> 2772
caaccttgtt ctatggatt

<212> DNA<213> Homo sapiens<220> <223> Cyclin G1 ribozyme binding site
<400> 2773
aaccttgttc tatggattc

<212> DNA<213> Homo sapiens<220> <223> Cyclin G1 ribozyme binding site
<400> 2774
ccttgttcta tggattcca

<212> DNA<213> Homo sapiens<220> <223> Cyclin G1 ribozyme binding site
<400> 2775
tctatggatt ccataatgt

<212> DNA<213> Homo sapiens<220> <223> Cyclin G1 ribozyme binding site
<400> 2776
ctatggattc cataatgtt

<212> DNA<213> Homo sapiens<220> <223> Cyclin G1 ribozyme binding site
<400> 2777

ggattccata atgttacaa

<212> DNA<213> Homo sapiens<220> <223> Cyclin G1 ribozyme binding site
<400> 2778
ccataatgtt acaatggat

<212> DNA<213> Homo sapiens<220> <223> Cyclin G1 ribozyme binding site
<400> 2779
cataatgtta caatggatt

<212> DNA<213> Homo sapiens<220> <223> Cyclin G1 ribozyme binding site
<400> 2780
acaatggatt taaagctat

<212> DNA<213> Homo sapiens<220> <223> Cyclin G1 ribozyme binding site
<400> 2781
caatggattt aagctatga

<212> DNA<213> Homo sapiens<220> <223> Cyclin G1 ribozyme binding site
<400> 2782
aatggattta agctatgaa

<212> DNA<213> Homo sapiens<220> <223> Cyclin G1 ribozyme binding site
<400> 2783
atttaagcta tgaagctca

<212> DNA<213> Homo sapiens<220> <223> Cyclin G1 ribozyme binding site
<400> 2784
tatgaagctc aaacatcac

<212> DNA<213> Homo sapiens<220> <223> Cyclin G1 ribozyme binding site
<400> 2785
ctcaaacatc acgagataa

<212> DNA<213> Homo sapiens<220> <223> Cyclin G1 ribozyme binding site
<400> 2786
tcacgagata agcatgatg

<212> DNA<213> Homo sapiens<220> <223> Cyclin G1 ribozyme binding site
<400> 2787
catgatggtc tcagcttgg

<212> DNA<213> Homo sapiens<220> <223> Cyclin H ribozyme binding site
<400> 2788
cacaacagta gtcagaagc

<212> DNA<213> Homo sapiens<220> <223> Cyclin H ribozyme binding site
<400> 2789
aacagtagtc agaagcggc

<212> DNA<213> Homo sapiens<220> <223> Cyclin H ribozyme binding site
<400> 2790
actggacctt ctccagcga

<212> DNA<213> Homo sapiens<220> <223> Cyclin H ribozyme binding site

<400> 2791
ggaccttctc cagcgagga

<212> DNA<213> Homo sapiens<220> <223> Cyclin H ribozyme binding site
<400> 2792
accgcaaatt cagatgcaa

<212> DNA<213> Homo sapiens<220> <223> Cyclin H ribozyme binding site
<400> 2793
ccgcaaattc agatgcaaa

<212> DNA<213> Homo sapiens<220> <223> Cyclin H ribozyme binding site
<400> 2794
cgggaaggtt cttccgaat

<212> DNA<213> Homo sapiens<220> <223> Cyclin H ribozyme binding site
<400> 2795
gggaaggttc ttccgaatg

<212> DNA<213> Homo sapiens<220> <223> Cyclin H ribozyme binding site
<400> 2796
gaaggttctt ccgaatgat

<212> DNA<213> Homo sapiens<220> <223> Cyclin H ribozyme binding site
<400> 2797
aaggttcttc cgaatgatc

<212> DNA<213> Homo sapiens<220> <223> Cyclin H ribozyme binding site
<400> 2798
ccgaatgatc cagtctttc

<212> DNA<213> Homo sapiens<220> <223> Cyclin H ribozyme binding site
<400> 2799
tgatccagtc tttcttgag

<212> DNA<213> Homo sapiens<220> <223> Cyclin H ribozyme binding site
<400> 2800
atccagtctt tcttgagcc

<212> DNA<213> Homo sapiens<220> <223> Cyclin H ribozyme binding site
<400> 2801
tccagtcttt cttgagcct

<212> DNA<213> Homo sapiens<220> <223> Cyclin H ribozyme binding site
<400> 2802
ccagtctttc ttgagcctc

<212> DNA<213> Homo sapiens<220> <223> Cyclin H ribozyme binding site
<400> 2803
agtctttctt gagcctcat

<212> DNA<213> Homo sapiens<220> <223> Cyclin H ribozyme binding site
<400> 2804
cttgagcctc atgaagaaa

<212> DNA<213> Homo sapiens<220> <223> Cyclin H ribozyme binding site
<400> 2805
aatgacactc tgcaaatac

<212> DNA<213> Homo sapiens<220> <223> Cyclin H ribozyme binding site
<400> 2806
tctgcaaata ctatgagaa

<212> DNA<213> Homo sapiens<220> <223> Cyclin H ribozyme binding site
<400> 2807
gcaaatacta tgagaaaag

<212> DNA<213> Homo sapiens<220> <223> Cyclin H ribozyme binding site
<400> 2808
agaaaaggtt attggaatt

<212> DNA<213> Homo sapiens<220> <223> Cyclin H ribozyme binding site
<400> 2809
gaaaaggtta ttggaattc

<212> DNA<213> Homo sapiens<220> <223> Cyclin H ribozyme binding site
<400> 2810
aaaggttatt ggaattctg

<212> DNA<213> Homo sapiens<220> <223> Cyclin H ribozyme binding site
<400> 2811
tattggaatt ctgttcggt

<212> DNA<213> Homo sapiens<220> <223> Cyclin H ribozyme binding site
<400> 2812
attggaattc tgttcggtg

<212> DNA<213> Homo sapiens<220> <223> Cyclin H ribozyme binding site
<400> 2813
gaattctgtt cggtgttta

<212> DNA<213> Homo sapiens<220> <223> Cyclin H ribozyme binding site
<400> 2814
aattctgttc ggtgtttaa

<212> DNA<213> Homo sapiens<220> <223> Cyclin H ribozyme binding site
<400> 2815
gttcggtgtt taagccagc

<212> DNA<213> Homo sapiens<220> <223> Cyclin H ribozyme binding site
<400> 2816
ttcggtgttt aagccagca

<212> DNA<213> Homo sapiens<220> <223> Cyclin H ribozyme binding site
<400> 2817
tcggtgttta agccagcaa

<212> DNA<213> Homo sapiens<220> <223> Cyclin H ribozyme binding site
<400> 2818
tgccaagatc tgttgtggg

<212> DNA<213> Homo sapiens<220> <223> Cyclin H ribozyme binding site
<400> 2819
aagatctgtt gtgggtacg

<212> DNA<213> Homo sapiens<220> <223> Cyclin H ribozyme binding site
<400> 2820
gttgtgggta cggcttgta

<212> DNA<213> Homo sapiens<220> <223> Cyclin H ribozyme binding site
<400> 2821
ggtacggctt gtatgtatt

<212> DNA<213> Homo sapiens<220> <223> Cyclin H ribozyme binding site
<400> 2822
acggcttgta tgtatttca

<212> DNA<213> Homo sapiens<220> <223> Cyclin H ribozyme binding site
<400> 2823
cttgtatgta tttcaaacg

<212> DNA<213> Homo sapiens<220> <223> Cyclin H ribozyme binding site
<400> 2824
tgtatgtatt tcaaacgtt

<212> DNA<213> Homo sapiens<220> <223> Cyclin H ribozyme binding site
<400> 2825
gtatgtattt caaacgttt

<212> DNA<213> Homo sapiens<220> <223> Cyclin H ribozyme binding site
<400> 2826
tatgtatttc aaacgtttt

<212> DNA<213> Homo sapiens<220> <223> Cyclin H ribozyme binding site
<400> 2827
ttcaaacgtt tttatctta

<212> DNA<213> Homo sapiens<220> <223> Cyclin H ribozyme binding site
<400> 2828
tcaaacgttt ttatcttaa

<212> DNA<213> Homo sapiens<220> <223> Cyclin H ribozyme binding site
<400> 2829
caaacgtttt tatcttaat

<212> DNA<213> Homo sapiens<220> <223> Cyclin H ribozyme binding site
<400> 2830
aaacgttttt atcttaata

<212> DNA<213> Homo sapiens<220> <223> Cyclin H ribozyme binding site
<400> 2831
aacgttttta tcttaataa

<212> DNA<213> Homo sapiens<220> <223> Cyclin H ribozyme binding site
<400> 2832

cgtttttatc ttaataact

<212> DNA<213> Homo sapiens<220> <223> Cyclin H ribozyme binding site
<400> 2833
tttttatctt aataactca

<212> DNA<213> Homo sapiens<220> <223> Cyclin H ribozyme binding site
<400> 2834
ttttatctta ataactcag

<212> DNA<213> Homo sapiens<220> <223> Cyclin H ribozyme binding site
<400> 2835
tatcttaata actcagtaa

<212> DNA<213> Homo sapiens<220> <223> Cyclin H ribozyme binding site
<400> 2836
ttaataactc agtaatgga

<212> DNA<213> Homo sapiens<220> <223> Cyclin H ribozyme binding site
<400> 2837
taactcagta atggaatat

<212> DNA<213> Homo sapiens<220> <223> Cyclin H ribozyme binding site
<400> 2838
taatggaata tcaccccag

<212> DNA<213> Homo sapiens<220> <223> Cyclin H ribozyme binding site
<400> 2839
atggaatatc accccagga

<212> DNA<213> Homo sapiens<220> <223> Cyclin H ribozyme binding site
<400> 2840
ccccaggata ataatgctc

<212> DNA<213> Homo sapiens<220> <223> Cyclin H ribozyme binding site
<400> 2841
caggataata atgctcact

<212> DNA<213> Homo sapiens<220> <223> Cyclin H ribozyme binding site
<400> 2842
aataatgctc acttgtgca

<212> DNA<213> Homo sapiens<220> <223> Cyclin H ribozyme binding site
<400> 2843
atgctcactt gtgcatttt

<212> DNA<213> Homo sapiens<220> <223> Cyclin H ribozyme binding site
<400> 2844
cttgtgcatt tttggcctg

<212> DNA<213> Homo sapiens<220> <223> Cyclin H ribozyme binding site
<400> 2845
ttgtgcattt ttggcctgc

<212> DNA<213> Homo sapiens<220> <223> Cyclin H ribozyme binding site

<400> 2846
tgtgcatttt tggcctgca

<212> DNA<213> Homo sapiens<220> <223> Cyclin H ribozyme binding site
<400> 2847
gtgcattttt ggcctgcaa

<212> DNA<213> Homo sapiens<220> <223> Cyclin H ribozyme binding site
<400> 2848
ctgcaaagta gatgaattc

<212> DNA<213> Homo sapiens<220> <223> Cyclin H ribozyme binding site
<400> 2849
tagatgaatt caatgtatc

<212> DNA<213> Homo sapiens<220> <223> Cyclin H ribozyme binding site
<400> 2850
agatgaattc aatgtatct

<212> DNA<213> Homo sapiens<220> <223> Cyclin H ribozyme binding site
<400> 2851
attcaatgta tctagtcct

<212> DNA<213> Homo sapiens<220> <223> Cyclin H ribozyme binding site
<400> 2852
tcaatgtatc tagtcctca

<212> DNA<213> Homo sapiens<220> <223> Cyclin H ribozyme binding site
<400> 2853
aatgtatcta gtcctcagt

<212> DNA<213> Homo sapiens<220> <223> Cyclin H ribozyme binding site
<400> 2854
gtatctagtc ctcagtttg

<212> DNA<213> Homo sapiens<220> <223> Cyclin H ribozyme binding site
<400> 2855
tctagtcctc agtttgttg

<212> DNA<213> Homo sapiens<220> <223> Cyclin H ribozyme binding site
<400> 2856
gtcctcagtt tgttggaaa

<212> DNA<213> Homo sapiens<220> <223> Cyclin H ribozyme binding site
<400> 2857
tcctcagttt gttggaaac

<212> DNA<213> Homo sapiens<220> <223> Cyclin H ribozyme binding site
<400> 2858
tcagtttgtt ggaaacctc

<212> DNA<213> Homo sapiens<220> <223> Cyclin H ribozyme binding site
<400> 2859
tggaaacctc cgggagagt

<212> DNA<213> Homo sapiens<220> <223> Cyclin H ribozyme binding site
<400> 2860
cgggagagtc ctcttggac

<212> DNA<213> Homo sapiens<220> <223> Cyclin H ribozyme binding site
<400> 2861
gagagtcctc ttggacagg

<212> DNA<213> Homo sapiens<220> <223> Cyclin H ribozyme binding site
<400> 2862
gagtcctctt ggacaggag

<212> DNA<213> Homo sapiens<220> <223> Cyclin H ribozyme binding site
<400> 2863
gaaggcactt gaacagata

<212> DNA<213> Homo sapiens<220> <223> Cyclin H ribozyme binding site
<400> 2864
tgaacagata ctggaatat

<212> DNA<213> Homo sapiens<220> <223> Cyclin H ribozyme binding site
<400> 2865
tactggaata tgaactact

<212> DNA<213> Homo sapiens<220> <223> Cyclin H ribozyme binding site
<400> 2866
atatgaacta cttcttata

<212> DNA<213> Homo sapiens<220> <223> Cyclin H ribozyme binding site
<400> 2867
tgaactactt cttatacag

<212> DNA<213> Homo sapiens<220> <223> Cyclin H ribozyme binding site
<400> 2868
actacttctt atacagcaa

<212> DNA<213> Homo sapiens<220> <223> Cyclin H ribozyme binding site
<400> 2869
ctacttctta tacagcaac

<212> DNA<213> Homo sapiens<220> <223> Cyclin H ribozyme binding site
<400> 2870
acttcttata cagcaactt

<212> DNA<213> Homo sapiens<220> <223> Cyclin H ribozyme binding site
<400> 2871
acagcaactt aatttccac

<212> DNA<213> Homo sapiens<220> <223> Cyclin H ribozyme binding site
<400> 2872
cagcaactta atttccacc

<212> DNA<213> Homo sapiens<220> <223> Cyclin H ribozyme binding site
<400> 2873
caacttaatt tccacctta

<212> DNA<213> Homo sapiens<220> <223> Cyclin H ribozyme binding site
<400> 2874
aacttaattt ccaccttat

<212> DNA<213> Homo sapiens<220> <223> Cyclin H ribozyme binding site
<400> 2875
acttaatttc caccttatt

<212> DNA<213> Homo sapiens<220> <223> Cyclin H ribozyme binding site
<400> 2876
tttccacctt attgtccac

<212> DNA<213> Homo sapiens<220> <223> Cyclin H ribozyme binding site
<400> 2877
ttccacctta ttgtccaca

<212> DNA<213> Homo sapiens<220> <223> Cyclin H ribozyme binding site
<400> 2878
ccaccttatt gtccacaat

<212> DNA<213> Homo sapiens<220> <223> Cyclin H ribozyme binding site
<400> 2879
ccttattgtc cacaatcct

<212> DNA<213> Homo sapiens<220> <223> Cyclin H ribozyme binding site
<400> 2880
gtccacaatc cttacagac

<212> DNA<213> Homo sapiens<220> <223> Cyclin H ribozyme binding site
<400> 2881
cacaatcctt acagaccat

<212> DNA<213> Homo sapiens<220> <223> Cyclin H ribozyme binding site
<400> 2882
acaatcctta cagaccatt

<212> DNA<213> Homo sapiens<220> <223> Cyclin H ribozyme binding site
<400> 2883
acagaccatt tgagggctt

<212> DNA<213> Homo sapiens<220> <223> Cyclin H ribozyme binding site
<400> 2884
cagaccattt gagggcttc

<212> DNA<213> Homo sapiens<220> <223> Cyclin H ribozyme binding site
<400> 2885
ttgagggctt cctcatcga

<212> DNA<213> Homo sapiens<220> <223> Cyclin H ribozyme binding site
<400> 2886
tgagggcttc ctcatcgac

<212> DNA<213> Homo sapiens<220> <223> Cyclin H ribozyme binding site
<400> 2887

gggcttcctc atcgactta

<212> DNA<213> Homo sapiens<220> <223> Cyclin H ribozyme binding site
<400> 2888
cttcctcatc gacttaaag

<212> DNA<213> Homo sapiens<220> <223> Cyclin H ribozyme binding site
<400> 2889
tcatcgactt aaagacccg

<212> DNA<213> Homo sapiens<220> <223> Cyclin H ribozyme binding site
<400> 2890
catcgactta aagacccgc

<212> DNA<213> Homo sapiens<220> <223> Cyclin H ribozyme binding site
<400> 2891
agacccgcta tcccatatt

<212> DNA<213> Homo sapiens<220> <223> Cyclin H ribozyme binding site
<400> 2892
acccgctatc ccatattgg

<212> DNA<213> Homo sapiens<220> <223> Cyclin H ribozyme binding site
<400> 2893
ctatcccata ttggagaat

<212> DNA<213> Homo sapiens<220> <223> Cyclin H ribozyme binding site
<400> 2894
atcccatatt ggagaatcc

<212> DNA<213> Homo sapiens<220> <223> Cyclin H ribozyme binding site
<400> 2895
ttggagaatc cagagattt

<212> DNA<213> Homo sapiens<220> <223> Cyclin H ribozyme binding site
<400> 2896
tccagagatt ttgaggaaa

<212> DNA<213> Homo sapiens<220> <223> Cyclin H ribozyme binding site
<400> 2897
ccagagattt tgaggaaaa

<212> DNA<213> Homo sapiens<220> <223> Cyclin H ribozyme binding site
<400> 2898
cagagatttt gaggaaaac

<212> DNA<213> Homo sapiens<220> <223> Cyclin H ribozyme binding site
<400> 2899
ctgatgactt tcttaatag

<212> DNA<213> Homo sapiens<220> <223> Cyclin H ribozyme binding site
<400> 2900
tgatgacttt cttaataga

<212> DNA<213> Homo sapiens<220> <223> Cyclin H ribozyme binding site

<400> 2901
gatgactttc ttaatagaa

<212> DNA<213> Homo sapiens<220> <223> Cyclin H ribozyme binding site
<400> 2902
tgactttctt aatagaatt

<212> DNA<213> Homo sapiens<220> <223> Cyclin H ribozyme binding site
<400> 2903
gactttctta atagaattg

<212> DNA<213> Homo sapiens<220> <223> Cyclin H ribozyme binding site
<400> 2904
tttcttaata gaattgcat

<212> DNA<213> Homo sapiens<220> <223> Cyclin H ribozyme binding site
<400> 2905
taatagaatt gcattgacg

<212> DNA<213> Homo sapiens<220> <223> Cyclin H ribozyme binding site
<400> 2906
gaattgcatt gacggatgc

<212> DNA<213> Homo sapiens<220> <223> Cyclin H ribozyme binding site
<400> 2907
acggatgctt accttttat

<212> DNA<213> Homo sapiens<220> <223> Cyclin H ribozyme binding site
<400> 2908
cggatgctta ccttttata

<212> DNA<213> Homo sapiens<220> <223> Cyclin H ribozyme binding site
<400> 2909
tgcttacctt ttatacaca

<212> DNA<213> Homo sapiens<220> <223> Cyclin H ribozyme binding site
<400> 2910
gcttaccttt tatacacac

<212> DNA<213> Homo sapiens<220> <223> Cyclin H ribozyme binding site
<400> 2911
cttacctttt atacacacc

<212> DNA<213> Homo sapiens<220> <223> Cyclin H ribozyme binding site
<400> 2912
ttacctttta tacacacct

<212> DNA<213> Homo sapiens<220> <223> Cyclin H ribozyme binding site
<400> 2913
accttttata cacaccttc

<212> DNA<213> Homo sapiens<220> <223> Cyclin H ribozyme binding site
<400> 2914
tacacacctt cccaaattg

<212> DNA<213> Homo sapiens<220> <223> Cyclin H ribozyme binding site
<400> 2915
acacaccttc ccaaattgc

<212> DNA<213> Homo sapiens<220> <223> Cyclin H ribozyme binding site
<400> 2916
ttcccaaatt gccctgact

<212> DNA<213> Homo sapiens<220> <223> Cyclin H ribozyme binding site
<400> 2917
gactgccatt ttatctagt

<212> DNA<213> Homo sapiens<220> <223> Cyclin H ribozyme binding site
<400> 2918
actgccattt tatctagtg

<212> DNA<213> Homo sapiens<220> <223> Cyclin H ribozyme binding site
<400> 2919
ctgccatttt atctagtgc

<212> DNA<213> Homo sapiens<220> <223> Cyclin H ribozyme binding site
<400> 2920
tgccatttta tctagtgcc

<212> DNA<213> Homo sapiens<220> <223> Cyclin H ribozyme binding site
<400> 2921
ccattttatc tagtgcctc

<212> DNA<213> Homo sapiens<220> <223> Cyclin H ribozyme binding site
<400> 2922
attttatcta gtgcctcca

<212> DNA<213> Homo sapiens<220> <223> Cyclin H ribozyme binding site
<400> 2923
ctagtgcctc cagggctgg

<212> DNA<213> Homo sapiens<220> <223> Cyclin H ribozyme binding site
<400> 2924
ggctggaatt actatggaa

<212> DNA<213> Homo sapiens<220> <223> Cyclin H ribozyme binding site
<400> 2925
gctggaatta ctatggaaa

<212> DNA<213> Homo sapiens<220> <223> Cyclin H ribozyme binding site
<400> 2926
ggaattacta tggaaagtt

<212> DNA<213> Homo sapiens<220> <223> Cyclin H ribozyme binding site
<400> 2927
atggaaagtt atttatcag

<212> DNA<213> Homo sapiens<220> <223> Cyclin H ribozyme binding site
<400> 2928
tggaaagtta tttatcaga

<212> DNA<213> Homo sapiens<220> <223> Cyclin H ribozyme binding site
<400> 2929
gaaagttatt tatcagaga

<212> DNA<213> Homo sapiens<220> <223> Cyclin H ribozyme binding site
<400> 2930
aaagttattt atcagagag

<212> DNA<213> Homo sapiens<220> <223> Cyclin H ribozyme binding site
<400> 2931
aagttattta tcagagagt

<212> DNA<213> Homo sapiens<220> <223> Cyclin H ribozyme binding site
<400> 2932
gttatttatc agagagtct

<212> DNA<213> Homo sapiens<220> <223> Cyclin H ribozyme binding site
<400> 2933
tcagagagtc tgatgctga

<212> DNA<213> Homo sapiens<220> <223> Cyclin H ribozyme binding site
<400> 2934
aacagaactt gcctgtcac

<212> DNA<213> Homo sapiens<220> <223> Cyclin H ribozyme binding site
<400> 2935
cttgcctgtc acagttact

<212> DNA<213> Homo sapiens<220> <223> Cyclin H ribozyme binding site
<400> 2936
tgtcacagtt actagatat

<212> DNA<213> Homo sapiens<220> <223> Cyclin H ribozyme binding site
<400> 2937
gtcacagtta ctagatata

<212> DNA<213> Homo sapiens<220> <223> Cyclin H ribozyme binding site
<400> 2938
acagttacta gatataatg

<212> DNA<213> Homo sapiens<220> <223> Cyclin H ribozyme binding site
<400> 2939
ttactagata taatgaaaa

<212> DNA<213> Homo sapiens<220> <223> Cyclin H ribozyme binding site
<400> 2940
actagatata atgaaaagc

<212> DNA<213> Homo sapiens<220> <223> Cyclin H ribozyme binding site
<400> 2941
tgagaaactt agtaaagaa

<212> DNA<213> Homo sapiens<220> <223> Cyclin H ribozyme binding site
<400> 2942

gagaaactta gtaaagaag

<212> DNA<213> Homo sapiens<220> <223> Cyclin H ribozyme binding site
<400> 2943
aaacttagta aagaagtat

<212> DNA<213> Homo sapiens<220> <223> Cyclin H ribozyme binding site
<400> 2944
taaagaagta tgaaccacc

<212> DNA<213> Homo sapiens<220> <223> Cyclin H ribozyme binding site
<400> 2945
cacccagatc tgaagaagt

<212> DNA<213> Homo sapiens<220> <223> Cyclin H ribozyme binding site
<400> 2946
tgaagaagtt gctgttctg

<212> DNA<213> Homo sapiens<220> <223> Cyclin H ribozyme binding site
<400> 2947
agttgctgtt ctgaaacag

<212> DNA<213> Homo sapiens<220> <223> Cyclin H ribozyme binding site
<400> 2948
gttgctgttc tgaaacaga

<212> DNA<213> Homo sapiens<220> <223> Cyclin H ribozyme binding site
<400> 2949
aacagaagtt ggagcgatg

<212> DNA<213> Homo sapiens<220> <223> Cyclin H ribozyme binding site
<400> 2950
gagcgatgtc attctgctg

<212> DNA<213> Homo sapiens<220> <223> Cyclin H ribozyme binding site
<400> 2951
cgatgtcatt ctgctgagc

<212> DNA<213> Homo sapiens<220> <223> Cyclin H ribozyme binding site
<400> 2952
gatgtcattc tgctgagct

<212> DNA<213> Homo sapiens<220> <223> Cyclin H ribozyme binding site
<400> 2953
tgctgagctt gcacttaac

<212> DNA<213> Homo sapiens<220> <223> Cyclin H ribozyme binding site
<400> 2954
gcttgcactt aacgtaatc

<212> DNA<213> Homo sapiens<220> <223> Cyclin H ribozyme binding site
<400> 2955
cttgcactta acgtaatca

<212> DNA<213> Homo sapiens<220> <223> Cyclin H ribozyme binding site

<400> 2956
acttaacgta atcacgaag

<212> DNA<213> Homo sapiens<220> <223> Cyclin H ribozyme binding site
<400> 2957
taacgtaatc acgaagaag

<212> DNA<213> Homo sapiens<220> <223> Cyclin H ribozyme binding site
<400> 2958
ggaaaggcta tgaagatga

<212> DNA<213> Homo sapiens<220> <223> Cyclin H ribozyme binding site
<400> 2959
gatgatgatt acgtctcaa

<212> DNA<213> Homo sapiens<220> <223> Cyclin H ribozyme binding site
<400> 2960
atgatgatta cgtctcaaa

<212> DNA<213> Homo sapiens<220> <223> Cyclin H ribozyme binding site
<400> 2961
tgattacgtc tcaaagaaa

<212> DNA<213> Homo sapiens<220> <223> Cyclin H ribozyme binding site
<400> 2962
attacgtctc aaagaaatc

<212> DNA<213> Homo sapiens<220> <223> Cyclin H ribozyme binding site
<400> 2963
caaagaaatc caaacatga

<212> DNA<213> Homo sapiens<220> <223> Cyclin H ribozyme binding site
<400> 2964
cgacctggta gaatctctc

<212> DNA<213> Homo sapiens<220> <223> Cyclin A1 ribozyme binding site
<400> 2965
ggtgttgttc cggacacat

<212> DNA<213> Homo sapiens<220> <223> Cyclin A1 ribozyme binding site
<400> 2966
cggacacata gaaagataa

<212> DNA<213> Homo sapiens<220> <223> Cyclin A1 ribozyme binding site
<400> 2967
tagaaagata acgacggga

<212> DNA<213> Homo sapiens<220> <223> Cyclin A1 ribozyme binding site
<400> 2968
gggccccgtt tggggtcca

<212> DNA<213> Homo sapiens<220> <223> Cyclin A1 ribozyme binding site
<400> 2969
ggccccgttt ggggtccag

<212> DNA<213> Homo sapiens<220> <223> Cyclin A1 ribozyme binding site
<400> 2970
gtttggggtc caggcaggt

<212> DNA<213> Homo sapiens<220> <223> Cyclin A1 ribozyme binding site
<400> 2971
caggcaggtt ttggggcct

<212> DNA<213> Homo sapiens<220> <223> Cyclin A1 ribozyme binding site
<400> 2972
aggcaggttt tggggcctc

<212> DNA<213> Homo sapiens<220> <223> Cyclin A1 ribozyme binding site
<400> 2973
ggcaggtttt ggggcctcc

<212> DNA<213> Homo sapiens<220> <223> Cyclin A1 ribozyme binding site
<400> 2974
ttggggcctc ctgtctggt

<212> DNA<213> Homo sapiens<220> <223> Cyclin A1 ribozyme binding site
<400> 2975
gcctcctgtc tggtgggag

<212> DNA<213> Homo sapiens<220> <223> Cyclin A1 ribozyme binding site
<400> 2976
gaccctgctc gtcacttgg

<212> DNA<213> Homo sapiens<220> <223> Cyclin A1 ribozyme binding site
<400> 2977
cctgctcgtc acttgggat

<212> DNA<213> Homo sapiens<220> <223> Cyclin A1 ribozyme binding site
<400> 2978
ctcgtcactt gggatggag

<212> DNA<213> Homo sapiens<220> <223> Cyclin A1 ribozyme binding site
<400> 2979
agaccggctt tcccgcaat

<212> DNA<213> Homo sapiens<220> <223> Cyclin A1 ribozyme binding site
<400> 2980
gaccggcttt cccgcaatc

<212> DNA<213> Homo sapiens<220> <223> Cyclin A1 ribozyme binding site
<400> 2981
accggctttc ccgcaatca

<212> DNA<213> Homo sapiens<220> <223> Cyclin A1 ribozyme binding site
<400> 2982
tcccgcaatc atgtaccct

<212> DNA<213> Homo sapiens<220> <223> Cyclin A1 ribozyme binding site
<400> 2983
caatcatgta ccctggatc

<212> DNA<213> Homo sapiens<220> <223> Cyclin A1 ribozyme binding site
<400> 2984
accctggatc ttttattgg

<212> DNA<213> Homo sapiens<220> <223> Cyclin A1 ribozyme binding site
<400> 2985
cctggatctt ttattgggg

<212> DNA<213> Homo sapiens<220> <223> Cyclin A1 ribozyme binding site
<400> 2986
ctggatcttt tattggggg

<212> DNA<213> Homo sapiens<220> <223> Cyclin A1 ribozyme binding site
<400> 2987
tggatctttt attgggggc

<212> DNA<213> Homo sapiens<220> <223> Cyclin A1 ribozyme binding site
<400> 2988
ggatctttta ttgggggct

<212> DNA<213> Homo sapiens<220> <223> Cyclin A1 ribozyme binding site
<400> 2989
atcttttatt gggggctgg

<212> DNA<213> Homo sapiens<220> <223> Cyclin A1 ribozyme binding site
<400> 2990
gagaagagta tctcagctg

<212> DNA<213> Homo sapiens<220> <223> Cyclin A1 ribozyme binding site
<400> 2991
gaagagtatc tcagctggg

<212> DNA<213> Homo sapiens<220> <223> Cyclin A1 ribozyme binding site
<400> 2992
agagtatctc agctgggaa

<212> DNA<213> Homo sapiens<220> <223> Cyclin A1 ribozyme binding site
<400> 2993
accggggctc ccagatttc

<212> DNA<213> Homo sapiens<220> <223> Cyclin A1 ribozyme binding site
<400> 2994
ctcccagatt tcgtcttcc

<212> DNA<213> Homo sapiens<220> <223> Cyclin A1 ribozyme binding site
<400> 2995
tcccagattt cgtcttcca

<212> DNA<213> Homo sapiens<220> <223> Cyclin A1 ribozyme binding site
<400> 2996
cccagatttc gtcttccag

<212> DNA<213> Homo sapiens<220> <223> Cyclin A1 ribozyme binding site
<400> 2997

agatttcgtc ttccagcag

<212> DNA<213> Homo sapiens<220> <223> Cyclin A1 ribozyme binding site
<400> 2998
atttcgtctt ccagcagca

<212> DNA<213> Homo sapiens<220> <223> Cyclin A1 ribozyme binding site
<400> 2999
tttcgtcttc cagcagcag

<212> DNA<213> Homo sapiens<220> <223> Cyclin A1 ribozyme binding site
<400> 3000
ccgtggagtc tgaagcaat

<212> DNA<213> Homo sapiens<220> <223> Cyclin A1 ribozyme binding site
<400> 3001
gagtggagtt gtgctggct

<212> DNA<213> Homo sapiens<220> <223> Cyclin A1 ribozyme binding site
<400> 3002
gtgctggcta cagtggccc

<212> DNA<213> Homo sapiens<220> <223> Cyclin A1 ribozyme binding site
<400> 3003
gcccgaggtc ccgatgctt

<212> DNA<213> Homo sapiens<220> <223> Cyclin A1 ribozyme binding site
<400> 3004
cccgatgctt gtcagatac

<212> DNA<213> Homo sapiens<220> <223> Cyclin A1 ribozyme binding site
<400> 3005
gatgcttgtc agatactca

<212> DNA<213> Homo sapiens<220> <223> Cyclin A1 ribozyme binding site
<400> 3006
ttgtcagata ctcaccaga

<212> DNA<213> Homo sapiens<220> <223> Cyclin A1 ribozyme binding site
<400> 3007
tcagatactc accagagcc

<212> DNA<213> Homo sapiens<220> <223> Cyclin A1 ribozyme binding site
<400> 3008
ggccaggatc ccccgcaga

<212> DNA<213> Homo sapiens<220> <223> Cyclin A1 ribozyme binding site
<400> 3009
gacagtgcta gggctgcta

<212> DNA<213> Homo sapiens<220> <223> Cyclin A1 ribozyme binding site
<400> 3010
agggctgcta actgcaaat

<212> DNA<213> Homo sapiens<220> <223> Cyclin A1 ribozyme binding site

<400> 3011
atgggcagta caggaggac

<212> DNA<213> Homo sapiens<220> <223> Cyclin A1 ribozyme binding site
<400> 3012
ccaggggatc acaagaatc

<212> DNA<213> Homo sapiens<220> <223> Cyclin A1 ribozyme binding site
<400> 3013
cacaagaatc aggtgttat

<212> DNA<213> Homo sapiens<220> <223> Cyclin A1 ribozyme binding site
<400> 3014
atcaggtgtt attctggat

<212> DNA<213> Homo sapiens<220> <223> Cyclin A1 ribozyme binding site
<400> 3015
tcaggtgtta ttctggatc

<212> DNA<213> Homo sapiens<220> <223> Cyclin A1 ribozyme binding site
<400> 3016
aggtgttatt ctggatcag

<212> DNA<213> Homo sapiens<220> <223> Cyclin A1 ribozyme binding site
<400> 3017
ggtgttattc tggatcaga

<212> DNA<213> Homo sapiens<220> <223> Cyclin A1 ribozyme binding site
<400> 3018
attctggatc agaaaatgc

<212> DNA<213> Homo sapiens<220> <223> Cyclin A1 ribozyme binding site
<400> 3019
aaaatgcctt ccctccagc

<212> DNA<213> Homo sapiens<220> <223> Cyclin A1 ribozyme binding site
<400> 3020
aaatgccttc cctccagct

<212> DNA<213> Homo sapiens<220> <223> Cyclin A1 ribozyme binding site
<400> 3021
gccttccctc cagctggaa

<212> DNA<213> Homo sapiens<220> <223> Cyclin A1 ribozyme binding site
<400> 3022
gaaagcactc cctgactgt

<212> DNA<213> Homo sapiens<220> <223> Cyclin A1 ribozyme binding site
<400> 3023
ctgtggggtc caagagccc

<212> DNA<213> Homo sapiens<220> <223> Cyclin A1 ribozyme binding site
<400> 3024
agcaagggtt tgacatcta

<212> DNA<213> Homo sapiens<220> <223> Cyclin A1 ribozyme binding site
<400> 3025
gcaagggttt gacatctac

<212> DNA<213> Homo sapiens<220> <223> Cyclin A1 ribozyme binding site
<400> 3026
gtttgacatc tacatggat

<212> DNA<213> Homo sapiens<220> <223> Cyclin A1 ribozyme binding site
<400> 3027
ttgacatcta catggatga

<212> DNA<213> Homo sapiens<220> <223> Cyclin A1 ribozyme binding site
<400> 3028
ggatgaacta gagcagggg

<212> DNA<213> Homo sapiens<220> <223> Cyclin A1 ribozyme binding site
<400> 3029
acagctgctc ggtcagaga

<212> DNA<213> Homo sapiens<220> <223> Cyclin A1 ribozyme binding site
<400> 3030
ctgctcggtc agagagggg

<212> DNA<213> Homo sapiens<220> <223> Cyclin A1 ribozyme binding site
<400> 3031
ggatggcatt tgaggatgt

<212> DNA<213> Homo sapiens<220> <223> Cyclin A1 ribozyme binding site
<400> 3032
gatggcattt gaggatgtg

<212> DNA<213> Homo sapiens<220> <223> Cyclin A1 ribozyme binding site
<400> 3033
aggatgtgta tgaagtaga

<212> DNA<213> Homo sapiens<220> <223> Cyclin A1 ribozyme binding site
<400> 3034
gtatgaagta gacaccggc

<212> DNA<213> Homo sapiens<220> <223> Cyclin A1 ribozyme binding site
<400> 3035
cggcacactc aagtcagac

<212> DNA<213> Homo sapiens<220> <223> Cyclin A1 ribozyme binding site
<400> 3036
cactcaagtc agacctgca

<212> DNA<213> Homo sapiens<220> <223> Cyclin A1 ribozyme binding site
<400> 3037
acctgcactt cctgctgga

<212> DNA<213> Homo sapiens<220> <223> Cyclin A1 ribozyme binding site
<400> 3038
cctgcacttc ctgctggat

<212> DNA<213> Homo sapiens<220> <223> Cyclin A1 ribozyme binding site
<400> 3039
ctgctggatt tcaacacag

<212> DNA<213> Homo sapiens<220> <223> Cyclin A1 ribozyme binding site
<400> 3040
tgctggattt caacacagt

<212> DNA<213> Homo sapiens<220> <223> Cyclin A1 ribozyme binding site
<400> 3041
gctggatttc aacacagtt

<212> DNA<213> Homo sapiens<220> <223> Cyclin A1 ribozyme binding site
<400> 3042
caacacagtt tcccctatg

<212> DNA<213> Homo sapiens<220> <223> Cyclin A1 ribozyme binding site
<400> 3043
aacacagttt cccctatgc

<212> DNA<213> Homo sapiens<220> <223> Cyclin A1 ribozyme binding site
<400> 3044
acacagtttc ccctatgct

<212> DNA<213> Homo sapiens<220> <223> Cyclin A1 ribozyme binding site
<400> 3045
gtttccccta tgctggtag

<212> DNA<213> Homo sapiens<220> <223> Cyclin A1 ribozyme binding site
<400> 3046
tatgctggta gattcatct

<212> DNA<213> Homo sapiens<220> <223> Cyclin A1 ribozyme binding site
<400> 3047
ctggtagatt catctctcc

<212> DNA<213> Homo sapiens<220> <223> Cyclin A1 ribozyme binding site
<400> 3048
tggtagattc atctctcct

<212> DNA<213> Homo sapiens<220> <223> Cyclin A1 ribozyme binding site
<400> 3049
tagattcatc tctcctctc

<212> DNA<213> Homo sapiens<220> <223> Cyclin A1 ribozyme binding site
<400> 3050
gattcatctc tcctctccc

<212> DNA<213> Homo sapiens<220> <223> Cyclin A1 ribozyme binding site
<400> 3051
ttcatctctc ctctcccag

<212> DNA<213> Homo sapiens<220> <223> Cyclin A1 ribozyme binding site
<400> 3052

atctctcctc tcccagtct

<212> DNA<213> Homo sapiens<220> <223> Cyclin A1 ribozyme binding site
<400> 3053
ctctcctctc ccagtctga

<212> DNA<213> Homo sapiens<220> <223> Cyclin A1 ribozyme binding site
<400> 3054
tctcccagtc tgaagatat

<212> DNA<213> Homo sapiens<220> <223> Cyclin A1 ribozyme binding site
<400> 3055
tctgaagata tatccagtc

<212> DNA<213> Homo sapiens<220> <223> Cyclin A1 ribozyme binding site
<400> 3056
tgaagatata tccagtctt

<212> DNA<213> Homo sapiens<220> <223> Cyclin A1 ribozyme binding site
<400> 3057
aagatatatc cagtcttgg

<212> DNA<213> Homo sapiens<220> <223> Cyclin A1 ribozyme binding site
<400> 3058
atatccagtc ttggcacag

<212> DNA<213> Homo sapiens<220> <223> Cyclin A1 ribozyme binding site
<400> 3059
atccagtctt ggcacagat

<212> DNA<213> Homo sapiens<220> <223> Cyclin A1 ribozyme binding site
<400> 3060
agatgtgata aatgtgact

<212> DNA<213> Homo sapiens<220> <223> Cyclin A1 ribozyme binding site
<400> 3061
tgactgaata tgctgaaga

<212> DNA<213> Homo sapiens<220> <223> Cyclin A1 ribozyme binding site
<400> 3062
tgaagaaatt tatcagtac

<212> DNA<213> Homo sapiens<220> <223> Cyclin A1 ribozyme binding site
<400> 3063
gaagaaattt atcagtacc

<212> DNA<213> Homo sapiens<220> <223> Cyclin A1 ribozyme binding site
<400> 3064
aagaaattta tcagtacct

<212> DNA<213> Homo sapiens<220> <223> Cyclin A1 ribozyme binding site
<400> 3065
gaaatttatc agtacctta

<212> DNA<213> Homo sapiens<220> <223> Cyclin A1 ribozyme binding site

<400> 3066
tttatcagta ccttaggga

<212> DNA<213> Homo sapiens<220> <223> Cyclin A1 ribozyme binding site
<400> 3067
tcagtacctt agggaagct

<212> DNA<213> Homo sapiens<220> <223> Cyclin A1 ribozyme binding site
<400> 3068
cagtacctta gggaagctg

<212> DNA<213> Homo sapiens<220> <223> Cyclin A1 ribozyme binding site
<400> 3069
agctgaaata aggcacaga

<212> DNA<213> Homo sapiens<220> <223> Cyclin A1 ribozyme binding site
<400> 3070
aagcacacta catgaagaa

<212> DNA<213> Homo sapiens<220> <223> Cyclin A1 ribozyme binding site
<400> 3071
gccagacatc acggaaggc

<212> DNA<213> Homo sapiens<220> <223> Cyclin A1 ribozyme binding site
<400> 3072
gcgcacgatt ctggtggac

<212> DNA<213> Homo sapiens<220> <223> Cyclin A1 ribozyme binding site
<400> 3073
cgcacgattc tggtggact

<212> DNA<213> Homo sapiens<220> <223> Cyclin A1 ribozyme binding site
<400> 3074
ggtggaggtt ggggaagaa

<212> DNA<213> Homo sapiens<220> <223> Cyclin A1 ribozyme binding site
<400> 3075
gggaagaata taaacttcg

<212> DNA<213> Homo sapiens<220> <223> Cyclin A1 ribozyme binding site
<400> 3076
gaagaatata aacttcgag

<212> DNA<213> Homo sapiens<220> <223> Cyclin A1 ribozyme binding site
<400> 3077
atataaactt cgagcagag

<212> DNA<213> Homo sapiens<220> <223> Cyclin A1 ribozyme binding site
<400> 3078
tataaacttc gagcagaga

<212> DNA<213> Homo sapiens<220> <223> Cyclin A1 ribozyme binding site
<400> 3079
agaccctgta tctggctgt

<212> DNA<213> Homo sapiens<220> <223> Cyclin A1 ribozyme binding site
<400> 3080
accctgtatc tggctgtca

<212> DNA<213> Homo sapiens<220> <223> Cyclin A1 ribozyme binding site
<400> 3081
tctggctgtc aacttcctg

<212> DNA<213> Homo sapiens<220> <223> Cyclin A1 ribozyme binding site
<400> 3082
ctgtcaactt cctggacag

<212> DNA<213> Homo sapiens<220> <223> Cyclin A1 ribozyme binding site
<400> 3083
tgtcaacttc ctggacagg

<212> DNA<213> Homo sapiens<220> <223> Cyclin A1 ribozyme binding site
<400> 3084
tggacaggtt cctttcatg

<212> DNA<213> Homo sapiens<220> <223> Cyclin A1 ribozyme binding site
<400> 3085
ggacaggttc ctttcatgt

<212> DNA<213> Homo sapiens<220> <223> Cyclin A1 ribozyme binding site
<400> 3086
caggttcctt tcatgtatg

<212> DNA<213> Homo sapiens<220> <223> Cyclin A1 ribozyme binding site
<400> 3087
aggttccttt catgtatgt

<212> DNA<213> Homo sapiens<220> <223> Cyclin A1 ribozyme binding site
<400> 3088
ggttcctttc atgtatgtc

<212> DNA<213> Homo sapiens<220> <223> Cyclin A1 ribozyme binding site
<400> 3089
ctttcatgta tgtctgttc

<212> DNA<213> Homo sapiens<220> <223> Cyclin A1 ribozyme binding site
<400> 3090
catgtatgtc tgttctgag

<212> DNA<213> Homo sapiens<220> <223> Cyclin A1 ribozyme binding site
<400> 3091
tatgtctgtt ctgagaggg

<212> DNA<213> Homo sapiens<220> <223> Cyclin A1 ribozyme binding site
<400> 3092
atgtctgttc tgagaggga

<212> DNA<213> Homo sapiens<220> <223> Cyclin A1 ribozyme binding site
<400> 3093
actgcagctc gtaggaaca

<212> DNA<213> Homo sapiens<220> <223> Cyclin A1 ribozyme binding site
<400> 3094
gcagctcgta ggaacagca

<212> DNA<213> Homo sapiens<220> <223> Cyclin A1 ribozyme binding site
<400> 3095
acagcagcta tgcttttgg

<212> DNA<213> Homo sapiens<220> <223> Cyclin A1 ribozyme binding site
<400> 3096
agctatgctt ttggcttcg

<212> DNA<213> Homo sapiens<220> <223> Cyclin A1 ribozyme binding site
<400> 3097
gctatgcttt tggcttcga

<212> DNA<213> Homo sapiens<220> <223> Cyclin A1 ribozyme binding site
<400> 3098
ctatgctttt ggcttcgaa

<212> DNA<213> Homo sapiens<220> <223> Cyclin A1 ribozyme binding site
<400> 3099
cttttggctt cgaaatatg

<212> DNA<213> Homo sapiens<220> <223> Cyclin A1 ribozyme binding site
<400> 3100
ttttggcttc gaaatatga

<212> DNA<213> Homo sapiens<220> <223> Cyclin A1 ribozyme binding site
<400> 3101
cttcgaaata tgaagagat

<212> DNA<213> Homo sapiens<220> <223> Cyclin A1 ribozyme binding site
<400> 3102
tgaagagata tatcctcct

<212> DNA<213> Homo sapiens<220> <223> Cyclin A1 ribozyme binding site
<400> 3103
aagagatata tcctcctga

<212> DNA<213> Homo sapiens<220> <223> Cyclin A1 ribozyme binding site
<400> 3104
gagatatatc ctcctgaag

<212> DNA<213> Homo sapiens<220> <223> Cyclin A1 ribozyme binding site
<400> 3105
atatatcctc ctgaagtag

<212> DNA<213> Homo sapiens<220> <223> Cyclin A1 ribozyme binding site
<400> 3106
tcctgaagta gacgagttt

<212> DNA<213> Homo sapiens<220> <223> Cyclin A1 ribozyme binding site
<400> 3107

tagacgagtt tgtctatat

<212> DNA<213> Homo sapiens<220> <223> Cyclin A1 ribozyme binding site
<400> 3108
agacgagttt gtctatatc

<212> DNA<213> Homo sapiens<220> <223> Cyclin A1 ribozyme binding site
<400> 3109
cgagtttgtc tatatcacc

<212> DNA<213> Homo sapiens<220> <223> Cyclin A1 ribozyme binding site
<400> 3110
agtttgtcta tatcaccga

<212> DNA<213> Homo sapiens<220> <223> Cyclin A1 ribozyme binding site
<400> 3111
tttgtctata tcaccgatg

<212> DNA<213> Homo sapiens<220> <223> Cyclin A1 ribozyme binding site
<400> 3112
tgtctatatc accgatgat

<212> DNA<213> Homo sapiens<220> <223> Cyclin A1 ribozyme binding site
<400> 3113
accgatgata catacacaa

<212> DNA<213> Homo sapiens<220> <223> Cyclin A1 ribozyme binding site
<400> 3114
atgatacata cacaaaacg

<212> DNA<213> Homo sapiens<220> <223> Cyclin A1 ribozyme binding site
<400> 3115
gacaactgtt aaaaatgga

<212> DNA<213> Homo sapiens<220> <223> Cyclin A1 ribozyme binding site
<400> 3116
acaactgtta aaaatggaa

<212> DNA<213> Homo sapiens<220> <223> Cyclin A1 ribozyme binding site
<400> 3117
tggaacactt gcttctgaa

<212> DNA<213> Homo sapiens<220> <223> Cyclin A1 ribozyme binding site
<400> 3118
acacttgctt ctgaaagtt

<212> DNA<213> Homo sapiens<220> <223> Cyclin A1 ribozyme binding site
<400> 3119
cacttgcttc tgaaagttc

<212> DNA<213> Homo sapiens<220> <223> Cyclin A1 ribozyme binding site
<400> 3120
tctgaaagtt ctagctttt

<212> DNA<213> Homo sapiens<220> <223> Cyclin A1 ribozyme binding site

<400> 3121
ctgaaagttc tagcttttg

<212> DNA<213> Homo sapiens<220> <223> Cyclin A1 ribozyme binding site
<400> 3122
gaaagttcta gcttttgat

<212> DNA<213> Homo sapiens<220> <223> Cyclin A1 ribozyme binding site
<400> 3123
gttctagctt ttgatctga

<212> DNA<213> Homo sapiens<220> <223> Cyclin A1 ribozyme binding site
<400> 3124
ttctagcttt tgatctgac

<212> DNA<213> Homo sapiens<220> <223> Cyclin A1 ribozyme binding site
<400> 3125
tctagctttt gatctgaca

<212> DNA<213> Homo sapiens<220> <223> Cyclin A1 ribozyme binding site
<400> 3126
gcttttgatc tgacagtac

<212> DNA<213> Homo sapiens<220> <223> Cyclin A1 ribozyme binding site
<400> 3127
tctgacagta ccaaccacc

<212> DNA<213> Homo sapiens<220> <223> Cyclin A1 ribozyme binding site
<400> 3128
ccaaccagtt tctccttca

<212> DNA<213> Homo sapiens<220> <223> Cyclin A1 ribozyme binding site
<400> 3129
caaccagttt ctccttcag

<212> DNA<213> Homo sapiens<220> <223> Cyclin A1 ribozyme binding site
<400> 3130
aaccagtttc tccttcagt

<212> DNA<213> Homo sapiens<220> <223> Cyclin A1 ribozyme binding site
<400> 3131
ccagtttctc cttcagtac

<212> DNA<213> Homo sapiens<220> <223> Cyclin A1 ribozyme binding site
<400> 3132
gtttctcctt cagtacttg

<212> DNA<213> Homo sapiens<220> <223> Cyclin A1 ribozyme binding site
<400> 3133
tttctccttc agtacttga

<212> DNA<213> Homo sapiens<220> <223> Cyclin A1 ribozyme binding site
<400> 3134
tccttcagta cttgaggcg

<212> DNA<213> Homo sapiens<220> <223> Cyclin A1 ribozyme binding site
<400> 3135
ttcagtactt gaggcgaca

<212> DNA<213> Homo sapiens<220> <223> Cyclin A1 ribozyme binding site
<400> 3136
agtgtgcgtc aggactgag

<212> DNA<213> Homo sapiens<220> <223> Cyclin A1 ribozyme binding site
<400> 3137
aacctggcta agtacgtag

<212> DNA<213> Homo sapiens<220> <223> Cyclin A1 ribozyme binding site
<400> 3138
tggctaagta cgtagcsga

<212> DNA<213> Homo sapiens<220> <223> Cyclin A1 ribozyme binding site
<400> 3139
taagtacgta gcsgagctg

<212> DNA<213> Homo sapiens<220> <223> Cyclin A1 ribozyme binding site
<400> 3140
gagctgagtc tacttgaag

<212> DNA<213> Homo sapiens<220> <223> Cyclin A1 ribozyme binding site
<400> 3141
gctgagtcta cttgaagca

<212> DNA<213> Homo sapiens<220> <223> Cyclin A1 ribozyme binding site
<400> 3142
gagtctactt gaagcagat

<212> DNA<213> Homo sapiens<220> <223> Cyclin A1 ribozyme binding site
<400> 3143
gaagcagatc cattcttga

<212> DNA<213> Homo sapiens<220> <223> Cyclin A1 ribozyme binding site
<400> 3144
cagatccatt cttgaaata

<212> DNA<213> Homo sapiens<220> <223> Cyclin A1 ribozyme binding site
<400> 3145
agatccattc ttgaaatat

<212> DNA<213> Homo sapiens<220> <223> Cyclin A1 ribozyme binding site
<400> 3146
atccattctt gaaatatct

<212> DNA<213> Homo sapiens<220> <223> Cyclin A1 ribozyme binding site
<400> 3147
tcttgaaata tcttccttc

<212> DNA<213> Homo sapiens<220> <223> Cyclin A1 ribozyme binding site
<400> 3148
ttgaaatatc ttccttcac

<212> DNA<213> Homo sapiens<220> <223> Cyclin A1 ribozyme binding site
<400> 3149
gaaatatctt ccttcactg

<212> DNA<213> Homo sapiens<220> <223> Cyclin A1 ribozyme binding site
<400> 3150
aaatatcttc cttcactga

<212> DNA<213> Homo sapiens<220> <223> Cyclin A1 ribozyme binding site
<400> 3151
tatcttcctt cactgatag

<212> DNA<213> Homo sapiens<220> <223> Cyclin A1 ribozyme binding site
<400> 3152
atcttccttc actgatagc

<212> DNA<213> Homo sapiens<220> <223> Cyclin A1 ribozyme binding site
<400> 3153
ttcactgata gctgcagca

<212> DNA<213> Homo sapiens<220> <223> Cyclin A1 ribozyme binding site
<400> 3154
gcagcagctt tttgcctgg

<212> DNA<213> Homo sapiens<220> <223> Cyclin A1 ribozyme binding site
<400> 3155
cagcagcttt ttgcctggc

<212> DNA<213> Homo sapiens<220> <223> Cyclin A1 ribozyme binding site
<400> 3156
agcagctttt tgcctggca

<212> DNA<213> Homo sapiens<220> <223> Cyclin A1 ribozyme binding site
<400> 3157
gcagcttttt gcctggcaa

<212> DNA<213> Homo sapiens<220> <223> Cyclin A1 ribozyme binding site
<400> 3158
tggcaaacta tactgtgaa

<212> DNA<213> Homo sapiens<220> <223> Cyclin A1 ribozyme binding site
<400> 3159
gcaaactata ctgtgaaca

<212> DNA<213> Homo sapiens<220> <223> Cyclin A1 ribozyme binding site
<400> 3160
acaagcactt ttggccaga

<212> DNA<213> Homo sapiens<220> <223> Cyclin A1 ribozyme binding site
<400> 3161
caagcacttt tggccagaa

<212> DNA<213> Homo sapiens<220> <223> Cyclin A1 ribozyme binding site
<400> 3162

aagcactttt ggccagaaa

<212> DNA<213> Homo sapiens<220> <223> Cyclin A1 ribozyme binding site
<400> 3163
agaaaccctt gctgcattt

<212> DNA<213> Homo sapiens<220> <223> Cyclin A1 ribozyme binding site
<400> 3164
ttgctgcatt tacagggta

<212> DNA<213> Homo sapiens<220> <223> Cyclin A1 ribozyme binding site
<400> 3165
tgctgcattt acagggtat

<212> DNA<213> Homo sapiens<220> <223> Cyclin A1 ribozyme binding site
<400> 3166
gctgcattta cagggtatt

<212> DNA<213> Homo sapiens<220> <223> Cyclin A1 ribozyme binding site
<400> 3167
ttacagggta ttcattaag

<212> DNA<213> Homo sapiens<220> <223> Cyclin A1 ribozyme binding site
<400> 3168
acagggtatt cattaagtg

<212> DNA<213> Homo sapiens<220> <223> Cyclin A1 ribozyme binding site
<400> 3169
cagggtattc attaagtga

<212> DNA<213> Homo sapiens<220> <223> Cyclin A1 ribozyme binding site
<400> 3170
ggtattcatt aagtgaaat

<212> DNA<213> Homo sapiens<220> <223> Cyclin A1 ribozyme binding site
<400> 3171
gtattcatta agtgaaatt

<212> DNA<213> Homo sapiens<220> <223> Cyclin A1 ribozyme binding site
<400> 3172
aagtgaaatt gtgccttgc

<212> DNA<213> Homo sapiens<220> <223> Cyclin A1 ribozyme binding site
<400> 3173
attgtgcctt gcctgagtg

<212> DNA<213> Homo sapiens<220> <223> Cyclin A1 ribozyme binding site
<400> 3174
gagtgagctt cataaagcg

<212> DNA<213> Homo sapiens<220> <223> Cyclin A1 ribozyme binding site
<400> 3175
agtgagcttc ataaagcgt

<212> DNA<213> Homo sapiens<220> <223> Cyclin A1 ribozyme binding site

<400> 3176
gagcttcata aagcgtact

<212> DNA<213> Homo sapiens<220> <223> Cyclin A1 ribozyme binding site
<400> 3177
ataaagcgta ccttgatat

<212> DNA<213> Homo sapiens<220> <223> Cyclin A1 ribozyme binding site
<400> 3178
agcgtacctt gatataccc

<212> DNA<213> Homo sapiens<220> <223> Cyclin A1 ribozyme binding site
<400> 3179
taccttgata taccccatc

<212> DNA<213> Homo sapiens<220> <223> Cyclin A1 ribozyme binding site
<400> 3180
ccttgatata ccccatcga

<212> DNA<213> Homo sapiens<220> <223> Cyclin A1 ribozyme binding site
<400> 3181
ataccccatc gacctcagc

<212> DNA<213> Homo sapiens<220> <223> Cyclin A1 ribozyme binding site
<400> 3182
catcgacctc agcaagcaa

<212> DNA<213> Homo sapiens<220> <223> Cyclin A1 ribozyme binding site
<400> 3183
gcaagcaatt agggagaag

<212> DNA<213> Homo sapiens<220> <223> Cyclin A1 ribozyme binding site
<400> 3184
caagcaatta gggagaagt

<212> DNA<213> Homo sapiens<220> <223> Cyclin A1 ribozyme binding site
<400> 3185
gggagaagta caaggcttc

<212> DNA<213> Homo sapiens<220> <223> Cyclin A1 ribozyme binding site
<400> 3186
tacaaggctt caaagtacc

<212> DNA<213> Homo sapiens<220> <223> Cyclin A1 ribozyme binding site
<400> 3187
acaaggcttc aaagtacct

<212> DNA<213> Homo sapiens<220> <223> Cyclin A1 ribozyme binding site
<400> 3188
cttcaaagta cctgtgtgt

<212> DNA<213> Homo sapiens<220> <223> Cyclin A1 ribozyme binding site
<400> 3189
tgtgtgtgtc cctcatgga

<212> DNA<213> Homo sapiens<220> <223> Cyclin A1 ribozyme binding site
<400> 3190
tgtgtccctc atggagcca

<212> DNA<213> Homo sapiens<220> <223> Cyclin A1 ribozyme binding site
<400> 3191
acctgcagtt cttcttcta

<212> DNA<213> Homo sapiens<220> <223> Cyclin A1 ribozyme binding site
<400> 3192
cctgcagttc ttcttctac

<212> DNA<213> Homo sapiens<220> <223> Cyclin A1 ribozyme binding site
<400> 3193
tgcagttctt cttctacaa

<212> DNA<213> Homo sapiens<220> <223> Cyclin A1 ribozyme binding site
<400> 3194
gcagttcttc ttctacaat

<212> DNA<213> Homo sapiens<220> <223> Cyclin A1 ribozyme binding site
<400> 3195
agttcttctt ctacaataa

<212> DNA<213> Homo sapiens<220> <223> Cyclin A1 ribozyme binding site
<400> 3196
gttcttcttc tacaataag

<212> DNA<213> Homo sapiens<220> <223> Cyclin A1 ribozyme binding site
<400> 3197
tcttcttcta caataagtt

<212> DNA<213> Homo sapiens<220> <223> Cyclin A1 ribozyme binding site
<400> 3198
ttctacaata agtttctga

<212> DNA<213> Homo sapiens<220> <223> Cyclin A1 ribozyme binding site
<400> 3199
acaataagtt tctgaatgg

<212> DNA<213> Homo sapiens<220> <223> Cyclin A1 ribozyme binding site
<400> 3200
caataagttt ctgaatgga

<212> DNA<213> Homo sapiens<220> <223> Cyclin A1 ribozyme binding site
<400> 3201
aataagtttc tgaatggaa

<212> DNA<213> Homo sapiens<220> <223> Cyclin A1 ribozyme binding site
<400> 3202
ggaagcactt ccagaactt

<212> DNA<213> Homo sapiens<220> <223> Cyclin A1 ribozyme binding site
<400> 3203
gaagcacttc cagaacttc

<212> DNA<213> Homo sapiens<220> <223> Cyclin A1 ribozyme binding site
<400> 3204
tccagaactt cacctccat

<212> DNA<213> Homo sapiens<220> <223> Cyclin A1 ribozyme binding site
<400> 3205
ccagaacttc acctccata

<212> DNA<213> Homo sapiens<220> <223> Cyclin A1 ribozyme binding site
<400> 3206
acttcacctc catatcaga

<212> DNA<213> Homo sapiens<220> <223> Cyclin A1 ribozyme binding site
<400> 3207
cacctccata tcagaagtg

<212> DNA<213> Homo sapiens<220> <223> Cyclin A1 ribozyme binding site
<400> 3208
cctccatatc agaagtgcc

<212> DNA<213> Homo sapiens<220> <223> Cyclin A1 ribozyme binding site
<400> 3209
agtgccaata atcgtcata

<212> DNA<213> Homo sapiens<220> <223> Cyclin A1 ribozyme binding site
<400> 3210
gccaataatc gtcataggc

<212> DNA<213> Homo sapiens<220> <223> Cyclin A1 ribozyme binding site
<400> 3211
aataatcgtc ataggcttc

<212> DNA<213> Homo sapiens<220> <223> Cyclin A1 ribozyme binding site
<400> 3212
aatcgtcata ggcttctgc

<212> DNA<213> Homo sapiens<220> <223> Cyclin A1 ribozyme binding site
<400> 3213
tcataggctt ctgcacgtt

<212> DNA<213> Homo sapiens<220> <223> Cyclin A1 ribozyme binding site
<400> 3214
cataggcttc tgcacgttg

<212> DNA<213> Homo sapiens<220> <223> Cyclin A1 ribozyme binding site
<400> 3215
tctgcacgtt ggatcaact

<212> DNA<213> Homo sapiens<220> <223> Cyclin A1 ribozyme binding site
<400> 3216
acgttggatc aactaatgt

<212> DNA<213> Homo sapiens<220> <223> Cyclin A1 ribozyme binding site
<400> 3217

ggatcaacta atgttgttt

<212> DNA<213> Homo sapiens<220> <223> Cyclin A1 ribozyme binding site
<400> 3218
aactaatgtt gtttacaat

<212> DNA<213> Homo sapiens<220> <223> Cyclin A1 ribozyme binding site
<400> 3219
taatgttgtt tacaatata

<212> DNA<213> Homo sapiens<220> <223> Cyclin A1 ribozyme binding site
<400> 3220
aatgttgttt acaatatag

<212> DNA<213> Homo sapiens<220> <223> Cyclin A1 ribozyme binding site
<400> 3221
atgttgttta caatataga

<212> DNA<213> Homo sapiens<220> <223> Cyclin A1 ribozyme binding site
<400> 3222
gtttacaata tagatgaca

<212> DNA<213> Homo sapiens<220> <223> Cyclin A1 ribozyme binding site
<400> 3223
ttacaatata gatgacatt

<212> DNA<213> Homo sapiens<220> <223> Cyclin A1 ribozyme binding site
<400> 3224
agatgacatt ttaaaaatg

<212> DNA<213> Homo sapiens<220> <223> Cyclin A1 ribozyme binding site
<400> 3225
gatgacattt taaaaatgt

<212> DNA<213> Homo sapiens<220> <223> Cyclin A1 ribozyme binding site
<400> 3226
atgacatttt aaaaatgta

<212> DNA<213> Homo sapiens<220> <223> Cyclin A1 ribozyme binding site
<400> 3227
tgacatttta aaaatgtaa

<212> DNA<213> Homo sapiens<220> <223> Cyclin A1 ribozyme binding site
<400> 3228
taaaaatgta aatgaattt

<212> DNA<213> Homo sapiens<220> <223> Cyclin A1 ribozyme binding site
<400> 3229
taaatgaatt tagtttccc

<212> DNA<213> Homo sapiens<220> <223> Cyclin A1 ribozyme binding site
<400> 3230
aaatgaattt agtttccct

<212> DNA<213> Homo sapiens<220> <223> Cyclin A1 ribozyme binding site

<400> 3231
aatgaattta gtttccctt

<212> DNA<213> Homo sapiens<220> <223> Cyclin A1 ribozyme binding site
<400> 3232
gaatttagtt tcccttaga

<212> DNA<213> Homo sapiens<220> <223> Cyclin A1 ribozyme binding site
<400> 3233
aatttagttt cccttagac

<212> DNA<213> Homo sapiens<220> <223> Cyclin A1 ribozyme binding site
<400> 3234
atttagtttc ccttagact

<212> DNA<213> Homo sapiens<220> <223> Cyclin A1 ribozyme binding site
<400> 3235
agtttccctt agactttag

<212> DNA<213> Homo sapiens<220> <223> Cyclin A1 ribozyme binding site
<400> 3236
gtttccctta gactttagt

<212> DNA<213> Homo sapiens<220> <223> Cyclin A1 ribozyme binding site
<400> 3237
ccttagactt tagtagttt

<212> DNA<213> Homo sapiens<220> <223> Cyclin A1 ribozyme binding site
<400> 3238
cttagacttt agtagtttg

<212> DNA<213> Homo sapiens<220> <223> Cyclin A1 ribozyme binding site
<400> 3239
ttagacttta gtagtttgt

<212> DNA<213> Homo sapiens<220> <223> Cyclin A1 ribozyme binding site
<400> 3240
gactttagta gtttgtaat

<212> DNA<213> Homo sapiens<220> <223> Cyclin A1 ribozyme binding site
<400> 3241
tttagtagtt tgtaatata

<212> DNA<213> Homo sapiens<220> <223> Cyclin A1 ribozyme binding site
<400> 3242
ttagtagttt gtaatatag

<212> DNA<213> Homo sapiens<220> <223> Cyclin A1 ribozyme binding site
<400> 3243
gtagtttgta atatagtcc

<212> DNA<213> Homo sapiens<220> <223> Cyclin A1 ribozyme binding site
<400> 3244
gtttgtaata tagtccaac

<212> DNA<213> Homo sapiens<220> <223> Cyclin A1 ribozyme binding site
<400> 3245
ttgtaatata gtccaacat

<212> DNA<213> Homo sapiens<220> <223> Cyclin A1 ribozyme binding site
<400> 3246
taatatagtc caacatttt

<212> DNA<213> Homo sapiens<220> <223> Cyclin A1 ribozyme binding site
<400> 3247
gtccaacatt ttttaaaca

<212> DNA<213> Homo sapiens<220> <223> Cyclin A1 ribozyme binding site
<400> 3248
tccaacattt tttaaacaa

<212> DNA<213> Homo sapiens<220> <223> Cyclin A1 ribozyme binding site
<400> 3249
ccaacatttt ttaaacaat

<212> DNA<213> Homo sapiens<220> <223> Cyclin A1 ribozyme binding site
<400> 3250
caacattttt taaacaata

<212> DNA<213> Homo sapiens<220> <223> Cyclin A1 ribozyme binding site
<400> 3251
aacatttttt aaacaataa

<212> DNA<213> Homo sapiens<220> <223> Cyclin A1 ribozyme binding site
<400> 3252
acatttttta aacaataaa

<212> DNA<213> Homo sapiens<220> <223> Cyclin A1 ribozyme binding site
<400> 3253
ttaaacaata aactgcttg

<212> DNA<213> Homo sapiens<220> <223> Cyclin A1 ribozyme binding site
<400> 3254
taaactgctt gtcttatga

<212> DNA<213> Homo sapiens<220> <223> Cyclin A1 ribozyme binding site
<400> 3255
actgcttgtc ttatgacaa

<212> DNA<213> Homo sapiens<220> <223> Cyclin A1 ribozyme binding site
<400> 3256
tgcttgtctt atgacaaaa

<212> DNA<213> Homo sapiens<220> <223> Cyclin A1 ribozyme binding site
<400> 3257
gcttgtctta tgacaaaaa

<212> DNA<213> Homo sapiens<220> <223> Cyclin B1 ribozyme binding site
<400> 3258
gctgggtgta ggtccttgg

<212> DNA<213> Homo sapiens<220> <223> Cyclin B1 ribozyme binding site
<400> 3259
ggtgtaggtc cttggctgg

<212> DNA<213> Homo sapiens<220> <223> Cyclin B1 ribozyme binding site
<400> 3260
gtaggtcctt ggctggtcg

<212> DNA<213> Homo sapiens<220> <223> Cyclin B1 ribozyme binding site
<400> 3261
ttggctggtc gggctccgg

<212> DNA<213> Homo sapiens<220> <223> Cyclin B1 ribozyme binding site
<400> 3262
ggtcgggctc cggtgttct

<212> DNA<213> Homo sapiens<220> <223> Cyclin B1 ribozyme binding site
<400> 3263
ctccggtgtt ctgcttctc

<212> DNA<213> Homo sapiens<220> <223> Cyclin B1 ribozyme binding site
<400> 3264
tccggtgttc tgcttctcc

<212> DNA<213> Homo sapiens<220> <223> Cyclin B1 ribozyme binding site
<400> 3265
tgttctgctt ctccccgct

<212> DNA<213> Homo sapiens<220> <223> Cyclin B1 ribozyme binding site
<400> 3266
gttctgcttc tccccgctg

<212> DNA<213> Homo sapiens<220> <223> Cyclin B1 ribozyme binding site
<400> 3267
tctgcttctc cccgctgag

<212> DNA<213> Homo sapiens<220> <223> Cyclin B1 ribozyme binding site
<400> 3268
catggcgctc cgagtcacc

<212> DNA<213> Homo sapiens<220> <223> Cyclin B1 ribozyme binding site
<400> 3269
gctccgagtc accaggaac

<212> DNA<213> Homo sapiens<220> <223> Cyclin B1 ribozyme binding site
<400> 3270
ccaggaactc gaaaattaa

<212> DNA<213> Homo sapiens<220> <223> Cyclin B1 ribozyme binding site
<400> 3271
ctcgaaaatt aatgctgaa

<212> DNA<213> Homo sapiens<220> <223> Cyclin B1 ribozyme binding site
<400> 3272

tcgaaaatta atgctgaaa

<212> DNA<213> Homo sapiens<220> <223> Cyclin B1 ribozyme binding site
<400> 3273
gctgaaaata aggcgaaga

<212> DNA<213> Homo sapiens<220> <223> Cyclin B1 ribozyme binding site
<400> 3274
ggcgaagatc aacatggca

<212> DNA<213> Homo sapiens<220> <223> Cyclin B1 ribozyme binding site
<400> 3275
aaagcgcgtt cctacggcc

<212> DNA<213> Homo sapiens<220> <223> Cyclin B1 ribozyme binding site
<400> 3276
aagcgcgttc ctacggccc

<212> DNA<213> Homo sapiens<220> <223> Cyclin B1 ribozyme binding site
<400> 3277
cgcgttccta cggcccctg

<212> DNA<213> Homo sapiens<220> <223> Cyclin B1 ribozyme binding site
<400> 3278
ctgcaacctc caagcccgg

<212> DNA<213> Homo sapiens<220> <223> Cyclin B1 ribozyme binding site
<400> 3279
agaacagctc ttggggaca

<212> DNA<213> Homo sapiens<220> <223> Cyclin B1 ribozyme binding site
<400> 3280
aacagctctt ggggacatt

<212> DNA<213> Homo sapiens<220> <223> Cyclin B1 ribozyme binding site
<400> 3281
tggggacatt ggtaacaaa

<212> DNA<213> Homo sapiens<220> <223> Cyclin B1 ribozyme binding site
<400> 3282 .
gacattggta acaaagtca

<212> DNA<213> Homo sapiens<220> <223> Cyclin B1 ribozyme binding site
<400> 3283
taacaaagtc agtgaacaa

<212> DNA<213> Homo sapiens<220> <223> Cyclin B1 ribozyme binding site
<400> 3284
aaaatgccta tgaagaagg

<212> DNA<213> Homo sapiens<220> <223> Cyclin B1 ribozyme binding site
<400> 3285
gcaaaacctt cagctactg

<212> DNA<213> Homo sapiens<220> <223> Cyclin B1 ribozyme binding site

<400> 3286
caaaaccttc agctactgg

<212> DNA<213> Homo sapiens<220> <223> Cyclin B1 ribozyme binding site
<400> 3287
ccttcagcta ctggaaaag

<212> DNA<213> Homo sapiens<220> <223> Cyclin B1 ribozyme binding site
<400> 3288
tggaaaagtc attgataaa

<212> DNA<213> Homo sapiens<220> <223> Cyclin B1 ribozyme binding site
<400> 3289
aaaagtcatt gataaaaaa

<212> DNA<213> Homo sapiens<220> <223> Cyclin B1 ribozyme binding site
<400> 3290
gtcattgata aaaaactac

<212> DNA<213> Homo sapiens<220> <223> Cyclin B1 ribozyme binding site
<400> 3291
taaaaaacta ccaaaacct

<212> DNA<213> Homo sapiens<220> <223> Cyclin B1 ribozyme binding site
<400> 3292
ccaaaacctc ttgaaaagg

<212> DNA<213> Homo sapiens<220> <223> Cyclin B1 ribozyme binding site
<400> 3293
aaaacctctt gaaaaggta

<212> DNA<213> Homo sapiens<220> <223> Cyclin B1 ribozyme binding site
<400> 3294
tgaaaaggta cctatgctg

<212> DNA<213> Homo sapiens<220> <223> Cyclin B1 ribozyme binding site
<400> 3295
aaggtaccta tgctggtgc

<212> DNA<213> Homo sapiens<220> <223> Cyclin B1 ribozyme binding site
<400> 3296
tgccagtgtc tgagccagt

<212> DNA<213> Homo sapiens<220> <223> Cyclin B1 ribozyme binding site
<400> 3297
tgagcctgtt aaagaagaa

<212> DNA<213> Homo sapiens<220> <223> Cyclin B1 ribozyme binding site
<400> 3298
gagcctgtta aagaagaaa

<212> DNA<213> Homo sapiens<220> <223> Cyclin B1 ribozyme binding site
<400> 3299
agaaaaactt tcgcctgag

<212> DNA<213> Homo sapiens<220> <223> Cyclin B1 ribozyme binding site
<400> 3300
gaaaaacttt cgcctgagc

<212> DNA<213> Homo sapiens<220> <223> Cyclin B1 ribozyme binding site
<400> 3301
aaaaactttc gcctgagcc

<212> DNA<213> Homo sapiens<220> <223> Cyclin B1 ribozyme binding site
<400> 3302
cctgagccta ttttggttg

<212> DNA<213> Homo sapiens<220> <223> Cyclin B1 ribozyme binding site
<400> 3303
tgagcctatt ttggttgat

<212> DNA<213> Homo sapiens<220> <223> Cyclin B1 ribozyme binding site
<400> 3304
gagcctattt tggttgata

<212> DNA<213> Homo sapiens<220> <223> Cyclin B1 ribozyme binding site
<400> 3305
agcctatttt ggttgatac

<212> DNA<213> Homo sapiens<220> <223> Cyclin B1 ribozyme binding site
<400> 3306
tattttggtt gatactgcc

<212> DNA<213> Homo sapiens<220> <223> Cyclin B1 ribozyme binding site
<400> 3307
ttggttgata ctgcctctc

<212> DNA<213> Homo sapiens<220> <223> Cyclin B1 ribozyme binding site
<400> 3308
atactgcctc tccaagccc

<212> DNA<213> Homo sapiens<220> <223> Cyclin B1 ribozyme binding site
<400> 3309
actgcctctc caagcccaa

<212> DNA<213> Homo sapiens<220> <223> Cyclin B1 ribozyme binding site
<400> 3310
tggaaacatc tggatgtgc

<212> DNA<213> Homo sapiens<220> <223> Cyclin B1 ribozyme binding site
<400> 3311
gacctgtgtc aggctttct

<212> DNA<213> Homo sapiens<220> <223> Cyclin B1 ribozyme binding site
<400> 3312
tgtcaggctt tctctgatg

<212> DNA<213> Homo sapiens<220> <223> Cyclin B1 ribozyme binding site
<400> 3313
gtcaggcttt ctctgatgt

<212> DNA<213> Homo sapiens<220> <223> Cyclin B1 ribozyme binding site
<400> 3314
tcaggctttc tctgatgta

<212> DNA<213> Homo sapiens<220> <223> Cyclin B1 ribozyme binding site
<400> 3315
aggctttctc tgatgtaat

<212> DNA<213> Homo sapiens<220> <223> Cyclin B1 ribozyme binding site
<400> 3316
ctctgatgta attcttgca

<212> DNA<213> Homo sapiens<220> <223> Cyclin B1 ribozyme binding site
<400> 3317
tgatgtaatt cttgcagta

<212> DNA<213> Homo sapiens<220> <223> Cyclin B1 ribozyme binding site
<400> 3318
gatgtaattc ttgcagtaa

<212> DNA<213> Homo sapiens<220> <223> Cyclin B1 ribozyme binding site
<400> 3319
tgtaattctt gcagtaaat

<212> DNA<213> Homo sapiens<220> <223> Cyclin B1 ribozyme binding site
<400> 3320
tcttgcagta aatgatgtg

<212> DNA<213> Homo sapiens<220> <223> Cyclin B1 ribozyme binding site
<400> 3321
ggagctgatc caaaccttt

<212> DNA<213> Homo sapiens<220> <223> Cyclin B1 ribozyme binding site
<400> 3322
tccaaacctt tgtagtgaa

<212> DNA<213> Homo sapiens<220> <223> Cyclin B1 ribozyme binding site
<400> 3323
ccaaaccttt gtagtgaat

<212> DNA<213> Homo sapiens<220> <223> Cyclin B1 ribozyme binding site
<400> 3324
aacctttgta gtgaatatg

<212> DNA<213> Homo sapiens<220> <223> Cyclin B1 ribozyme binding site
<400> 3325
gtagtgaata tgtgaaaga

<212> DNA<213> Homo sapiens<220> <223> Cyclin B1 ribozyme binding site
<400> 3326
gtgaaagata tttatgctt

<212> DNA<213> Homo sapiens<220> <223> Cyclin B1 ribozyme binding site
<400> 3327

gaaagatatt tatgcttat

<212> DNA<213> Homo sapiens<220> <223> Cyclin B1 ribozyme binding site
<400> 3328
aaagatattt atgcttatc

<212> DNA<213> Homo sapiens<220> <223> Cyclin B1 ribozyme binding site
<400> 3329
aagatattta tgcttatct

<212> DNA<213> Homo sapiens<220> <223> Cyclin B1 ribozyme binding site
<400> 3330
atttatgctt atctgagac

<212> DNA<213> Homo sapiens<220> <223> Cyclin B1 ribozyme binding site
<400> 3331
tttatgctta tctgagaca

<212> DNA<213> Homo sapiens<220> <223> Cyclin B1 ribozyme binding site
<400> 3332
tatgcttatc tgagacaac

<212> DNA<213> Homo sapiens<220> <223> Cyclin B1 ribozyme binding site
<400> 3333
gagacaactt gaggaagag

<212> DNA<213> Homo sapiens<220> <223> Cyclin B1 ribozyme binding site
<400> 3334
gcaagcagtc agaccaaaa

<212> DNA<213> Homo sapiens<220> <223> Cyclin B1 ribozyme binding site
<400> 3335
gaccaaaata cctactggg

<212> DNA<213> Homo sapiens<220> <223> Cyclin B1 ribozyme binding site
<400> 3336
aaaataccta ctgggtcgg

<212> DNA<213> Homo sapiens<220> <223> Cyclin B1 ribozyme binding site
<400> 3337
ctactgggtc gggaagtca

<212> DNA<213> Homo sapiens<220> <223> Cyclin B1 ribozyme binding site
<400> 3338
tcgggaagtc actggaaac

<212> DNA<213> Homo sapiens<220> <223> Cyclin B1 ribozyme binding site
<400> 3339
gagagccatc ctaattgac

<212> DNA<213> Homo sapiens<220> <223> Cyclin B1 ribozyme binding site
<400> 3340
agccatccta attgactgg

<212> DNA<213> Homo sapiens<220> <223> Cyclin B1 ribozyme binding site

<400> 3341
catcctaatt gactggcta

<212> DNA<213> Homo sapiens<220> <223> Cyclin B1 ribozyme binding site
<400> 3342
tgactggcta gtacaggtt

<212> DNA<213> Homo sapiens<220> <223> Cyclin B1 ribozyme binding site
<400> 3343
ctggctagta caggttcaa

<212> DNA<213> Homo sapiens<220> <223> Cyclin B1 ribozyme binding site
<400> 3344
agtacaggtt caaatgaaa

<212> DNA<213> Homo sapiens<220> <223> Cyclin B1 ribozyme binding site
<400> 3345
gtacaggttc aaatgaaat

<212> DNA<213> Homo sapiens<220> <223> Cyclin B1 ribozyme binding site
<400> 3346
aaatgaaatt caggttgtt

<212> DNA<213> Homo sapiens<220> <223> Cyclin B1 ribozyme binding site
<400> 3347
aatgaaattc aggttgttg

<212> DNA<213> Homo sapiens<220> <223> Cyclin B1 ribozyme binding site
<400> 3348
aattcaggtt gttgcagga

<212> DNA<213> Homo sapiens<220> <223> Cyclin B1 ribozyme binding site
<400> 3349
tcaggttgtt gcaggagac

<212> DNA<213> Homo sapiens<220> <223> Cyclin B1 ribozyme binding site
<400> 3350
agaccatgta catgactgt

<212> DNA<213> Homo sapiens<220> <223> Cyclin B1 ribozyme binding site
<400> 3351
catgactgtc tccattatt

<212> DNA<213> Homo sapiens<220> <223> Cyclin B1 ribozyme binding site
<400> 3352
tgactgtctc cattattga

<212> DNA<213> Homo sapiens<220> <223> Cyclin B1 ribozyme binding site
<400> 3353
tgtctccatt attgatcgg

<212> DNA<213> Homo sapiens<220> <223> Cyclin B1 ribozyme binding site
<400> 3354
gtctccatta ttgatcggt

<212> DNA<213> Homo sapiens<220> <223> Cyclin B1 ribozyme binding site
<400> 3355
ctccattatt gatcggttc

<212> DNA<213> Homo sapiens<220> <223> Cyclin B1 ribozyme binding site
<400> 3356
attattgatc ggttcatgc

<212> DNA<213> Homo sapiens<220> <223> Cyclin B1 ribozyme binding site
<400> 3357
ttgatcggtt catgcagaa

<212> DNA<213> Homo sapiens<220> <223> Cyclin B1 ribozyme binding site
<400> 3358
tgatcggttc atgcagaat

<212> DNA<213> Homo sapiens<220> <223> Cyclin B1 ribozyme binding site
<400> 3359
atgcagaata attgtgtgc

<212> DNA<213> Homo sapiens<220> <223> Cyclin B1 ribozyme binding site
<400> 3360
cagaataatt gtgtgccca

<212> DNA<213> Homo sapiens<220> <223> Cyclin B1 ribozyme binding site
<400> 3361
gcagctggtt ggtgtcact

<212> DNA<213> Homo sapiens<220> <223> Cyclin B1 ribozyme binding site
<400> 3362
ggttggtgtc actgccatg

<212> DNA<213> Homo sapiens<220> <223> Cyclin B1 ribozyme binding site
<400> 3363
ctgccatgtt tattgcaag

<212> DNA<213> Homo sapiens<220> <223> Cyclin B1 ribozyme binding site
<400> 3364
tgccatgttt attgcaagc

<212> DNA<213> Homo sapiens<220> <223> Cyclin B1 ribozyme binding site
<400> 3365
gccatgttta ttgcaagca

<212> DNA<213> Homo sapiens<220> <223> Cyclin B1 ribozyme binding site
<400> 3366
catgtttatt gcaagcaaa

<212> DNA<213> Homo sapiens<220> <223> Cyclin B1 ribozyme binding site
<400> 3367
caagcaaata tgaagaaat

<212> DNA<213> Homo sapiens<220> <223> Cyclin B1 ribozyme binding site
<400> 3368
aagaaatgta ccctccaga

<212> DNA<213> Homo sapiens<220> <223> Cyclin B1 ribozyme binding site
<400> 3369
atgtaccctc cagaaattg

<212> DNA<213> Homo sapiens<220> <223> Cyclin B1 ribozyme binding site
<400> 3370
tccagaaatt ggtgacttt

<212> DNA<213> Homo sapiens<220> <223> Cyclin B1 ribozyme binding site
<400> 3371
ttggtgactt tgcttttgt

<212> DNA<213> Homo sapiens<220> <223> Cyclin B1 ribozyme binding site
<400> 3372
tggtgacttt gcttttgtg

<212> DNA<213> Homo sapiens<220> <223> Cyclin B1 ribozyme binding site
<400> 3373
gactttgctt ttgtgactg

<212> DNA<213> Homo sapiens<220> <223> Cyclin B1 ribozyme binding site
<400> 3374
actttgcttt tgtgactga

<212> DNA<213> Homo sapiens<220> <223> Cyclin B1 ribozyme binding site
<400> 3375
ctttgctttt gtgactgac

<212> DNA<213> Homo sapiens<220> <223> Cyclin B1 ribozyme binding site
<400> 3376
gacaacactt atactaagc

<212> DNA<213> Homo sapiens<220> <223> Cyclin B1 ribozyme binding site
<400> 3377
acaacactta tactaagca

<212> DNA<213> Homo sapiens<220> <223> Cyclin B1 ribozyme binding site
<400> 3378
aacacttata ctaagcacc

<212> DNA<213> Homo sapiens<220> <223> Cyclin B1 ribozyme binding site
<400> 3379
acttatacta agcaccaaa

<212> DNA<213> Homo sapiens<220> <223> Cyclin B1 ribozyme binding site
<400> 3380
gcaccaaatc agacagatg

<212> DNA<213> Homo sapiens<220> <223> Cyclin B1 ribozyme binding site
<400> 3381
aatgaagatt ctaagagct

<212> DNA<213> Homo sapiens<220> <223> Cyclin B1 ribozyme binding site
<400> 3382

atgaagattc taagagctt

<212> DNA<213> Homo sapiens<220> <223> Cyclin B1 ribozyme binding site
<400> 3383
gaagattcta agagcttta

<212> DNA<213> Homo sapiens<220> <223> Cyclin B1 ribozyme binding site
<400> 3384
ctaagagctt taaactttg

<212> DNA<213> Homo sapiens<220> <223> Cyclin B1 ribozyme binding site
<400> 3385
taagagcttt aaactttgg

<212> DNA<213> Homo sapiens<220> <223> Cyclin B1 ribozyme binding site
<400> 3386
aagagcttta aactttggt

<212> DNA<213> Homo sapiens<220> <223> Cyclin B1 ribozyme binding site
<400> 3387
ctttaaactt tggtctggg

<212> DNA<213> Homo sapiens<220> <223> Cyclin B1 ribozyme binding site
<400> 3388
tttaaacttt ggtctgggt

<212> DNA<213> Homo sapiens<220> <223> Cyclin B1 ribozyme binding site
<400> 3389
aactttggtc tgggtcggc

<212> DNA<213> Homo sapiens<220> <223> Cyclin B1 ribozyme binding site
<400> 3390
ggtctgggtc ggcctctac

<212> DNA<213> Homo sapiens<220> <223> Cyclin B1 ribozyme binding site
<400> 3391
ggtcggcctc tacctttgc

<212> DNA<213> Homo sapiens<220> <223> Cyclin B1 ribozyme binding site
<400> 3392
tcggcctcta cctttgcac

<212> DNA<213> Homo sapiens<220> <223> Cyclin B1 ribozyme binding site
<400> 3393
cctctacctt tgcacttcc

<212> DNA<213> Homo sapiens<220> <223> Cyclin B1 ribozyme binding site
<400> 3394
ctctaccttt gcacttcct

<212> DNA<213> Homo sapiens<220> <223> Cyclin B1 ribozyme binding site
<400> 3395
ctttgcactt ccttcggag

<212> DNA<213> Homo sapiens<220> <223> Cyclin B1 ribozyme binding site

<400> 3396
tttgcacttc cttcggaga

<212> DNA<213> Homo sapiens<220> <223> Cyclin B1 ribozyme binding site
<400> 3397
gcacttcctt cggagagca

<212> DNA<213> Homo sapiens<220> <223> Cyclin B1 ribozyme binding site
<400> 3398
cacttccttc ggagagcat

<212> DNA<213> Homo sapiens<220> <223> Cyclin B1 ribozyme binding site
<400> 3399
ggagagcatc taagattgg

<212> DNA<213> Homo sapiens<220> <223> Cyclin B1 ribozyme binding site
<400> 3400
agagcatcta agattggag

<212> DNA<213> Homo sapiens<220> <223> Cyclin B1 ribozyme binding site
<400> 3401
atctaagatt ggagaggtt

<212> DNA<213> Homo sapiens<220> <223> Cyclin B1 ribozyme binding site
<400> 3402
tggagaggtt gatgtcgag

<212> DNA<213> Homo sapiens<220> <223> Cyclin B1 ribozyme binding site
<400> 3403
ggttgatgtc gagcaacat

<212> DNA<213> Homo sapiens<220> <223> Cyclin B1 ribozyme binding site
<400> 3404
gagcaacata ctttggcca

<212> DNA<213> Homo sapiens<220> <223> Cyclin B1 ribozyme binding site
<400> 3405
caacatactt tggccaaat

<212> DNA<213> Homo sapiens<220> <223> Cyclin B1 ribozyme binding site
<400> 3406
aacatacttt ggccaaata

<212> DNA<213> Homo sapiens<220> <223> Cyclin B1 ribozyme binding site
<400> 3407
tggccaaata cctgatgga

<212> DNA<213> Homo sapiens<220> <223> Cyclin B1 ribozyme binding site
<400> 3408
gatggaacta actatgttg

<212> DNA<213> Homo sapiens<220> <223> Cyclin B1 ribozyme binding site
<400> 3409
gaactaacta tgttggact

<212> DNA<213> Homo sapiens<220> <223> Cyclin B1 ribozyme binding site
<400> 3410
taactatgtt ggactatga

<212> DNA<213> Homo sapiens<220> <223> Cyclin B1 ribozyme binding site
<400> 3411
tgttggacta tgacatggt

<212> DNA<213> Homo sapiens<220> <223> Cyclin B1 ribozyme binding site
<400> 3412
tggtgcactt tcctccttc

<212> DNA<213> Homo sapiens<220> <223> Cyclin B1 ribozyme binding site
<400> 3413
ggtgcacttt cctccttct

<212> DNA<213> Homo sapiens<220> <223> Cyclin B1 ribozyme binding site
<400> 3414
gtgcactttc ctccttctc

<212> DNA<213> Homo sapiens<220> <223> Cyclin B1 ribozyme binding site
<400> 3415
cactttcctc cttctcaaa

<212> DNA<213> Homo sapiens<220> <223> Cyclin B1 ribozyme binding site
<400> 3416
tttcctcctt ctcaaattg

<212> DNA<213> Homo sapiens<220> <223> Cyclin B1 ribozyme binding site
<400> 3417
ttcctccttc tcaaattgc

<212> DNA<213> Homo sapiens<220> <223> Cyclin B1 ribozyme binding site
<400> 3418
cctccttctc aaattgcag

<212> DNA<213> Homo sapiens<220> <223> Cyclin B1 ribozyme binding site
<400> 3419
ttctcaaatt gcagcagga

<212> DNA<213> Homo sapiens<220> <223> Cyclin B1 ribozyme binding site
<400> 3420
gcaggagctt tttgcttag

<212> DNA<213> Homo sapiens<220> <223> Cyclin B1 ribozyme binding site
<400> 3421
caggagcttt ttgcttagc

<212> DNA<213> Homo sapiens<220> <223> Cyclin B1 ribozyme binding site
<400> 3422
aggagctttt tgcttagca

<212> DNA<213> Homo sapiens<220> <223> Cyclin B1 ribozyme binding site
<400> 3423
ggagcttttt gcttagcac

<212> DNA<213> Homo sapiens<220> <223> Cyclin B1 ribozyme binding site
<400> 3424
cttttgctt agcactgaa

<212> DNA<213> Homo sapiens<220> <223> Cyclin B1 ribozyme binding site
<400> 3425
ttttgctta gcactgaaa

<212> DNA<213> Homo sapiens<220> <223> Cyclin B1 ribozyme binding site
<400> 3426
actgaaaatt ctggataat

<212> DNA<213> Homo sapiens<220> <223> Cyclin B1 ribozyme binding site
<400> 3427
ctgaaaattc tggataatg

<212> DNA<213> Homo sapiens<220> <223> Cyclin B1 ribozyme binding site
<400> 3428
attctggata atggtgaat

<212> DNA<213> Homo sapiens<220> <223> Cyclin B1 ribozyme binding site
<400> 3429
acaccaactc tacaacatt

<212> DNA<213> Homo sapiens<220> <223> Cyclin B1 ribozyme binding site
<400> 3430
accaactcta caacattac

<212> DNA<213> Homo sapiens<220> <223> Cyclin B1 ribozyme binding site
<400> 3431
ctacaacatt acctgtcat

<212> DNA<213> Homo sapiens<220> <223> Cyclin B1 ribozyme binding site
<400> 3432
tacaacatta cctgtcata

<212> DNA<213> Homo sapiens<220> <223> Cyclin B1 ribozyme binding site
<400> 3433
attacctgtc atatactga

<212> DNA<213> Homo sapiens<220> <223> Cyclin B1 ribozyme binding site
<400> 3434
acctgtcata tactgaaga

<212> DNA<213> Homo sapiens<220> <223> Cyclin B1 ribozyme binding site
<400> 3435
ctgtcatata ctgaagaat

<212> DNA<213> Homo sapiens<220> <223> Cyclin B1 ribozyme binding site
<400> 3436
ctgaagaatc tcttcttcc

<212> DNA<213> Homo sapiens<220> <223> Cyclin B1 ribozyme binding site
<400> 3437

gaagaatctc ttcttccag

<212> DNA<213> Homo sapiens<220> <223> Cyclin B1 ribozyme binding site
<400> 3438
agaatctctt cttccagtt

<212> DNA<213> Homo sapiens<220> <223> Cyclin B1 ribozyme binding site
<400> 3439
gaatctcttc ttccagtta

<212> DNA<213> Homo sapiens<220> <223> Cyclin B1 ribozyme binding site
<400> 3440
atctcttctt ccagttatg

<212> DNA<213> Homo sapiens<220> <223> Cyclin B1 ribozyme binding site
<400> 3441
tctcttcttc cagttatgc

<212> DNA<213> Homo sapiens<220> <223> Cyclin B1 ribozyme binding site
<400> 3442
tcttccagtt atgcagcac

<212> DNA<213> Homo sapiens<220> <223> Cyclin B1 ribozyme binding site
<400> 3443
cttccagtta tgcagcacc

<212> DNA<213> Homo sapiens<220> <223> Cyclin B1 ribozyme binding site
<400> 3444
cacctggcta agaatgtag

<212> DNA<213> Homo sapiens<220> <223> Cyclin B1 ribozyme binding site
<400> 3445
taagaatgta gtcatggta

<212> DNA<213> Homo sapiens<220> <223> Cyclin B1 ribozyme binding site
<400> 3446
gaatgtagtc atggtaaat

<212> DNA<213> Homo sapiens<220> <223> Cyclin B1 ribozyme binding site
<400> 3447
agtcatggta aatcaagga

<212> DNA<213> Homo sapiens<220> <223> Cyclin B1 ribozyme binding site
<400> 3448
atggtaaatc aaggactta

<212> DNA<213> Homo sapiens<220> <223> Cyclin B1 ribozyme binding site
<400> 3449
tcaaggactt acaaagcac

<212> DNA<213> Homo sapiens<220> <223> Cyclin B1 ribozyme binding site
<400> 3450
caaggactta caaagcaca

<212> DNA<213> Homo sapiens<220> <223> Cyclin B1 ribozyme binding site

<400> 3451
catgactgtc aagaacaag

<212> DNA<213> Homo sapiens<220> <223> Cyclin B1 ribozyme binding site
<400> 3452
agaacaagta tgccacatc

<212> DNA<213> Homo sapiens<220> <223> Cyclin B1 ribozyme binding site
<400> 3453
atgccacatc gaagcatgc

<212> DNA<213> Homo sapiens<220> <223> Cyclin B1 ribozyme binding site
<400> 3454
aagcatgcta agatcagca

<212> DNA<213> Homo sapiens<220> <223> Cyclin B1 ribozyme binding site
<400> 3455
tgctaagatc agcactcta

<212> DNA<213> Homo sapiens<220> <223> Cyclin B1 ribozyme binding site
<400> 3456
atcagcactc taccacagc

<212> DNA<213> Homo sapiens<220> <223> Cyclin B1 ribozyme binding site
<400> 3457
cagcactcta ccacagctg

<212> DNA<213> Homo sapiens<220> <223> Cyclin B1 ribozyme binding site
<400> 3458
cagctgaatt ctgcactag

<212> DNA<213> Homo sapiens<220> <223> Cyclin B1 ribozyme binding site
<400> 3459
agctgaattc tgcactagt

<212> DNA<213> Homo sapiens<220> <223> Cyclin B1 ribozyme binding site
<400> 3460
ttctgcacta gttcaagat

<212> DNA<213> Homo sapiens<220> <223> Cyclin B1 ribozyme binding site
<400> 3461
tgcactagtt caagattta

<212> DNA<213> Homo sapiens<220> <223> Cyclin B1 ribozyme binding site
<400> 3462
gcactagttc aagatttag

<212> DNA<213> Homo sapiens<220> <223> Cyclin B1 ribozyme binding site
<400> 3463
gttcaagatt tagccaagg

<212> DNA<213> Homo sapiens<220> <223> Cyclin B1 ribozyme binding site
<400> 3464
ttcaagattt agccaaggc

<212> DNA<213> Homo sapiens<220> <223> Cyclin B1 ribozyme binding site
<400> 3465
tcaagattta gccaaggct

<212> DNA<213> Homo sapiens<220> <223> Cyclin B1 ribozyme binding site
<400> 3466
caaaggtgta acttgtaaa

<212> DNA<213> Homo sapiens<220> <223> Cyclin B1 ribozyme binding site
<400> 3467
ggtgtaactt gtaaacttg

<212> DNA<213> Homo sapiens<220> <223> Cyclin B1 ribozyme binding site
<400> 3468
gtaacttgta aacttgagt

<212> DNA<213> Homo sapiens<220> <223> Cyclin B1 ribozyme binding site
<400> 3469
ttgtaaactt gagttggag

<212> DNA<213> Homo sapiens<220> <223> Cyclin B1 ribozyme binding site
<400> 3470
aacttgagtt ggagtacta

<212> DNA<213> Homo sapiens<220> <223> Cyclin B1 ribozyme binding site
<400> 3471
agttggagta ctatacttt

<212> DNA<213> Homo sapiens<220> <223> Cyclin B1 ribozyme binding site
<400> 3472
tggagtacta tactttaca

<212> DNA<213> Homo sapiens<220> <223> Cyclin B1 ribozyme binding site
<400> 3473
gagtactata ctttacaaa

<212> DNA<213> Homo sapiens<220> <223> Cyclin B1 ribozyme binding site
<400> 3474
tactatactt tacaaacta

<212> DNA<213> Homo sapiens<220> <223> Cyclin B1 ribozyme binding site
<400> 3475
actatacttt acaaactaa

<212> DNA<213> Homo sapiens<220> <223> Cyclin B1 ribozyme binding site
<400> 3476
ctatacttta caaactaaa

<212> DNA<213> Homo sapiens<220> <223> Cyclin B1 ribozyme binding site
<400> 3477
ttacaaacta aaattggca

<212> DNA<213> Homo sapiens<220> <223> Cyclin B1 ribozyme binding site
<400> 3478
aactaaaatt ggcacatgt

<212> DNA<213> Homo sapiens<220> <223> Cdc25 hs ribozyme binding site
<400> 3479
aggaagactc tgagtccga

<212> DNA<213> Homo sapiens<220> <223> Cdc25 hs ribozyme binding site
<400> 3480
actctgagtc cgacgttgg

<212> DNA<213> Homo sapiens<220> <223> Cdc25 hs ribozyme binding site
<400> 3481
gtccgacgtt ggcctaccc

<212> DNA<213> Homo sapiens<220> <223> Cdc25 hs ribozyme binding site
<400> 3482
cgttggccta cccagtcgg

<212> DNA<213> Homo sapiens<220> <223> Cdc25 hs ribozyme binding site
<400> 3483
ctacccagtc ggaaggcag

<212> DNA<213> Homo sapiens<220> <223> Cdc25 hs ribozyme binding site
<400> 3484
agctgcaatc tagttaact

<212> DNA<213> Homo sapiens<220> <223> Cdc25 hs ribozyme binding site
<400> 3485
ctgcaatcta gttaactac

<212> DNA<213> Homo sapiens<220> <223> Cdc25 hs ribozyme binding site
<400> 3486
caatctagtt aactacctc

<212> DNA<213> Homo sapiens<220> <223> Cdc25 hs ribozyme binding site
<400> 3487
aatctagtta actacctcc

<212> DNA<213> Homo sapiens<220> <223> Cdc25 hs ribozyme binding site
<400> 3488
tagttaacta cctcctttc

<212> DNA<213> Homo sapiens<220> <223> Cdc25 hs ribozyme binding site
<400> 3489
taactacctc ctttcccct

<212> DNA<213> Homo sapiens<220> <223> Cdc25 hs ribozyme binding site
<400> 3490
ctacctcctt tcccctaga

<212> DNA<213> Homo sapiens<220> <223> Cdc25 hs ribozyme binding site
<400> 3491
tacctccttt cccctagat

<212> DNA<213> Homo sapiens<220> <223> Cdc25 hs ribozyme binding site
<400> 3492

acctcctttc ccctagatt

<212> DNA<213> Homo sapiens<220> <223> Cdc25 hs ribozyme binding site
<400> 3493
ctttccccta gatttcctt

<212> DNA<213> Homo sapiens<220> <223> Cdc25 hs ribozyme binding site
<400> 3494
cccctagatt tcctttcat

<212> DNA<213> Homo sapiens<220> <223> Cdc25 hs ribozyme binding site
<400> 3495
ccctagattt cctttcatt

<212> DNA<213> Homo sapiens<220> <223> Cdc25 hs ribozyme binding site
<400> 3496
cctagatttc ctttcattc

<212> DNA<213> Homo sapiens<220> <223> Cdc25 hs ribozyme binding site
<400> 3497
agatttcctt tcattctgc

<212> DNA<213> Homo sapiens<220> <223> Cdc25 hs ribozyme binding site
<400> 3498
gatttccttt cattctgct

<212> DNA<213> Homo sapiens<220> <223> Cdc25 hs ribozyme binding site
<400> 3499
atttcctttc attctgctc

<212> DNA<213> Homo sapiens<220> <223> Cdc25 hs ribozyme binding site
<400> 3500
tcctttcatt ctgctcaag

<212> DNA<213> Homo sapiens<220> <223> Cdc25 hs ribozyme binding site
<400> 3501
cctttcattc tgctcaagt

<212> DNA<213> Homo sapiens<220> <223> Cdc25 hs ribozyme binding site
<400> 3502
cattctgctc aagtcttcg

<212> DNA<213> Homo sapiens<220> <223> Cdc25 hs ribozyme binding site
<400> 3503
tgctcaagtc ttcgcctgt

<212> DNA<213> Homo sapiens<220> <223> Cdc25 hs ribozyme binding site
<400> 3504
ctcaagtctt cgcctgtgt

<212> DNA<213> Homo sapiens<220> <223> Cdc25 hs ribozyme binding site
<400> 3505
tcaagtcttc gcctgtgtc

<212> DNA<213> Homo sapiens<220> <223> Cdc25 hs ribozyme binding site

<400> 3506
cgcctgtgtc cgatcccta

<212> DNA<213> Homo sapiens<220> <223> Cdc25 hs ribozyme binding site
<400> 3507
gtgtccgatc cctatctac

<212> DNA<213> Homo sapiens<220> <223> Cdc25 hs ribozyme binding site
<400> 3508
ccgatcccta tctactttc

<212> DNA<213> Homo sapiens<220> <223> Cdc25 hs ribozyme binding site
<400> 3509
gatccctatc tactttctc

<212> DNA<213> Homo sapiens<220> <223> Cdc25 hs ribozyme binding site
<400> 3510
tccctatcta ctttctctc

<212> DNA<213> Homo sapiens<220> <223> Cdc25 hs ribozyme binding site
<400> 3511
ctatctactt tctctcctc

<212> DNA<213> Homo sapiens<220> <223> Cdc25 hs ribozyme binding site
<400> 3512
tatctacttt ctctcctct

<212> DNA<213> Homo sapiens<220> <223> Cdc25 hs ribozyme binding site
<400> 3513
atctactttc tctcctctt

<212> DNA<213> Homo sapiens<220> <223> Cdc25 hs ribozyme binding site
<400> 3514
ctactttctc tcctcttgt

<212> DNA<213> Homo sapiens<220> <223> Cdc25 hs ribozyme binding site
<400> 3515
actttctctc ctcttgtag

<212> DNA<213> Homo sapiens<220> <223> Cdc25 hs ribozyme binding site
<400> 3516
ttctctcctc ttgtagcaa

<212> DNA<213> Homo sapiens<220> <223> Cdc25 hs ribozyme binding site
<400> 3517
ctctcctctt gtagcaagc

<212> DNA<213> Homo sapiens<220> <223> Cdc25 hs ribozyme binding site
<400> 3518
tcctcttgta gcaagcctc

<212> DNA<213> Homo sapiens<220> <223> Cdc25 hs ribozyme binding site
<400> 3519
agcaagcctc agactccag

<212> DNA<213> Homo sapiens<220> <223> Cdc25 hs ribozyme binding site
<400> 3520
cctcagactc caggcttga

<212> DNA<213> Homo sapiens<220> <223> Cdc25 hs ribozyme binding site
<400> 3521
ctccaggctt gagctaggt

<212> DNA<213> Homo sapiens<220> <223> Cdc25 hs ribozyme binding site
<400> 3522
gcttgagcta ggttttgtt

<212> DNA<213> Homo sapiens<220> <223> Cdc25 hs ribozyme binding site
<400> 3523
gagctaggtt ttgttttc

<212> DNA<213> Homo sapiens<220> <223> Cdc25 hs ribozyme binding site
<400> 3524
agctaggttt tgttttct

<212> DNA<213> Homo sapiens<220> <223> Cdc25 hs ribozyme binding site
<400> 3525
gctaggtttt gttttctc

<212> DNA<213> Homo sapiens<220> <223> Cdc25 hs ribozyme binding site
<400> 3526
aggttttgtt tttctcctg

<212> DNA<213> Homo sapiens<220> <223> Cdc25 hs ribozyme binding site
<400> 3527
ggttttgttt ttctcctgg

<212> DNA<213> Homo sapiens<220> <223> Cdc25 hs ribozyme binding site
<400> 3528
gttttgtttt tctcctggt

<212> DNA<213> Homo sapiens<220> <223> Cdc25 hs ribozyme binding site
<400> 3529
ttttgttttt ctcctggtg

<212> DNA<213> Homo sapiens<220> <223> Cdc25 hs ribozyme binding site
<400> 3530
tttgtttttc tcctggtga

<212> DNA<213> Homo sapiens<220> <223> Cdc25 hs ribozyme binding site
<400> 3531
tgtttttctc ctggtgaga

<212> DNA<213> Homo sapiens<220> <223> Cdc25 hs ribozyme binding site
<400> 3532
ggtgagaatt cgaagacca

<212> DNA<213> Homo sapiens<220> <223> Cdc25 hs ribozyme binding site
<400> 3533
gtgagaattc gaagaccat

<212> DNA<213> Homo sapiens<220> <223> Cdc25 hs ribozyme binding site
<400> 3534
agaccatgtc tacggaact

<212> DNA<213> Homo sapiens<220> <223> Cdc25 hs ribozyme binding site
<400> 3535
accatgtcta cggaactct

<212> DNA<213> Homo sapiens<220> <223> Cdc25 hs ribozyme binding site
<400> 3536
tacggaactc ttctcatcc

<212> DNA<213> Homo sapiens<220> <223> Cdc25 hs ribozyme binding site
<400> 3537
cggaactctt ctcatccac

<212> DNA<213> Homo sapiens<220> <223> Cdc25 hs ribozyme binding site
<400> 3538
ggaactcttc tcatccaca

<212> DNA<213> Homo sapiens<220> <223> Cdc25 hs ribozyme binding site
<400> 3539
aactcttctc atccacaag

<212> DNA<213> Homo sapiens<220> <223> Cdc25 hs ribozyme binding site
<400> 3540
tcttctcatc cacaagaga

<212> DNA<213> Homo sapiens<220> <223> Cdc25 hs ribozyme binding site
<400> 3541
aaggaagctc tggctcagg

<212> DNA<213> Homo sapiens<220> <223> Cdc25 hs ribozyme binding site
<400> 3542
gctctggctc aggacccag

<212> DNA<213> Homo sapiens<220> <223> Cdc25 hs ribozyme binding site
<400> 3543
ggacccagtt ttaggtcta

<212> DNA<213> Homo sapiens<220> <223> Cdc25 hs ribozyme binding site
<400> 3544
gacccagttt taggtctaa

<212> DNA<213> Homo sapiens<220> <223> Cdc25 hs ribozyme binding site
<400> 3545
acccagtttt aggtctaat

<212> DNA<213> Homo sapiens<220> <223> Cdc25 hs ribozyme binding site
<400> 3546
cccagtttta ggtctaatc

<212> DNA<213> Homo sapiens<220> <223> Cdc25 hs ribozyme binding site
<400> 3547

gttttaggtc taacaaagg

<212> DNA<213> Homo sapiens<220> <223> Cdc25 hs ribozyme binding site
<400> 3548
tttaggtcta atcaaagga

<212> DNA<213> Homo sapiens<220> <223> Cdc25 hs ribozyme binding site
<400> 3549
aggtctaatc aaaggaaaa

<212> DNA<213> Homo sapiens<220> <223> Cdc25 hs ribozyme binding site
<400> 3550
ggaaaatgtt aaacctgct

<212> DNA<213> Homo sapiens<220> <223> Cdc25 hs ribozyme binding site
<400> 3551
gaaaatgtta aacctgctc

<212> DNA<213> Homo sapiens<220> <223> Cdc25 hs ribozyme binding site
<400> 3552
aaacctgctc ctggagaga

<212> DNA<213> Homo sapiens<220> <223> Cdc25 hs ribozyme binding site
<400> 3553
agagacactt cctttaccg

<212> DNA<213> Homo sapiens<220> <223> Cdc25 hs ribozyme binding site
<400> 3554
gagacacttc ctttaccgt

<212> DNA<213> Homo sapiens<220> <223> Cdc25 hs ribozyme binding site
<400> 3555
acacttcctt taccgtctg

<212> DNA<213> Homo sapiens<220> <223> Cdc25 hs ribozyme binding site
<400> 3556
cacttccttt accgtctgt

<212> DNA<213> Homo sapiens<220> <223> Cdc25 hs ribozyme binding site
<400> 3557
acttccttta ccgtctgtc

<212> DNA<213> Homo sapiens<220> <223> Cdc25 hs ribozyme binding site
<400> 3558
ctttaccgtc tgtccagat

<212> DNA<213> Homo sapiens<220> <223> Cdc25 hs ribozyme binding site
<400> 3559
accgtctgtc cagatgtcc

<212> DNA<213> Homo sapiens<220> <223> Cdc25 hs ribozyme binding site
<400> 3560
tccagatgtc cctagaact

<212> DNA<213> Homo sapiens<220> <223> Cdc25 hs ribozyme binding site

<400> 3561
gatgtcccta gaactc[?]ag

<212> DNA<213> Homo sapiens<220> <223> Cdc25 hs ribozyme binding site
<400> 3562
cctagaactc cagtgggca

<212> DNA<213> Homo sapiens<220> <223> Cdc25 hs ribozyme binding site
<400> 3563
tgggcaaatt tcttggtga

<212> DNA<213> Homo sapiens<220> <223> Cdc25 hs ribozyme binding site
<400> 3564
gggcaaattt cttggtgat

<212> DNA<213> Homo sapiens<220> <223> Cdc25 hs ribozyme binding site
<400> 3565
ggcaaatttc ttggtgatt

<212> DNA<213> Homo sapiens<220> <223> Cdc25 hs ribozyme binding site
<400> 3566
caaatttctt ggtgattct

<212> DNA<213> Homo sapiens<220> <223> Cdc25 hs ribozyme binding site
<400> 3567
cttggtgatt ctgcaaacc

<212> DNA<213> Homo sapiens<220> <223> Cdc25 hs ribozyme binding site
<400> 3568
ttggtgattc tgcaaacct

<212> DNA<213> Homo sapiens<220> <223> Cdc25 hs ribozyme binding site
<400> 3569
tgcaaaccta agcattttg

<212> DNA<213> Homo sapiens<220> <223> Cdc25 hs ribozyme binding site
<400> 3570
cctaagcatt ttgtctgga

<212> DNA<213> Homo sapiens<220> <223> Cdc25 hs ribozyme binding site
<400> 3571
ctaagcattt tgtctggag

<212> DNA<213> Homo sapiens<220> <223> Cdc25 hs ribozyme binding site
<400> 3572
taagcatttt gtctggagg

<212> DNA<213> Homo sapiens<220> <223> Cdc25 hs ribozyme binding site
<400> 3573
gcattttgtc tggaggaac

<212> DNA<213> Homo sapiens<220> <223> Cdc25 hs ribozyme binding site
<400> 3574
ccaaaatgtt gcctcgatc

<212> DNA<213> Homo sapiens<220> <223> Cdc25 hs ribozyme binding site
<400> 3575
atgttgcctc gatctttcg

<212> DNA<213> Homo sapiens<220> <223> Cdc25 hs ribozyme binding site
<400> 3576
tgcctcgatc tttcgaatc

<212> DNA<213> Homo sapiens<220> <223> Cdc25 hs ribozyme binding site
<400> 3577
cctcgatctt tcgaatctt

<212> DNA<213> Homo sapiens<220> <223> Cdc25 hs ribozyme binding site
<400> 3578
ctcgatcttt cgaatctta

<212> DNA<213> Homo sapiens<220> <223> Cdc25 hs ribozyme binding site
<400> 3579
tcgatctttc gaatcttag

<212> DNA<213> Homo sapiens<220> <223> Cdc25 hs ribozyme binding site
<400> 3580
ctttcgaatc ttagcagtg

<212> DNA<213> Homo sapiens<220> <223> Cdc25 hs ribozyme binding site
<400> 3581
ttcgaatctt agcagtggg

<212> DNA<213> Homo sapiens<220> <223> Cdc25 hs ribozyme binding site
<400> 3582
tcgaatctta gcagtgggg

<212> DNA<213> Homo sapiens<220> <223> Cdc25 hs ribozyme binding site
<400> 3583
tggggagata actgccact

<212> DNA<213> Homo sapiens<220> <223> Cdc25 hs ribozyme binding site
<400> 3584
actgccactc agcttacca

<212> DNA<213> Homo sapiens<220> <223> Cdc25 hs ribozyme binding site
<400> 3585
cactcagctt accacttct

<212> DNA<213> Homo sapiens<220> <223> Cdc25 hs ribozyme binding site
<400> 3586
actcagctta ccacttctg

<212> DNA<213> Homo sapiens<220> <223> Cdc25 hs ribozyme binding site
<400> 3587
cttaccactt ctgcagacc

<212> DNA<213> Homo sapiens<220> <223> Cdc25 hs ribozyme binding site
<400> 3588
ttaccacttc tgcagacct

<212> DNA<213> Homo sapiens<220> <223> Cdc25 hs ribozyme binding site
<400> 3589
tgcagacctt gatgaaact

<212> DNA<213> Homo sapiens<220> <223> Cdc25 hs ribozyme binding site
<400> 3590
gaaactggtc acctggatt

<212> DNA<213> Homo sapiens<220> <223> Cdc25 hs ribozyme binding site
<400> 3591
cacctggatt cttcaggac

<212> DNA<213> Homo sapiens<220> <223> Cdc25 hs ribozyme binding site
<400> 3592
acctggattc ttcaggact

<212> DNA<213> Homo sapiens<220> <223> Cdc25 hs ribozyme binding site
<400> 3593
ctggattctt caggacttc

<212> DNA<213> Homo sapiens<220> <223> Cdc25 hs ribozyme binding site
<400> 3594
tggattcttc aggacttca

<212> DNA<213> Homo sapiens<220> <223> Cdc25 hs ribozyme binding site
<400> 3595
ttcaggactt caggaagtg

<212> DNA<213> Homo sapiens<220> <223> Cdc25 hs ribozyme binding site
<400> 3596
tcaggacttc aggaagtgc

<212> DNA<213> Homo sapiens<220> <223> Cdc25 hs ribozyme binding site
<400> 3597
gaagtgcatt tagctggga

<212> DNA<213> Homo sapiens<220> <223> Cdc25 hs ribozyme binding site
<400> 3598
aagtgcattt acgtgggat

<212> DNA<213> Homo sapiens<220> <223> Cdc25 hs ribozyme binding site
<400> 3599
agtgcattta gctgggatg

<212> DNA<213> Homo sapiens<220> <223> Cdc25 hs ribozyme binding site
<400> 3600
gggatgaatc atgaccagc

<212> DNA<213> Homo sapiens<220> <223> Cdc25 hs ribozyme binding site
<400> 3601
ccagcaccta atgaaatgt

<212> DNA<213> Homo sapiens<220> <223> Cdc25 hs ribozyme binding site
<400> 3602

atgaaatgta gcccagcac

<212> DNA<213> Homo sapiens<220> <223> Cdc25 hs ribozyme binding site
<400> 3603
agcacagctt ctttgtagc

<212> DNA<213> Homo sapiens<220> <223> Cdc25 hs ribozyme binding site
<400> 3604
gcacagcttc tttgtagca

<212> DNA<213> Homo sapiens<220> <223> Cdc25 hs ribozyme binding site
<400> 3605
acagcttctt tgtagcact

<212> DNA<213> Homo sapiens<220> <223> Cdc25 hs ribozyme binding site
<400> 3606
cagcttcttt gtagcactc

<212> DNA<213> Homo sapiens<220> <223> Cdc25 hs ribozyme binding site
<400> 3607
cttctttgta gcactccga

<212> DNA<213> Homo sapiens<220> <223> Cdc25 hs ribozyme binding site
<400> 3608
tgtagcactc cgaatggtt

<212> DNA<213> Homo sapiens<220> <223> Cdc25 hs ribozyme binding site
<400> 3609
ccgaatggtt tggaccgtg

<212> DNA<213> Homo sapiens<220> <223> Cdc25 hs ribozyme binding site
<400> 3610
cgaatggttt ggaccgtgg

<212> DNA<213> Homo sapiens<220> <223> Cdc25 hs ribozyme binding site
<400> 3611
cgtggccata gaaagagag

<212> DNA<213> Homo sapiens<220> <223> Cdc25 hs ribozyme binding site
<400> 3612
gcaatgtgta gttcatctg

<212> DNA<213> Homo sapiens<220> <223> Cdc25 hs ribozyme binding site
<400> 3613
atgtgtagtt catctgcaa

<212> DNA<213> Homo sapiens<220> <223> Cdc25 hs ribozyme binding site
<400> 3614
tgtgtagttc atctgcaaa

<212> DNA<213> Homo sapiens<220> <223> Cdc25 hs ribozyme binding site
<400> 3615
gtagttcatc tgcaaataa

<212> DNA<213> Homo sapiens<220> <223> Cdc25 hs ribozyme binding site

<400> 3616
tctgcaaata aagaaaatg

<212> DNA<213> Homo sapiens<220> <223> Cdc25 hs ribozyme binding site
<400> 3617
atggaaactt ggtggacag

<212> DNA<213> Homo sapiens<220> <223> Cdc25 hs ribozyme binding site
<400> 3618
aaatgaaata tttgggcag

<212> DNA<213> Homo sapiens<220> <223> Cdc25 hs ribozyme binding site
<400> 3619
atgaaatatt tgggcagtc

<212> DNA<213> Homo sapiens<220> <223> Cdc25 hs ribozyme binding site
<400> 3620
tgaaatattt gggcagtcc

<212> DNA<213> Homo sapiens<220> <223> Cdc25 hs ribozyme binding site
<400> 3621
ttgggcagtc ccattacta

<212> DNA<213> Homo sapiens<220> <223> Cdc25 hs ribozyme binding site
<400> 3622
cagtcccatt actactgtt

<212> DNA<213> Homo sapiens<220> <223> Cdc25 hs ribozyme binding site
<400> 3623
agtcccatta ctactgttc

<212> DNA<213> Homo sapiens<220> <223> Cdc25 hs ribozyme binding site
<400> 3624
cccattacta ctgttccaa

<212> DNA<213> Homo sapiens<220> <223> Cdc25 hs ribozyme binding site
<400> 3625
tactactgtt ccaaaattg

<212> DNA<213> Homo sapiens<220> <223> Cdc25 hs ribozyme binding site
<400> 3626
ttccaaaatt ggataaaaa

<212> DNA<213> Homo sapiens<220> <223> Cdc25 hs ribozyme binding site
<400> 3627
aaattggata aaaatccaa

<212> DNA<213> Homo sapiens<220> <223> Cdc25 hs ribozyme binding site
<400> 3628
gataaaaatc caaacctag

<212> DNA<213> Homo sapiens<220> <223> Cdc25 hs ribozyme binding site
<400> 3629
tccaaaccta ggagaagac

<212> DNA<213> Homo sapiens<220> <223> Cdc25 hs ribozyme binding site
<400> 3630
agaagagatt tcagatgaa

<212> DNA<213> Homo sapiens<220> <223> Cdc25 hs ribozyme binding site
<400> 3631
gaagagattt cagatgaat

<212> DNA<213> Homo sapiens<220> <223> Cdc25 hs ribozyme binding site
<400> 3632
aagagatttc agatgaatt

<212> DNA<213> Homo sapiens<220> <223> Cdc25 hs ribozyme binding site
<400> 3633
cagatgaatt aatggagtt

<212> DNA<213> Homo sapiens<220> <223> Cdc25 hs ribozyme binding site
<400> 3634
agatgaatta atggagttt

<212> DNA<213> Homo sapiens<220> <223> Cdc25 hs ribozyme binding site
<400> 3635
taatggagtt ttccctgaa

<212> DNA<213> Homo sapiens<220> <223> Cdc25 hs ribozyme binding site
<400> 3636
aatggagttt tccctgaaa

<212> DNA<213> Homo sapiens<220> <223> Cdc25 hs ribozyme binding site
<400> 3637
atggagtttt ccctgaaag

<212> DNA<213> Homo sapiens<220> <223> Cdc25 hs ribozyme binding site
<400> 3638
tggagttttc cctgaaaga

<212> DNA<213> Homo sapiens<220> <223> Cdc25 hs ribozyme binding site
<400> 3639
ctgaaagatc aagaagcaa

<212> DNA<213> Homo sapiens<220> <223> Cdc25 hs ribozyme binding site
<400> 3640
aagtggccta tatcgctcc

<212> DNA<213> Homo sapiens<220> <223> Cdc25 hs ribozyme binding site
<400> 3641
gtggcctata tcgctcccc

<212> DNA<213> Homo sapiens<220> <223> Cdc25 hs ribozyme binding site
<400> 3642
ggcctatatc gctccccgt

<212> DNA<213> Homo sapiens<220> <223> Cdc25 hs ribozyme binding site
<400> 3643
tatatcgctc cccgtcgat

<212> DNA<213> Homo sapiens<220> <223> Cdc25 hs ribozyme binding site
<400> 3644
gctccccgtc gatgccaga

<212> DNA<213> Homo sapiens<220> <223> Cdc25 hs ribozyme binding site
<400> 3645
cagagaactt gaacaggcc

<212> DNA<213> Homo sapiens<220> <223> Cdc25 hs ribozyme binding site
<400> 3646
tggaaaaatt caaggacaa

<212> DNA<213> Homo sapiens<220> <223> Cdc25 hs ribozyme binding site
<400> 3647
ggaaaaattc aaggacaac

<212> DNA<213> Homo sapiens<220> <223> Cdc25 hs ribozyme binding site
<400> 3648
caacacaata ccagataaa

<212> DNA<213> Homo sapiens<220> <223> Cdc25 hs ribozyme binding site
<400> 3649
ataccagata aagttaaaa

<212> DNA<213> Homo sapiens<220> <223> Cdc25 hs ribozyme binding site
<400> 3650
agataaagtt aaaaaaaag

<212> DNA<213> Homo sapiens<220> <223> Cdc25 hs ribozyme binding site
<400> 3651
gataaagtta aaaaaaagt

<212> DNA<213> Homo sapiens<220> <223> Cdc25 hs ribozyme binding site
<400> 3652
aaaaaaagta tttttctgg

<212> DNA<213> Homo sapiens<220> <223> Cdc25 hs ribozyme binding site
<400> 3653
aaaaagtatt tttctggcc

<212> DNA<213> Homo sapiens<220> <223> Cdc25 hs ribozyme binding site
<400> 3654
aaaagtattt ttctggcca

<212> DNA<213> Homo sapiens<220> <223> Cdc25 hs ribozyme binding site
<400> 3655
aaagtatttt tctggccaa

<212> DNA<213> Homo sapiens<220> <223> Cdc25 hs ribozyme binding site
<400> 3656
aagtattttt ctggccaag

<212> DNA<213> Homo sapiens<220> <223> Cdc25 hs ribozyme binding site
<400> 3657

agtatttttc tggccaagg

<212> DNA<213> Homo sapiens<220> <223> Cdc25 hs ribozyme binding site
<400> 3658
aggaaagctc aggaagggc

<212> DNA<213> Homo sapiens<220> <223> Cdc25 hs ribozyme binding site
<400> 3659
ggaagggctt atgtttaaa

<212> DNA<213> Homo sapiens<220> <223> Cdc25 hs ribozyme binding site
<400> 3660
gaagggctta tgtttaaag

<212> DNA<213> Homo sapiens<220> <223> Cdc25 hs ribozyme binding site
<400> 3661
ggcttatgtt taaagaaga

<212> DNA<213> Homo sapiens<220> <223> Cdc25 hs ribozyme binding site
<400> 3662
gcttatgttt aaagaagac

<212> DNA<213> Homo sapiens<220> <223> Cdc25 hs ribozyme binding site
<400> 3663
cttatgttta aagaagaca

<212> DNA<213> Homo sapiens<220> <223> Cdc25 hs ribozyme binding site
<400> 3664
gaagacagtc tctctgtgt

<212> DNA<213> Homo sapiens<220> <223> Cdc25 hs ribozyme binding site
<400> 3665
agacagtctc tctgtgtga

<212> DNA<213> Homo sapiens<220> <223> Cdc25 hs ribozyme binding site
<400> 3666
acagtctctc tgtgtgaca

<212> DNA<213> Homo sapiens<220> <223> Cdc25 hs ribozyme binding site
<400> 3667
gtgtgacatt actatcact

<212> DNA<213> Homo sapiens<220> <223> Cdc25 hs ribozyme binding site
<400> 3668
tgtgacatta ctatcactc

<212> DNA<213> Homo sapiens<220> <223> Cdc25 hs ribozyme binding site
<400> 3669
gacattacta tcactcaga

<212> DNA<213> Homo sapiens<220> <223> Cdc25 hs ribozyme binding site
<400> 3670
cattactatc actcagatg

<212> DNA<213> Homo sapiens<220> <223> Cdc25 hs ribozyme binding site

<400> 3671
actatcactc agatgctgg

<212> DNA<213> Homo sapiens<220> <223> Cdc25 hs ribozyme binding site
<400> 3672
gaggaagatt ctaaccagg

<212> DNA<213> Homo sapiens<220> <223> Cdc25 hs ribozyme binding site
<400> 3673
aggaagattc taaccaggg

<212> DNA<213> Homo sapiens<220> <223> Cdc25 hs ribozyme binding site
<400> 3674
gaagattcta accaggggc

<212> DNA<213> Homo sapiens<220> <223> Cdc25 hs ribozyme binding site
<400> 3675
gcacctgatt ggtgatttt

<212> DNA<213> Homo sapiens<220> <223> Cdc25 hs ribozyme binding site
<400> 3676
attggtgatt tttccaagg

<212> DNA<213> Homo sapiens<220> <223> Cdc25 hs ribozyme binding site
<400> 3677
ttggtgattt ttccaaggt

<212> DNA<213> Homo sapiens<220> <223> Cdc25 hs ribozyme binding site
<400> 3678
tggtgatttt tccaaggta

<212> DNA<213> Homo sapiens<220> <223> Cdc25 hs ribozyme binding site
<400> 3679
ggtgattttt ccaaggtat

<212> DNA<213> Homo sapiens<220> <223> Cdc25 hs ribozyme binding site
<400> 3680
gtgatttttc caaggtatg

<212> DNA<213> Homo sapiens<220> <223> Cdc25 hs ribozyme binding site
<400> 3681
ttccaaggta tgtgcgctg

<212> DNA<213> Homo sapiens<220> <223> Cdc25 hs ribozyme binding site
<400> 3682
caaccgtgtc agggaaaca

<212> DNA<213> Homo sapiens<220> <223> Cdc25 hs ribozyme binding site
<400> 3683
caccaagatc tgaagtatg

<212> DNA<213> Homo sapiens<220> <223> Cdc25 hs ribozyme binding site
<400> 3684
atctgaagta tgtcaaccc

<212> DNA<213> Homo sapiens<220> <223> Cdc25 hs ribozyme binding site
<400> 3685
gaagtatgtc aacccagaa

<212> DNA<213> Homo sapiens<220> <223> Cdc25 hs ribozyme binding site
<400> 3686
tggctgcctt actgtcggg

<212> DNA<213> Homo sapiens<220> <223> Cdc25 hs ribozyme binding site
<400> 3687
ggctgcctta ctgtcgggg

<212> DNA<213> Homo sapiens<220> <223> Cdc25 hs ribozyme binding site
<400> 3688
ccttactgtc ggggaagtt

<212> DNA<213> Homo sapiens<220> <223> Cdc25 hs ribozyme binding site
<400> 3689
cggggaagtt ccagggtct

<212> DNA<213> Homo sapiens<220> <223> Cdc25 hs ribozyme binding site
<400> 3690
ggggaagttc cagggtctg

<212> DNA<213> Homo sapiens<220> <223> Cdc25 hs ribozyme binding site
<400> 3691
ttccagggtc tgattgaga

<212> DNA<213> Homo sapiens<220> <223> Cdc25 hs ribozyme binding site
<400> 3692
gggtctgatt gagaagttt

<212> DNA<213> Homo sapiens<220> <223> Cdc25 hs ribozyme binding site
<400> 3693
ttgagaagtt ttatgtcat

<212> DNA<213> Homo sapiens<220> <223> Cdc25 hs ribozyme binding site
<400> 3694
tgagaagttt tatgtcatt

<212> DNA<213> Homo sapiens<220> <223> Cdc25 hs ribozyme binding site
<400> 3695
gagaagtttt atgtcattg

<212> DNA<213> Homo sapiens<220> <223> Cdc25 hs ribozyme binding site
<400> 3696
agaagtttta tgtcattga

<212> DNA<213> Homo sapiens<220> <223> Cdc25 hs ribozyme binding site
<400> 3697
gttttatgtc attgattgt

<212> DNA<213> Homo sapiens<220> <223> Cdc25 hs ribozyme binding site
<400> 3698
ttatgtcatt gattgtcgc

<212> DNA<213> Homo sapiens<220> <223> Cdc25 hs ribozyme binding site
<400> 3699
gtcattgatt gtcgctatc

<212> DNA<213> Homo sapiens<220> <223> Cdc25 hs ribozyme binding site
<400> 3700
attgattgtc gctatccat

<212> DNA<213> Homo sapiens<220> <223> Cdc25 hs ribozyme binding site
<400> 3701
attgtcgcta tccatatga

<212> DNA<213> Homo sapiens<220> <223> Cdc25 hs ribozyme binding site
<400> 3702
tgtcgctatc catatgagt

<212> DNA<213> Homo sapiens<220> <223> Cdc25 hs ribozyme binding site
<400> 3703
gctatccata tgagtatct

<212> DNA<213> Homo sapiens<220> <223> Cdc25 hs ribozyme binding site
<400> 3704
catatgagta tctgggagg

<212> DNA<213> Homo sapiens<220> <223> Cdc25 hs ribozyme binding site
<400> 3705
tatgagtatc tgggaggac

<212> DNA<213> Homo sapiens<220> <223> Cdc25 hs ribozyme binding site
<400> 3706
aggacacatc cagggagcc

<212> DNA<213> Homo sapiens<220> <223> Cdc25 hs ribozyme binding site
<400> 3707
agggagcctt aaacttata

<212> DNA<213> Homo sapiens<220> <223> Cdc25 hs ribozyme binding site
<400> 3708
gggagcctta aacttatat

<212> DNA<213> Homo sapiens<220> <223> Cdc25 hs ribozyme binding site
<400> 3709
ccttaaactt atatagtca

<212> DNA<213> Homo sapiens<220> <223> Cdc25 hs ribozyme binding site
<400> 3710
cttaaactta tatagtcag

<212> DNA<213> Homo sapiens<220> <223> Cdc25 hs ribozyme binding site
<400> 3711
taaacttata tagtcagga

<212> DNA<213> Homo sapiens<220> <223> Cdc25 hs ribozyme binding site
<400> 3712

aacttatata gtcaggaag

<212> DNA<213> Homo sapiens<220> <223> Cdc25 hs ribozyme binding site
<400> 3713
ttatatagtc aggaagaac

<212> DNA<213> Homo sapiens<220> <223> Cdc25 hs ribozyme binding site
<400> 3714
aagaactgtt taacttctt

<212> DNA<213> Homo sapiens<220> <223> Cdc25 hs ribozyme binding site
<400> 3715
agaactgttt aacttcttt

<212> DNA<213> Homo sapiens<220> <223> Cdc25 hs ribozyme binding site
<400> 3716
gaactgttta acttctttc

<212> DNA<213> Homo sapiens<220> <223> Cdc25 hs ribozyme binding site
<400> 3717
tgtttaactt ctttctgaa

<212> DNA<213> Homo sapiens<220> <223> Cdc25 hs ribozyme binding site
<400> 3718
gtttaacttc tttctgaag

<212> DNA<213> Homo sapiens<220> <223> Cdc25 hs ribozyme binding site
<400> 3719
ttaacttctt tctgaagaa

<212> DNA<213> Homo sapiens<220> <223> Cdc25 hs ribozyme binding site
<400> 3720
taacttcttt ctgaagaag

<212> DNA<213> Homo sapiens<220> <223> Cdc25 hs ribozyme binding site
<400> 3721
aacttctttc tgaagaagc

<212> DNA<213> Homo sapiens<220> <223> Cdc25 hs ribozyme binding site
<400> 3722
gaagcccatc gtccctttg

<212> DNA<213> Homo sapiens<220> <223> Cdc25 hs ribozyme binding site
<400> 3723
gcccatcgtc cctttggac

<212> DNA<213> Homo sapiens<220> <223> Cdc25 hs ribozyme binding site
<400> 3724
atcgtccctt tggacaccc

<212> DNA<213> Homo sapiens<220> <223> Cdc25 hs ribozyme binding site
<400> 3725
tcgtcccttt ggacaccca

<212> DNA<213> Homo sapiens<220> <223> Cdc25 hs ribozyme binding site

<400> 3726
gaagagaata atcatcgtg

<212> DNA<213> Homo sapiens<220> <223> Cdc25 hs ribozyme binding site
<400> 3727
gagaataatc atcgtgttc

<212> DNA<213> Homo sapiens<220> <223> Cdc25 hs ribozyme binding site
<400> 3728
aataatcatc gtgttccac

<212> DNA<213> Homo sapiens<220> <223> Cdc25 hs ribozyme binding site
<400> 3729
tcatcgtgtt ccactgtga

<212> DNA<213> Homo sapiens<220> <223> Cdc25 hs ribozyme binding site
<400> 3730
catcgtgttc cactgtgaa

<212> DNA<213> Homo sapiens<220> <223> Cdc25 hs ribozyme binding site
<400> 3731
actgtgaatt ctcctcaga

<212> DNA<213> Homo sapiens<220> <223> Cdc25 hs ribozyme binding site
<400> 3732
ctgtgaattc tcctcagag

<212> DNA<213> Homo sapiens<220> <223> Cdc25 hs ribozyme binding site
<400> 3733
gtgaattctc ctcagagag

<212> DNA<213> Homo sapiens<220> <223> Cdc25 hs ribozyme binding site
<400> 3734
aattctcctc agagagggg

<212> DNA<213> Homo sapiens<220> <223> Cdc25 hs ribozyme binding site
<400> 3735
tgccgctgtc tgcgtgaag

<212> DNA<213> Homo sapiens<220> <223> Cdc25 hs ribozyme binding site
<400> 3736
aggacaggtc tctgaacca

<212> DNA<213> Homo sapiens<220> <223> Cdc25 hs ribozyme binding site
<400> 3737
gacaggtctc tgaaccagt

<212> DNA<213> Homo sapiens<220> <223> Cdc25 hs ribozyme binding site
<400> 3738
tgaaccagta tcctgcatt

<212> DNA<213> Homo sapiens<220> <223> Cdc25 hs ribozyme binding site
<400> 3739
aaccagtatc ctgcattgt

<212> DNA<213> Homo sapiens<220> <223> Cdc25 hs ribozyme binding site
<400> 3740
atcctgcatt gtactaccc

<212> DNA<213> Homo sapiens<220> <223> Cdc25 hs ribozyme binding site
<400> 3741
ctgcattgta ctacccaga

<212> DNA<213> Homo sapiens<220> <223> Cdc25 hs ribozyme binding site
<400> 3742
cattgtacta cccagagct

<212> DNA<213> Homo sapiens<220> <223> Cdc25 hs ribozyme binding site
<400> 3743
cccagagcta tatatcctt

<212> DNA<213> Homo sapiens<220> <223> Cdc25 hs ribozyme binding site
<400> 3744
cagagctata tatccttaa

<212> DNA<213> Homo sapiens<220> <223> Cdc25 hs ribozyme binding site
<400> 3745
gagctatata tccttaaag

<212> DNA<213> Homo sapiens<220> <223> Cdc25 hs ribozyme binding site
<400> 3746
gctatatatc cttaaaggc

<212> DNA<213> Homo sapiens<220> <223> Cdc25 hs ribozyme binding site
<400> 3747
atatatcctt aaaggcggc

<212> DNA<213> Homo sapiens<220> <223> Cdc25 hs ribozyme binding site
<400> 3748
tatatcctta aaggcggct

<212> DNA<213> Homo sapiens<220> <223> Cdc25 hs ribozyme binding site
<400> 3749
aaggcggcta cagagactt

<212> DNA<213> Homo sapiens<220> <223> Cdc25 hs ribozyme binding site
<400> 3750
acagagactt ctttccaga

<212> DNA<213> Homo sapiens<220> <223> Cdc25 hs ribozyme binding site
<400> 3751
cagagacttc tttccagaa

<212> DNA<213> Homo sapiens<220> <223> Cdc25 hs ribozyme binding site
<400> 3752
gagacttctt tccagaata

<212> DNA<213> Homo sapiens<220> <223> Cdc25 hs ribozyme binding site
<400> 3753
agacttcttt ccagaatat

<212> DNA<213> Homo sapiens<220> <223> Cdc25 hs ribozyme binding site
<400> 3754
gacttctttc cagaatata

<212> DNA<213> Homo sapiens<220> <223> Cdc25 hs ribozyme binding site
<400> 3755
ttccagaata tatggaact

<212> DNA<213> Homo sapiens<220> <223> Cdc25 hs ribozyme binding site
<400> 3756
ccagaatata tggaactgt

<212> DNA<213> Homo sapiens<220> <223> Cdc25 hs ribozyme binding site
<400> 3757
cacagagcta ctgccctat

<212> DNA<213> Homo sapiens<220> <223> Cdc25 hs ribozyme binding site
<400> 3758
tactgcccta tgcatcatc

<212> DNA<213> Homo sapiens<220> <223> Cdc25 hs ribozyme binding site
<400> 3759
cctatgcatc atcaggacc

<212> DNA<213> Homo sapiens<220> <223> Cdc25 hs ribozyme binding site
<400> 3760
atgcatcatc aggaccaca

<212> DNA<213> Homo sapiens<220> <223> Cdc25 hs ribozyme binding site
<400> 3761
agactgagtt gctgaggtg

<212> DNA<213> Homo sapiens<220> <223> Cdc25 hs ribozyme binding site
<400> 3762
ctgaggtgtc gaagccaga

<212> DNA<213> Homo sapiens<220> <223> Cdc25 hs ribozyme binding site
<400> 3763
ggagcagatt gcccttctg

<212> DNA<213> Homo sapiens<220> <223> Cdc25 hs ribozyme binding site
<400> 3764
gattgccctt ctggtgaag

<212> DNA<213> Homo sapiens<220> <223> Cdc25 hs ribozyme binding site
<400> 3765
attgcccttc tggtgaagg

<212> DNA<213> Homo sapiens<220> <223> Cdc25 hs ribozyme binding site
<400> 3766
gcccatgata acattccag

<212> DNA<213> Homo sapiens<220> <223> Cdc25 hs ribozyme binding site
<400> 3767

tgataacatt ccagccact

<212> DNA<213> Homo sapiens<220> <223> Cdc25 hs ribozyme binding site
<400> 3768
gataacattc cagccactg

<212> DNA<213> Homo sapiens<220> <223> Cdc25 hs ribozyme binding site
<400> 3769
ctggctgcta acaagtcac

<212> DNA<213> Homo sapiens<220> <223> Cdc25 hs ribozyme binding site
<400> 3770
ctaacaagtc accaaaaag

<212> DNA<213> Homo sapiens<220> <223> Cdc25 hs ribozyme binding site
<400> 3771
aagaggcctt ctggatggc

<212> DNA<213> Homo sapiens<220> <223> Cdc25 hs ribozyme binding site
<400> 3772
acccaagatt attaaaaga

<212> DNA<213> Homo sapiens<220> <223> Cdc25 hs ribozyme binding site
<400> 3773
cccaagatta ttaaaagat

<212> DNA<213> Homo sapiens<220> <223> Cdc25 hs ribozyme binding site
<400> 3774
caagattatt aaaagatgt

<212> DNA<213> Homo sapiens<220> <223> Cdc25 hs ribozyme binding site
<400> 3775
aagattatta aaagatgtc

<212> DNA<213> Homo sapiens<220> <223> Cdc25 hs ribozyme binding site
<400> 3776
aaaagatgtc tctgcaaac

<212> DNA<213> Homo sapiens<220> <223> Cdc25 hs ribozyme binding site
<400> 3777
aagatgtctc tgcaaacca

<212> DNA<213> Homo sapiens<220> <223> Cdc25 hs ribozyme binding site
<400> 3778
caacaggcta ccaacttgt

<212> DNA<213> Homo sapiens<220> <223> Cdc25 hs ribozyme binding site
<400> 3779
ctaccaactt gtatccagg

<212> DNA<213> Homo sapiens<220> <223> Cdc25 hs ribozyme binding site
<400> 3780
ccaacttgta tccaggcct

<212> DNA<213> Homo sapiens<220> <223> Cdc25 hs ribozyme binding site

<400> 3781
aacttgtatc caggcctgg

<212> DNA<213> Homo sapiens<220> <223> Cdc25 hs ribozyme binding site
<400> 3782
ggaatggatt aggtttcag

<212> DNA<213> Homo sapiens<220> <223> Cdc25 hs ribozyme binding site
<400> 3783
gaatggatta ggtttcagc

<212> DNA<213> Homo sapiens<220> <223> Cdc25 hs ribozyme binding site
<400> 3784
ggattaggtt tcagcagag

<212> DNA<213> Homo sapiens<220> <223> Cdc25 hs ribozyme binding site
<400> 3785
gattaggttt cagcagagc

<212> DNA<213> Homo sapiens<220> <223> Cdc25 hs ribozyme binding site
<400> 3786
attaggtttc agcagagct

<212> DNA<213> Homo sapiens<220> <223> Cdc25 hs ribozyme binding site
<400> 3787
tggcagagtc ctggagctg

<212> DNA<213> Homo sapiens<220> <223> Cdc25 hs ribozyme binding site
<400> 3788
gagctggctc tataaggca

<212> DNA<213> Homo sapiens<220> <223> Cdc25 hs ribozyme binding site
<400> 3789
gctggctcta taaggcagc

<212> DNA<213> Homo sapiens<220> <223> Cdc25 hs ribozyme binding site
<400> 3790
tggctctata aggcagcct

<212> DNA<213> Homo sapiens<220> <223> Cdc25 hs ribozyme binding site
<400> 3791
aggcagcctt gagttgcat

<212> DNA<213> Homo sapiens<220> <223> Cdc25 hs ribozyme binding site
<400> 3792
gagttgcata gagatttgt

<212> DNA<213> Homo sapiens<220> <223> Cdc25 hs ribozyme binding site
<400> 3793
catagagatt tgtattggt

<212> DNA<213> Homo sapiens<220> <223> Cdc25 hs ribozyme binding site
<400> 3794
atagagattt gtattggtt

<212> DNA<213> Homo sapiens<220> <223> Cdc25 hs ribozyme binding site
<400> 3795
gagatttgta ttggttcag

<212> DNA<213> Homo sapiens<220> <223> Cdc25 hs ribozyme binding site
<400> 3796
gatttgtatt ggttcaggg

<212> DNA<213> Homo sapiens<220> <223> Cdc25 hs ribozyme binding site
<400> 3797
tgtattggtt cagggaact

<212> DNA<213> Homo sapiens<220> <223> Cdc25 hs ribozyme binding site
<400> 3798
gtattggttc agggaactc

<212> DNA<213> Homo sapiens<220> <223> Cdc25 hs ribozyme binding site
<400> 3799
cagggaactc tggcattcc

<212> DNA<213> Homo sapiens<220> <223> Cdc25 hs ribozyme binding site
<400> 3800
ctctggcatt ccttttccc

<212> DNA<213> Homo sapiens<220> <223> Cdc25 hs ribozyme binding site
<400> 3801
tctggcattc cttttccca

<212> DNA<213> Homo sapiens<220> <223> Cdc25 hs ribozyme binding site
<400> 3802
ggcattcctt ttcccaact

<212> DNA<213> Homo sapiens<220> <223> Cdc25 hs ribozyme binding site
<400> 3803
gcattccttt tcccaactc

<212> DNA<213> Homo sapiens<220> <223> Cdc25 hs ribozyme binding site
<400> 3804
cattcctttt cccaactcc

<212> DNA<213> Homo sapiens<220> <223> Cdc25 hs ribozyme binding site
<400> 3805
attccttttc ccaactcct

<212> DNA<213> Homo sapiens<220> <223> Cdc25 hs ribozyme binding site
<400> 3806
ttcccaactc ctcatgtct

<212> DNA<213> Homo sapiens<220> <223> Cdc25 hs ribozyme binding site
<400> 3807
ccaactcctc atgtcttct

<212> DNA<213> Homo sapiens<220> <223> Cdc25 hs ribozyme binding site
<400> 3808
tcctcatgtc ttctcacaa

<212> DNA<213> Homo sapiens<220> <223> Cdc25 hs ribozyme binding site
<400> 3809
ctcatgtctt ctcacaagc

<212> DNA<213> Homo sapiens<220> <223> Cdc25 hs ribozyme binding site
<400> 3810
tcatgtcttc tcacaagcc

<212> DNA<213> Homo sapiens<220> <223> Cdc25 hs ribozyme binding site
<400> 3811
atgtcttctc acaagccag

<212> DNA<213> Homo sapiens<220> <223> Cdc25 hs ribozyme binding site
<400> 3812
cagccaactc tttctctct

<212> DNA<213> Homo sapiens<220> <223> Cdc25 hs ribozyme binding site
<400> 3813
gccaactctt tctctctgg

<212> DNA<213> Homo sapiens<220> <223> Cdc25 hs ribozyme binding site
<400> 3814
ccaactcttt ctctctggg

<212> DNA<213> Homo sapiens<220> <223> Cdc25 hs ribozyme binding site
<400> 3815
caactctttc tctctgggc

<212> DNA<213> Homo sapiens<220> <223> Cdc25 hs ribozyme binding site
<400> 3816
actctttctc tctgggctt

<212> DNA<213> Homo sapiens<220> <223> Cdc25 hs ribozyme binding site
<400> 3817
tctttctctc tgggcttcg

<212> DNA<213> Homo sapiens<220> <223> Cdc25 hs ribozyme binding site
<400> 3818
ctctgggctt cgggctatg

<212> DNA<213> Homo sapiens<220> <223> Cdc25 hs ribozyme binding site
<400> 3819
tctgggcttc gggctatgc

<212> DNA<213> Homo sapiens<220> <223> Cdc25 hs ribozyme binding site
<400> 3820
cttcgggcta tgcaagagc

<212> DNA<213> Homo sapiens<220> <223> Cdc25 hs ribozyme binding site
<400> 3821
caagagcgtt gtctacctt

<212> DNA<213> Homo sapiens<220> <223> Cdc25 hs ribozyme binding site
<400> 3822

gagcgttgtc taccttctt

<212> DNA<213> Homo sapiens<220> <223> Cdc25 hs ribozyme binding site
<400> 3823
gcgttgtcta ccttctttc

<212> DNA<213> Homo sapiens<220> <223> Cdc25 hs ribozyme binding site
<400> 3824
tgtctacctt ctttctttg

<212> DNA<213> Homo sapiens<220> <223> Cdc25 hs ribozyme binding site
<400> 3825
gtctaccttc tttctttgt

<212> DNA<213> Homo sapiens<220> <223> Cdc25 hs ribozyme binding site
<400> 3826
ctaccttctt tctttgtat

<212> DNA<213> Homo sapiens<220> <223> Cdc25 hs ribozyme binding site
<400> 3827
taccttcttt ctttgtatt

<212> DNA<213> Homo sapiens<220> <223> Cdc25 hs ribozyme binding site
<400> 3828
accttctttc tttgtattt

<212> DNA<213> Homo sapiens<220> <223> Cdc25 hs ribozyme binding site
<400> 3829
cttctttctt tgtattttc

<212> DNA<213> Homo sapiens<220> <223> Cdc25 hs ribozyme binding site
<400> 3830
ttctttcttt gtattttcc

<212> DNA<213> Homo sapiens<220> <223> Cdc25 hs ribozyme binding site
<400> 3831
tttctttgta ttttccttc

<212> DNA<213> Homo sapiens<220> <223> Cdc25 hs ribozyme binding site
<400> 3832
tctttgtatt ttccttctt

<212> DNA<213> Homo sapiens<220> <223> Cdc25 hs ribozyme binding site
<400> 3833
ctttgtattt tccttcttt

<212> DNA<213> Homo sapiens<220> <223> Cdc25 hs ribozyme binding site
<400> 3834
tttgtatttt ccttctttg

<212> DNA<213> Homo sapiens<220> <223> Cdc25 hs ribozyme binding site
<400> 3835
ttgtattttc cttctttgt

<212> DNA<213> Homo sapiens<220> <223> Cdc25 hs ribozyme binding site

<400> 3836
tattttcctt ctttgtttc

<212> DNA<213> Homo sapiens<220> <223> Cdc25 hs ribozyme binding site
<400> 3837
attttccttc tttgtttcc

<212> DNA<213> Homo sapiens<220> <223> Cdc25 hs ribozyme binding site
<400> 3838
tttccttctt tgtttcccc

<212> DNA<213> Homo sapiens<220> <223> Cdc25 hs ribozyme binding site
<400> 3839
ttccttcttt gtttccccc

<212> DNA<213> Homo sapiens<220> <223> Cdc25 hs ribozyme binding site
<400> 3840
cttctttgtt tccccctct

<212> DNA<213> Homo sapiens<220> <223> Cdc25 hs ribozyme binding site
<400> 3841
ttctttgttt ccccctctt

<212> DNA<213> Homo sapiens<220> <223> Cdc25 hs ribozyme binding site
<400> 3842
tctttgtttc cccctcttt

<212> DNA<213> Homo sapiens<220> <223> Cdc25 hs ribozyme binding site
<400> 3843
tttccccctc tttcttttt

<212> DNA<213> Homo sapiens<220> <223> Cdc25 hs ribozyme binding site
<400> 3844
tccccctctt tcttttta

<212> DNA<213> Homo sapiens<220> <223> Cdc25 hs ribozyme binding site
<400> 3845
ccccctcttt cttttttaa

<212> DNA<213> Homo sapiens<220> <223> Cdc25 hs ribozyme binding site
<400> 3846
cccctctttc tttttaaa

<212> DNA<213> Homo sapiens<220> <223> Cdc25 hs ribozyme binding site
<400> 3847
cctctttctt ttttaaaaa

<212> DNA<213> Homo sapiens<220> <223> Cdc25 hs ribozyme binding site
<400> 3848
ctctttcttt tttaaaaat

<212> DNA<213> Homo sapiens<220> <223> Cdc25 hs ribozyme binding site
<400> 3849
tctttctttt ttaaaaatg

<212> DNA<213> Homo sapiens<220> <223> Cdc25 hs ribozyme binding site
<400> 3850
ctttcttttt taaaaatgg

<212> DNA<213> Homo sapiens<220> <223> Cdc25 hs ribozyme binding site
<400> 3851
tttcttttt aaaaatgga

<212> DNA<213> Homo sapiens<220> <223> Cdc25 hs ribozyme binding site
<400> 3852
ttcttttta aaaatggaa

<212> DNA<213> Homo sapiens<220> <223> Cdc25 hs ribozyme binding site
<400> 3853
tggaaaaata aacactaca

<212> DNA<213> Homo sapiens<220> <223> Cdc25 hs ribozyme binding site
<400> 3854
ataaacacta cagaatgag

<212> DNA<213> Homo sapiens<220> <223> PCNA HH ribozyme binding site
<400> 3855
ttaaacggtt gcaggcgta

<212> DNA<213> Homo sapiens<220> <223> PCNA HH ribozyme binding site
<400> 3856
tgcaggcgta gagagtggt

<212> DNA<213> Homo sapiens<220> <223> PCNA HH ribozyme binding site
<400> 3857
gagagtggtc gttgtcttt

<212> DNA<213> Homo sapiens<220> <223> PCNA HH ribozyme binding site
<400> 3858
agtggtcgtt gtctttcta

<212> DNA<213> Homo sapiens<220> <223> PCNA HH ribozyme binding site
<400> 3859
ggtcgttgtc tttctaggt

<212> DNA<213> Homo sapiens<220> <223> PCNA HH ribozyme binding site
<400> 3860
tcgttgtctt tctaggtct

<212> DNA<213> Homo sapiens<220> <223> PCNA HH ribozyme binding site
<400> 3861
cgttgtcttt ctaggtctc

<212> DNA<213> Homo sapiens<220> <223> PCNA HH ribozyme binding site
<400> 3862
gttgtctttc taggtctca

<212> DNA<213> Homo sapiens<220> <223> PCNA HH ribozyme binding site
<400> 3863
tgtctttcta ggtctcagc

<212> DNA<213> Homo sapiens<220> <223> PCNA HH ribozyme binding site
<400> 3864
tttctaggtc tcagccggt

<212> DNA<213> Homo sapiens<220> <223> PCNA HH ribozyme binding site
<400> 3865
tctaggtctc agccggtcg

<212> DNA<213> Homo sapiens<220> <223> PCNA HH ribozyme binding site
<400> 3866
tcagccggtc gtcgcgacg

<212> DNA<213> Homo sapiens<220> <223> PCNA HH ribozyme binding site
<400> 3867
gccggtcgtc gcgacgttc

<212> DNA<213> Homo sapiens<220> <223> PCNA HH ribozyme binding site
<400> 3868
tcgcgacgtt cgcccgctc

<212> DNA<213> Homo sapiens<220> <223> PCNA HH ribozyme binding site
<400> 3869
cgcgacgttc gcccgctct

<212> DNA<213> Homo sapiens<220> <223> PCNA HH ribozyme binding site
<400> 3870
tcgcccgctc tgaggctcc

<212> DNA<213> Homo sapiens<220> <223> PCNA HH ribozyme binding site
<400> 3871
tctgaggctc ctgaagccg

<212> DNA<213> Homo sapiens<220> <223> PCNA HH ribozyme binding site
<400> 3872
gccgaaacta gctagactt

<212> DNA<213> Homo sapiens<220> <223> PCNA HH ribozyme binding site
<400> 3873
aaactagcta gactttcct

<212> DNA<213> Homo sapiens<220> <223> PCNA HH ribozyme binding site
<400> 3874
agctagactt tcctccttc

<212> DNA<213> Homo sapiens<220> <223> PCNA HH ribozyme binding site
<400> 3875
gctagacttt cctccttcc

<212> DNA<213> Homo sapiens<220> <223> PCNA HH ribozyme binding site
<400> 3876
ctagactttc ctccttccc

<212> DNA<213> Homo sapiens<220> <223> PCNA HH ribozyme binding site
<400> 3877

gactttcctc cttcccgcc

<212> DNA<213> Homo sapiens<220> <223> PCNA HH ribozyme binding site
<400> 3878
tttcctcctt cccgcctgc

<212> DNA<213> Homo sapiens<220> <223> PCNA HH ribozyme binding site
<400> 3879
ttcctccttc ccgcctgcc

<212> DNA<213> Homo sapiens<220> <223> PCNA HH ribozyme binding site
<400> 3880
cctgcctgta gcggcgttg

<212> DNA<213> Homo sapiens<220> <223> PCNA HH ribozyme binding site
<400> 3881
tagcggcgtt gccactccg

<212> DNA<213> Homo sapiens<220> <223> PCNA HH ribozyme binding site
<400> 3882
gttgccactc cgccaccat

<212> DNA<213> Homo sapiens<220> <223> PCNA HH ribozyme binding site
<400> 3883
ccaccatgtt cgaggcgcg

<212> DNA<213> Homo sapiens<220> <223> PCNA HH ribozyme binding site
<400> 3884
caccatgttc gaggcgcgc

<212> DNA<213> Homo sapiens<220> <223> PCNA HH ribozyme binding site
<400> 3885
gcgcctggtc cagggctcc

<212> DNA<213> Homo sapiens<220> <223> PCNA HH ribozyme binding site
<400> 3886
tccagggctc catcctcaa

<212> DNA<213> Homo sapiens<220> <223> PCNA HH ribozyme binding site
<400> 3887
gggctccatc ctcaagaag

<212> DNA<213> Homo sapiens<220> <223> PCNA HH ribozyme binding site
<400> 3888
ctccatcctc aagaaggtg

<212> DNA<213> Homo sapiens<220> <223> PCNA HH ribozyme binding site
<400> 3889
agaaggtgtt ggaggcact

<212> DNA<213> Homo sapiens<220> <223> PCNA HH ribozyme binding site
<400> 3890
ggaggcactc aaggacctc

<212> DNA<213> Homo sapiens<220> <223> PCNA HH ribozyme binding site

<400> 3891
caaggacctc atcaacgag

<212> DNA<213> Homo sapiens<220> <223> PCNA HH ribozyme binding site
<400> 3892
ggacctcatc aacgaggcc

<212> DNA<213> Homo sapiens<220> <223> PCNA HH ribozyme binding site
<400> 3893
tgctgggata ttagctcca

<212> DNA<213> Homo sapiens<220> <223> PCNA HH ribozyme binding site
<400> 3894
ctgggatatt agctccagc

<212> DNA<213> Homo sapiens<220> <223> PCNA HH ribozyme binding site
<400> 3895
tgggatatta gctccagcg

<212> DNA<213> Homo sapiens<220> <223> PCNA HH ribozyme binding site
<400> 3896
atattagctc cagcggtgt

<212> DNA<213> Homo sapiens<220> <223> PCNA HH ribozyme binding site
<400> 3897
cagcggtgta aacctgcag

<212> DNA<213> Homo sapiens<220> <223> PCNA HH ribozyme binding site
<400> 3898
gcatggactc gtcccacgt

<212> DNA<213> Homo sapiens<220> <223> PCNA HH ribozyme binding site
<400> 3899
tggactcgtc ccacgtctc

<212> DNA<213> Homo sapiens<220> <223> PCNA HH ribozyme binding site
<400> 3900
gtcccacgtc tctttggtg

<212> DNA<213> Homo sapiens<220> <223> PCNA HH ribozyme binding site
<400> 3901
cccacgtctc tttggtgca

<212> DNA<213> Homo sapiens<220> <223> PCNA HH ribozyme binding site
<400> 3902
cacgtctctt tggtgcagc

<212> DNA<213> Homo sapiens<220> <223> PCNA HH ribozyme binding site
<400> 3903
acgtctcttt ggtgcagct

<212> DNA<213> Homo sapiens<220> <223> PCNA HH ribozyme binding site
<400> 3904
ggtgcagctc accctgcgg

<212> DNA<213> Homo sapiens<220> <223> PCNA HH ribozyme binding site
<400> 3905
ccctgcggtc tgagggctt

<212> DNA<213> Homo sapiens<220> <223> PCNA HH ribozyme binding site
<400> 3906
ctgagggctt cgacaccta

<212> DNA<213> Homo sapiens<220> <223> PCNA HH ribozyme binding site
<400> 3907
tgagggcttc gacacctac

<212> DNA<213> Homo sapiens<220> <223> PCNA HH ribozyme binding site
<400> 3908
tcgacaccta ccgctgcga

<212> DNA<213> Homo sapiens<220> <223> PCNA HH ribozyme binding site
<400> 3909
cgtgaacctc accagtatg

<212> DNA<213> Homo sapiens<220> <223> PCNA HH ribozyme binding site
<400> 3910
ctcaccagta tgtccaaaa

<212> DNA<213> Homo sapiens<220> <223> PCNA HH ribozyme binding site
<400> 3911
ccagtatgtc caaaatact

<212> DNA<213> Homo sapiens<220> <223> PCNA HH ribozyme binding site
<400> 3912
gtccaaaata ctaaaatgc

<212> DNA<213> Homo sapiens<220> <223> PCNA HH ribozyme binding site
<400> 3913
caaaatacta aaatgcgcc

<212> DNA<213> Homo sapiens<220> <223> PCNA HH ribozyme binding site
<400> 3914
aatgaagata tcattacac

<212> DNA<213> Homo sapiens<220> <223> PCNA HH ribozyme binding site
<400> 3915
tgaagatatc attacacta

<212> DNA<213> Homo sapiens<220> <223> PCNA HH ribozyme binding site
<400> 3916
agatatcatt acactaagg

<212> DNA<213> Homo sapiens<220> <223> PCNA HH ribozyme binding site
<400> 3917
gatatcatta cactaaggg

<212> DNA<213> Homo sapiens<220> <223> PCNA HH ribozyme binding site
<400> 3918
cattacacta agggccgaa

<212> DNA<213> Homo sapiens<220> <223> PCNA HH ribozyme binding site
<400> 3919
gccgaagata acgcggata

<212> DNA<213> Homo sapiens<220> <223> PCNA HH ribozyme binding site
<400> 3920
aacgcggata ccttggcgc

<212> DNA<213> Homo sapiens<220> <223> PCNA HH ribozyme binding site
<400> 3921
cggatacctt ggcgctagt

<212> DNA<213> Homo sapiens<220> <223> PCNA HH ribozyme binding site
<400> 3922
cttggcgcta gtatttgaa

<212> DNA<213> Homo sapiens<220> <223> PCNA HH ribozyme binding site
<400> 3923
ggcgctagta tttgaagca

<212> DNA<213> Homo sapiens<220> <223> PCNA HH ribozyme binding site
<400> 3924
cgctagtatt tgaagcacc

<212> DNA<213> Homo sapiens<220> <223> PCNA HH ribozyme binding site
<400> 3925
gctagtattt gaagcacca

<212> DNA<213> Homo sapiens<220> <223> PCNA HH ribozyme binding site
<400> 3926
ggagaaagtt tcagactat

<212> DNA<213> Homo sapiens<220> <223> PCNA HH ribozyme binding site
<400> 3927
gagaaagttt cagactatg

<212> DNA<213> Homo sapiens<220> <223> PCNA HH ribozyme binding site
<400> 3928
agaaagtttc agactatga

<212> DNA<213> Homo sapiens<220> <223> PCNA HH ribozyme binding site
<400> 3929
tttcagacta tgaaatgaa

<212> DNA<213> Homo sapiens<220> <223> PCNA HH ribozyme binding site
<400> 3930
aaatgaagtt gatggattt

<212> DNA<213> Homo sapiens<220> <223> PCNA HH ribozyme binding site
<400> 3931
ttgatggatt tagatgttg

<212> DNA<213> Homo sapiens<220> <223> PCNA HH ribozyme binding site
<400> 3932

tgatggattt agatgttga

<212> DNA<213> Homo sapiens<220> <223> PCNA HH ribozyme binding site
<400> 3933
gatggattta gatgttgaa

<212> DNA<213> Homo sapiens<220> <223> PCNA HH ribozyme binding site
<400> 3934
tttagatgtt gaacaactt

<212> DNA<213> Homo sapiens<220> <223> PCNA HH ribozyme binding site
<400> 3935
tgaacaactt ggaattcca

<212> DNA<213> Homo sapiens<220> <223> PCNA HH ribozyme binding site
<400> 3936
acttggaatt ccagaacag

<212> DNA<213> Homo sapiens<220> <223> PCNA HH ribozyme binding site
<400> 3937
cttggaattc cagaacagg

<212> DNA<213> Homo sapiens<220> <223> PCNA HH ribozyme binding site
<400> 3938
aacaggagta cagctgtgt

<212> DNA<213> Homo sapiens<220> <223> PCNA HH ribozyme binding site
<400> 3939
cagctgtgta gtaaagatg

<212> DNA<213> Homo sapiens<220> <223> PCNA HH ribozyme binding site
<400> 3940
ctgtgtagta aagatgcct

<212> DNA<213> Homo sapiens<220> <223> PCNA HH ribozyme binding site
<400> 3941
aagatgcctt ctggtgaat

<212> DNA<213> Homo sapiens<220> <223> PCNA HH ribozyme binding site
<400> 3942
agatgccttc tggtgaatt

<212> DNA<213> Homo sapiens<220> <223> PCNA HH ribozyme binding site
<400> 3943
ctggtgaatt tgcacgtat

<212> DNA<213> Homo sapiens<220> <223> PCNA HH ribozyme binding site
<400> 3944
tggtgaattt gcacgtata

<212> DNA<213> Homo sapiens<220> <223> PCNA HH ribozyme binding site
<400> 3945
tttgcacgta tatgccgag

<212> DNA<213> Homo sapiens<220> <223> PCNA HH ribozyme binding site

<400> 3946
tgcacgtata tgccgagat

<212> DNA<213> Homo sapiens<220> <223> PCNA HH ribozyme binding site
<400> 3947
tgccgagatc tcagccata

<212> DNA<213> Homo sapiens<220> <223> PCNA HH ribozyme binding site
<400> 3948
ccgagatctc agccatatt

<212> DNA<213> Homo sapiens<220> <223> PCNA HH ribozyme binding site
<400> 3949
ctcagccata ttggagatg

<212> DNA<213> Homo sapiens<220> <223> PCNA HH ribozyme binding site
<400> 3950
cagccatatt ggagatgct

<212> DNA<213> Homo sapiens<220> <223> PCNA HH ribozyme binding site
<400> 3951
agatgctgtt gtaatttcc

<212> DNA<213> Homo sapiens<220> <223> PCNA HH ribozyme binding site
<400> 3952
tgctgttgta atttcctgt

<212> DNA<213> Homo sapiens<220> <223> PCNA HH ribozyme binding site
<400> 3953
tgttgtaatt tcctgtgca

<212> DNA<213> Homo sapiens<220> <223> PCNA HH ribozyme binding site
<400> 3954
gttgtaattt cctgtgcaa

<212> DNA<213> Homo sapiens<220> <223> PCNA HH ribozyme binding site
<400> 3955
ttgtaatttc ctgtgcaaa

<212> DNA<213> Homo sapiens<220> <223> PCNA HH ribozyme binding site
<400> 3956
gagtgaaatt ttctgcaag

<212> DNA<213> Homo sapiens<220> <223> PCNA HH ribozyme binding site
<400> 3957
agtgaaattt tctgcaagt

<212> DNA<213> Homo sapiens<220> <223> PCNA HH ribozyme binding site
<400> 3958
gtgaaatttt ctgcaagtg

<212> DNA<213> Homo sapiens<220> <223> PCNA HH ribozyme binding site
<400> 3959
tgaaattttc tgcaagtgg

<212> DNA<213> Homo sapiens<220> <223> PCNA HH ribozyme binding site
<400> 3960
tggagaactt ggaaatgga

<212> DNA<213> Homo sapiens<220> <223> PCNA HH ribozyme binding site
<400> 3961
tggaaacatt aaattgtca

<212> DNA<213> Homo sapiens<220> <223> PCNA HH ribozyme binding site
<400> 3962
ggaaacatta aattgtcac

<212> DNA<213> Homo sapiens<220> <223> PCNA HH ribozyme binding site
<400> 3963
acattaaatt gtcacagac

<212> DNA<213> Homo sapiens<220> <223> PCNA HH ribozyme binding site
<400> 3964
ttaaattgtc acagacaag

<212> DNA<213> Homo sapiens<220> <223> PCNA HH ribozyme binding site
<400> 3965
cagacaagta atgtcgata

<212> DNA<213> Homo sapiens<220> <223> PCNA HH ribozyme binding site
<400> 3966
aagtaatgtc gataaagag

<212> DNA<213> Homo sapiens<220> <223> PCNA HH ribozyme binding site
<400> 3967
aatgtcgata aagaggagg

<212> DNA<213> Homo sapiens<220> <223> PCNA HH ribozyme binding site
<400> 3968
ggaagctgtt accatagag

<212> DNA<213> Homo sapiens<220> <223> PCNA HH ribozyme binding site
<400> 3969
gaagctgtta ccatagaga

<212> DNA<213> Homo sapiens<220> <223> PCNA HH ribozyme binding site
<400> 3970
tgttaccata gagatgaat

<212> DNA<213> Homo sapiens<220> <223> PCNA HH ribozyme binding site
<400> 3971
tgaaccagtt caactaact

<212> DNA<213> Homo sapiens<220> <223> PCNA HH ribozyme binding site
<400> 3972
gaaccagttc aactaactt

<212> DNA<213> Homo sapiens<220> <223> PCNA HH ribozyme binding site
<400> 3973
agttcaacta acttttgca

<212> DNA<213> Homo sapiens<220> <223> PCNA HH ribozyme binding site
<400> 3974
caactaactt ttgcactga

<212> DNA<213> Homo sapiens<220> <223> PCNA HH ribozyme binding site
<400> 3975
aactaacttt tgcactgag

<212> DNA<213> Homo sapiens<220> <223> PCNA HH ribozyme binding site
<400> 3976
actaactttt gcactgagg

<212> DNA<213> Homo sapiens<220> <223> PCNA HH ribozyme binding site
<400> 3977
cactgaggta cctgaactt

<212> DNA<213> Homo sapiens<220> <223> PCNA HH ribozyme binding site
<400> 3978
acctgaactt ctttacaaa

<212> DNA<213> Homo sapiens<220> <223> PCNA HH ribozyme binding site
<400> 3979
cctgaacttc tttacaaaa

<212> DNA<213> Homo sapiens<220> <223> PCNA HH ribozyme binding site
<400> 3980
tgaacttctt tacaaaagc

<212> DNA<213> Homo sapiens<220> <223> PCNA HH ribozyme binding site
<400> 3981
gaacttcttt acaaaagcc

<212> DNA<213> Homo sapiens<220> <223> PCNA HH ribozyme binding site
<400> 3982
aacttcttta caaaagcca

<212> DNA<213> Homo sapiens<220> <223> PCNA HH ribozyme binding site
<400> 3983
aaagccactc cactctctt

<212> DNA<213> Homo sapiens<220> <223> PCNA HH ribozyme binding site
<400> 3984
cactccactc tcttcaacg

<212> DNA<213> Homo sapiens<220> <223> PCNA HH ribozyme binding site
<400> 3985
ctccactctc ttcaacggt

<212> DNA<213> Homo sapiens<220> <223> PCNA HH ribozyme binding site
<400> 3986
ccactctctt caacggtga

<212> DNA<213> Homo sapiens<220> <223> PCNA HH ribozyme binding site
<400> 3987

cactctcttc aacggtgac

<212> DNA<213> Homo sapiens<220> <223> PCNA HH ribozyme binding site
<400> 3988
ggtgacactc agtatgtct

<212> DNA<213> Homo sapiens<220> <223> PCNA HH ribozyme binding site
<400> 3989
acactcagta tgtctgcag

<212> DNA<213> Homo sapiens<220> <223> PCNA HH ribozyme binding site
<400> 3990
tcagtatgtc tgcagatgt

<212> DNA<213> Homo sapiens<220> <223> PCNA HH ribozyme binding site
<400> 3991
tgcagatgta ccccttgtt

<212> DNA<213> Homo sapiens<220> <223> PCNA HH ribozyme binding site
<400> 3992
tgtacccctt gttgtagag

<212> DNA<213> Homo sapiens<220> <223> PCNA HH ribozyme binding site
<400> 3993
accccttgtt gtagagtat

<212> DNA<213> Homo sapiens<220> <223> PCNA HH ribozyme binding site
<400> 3994
ccttgttgta gagtataaa

<212> DNA<213> Homo sapiens<220> <223> PCNA HH ribozyme binding site
<400> 3995
ttgtagagta taaaattgc

<212> DNA<213> Homo sapiens<220> <223> PCNA HH ribozyme binding site
<400> 3996
gtagagtata aaattgcgg

<212> DNA<213> Homo sapiens<220> <223> PCNA HH ribozyme binding site
<400> 3997
gtataaaatt gcggatatg

<212> DNA<213> Homo sapiens<220> <223> PCNA HH ribozyme binding site
<400> 3998
attgcggata tgggacact

<212> DNA<213> Homo sapiens<220> <223> PCNA HH ribozyme binding site
<400> 3999
tgggacactt aaaatacta

<212> DNA<213> Homo sapiens<220> <223> PCNA HH ribozyme binding site
<400> 4000
gggacactta aaatactac

<212> DNA<213> Homo sapiens<220> <223> PCNA HH ribozyme binding site

<400> 4001
acttaaaata ctacttggc

<212> DNA<213> Homo sapiens<220> <223> PCNA HH ribozyme binding site
<400> 4002
taaaatacta cttggctcc

<212> DNA<213> Homo sapiens<220> <223> PCNA HH ribozyme binding site
<400> 4003
aatactactt ggctcccaa

<212> DNA<213> Homo sapiens<220> <223> PCNA HH ribozyme binding site
<400> 4004
tacttggctc ccaagatcg

<212> DNA<213> Homo sapiens<220> <223> PCNA HH ribozyme binding site
<400> 4005
tcccaagatc gaggatgaa

<212> DNA<213> Homo sapiens<220> <223> PCNA HH ribozyme binding site
<400> 4006
aagaaggatc ttaggcatt

<212> DNA<213> Homo sapiens<220> <223> PCNA HH ribozyme binding site
<400> 4007
gaaggatctt aggcattct

<212> DNA<213> Homo sapiens<220> <223> PCNA HH ribozyme binding site
<400> 4008
aaggatctta ggcattctt

<212> DNA<213> Homo sapiens<220> <223> PCNA HH ribozyme binding site
<400> 4009
cttaggcatt cttaaaatt

<212> DNA<213> Homo sapiens<220> <223> PCNA HH ribozyme binding site
<400> 4010
ttaggcattc ttaaaattc

<212> DNA<213> Homo sapiens<220> <223> PCNA HH ribozyme binding site
<400> 4011
aggcattctt aaaattcaa

<212> DNA<213> Homo sapiens<220> <223> PCNA HH ribozyme binding site
<400> 4012
ggcattctta aaattcaag

<212> DNA<213> Homo sapiens<220> <223> PCNA HH ribozyme binding site
<400> 4013
tcttaaaatt caagaaaat

<212> DNA<213> Homo sapiens<220> <223> PCNA HH ribozyme binding site
<400> 4014
cttaaaattc aagaaaata

<212> DNA<213> Homo sapiens<220> <223> PCNA HH ribozyme binding site
<400> 4015
caagaaaata aaactaagc

<212> DNA<213> Homo sapiens<220> <223> PCNA HH ribozyme binding site
<400> 4016
aataaaacta agctctttg

<212> DNA<213> Homo sapiens<220> <223> PCNA HH ribozyme binding site
<400> 4017
aactaagctc tttgagaac

<212> DNA<213> Homo sapiens<220> <223> PCNA HH ribozyme binding site
<400> 4018
ctaagctctt tgagaactg

<212> DNA<213> Homo sapiens<220> <223> PCNA HH ribozyme binding site
<400> 4019
taagctcttt gagaactgc

<212> DNA<213> Homo sapiens<220> <223> PCNA HH ribozyme binding site
<400> 4020
agaactgctt ctaagatgc

<212> DNA<213> Homo sapiens<220> <223> PCNA HH ribozyme binding site
<400> 4021
gaactgcttc taagatgcc

<212> DNA<213> Homo sapiens<220> <223> PCNA HH ribozyme binding site
<400> 4022
actgcttcta agatgccag

<212> DNA<213> Homo sapiens<220> <223> PCNA HH ribozyme binding site
<400> 4023
tgccagcata tactgaagt

<212> DNA<213> Homo sapiens<220> <223> PCNA HH ribozyme binding site
<400> 4024
ccagcatata ctgaagtct

<212> DNA<213> Homo sapiens<220> <223> PCNA HH ribozyme binding site
<400> 4025
tactgaagtc ttttctgtc

<212> DNA<213> Homo sapiens<220> <223> PCNA HH ribozyme binding site
<400> 4026
ctgaagtctt ttctgtcac

<212> DNA<213> Homo sapiens<220> <223> PCNA HH ribozyme binding site
<400> 4027
tgaagtcttt tctgtcacc

<212> DNA<213> Homo sapiens<220> <223> PCNA HH ribozyme binding site
<400> 4028
gaagtctttt ctgtcacca

<212> DNA<213> Homo sapiens<220> <223> PCNA HH ribozyme binding site
<400> 4029
aagtcttttc tgtcaccaa

<212> DNA<213> Homo sapiens<220> <223> PCNA HH ribozyme binding site
<400> 4030
cttttctgtc accaaattt

<212> DNA<213> Homo sapiens<220> <223> PCNA HH ribozyme binding site
<400> 4031
tcaccaaatt tgtacctct

<212> DNA<213> Homo sapiens<220> <223> PCNA HH ribozyme binding site
<400> 4032
caccaaattt gtacctcta

<212> DNA<213> Homo sapiens<220> <223> PCNA HH ribozyme binding site
<400> 4033
caaatttgta cctctaagt

<212> DNA<213> Homo sapiens<220> <223> PCNA HH ribozyme binding site
<400> 4034
tttgtacctc taagtacat

<212> DNA<213> Homo sapiens<220> <223> PCNA HH ribozyme binding site
<400> 4035
tgtacctcta agtacatat

<212> DNA<213> Homo sapiens<220> <223> PCNA HH ribozyme binding site
<400> 4036
cctctaagta catatgtag

<212> DNA<213> Homo sapiens<220> <223> PCNA HH ribozyme binding site
<400> 4037
taagtacata tgtagatat

<212> DNA<213> Homo sapiens<220> <223> PCNA HH ribozyme binding site
<400> 4038
tacatatgta gatattgtt

<212> DNA<213> Homo sapiens<220> <223> PCNA HH ribozyme binding site
<400> 4039
tatgtagata ttgttttct

<212> DNA<213> Homo sapiens<220> <223> PCNA HH ribozyme binding site
<400> 4040
tgtagatatt gttttctgt

<212> DNA<213> Homo sapiens<220> <223> PCNA HH ribozyme binding site
<400> 4041
agatattgtt ttctgtaaa

<212> DNA<213> Homo sapiens<220> <223> PCNA HH ribozyme binding site
<400> 4042

gatattgttt tctgtaaat

<212> DNA<213> Homo sapiens<220> <223> PCNA HH ribozyme binding site
<400> 4043
atattgtttt ctgtaaata

<212> DNA<213> Homo sapiens<220> <223> PCNA HH ribozyme binding site
<400> 4044
tattgttttc tgtaaataa

<212> DNA<213> Homo sapiens<220> <223> PCNA HH ribozyme binding site
<400> 4045
gttttctgta aataaccta

<212> DNA<213> Homo sapiens<220> <223> PCNA HH ribozyme binding site
<400> 4046
tctgtaaata acctatttt

<212> DNA<213> Homo sapiens<220> <223> PCNA HH ribozyme binding site
<400> 4047
aaataaccta tttttttc

<212> DNA<213> Homo sapiens<220> <223> PCNA HH ribozyme binding site
<400> 4048
ataacctatt tttttctc

<212> DNA<213> Homo sapiens<220> <223> PCNA HH ribozyme binding site
<400> 4049
taacctattt tttttctct

<212> DNA<213> Homo sapiens<220> <223> PCNA HH ribozyme binding site
<400> 4050
aacctatttt ttttctcta

<212> DNA<213> Homo sapiens<220> <223> PCNA HH ribozyme binding site
<400> 4051
acctattttt tttctctat

<212> DNA<213> Homo sapiens<220> <223> PCNA HH ribozyme binding site
<400> 4052
cctatttttt ttctctatt

<212> DNA<213> Homo sapiens<220> <223> PCNA HH ribozyme binding site
<400> 4053
ctattttttt tctctattc

<212> DNA<213> Homo sapiens<220> <223> PCNA HH ribozyme binding site
<400> 4054
tattttttt ctctattct

<212> DNA<213> Homo sapiens<220> <223> PCNA HH ribozyme binding site
<400> 4055
atttttttc tctattctc

<212> DNA<213> Homo sapiens<220> <223> PCNA HH ribozyme binding site

<400> 4056
tttttttctc tattctctc

<212> DNA<213> Homo sapiens<220> <223> PCNA HH ribozyme binding site
<400> 4057
tttttctcta ttctctcca

<212> DNA<213> Homo sapiens<220> <223> PCNA HH ribozyme binding site
<400> 4058
tttctctatt ctctccaat

<212> DNA<213> Homo sapiens<220> <223> PCNA HH ribozyme binding site
<400> 4059
ttctctattc tctccaatt

<212> DNA<213> Homo sapiens<220> <223> PCNA HH ribozyme binding site
<400> 4060
ctctattctc tccaatttg

<212> DNA<213> Homo sapiens<220> <223> PCNA HH ribozyme binding site
<400> 4061
ctattctctc caatttgtt

<212> DNA<213> Homo sapiens<220> <223> PCNA HH ribozyme binding site
<400> 4062
ctctccaatt tgtttaaag

<212> DNA<213> Homo sapiens<220> <223> PCNA HH ribozyme binding site
<400> 4063
tctccaattt gtttaaaga

<212> DNA<213> Homo sapiens<220> <223> PCNA HH ribozyme binding site
<400> 4064
ccaatttgtt taaagaata

<212> DNA<213> Homo sapiens<220> <223> PCNA HH ribozyme binding site
<400> 4065
caatttgttt aaagaataa

<212> DNA<213> Homo sapiens<220> <223> PCNA HH ribozyme binding site
<400> 4066
aatttgttta aagaataaa

<212> DNA<213> Homo sapiens<220> <223> PCNA HH ribozyme binding site
<400> 4067
ttaaagaata aagtccaaa

<212> DNA<213> Homo sapiens<220> <223> PCNA HH ribozyme binding site
<400> 4068
gaataaagtc caaagtctg

<212> DNA<213> Homo sapiens<220> <223> PCNA HH ribozyme binding site
<400> 4069
gtccaaagtc tgatctggt

<212> DNA<213> Homo sapiens<220> <223> PCNA HH ribozyme binding site
<400> 4070
aagtctgatc tggtctagt

<212> DNA<213> Homo sapiens<220> <223> PCNA HH ribozyme binding site
<400> 4071
tgatctggtc tagttaacc

<212> DNA<213> Homo sapiens<220> <223> PCNA HH ribozyme binding site
<400> 4072
atctggtcta gttaaccta

<212> DNA<213> Homo sapiens<220> <223> PCNA HH ribozyme binding site
<400> 4073
tggtctagtt aacctagaa

<212> DNA<213> Homo sapiens<220> <223> PCNA HH ribozyme binding site
<400> 4074
ggtctagtta acctagaag

<212> DNA<213> Homo sapiens<220> <223> PCNA HH ribozyme binding site
<400> 4075
agttaaccta gaagtattt

<212> DNA<213> Homo sapiens<220> <223> PCNA HH ribozyme binding site
<400> 4076
cctagaagta tttttgtct

<212> DNA<213> Homo sapiens<220> <223> PCNA HH ribozyme binding site
<400> 4077
tagaagtatt tttgtctct

<212> DNA<213> Homo sapiens<220> <223> PCNA HH ribozyme binding site
<400> 4078
agaagtattt ttgtctctt

<212> DNA<213> Homo sapiens<220> <223> PCNA HH ribozyme binding site
<400> 4079
gaagtatttt tgtctctta

<212> DNA<213> Homo sapiens<220> <223> PCNA HH ribozyme binding site
<400> 4080
aagtattttt gtctcttag

<212> DNA<213> Homo sapiens<220> <223> PCNA HH ribozyme binding site
<400> 4081
tatttttgtc tcttagaaa

<212> DNA<213> Homo sapiens<220> <223> PCNA HH ribozyme binding site
<400> 4082
tttttgtctc ttagaaata

<212> DNA<213> Homo sapiens<220> <223> PCNA HH ribozyme binding site
<400> 4083
tttgtctctt agaaatact

<212> DNA<213> Homo sapiens<220> <223> PCNA HH ribozyme binding site
<400> 4084
ttgtctctta gaaatactt

<212> DNA<213> Homo sapiens<220> <223> PCNA HH ribozyme binding site
<400> 4085
cttagaaata cttgtgatt

<212> DNA<213> Homo sapiens<220> <223> PCNA HH ribozyme binding site
<400> 4086
agaaatactt gtgattttt

<212> DNA<213> Homo sapiens<220> <223> PCNA HH ribozyme binding site
<400> 4087
acttgtgatt tttataata

<212> DNA<213> Homo sapiens<220> <223> PCNA HH ribozyme binding site
<400> 4088
cttgtgattt ttataatac

<212> DNA<213> Homo sapiens<220> <223> PCNA HH ribozyme binding site
<400> 4089
ttgtgatttt tataataca

<212> DNA<213> Homo sapiens<220> <223> PCNA HH ribozyme binding site
<400> 4090
tgtgattttt ataatacaa

<212> DNA<213> Homo sapiens<220> <223> PCNA HH ribozyme binding site
<400> 4091
gtgattttta taatacaaa

<212> DNA<213> Homo sapiens<220> <223> PCNA HH ribozyme binding site
<400> 4092
gatttttata atacaaaag

<212> DNA<213> Homo sapiens<220> <223> PCNA HH ribozyme binding site
<400> 4093
ttttataata caaaagggt

<212> DNA<213> Homo sapiens<220> <223> PCNA HH ribozyme binding site
<400> 4094
caaaagggtc ttgactcta

<212> DNA<213> Homo sapiens<220> <223> PCNA HH ribozyme binding site
<400> 4095
aaagggtctt gactctaaa

<212> DNA<213> Homo sapiens<220> <223> PCNA HH ribozyme binding site
<400> 4096
gtcttgactc taaatgcag

<212> DNA<213> Homo sapiens<220> <223> PCNA HH ribozyme binding site
<400> 4097

cttgactcta aatgcagtt

<212> DNA<213> Homo sapiens<220> <223> PCNA HH ribozyme binding site
<400> 4098
aaatgcagtt ttaagaagt

<212> DNA<213> Homo sapiens<220> <223> PCNA HH ribozyme binding site
<400> 4099
aatgcagttt taagaagtg

<212> DNA<213> Homo sapiens<220> <223> PCNA HH ribozyme binding site
<400> 4100
atgcagtttt aagaagtgt

<212> DNA<213> Homo sapiens<220> <223> PCNA HH ribozyme binding site
<400> 4101
tgcagtttta agaagtgtt

<212> DNA<213> Homo sapiens<220> <223> PCNA HH ribozyme binding site
<400> 4102
aagaagtgtt tttgaattt

<212> DNA<213> Homo sapiens<220> <223> PCNA HH ribozyme binding site
<400> 4103
agaagtgttt ttgaattta

<212> DNA<213> Homo sapiens<220> <223> PCNA HH ribozyme binding site
<400> 4104
gaagtgtttt tgaatttaa

<212> DNA<213> Homo sapiens<220> <223> PCNA HH ribozyme binding site
<400> 4105
aagtgttttt gaatttaaa

<212> DNA<213> Homo sapiens<220> <223> PCNA HH ribozyme binding site
<400> 4106
tttttgaatt taaataaag

<212> DNA<213> Homo sapiens<220> <223> PCNA HH ribozyme binding site
<400> 4107
ttttgaattt aaataaagt

<212> DNA<213> Homo sapiens<220> <223> PCNA HH ribozyme binding site
<400> 4108
tttgaattta aataaagtt

<212> DNA<213> Homo sapiens<220> <223> PCNA HH ribozyme binding site
<400> 4109
aatttaaata aagttactt

<212> DNA<213> Homo sapiens<220> <223> PCNA HH ribozyme binding site
<400> 4110
aaataaagtt acttgaatt

<212> DNA<213> Homo sapiens<220> <223> PCNA HH ribozyme binding site

<400> 4111
aataaagtta cttgaattt

<212> DNA<213> Homo sapiens<220> <223> PCNA HH ribozyme binding site
<400> 4112
aaagttactt gaatttcaa

<212> DNA<213> Homo sapiens<220> <223> PCNA HH ribozyme binding site
<400> 4113
tacttgaatt tcaaacaaa

<212> DNA<213> Homo sapiens<220> <223> PCNA HH ribozyme binding site
<400> 4114
acttgaattt caaacaaaa

<212> DNA<213> Homo sapiens<220> <223> PCNA HH ribozyme binding site
<400> 4115
cttgaatttc aaacaaaaa

<212> DNA<213> Homo sapiens
<400> 4116
gacccctgag aaaggcacca gagaaacaca cttggtggta tattacctgg ta

<212> DNA<213> Homo sapiens
<400> 4117
gagccctgag aaaggcacca gagaaacaca cttggtggta tattacctgg ta

<212> DNA<213> Homo sapiens
<400> 4118
gagccctgag aaaggcacca gcgaaacaca cttggtggca tattacctgg ta

<212> DNA<213> Homo sapiens
<400> 4119
gagccctgag aaaggcacca gcgaaacaca cggacuucgg tccgtggcat attacctggt a

<212> DNA<213> Homo sapiens<220> <223> General Hairpin Ribozyme Site<220>
<400> 4120
nnnbngucnn nnnn

<212> DNA<213> Homo sapiens<220> <223> General Hairpin Ribozyme Site<220>
<400> 4121
nnnbngucnn nnnnn

<210> 4122<212> DNA<213> Homo sapiens<220> <223> General Hairpin Ribozyme
<400> 4122
nnnbngucnn nnnnnn

<210> 4123<212> DNA<213> Homo sapiens<220> <223> General Hairpin Ribozyme
<400> 4123
nnnbngucnn nnnnnnn

<210> 4124<212> DNA<213> Homo sapiens<220> <223> General Hairpin Ribozyme
<400> 4124
nnnbngucnn nnnnnnnn

<212> DNA<213> Homo sapiens<220> <223> Hairpin ribozyme recognition site
for cdc 2 kinase
<400> 4125
acttcgtcat ccaaat                                                    16

<212> DNA<213> Homo sapiens<220> <223> Hairpin ribozyme recognition site
for cdc 2 kinase
<400> 4126
atatagtcag tcttca                                                    16

<212> DNA<213> Homo sapiens<220> <223> Hairpin ribozyme recognition site
for cdc 2 kinase
<400> 4127
agtcagtctt caggat                                                    16

<212> DNA<213> Homo sapiens<220> <223> Hairpin ribozyme recognition site
for cdc 2 kinase
<400> 4128
tcctggtcag tacatg                                                    16

<212> DNA<213> Homo sapiens<220> <223> Hairpin ribozyme recognition site
for cdc 2 kinase
<400> 4129
gttttgtcac tctaga                                                    16

<212> DNA<213> Homo sapiens<220> <223> Hairpin ribozyme recognition site
for cdc 2 kinase
<400> 4130
ctggggtcag ctcgtt                                                    16

<210> <211> <212> DNA<213> Homo sapiens<220> <223> <400>

<212> DNA<213> Homo sapiens<220> <223> Hairpin ribozyme recognition site
for cyclin B1
<400> 4131
tccgagtcac caggaa                                                    16

<212> DNA<213> Homo sapiens<220> <223> Hairpin ribozyme recognition site
for cyclin B1
<400> 4132
ccagtgtctg agccag                                                    16

<212> DNA<213> Homo sapiens<220> <223> Hairpin ribozyme recognition site
for cyclin B1
<400> 4133
cctgtgtcag gctttc                                                    16

<212> DNA<213> Homo sapiens<220> <223> Hairpin ribozyme recognition site
for cyclin B1
<400> 4134
aagcagtcag accaaa                                                    16

<212> DNA<213> Homo sapiens<220> <223> Hairpin ribozyme recognition site
for cyclin B1
<400> 4135

actgggtcgg gaagtc                                                16

<212> DNA<213> Homo sapiens<220> <223> Hairpin ribozyme recognition site
for cyclin B1
<400> 4136
tgactgtctc cattat                                                16

<212> DNA<213> Homo sapiens<220> <223> Hairpin ribozyme recognition site
for cyclin B1
<400> 4137
ttggtgtcac tgccat                                                16

<212> DNA<213> Homo sapiens<220> <223> Hairpin ribozyme recognition site
for cyclin B1
<400> 4138
ctttggtctg ggtcgg                                                16

<212> DNA<213> Homo sapiens<220> <223> Hairpin ribozyme recognition site
for cyclin B1
<400> 4139
tctgggtcgg cctcta                                                16

<212> DNA<213> Homo sapiens<220> <223> Hairpin ribozyme recognition site
for cyclin B1
<400> 4140
tacctgtcat atactg                                                16

<212> DNA<213> Homo sapiens<220> <223> Hairpin ribozyme recognition site
for cyclin B1
<400> 4141
atgtagtcat ggtaaa                                                16

<212> DNA<213> Homo sapiens<220> <223> Hairpin ribozyme recognition site
for cyclin B1
<400> 4142
tgactgtcaa gaacaa                                                16

<212> DNA<213> Homo sapiens<220> <223> Hairpin ribozyme recognition site
for PCNA
<400> 4143
gagtggtcgt tgtctt                                                16

<212> DNA<213> Homo sapiens<220> <223> Hairpin ribozyme recognition site
for PCNA
<400> 4144
tcgttgtctt tctagg                                                16

<212> DNA<213> Homo sapiens<220> <223> Hairpin ribozyme recognition site
for PCNA
<400> 4145
gcctggtcca gggctc                                                16

<212> DNA<213> Homo sapiens<220> <223> Hairpin ribozyme recognition site
for PCNA
<400> 4146

gactcgtccc acgtct                                                16

<212> DNA<213> Homo sapiens<220> <223> Hairpin ribozyme recognition site
for PCNA
<400> 4147
ctgcggtctg agggct                                                16

<212> DNA<213> Homo sapiens<220> <223> Hairpin ribozyme recognition site
for PCNA
<400> 4148
aaattgtcac agacaa                                                16

<212> DNA<213> Homo sapiens<220> <223> Hairpin ribozyme recognition site
for PCNA
<400> 4149
tttctgtcac caaatt                                                16

<212> DNA<213> Homo sapiens<220> <223> Hairpin ribozyme recognition site
for PCNA
<400> 4150
atctggtcta gttaac                                                16

<212> DNA<213> Homo sapiens<220> <223> Hairpin ribozyme recognition site
for PCNA
<400> 4151
ttttgtctc ttagaa                                                16

<212> DNA<213> Homo sapiens<220> <223> Hairpin ribozyme recognition site
for PCNA
<400> 4152
aaagggtctt gactct                                                16

<212> DNA<213> Homo sapiens<220> <223> Hairpin ribozyme recognition site
for lysyl oxidase
<400> 4153
ccgccgtccc tggtgc                                                16

<212> DNA<213> Homo sapiens<220> <223> Hairpin ribozyme recognition site
for lysyl oxidase
<400> 4154
ctggagtcac cgctgg                                                16

<212> DNA<213> Homo sapiens<220> <223> Hairpin ribozyme recognition site
for lysyl oxidase
<400> 4155
cgcccgtcac tggttc                                                16

<212> DNA<213> Homo sapiens<220> <223> Hairpin ribozyme recognition site
for lysyl oxidase
<400> 4156
gtacggtctc ccagac                                                16

<212> DNA<213> Homo sapiens<220> <223> Hairpin ribozyme recognition site
for lysyl oxidase
<400> 4157

caggcgtcca cgtacg 16

<212> DNA<213> Homo sapiens<220> <223> Hairpin ribozyme recognition site
for lysyl oxidase
<400> 4158
aaactgtctg gccagt 16

<212> DNA<213> Homo sapiens<220> <223> Hairpin ribozyme recognition site
for lysyl oxidase
<400> 4159
tttctgtctt gaagac 16

<212> DNA<213> Homo sapiens<220> <223> Hammerhead ribozyme recognition site
for cdc 2 kinase
<400> 4160
tacaggtcaa gtggta 16

<212> DNA<213> Homo sapiens<220> <223> Hammerhead ribozyme recognition site
for cdc 2 kinase
<400> 4161
aaatttctct attaaag 17

<212> DNA<213> Homo sapiens<220> <223> Hammerhead ribozyme recognition site
for cdc 2 kinase
<400> 4162
agtcagtctt caggat 16

<212> DNA<213> Homo sapiens<220> <223> Hammerhead ribozyme recognition site
for cdc 2 kinase
<400> 4163
ctggggtcag ctcgtt 16

<212> DNA<213> Homo sapiens<220> <223> Hammerhead ribozyme recognition site
for cdc 2 kinase
<400> 4164
cgcggaataa taagccgg 18

<212> DNA<213> Homo sapiens<220> <223> Hammerhead ribozyme recognition site
for cdc 2 kinase
<400> 4165
ggaataataa gccgggat 18

<212> DNA<213> Homo sapiens<220> <223> Hammerhead ribozyme recognition site
for cdc 2 kinase
<400> 4166
gccgggatct accatacc 18

<212> DNA<213> Homo sapiens<220> <223> Hammerhead ribozyme recognition site
for cdc 2 kinase
<400> 4167
cgggatctac cataccat 18

<212> DNA<213> Homo sapiens<220> <223> Hammerhead ribozyme recognition site
for cdc 2 kinase
<400> 4168

tctaccatac cattgact 18

<212> DNA<213> Homo sapiens<220> <223> Hammerhead ribozyme recognition site
for cdc 2 kinase
<400> 4169
cataccattg actaacta 18

<212> DNA<213> Homo sapiens<220> <223> Hammerhead ribozyme recognition site
for cdc 2 kinase
<400> 4170
cattgactaa ctatggaa 18

<212> DNA<213> Homo sapiens<220> <223> Hammerhead ribozyme recognition site
for cdc 2 kinase
<400> 4171
gactaactat ggaagatt 18

<212> DNA<213> Homo sapiens<220> <223> Hammerhead ribozyme recognition site
for cdc 2 kinase
<400> 4172
tggaagatta taccaaaa 18

<212> DNA<213> Homo sapiens<220> <223> Hammerhead ribozyme recognition site
for cdc 2 kinase
<400> 4173
ggaagattat accaaaat 18

<212> DNA<213> Homo sapiens<220> <223> Hammerhead ribozyme recognition site
for cdc 2 kinase
<400> 4174
aagattatac caaaatag 18

<212> DNA<213> Homo sapiens<220> <223> Hammerhead ribozyme recognition site
for cdc 2 kinase
<400> 4175
accaaaatag agaaaatt 18

<212> DNA<213> Homo sapiens<220> <223> Hammerhead ribozyme recognition site
for cdc 2 kinase
<400> 4176
gagaaaattg gagaaggt 18

<212> DNA<213> Homo sapiens<220> <223> Hammerhead ribozyme recognition site
for cdc 2 kinase
<400> 4177
gagaaggtac ctatggag 18

<212> DNA<213> Homo sapiens<220> <223> Hammerhead ribozyme recognition site
for cdc 2 kinase
<400> 4178
aggtacctat ggagttgtg 19

<212> DNA<213> Homo sapiens<220> <223> Hammerhead ribozyme recognition site
for cdc 2 kinase
<400> 4179

tatggagttg tgtataag 18

<212> DNA<213> Homo sapiens<220> <223> Hammerhead ribozyme recognition site
for cdc 2 kinase
<400> 4180
agttgtgtat aagggtag 18

<212> DNA<213> Homo sapiens<220> <223> Hammerhead ribozyme recognition site
for cdc 2 kinase
<400> 4181
ttgtgtataa gggtagac 18

<212> DNA<213> Homo sapiens<220> <223> Hammerhead ribozyme recognition site
for cdc 2 kinase
<400> 4182
ataagggtag acacaaaa 18

<212> DNA<213> Homo sapiens<220> <223> Hammerhead ribozyme recognition site
for cdc 2 kinase
<400> 4183
acaaaactac aggtcaag 18

<212> DNA<213> Homo sapiens<220> <223> Hammerhead ribozyme recognition site
for cdc 2 kinase
<400> 4184
ctacaggtca agtggtag 18

<212> DNA<213> Homo sapiens<220> <223> Hammerhead ribozyme recognition site
for cdc 2 kinase
<400> 4185
caagtggtag ccatgaaa 18

<212> DNA<213> Homo sapiens<220> <223> Hammerhead ribozyme recognition site
for cdc 2 kinase
<400> 4186
aaaaaaatca gactagaa 18

<212> DNA<213> Homo sapiens<220> <223> Hammerhead ribozyme recognition site
for cdc 2 kinase
<400> 4187
atcagactag aaagtgaa 18

<212> DNA<213> Homo sapiens<220> <223> Hammerhead ribozyme recognition site
for cdc 2 kinase
<400> 4188
gaaggggttc ctagtact 18

<212> DNA<213> Homo sapiens<220> <223> Hammerhead ribozyme recognition site
for cdc 2 kinase
<400> 4189
aaggggttcc tagtactg 18

<212> DNA<213> Homo sapiens<220> <223> Hammerhead ribozyme recognition site
for cdc 2 kinase
<400> 4190

gggttcctag tactgcaa 18

<212> DNA<213> Homo sapiens<220> <223> Hammerhead ribozyme recognition site
for cdc 2 kinase
<400> 4191
ttcctagtac tgcaattc 18

<212> DNA<213> Homo sapiens<220> <223> Hammerhead ribozyme recognition site
for cdc 2 kinase
<400> 4192
actgcaattc gggaaatt 18

<212> DNA<213> Homo sapiens<220> <223> Hammerhead ribozyme recognition site
for cdc 2 kinase
<400> 4193
ctgcaattcg ggaaattt 18

<212> DNA<213> Homo sapiens<220> <223> Hammerhead ribozyme recognition site
for cdc 2 kinase
<400> 4194
cgggaaattt ctctatta 18

<212> DNA<213> Homo sapiens<220> <223> Hammerhead ribozyme recognition site
for cdc 2 kinase
<400> 4195
gggaaatttc tctattaa 18

<212> DNA<213> Homo sapiens<220> <223> Hammerhead ribozyme recognition site
for cdc 2 kinase
<400> 4196
ggaaatttct ctattaaa 18

<212> DNA<213> Homo sapiens<220> <223> Hammerhead ribozyme recognition site
for cdc 2 kinase
<400> 4197
aaatttctct attaaagg 18

<212> DNA<213> Homo sapiens<220> <223> Hammerhead ribozyme recognition site
for cdc 2 kinase
<400> 4198
atttctctat taaaggaa 18

<212> DNA<213> Homo sapiens<220> <223> Hammerhead ribozyme recognition site
for cdc 2 kinase
<400> 4199
ttctctatta aaggaact 18

<212> DNA<213> Homo sapiens<220> <223> Hammerhead ribozyme recognition site
for cdc 2 kinase
<400> 4200
tctctattaa aggaactt 18

<212> DNA<213> Homo sapiens<220> <223> Hammerhead ribozyme recognition site
for cdc 2 kinase
<400> 4201

aaggaacttc gtcatcca 18

<212> DNA<213> Homo sapiens<220> <223> Hammerhead ribozyme recognition site
for cdc 2 kinase
<400> 4202
aggaacttcg tcatccaa 18

<212> DNA<213> Homo sapiens<220> <223> Hammerhead ribozyme recognition site
for cdc 2 kinase
<400> 4203
aacttcgtca tccaaata 18

<212> DNA<213> Homo sapiens<220> <223> Hammerhead ribozyme recognition site
for cdc 2 kinase
<400> 4204
ttcgtcatcc aaatatag 18

<212> DNA<213> Homo sapiens<220> <223> Hammerhead ribozyme recognition site
for cdc 2 kinase
<400> 4205
atccaaatat agtcagtc 18

<212> DNA<213> Homo sapiens<220> <223> Hammerhead ribozyme recognition site
for cdc 2 kinase
<400> 4206
ccaaatatag tcagtctt 18

<212> DNA<213> Homo sapiens<220> <223> Hammerhead ribozyme recognition site
for cdc 2 kinase
<400> 4207
aatatagtca gtcttcag 18

<212> DNA<213> Homo sapiens<220> <223> Hammerhead ribozyme recognition site
for cdc 2 kinase
<400> 4208
tagtcagtct tcaggatg 18

<212> DNA<213> Homo sapiens<220> <223> Hammerhead ribozyme recognition site
for cdc 2 kinase
<400> 4209
gtcagtcttc aggatgtg 18

<212> DNA<213> Homo sapiens<220> <223> Hammerhead ribozyme recognition site
for cdc 2 kinase
<400> 4210
tcagtcttca ggatgtgc 18

<212> DNA<213> Homo sapiens<220> <223> Hammerhead ribozyme recognition site
for cdc 2 kinase
<400> 4211
gatgtgctta tgcaggatt 18

<212> DNA<213> Homo sapiens<220> <223> Hammerhead ribozyme recognition site
for cdc 2 kinase
<400> 4212

atgtgcttat gcaggattc 18

<212> DNA<213> Homo sapiens<220> <223> Hammerhead ribozyme recognition site
for cdc 2 kinase
<400> 4213
tgcaggattc caggttat 18

<212> DNA<213> Homo sapiens<220> <223> Hammerhead ribozyme recognition site
for cdc 2 kinase
<400> 4214
gcaggattcc aggttata 18

<212> DNA<213> Homo sapiens<220> <223> Hammerhead ribozyme recognition site
for cdc 2 kinase
<400> 4215
ttccaggtta tatctcat 18

<212> DNA<213> Homo sapiens<220> <223> Hammerhead ribozyme recognition site
for cdc 2 kinase
<400> 4216
tccaggttat atctcatc 18

<212> DNA<213> Homo sapiens<220> <223> Hammerhead ribozyme recognition site
for cdc 2 kinase
<400> 4217
caggttatat ctcatctt 18

<212> DNA<213> Homo sapiens<220> <223> Hammerhead ribozyme recognition site
for cdc 2 kinase
<400> 4218
ggttatatct catctttg 18

<212> DNA<213> Homo sapiens<220> <223> Hammerhead ribozyme recognition site
for cdc 2 kinase
<400> 4219
ttatatctca tctttgag 18

<212> DNA<213> Homo sapiens<220> <223> Hammerhead ribozyme recognition site
for cdc 2 kinase
<400> 4220
tatctcatct ttgagttt 18

<212> DNA<213> Homo sapiens<220> <223> Hammerhead ribozyme recognition site
for cdc 2 kinase
<400> 4221
tctcatcttt gagtttct 18

<212> DNA<213> Homo sapiens<220> <223> Hammerhead ribozyme recognition site
for cdc 2 kinase
<400> 4222
ctcatctttg agtttctt 18

<212> DNA<213> Homo sapiens<220> <223> Hammerhead ribozyme recognition site
for cdc 2 kinase
<400> 4223

ctttgagttt ctttccat 18

<212> DNA<213> Homo sapiens<220> <223> Hammerhead ribozyme recognition site
for cdc 2 kinase
<400> 4224
tttgagtttc tttccatg 18

<212> DNA<213> Homo sapiens<220> <223> Hammerhead ribozyme recognition site
for cdc 2 kinase
<400> 4225
ttgagtttct ttccatgg 18

<212> DNA<213> Homo sapiens<220> <223> Hammerhead ribozyme recognition site
for cdc 2 kinase
<400> 4226
gagtttcttt ccatggat 18

<212> DNA<213> Homo sapiens<220> <223> Hammerhead ribozyme recognition site
for cdc 2 kinase
<400> 4227
agtttctttc catggatc 18

<212> DNA<213> Homo sapiens<220> <223> Hammerhead ribozyme recognition site
for cdc 2 kinase
<400> 4228
ccatggatct gaagaaat 18

<212> DNA<213> Homo sapiens<220> <223> Hammerhead ribozyme recognition site
for cdc 2 kinase
<400> 4229
gaagaaatac ttggattc 18

<212> DNA<213> Homo sapiens<220> <223> Hammerhead ribozyme recognition site
for cdc 2 kinase
<400> 4230
gaaatacttg gattctat 18

<212> DNA<213> Homo sapiens<220> <223> Hammerhead ribozyme recognition site
for cdc 2 kinase
<400> 4231
acttggattc tatccctc 18

<212> DNA<213> Homo sapiens<220> <223> Hammerhead ribozyme recognition site
for cdc 2 kinase
<400> 4232
cttggattct atccctcc 18

<212> DNA<213> Homo sapiens<220> <223> Hammerhead ribozyme recognition site
for cdc 2 kinase
<400> 4233
tggattctat ccctcctg 18

<212> DNA<213> Homo sapiens<220> <223> Hammerhead ribozyme recognition site
for cdc 2 kinase
<400> 4234

gattctatcc ctcctggt 18

<212> DNA<213> Homo sapiens<220> <223> Hammerhead ribozyme recognition site
for cdc 2 kinase
<400> 4235
ctatccctcc tggtcagt 18

<212> DNA<213> Homo sapiens<220> <223> Hammerhead ribozyme recognition site
for cdc 2 kinase
<400> 4236
ctcctggtca gtacatgg 18

<212> DNA<213> Homo sapiens<220> <223> Hammerhead ribozyme recognition site
for cdc 2 kinase
<400> 4237
tggtcagtac atggattc 18

<212> DNA<213> Homo sapiens<220> <223> Hammerhead ribozyme recognition site
for cdc 2 kinase
<400> 4238
acatggattc ttcacttg 18

<212> DNA<213> Homo sapiens<220> <223> Hammerhead ribozyme recognition site
for cdc 2 kinase
<400> 4239
catggattct tcacttgt 18

<212> DNA<213> Homo sapiens<220> <223> Hammerhead ribozyme recognition site
for cdc 2 kinase
<400> 4240
tggattcttc acttgtta 18

<212> DNA<213> Homo sapiens<220> <223> Hammerhead ribozyme recognition site
for cdc 2 kinase
<400> 4241
ggattcttca cttgttaa 18

<212> DNA<213> Homo sapiens<220> <223> Hammerhead ribozyme recognition site
for cdc 2 kinase
<400> 4242
tcttcacttg ttaagagt 18

<212> DNA<213> Homo sapiens<220> <223> Hammerhead ribozyme recognition site
for cdc 2 kinase
<400> 4243
tcacttgtta agagttat 18

<212> DNA<213> Homo sapiens<220> <223> Hammerhead ribozyme recognition site
for cdc 2 kinase
<400> 4244
cacttgttaa gagttatt 18

<212> DNA<213> Homo sapiens<220> <223> Hammerhead ribozyme recognition site
for cdc 2 kinase
<400> 4245

ttaagagtta tttatacc 18

<212> DNA<213> Homo sapiens<220> <223> Hammerhead ribozyme recognition site
for cdc 2 kinase
<400> 4246
taagagttat ttatacca 18

<212> DNA<213> Homo sapiens<220> <223> Hammerhead ribozyme recognition site
for cdc 2 kinase
<400> 4247
agagttattt ataccaaa 18

<212> DNA<213> Homo sapiens<220> <223> Hammerhead ribozyme recognition site
for cdc 2 kinase
<400> 4248
gagttattta taccaaat 18

<212> DNA<213> Homo sapiens<220> <223> Hammerhead ribozyme recognition site
for cdc 2 kinase
<400> 4249
agttatttat accaaatc 18

<212> DNA<213> Homo sapiens<220> <223> Hammerhead ribozyme recognition site
for cdc 2 kinase
<400> 4250
ttatttatac caaatcct 18

<212> DNA<213> Homo sapiens<220> <223> Hammerhead ribozyme recognition site
for cdc 2 kinase
<400> 4251
caaatcctac aggggatt 18

<212> DNA<213> Homo sapiens<220> <223> Hammerhead ribozyme recognition site
for cdc 2 kinase
<400> 4252
caggggattg tgttttgt 18

<212> DNA<213> Homo sapiens<220> <223> Hammerhead ribozyme recognition site
for cdc 2 kinase
<400> 4253
gattgtgttt tgtcactc 18

<212> DNA<213> Homo sapiens<220> <223> Hammerhead ribozyme recognition site
for cdc 2 kinase
<400> 4254
attgtgtttt gtcactct 18

<212> DNA<213> Homo sapiens<220> <223> Hammerhead ribozyme recognition site
for cdc 2 kinase
<400> 4255
ttgtgttttg tcactcta 18

<212> DNA<213> Homo sapiens<220> <223> Hammerhead ribozyme recognition site
for cdc 2 kinase
<400> 4256

tgttttgtca ctctagaa 18

<212> DNA<213> Homo sapiens<220> <223> Hammerhead ribozyme recognition site
for cdc 2 kinase
<400> 4257
ttgtcactct agaagagt 18

<212> DNA<213> Homo sapiens<220> <223> Hammerhead ribozyme recognition site
for cdc 2 kinase
<400> 4258
gtcactctag aagagttc 18

<212> DNA<213> Homo sapiens<220> <223> Hammerhead ribozyme recognition site
for cdc 2 kinase
<400> 4259
agaagagttc ttcacaga 18

<212> DNA<213> Homo sapiens<220> <223> Hammerhead ribozyme recognition site
for cdc 2 kinase
<400> 4260
gaagagttct tcacagag 18

<212> DNA<213> Homo sapiens<220> <223> Hammerhead ribozyme recognition site
for cdc 2 kinase
<400> 4261
agagttcttc acagagac 18

<212> DNA<213> Homo sapiens<220> <223> Hammerhead ribozyme recognition site
for cdc 2 kinase
<400> 4262
cagagactta aaacctca 18

<212> DNA<213> Homo sapiens<220> <223> Hammerhead ribozyme recognition site
for cdc 2 kinase
<400> 4263
agagacttaa aacctcaa 18

<212> DNA<213> Homo sapiens<220> <223> Hammerhead ribozyme recognition site
for cdc 2 kinase
<400> 4264
taaaacctca aaatctct 18

<212> DNA<213> Homo sapiens<220> <223> Hammerhead ribozyme recognition site
for cdc 2 kinase
<400> 4265
ctcaaaatct cttgattg 18

<212> DNA<213> Homo sapiens<220> <223> Hammerhead ribozyme recognition site
for cdc 2 kinase
<400> 4266
caaaatctct tgattgat 18

<212> DNA<213> Homo sapiens<220> <223> Hammerhead ribozyme recognition site
for cdc 2 kinase
<400> 4267

aaatctcttg attgatga                                                18

<212> DNA<213> Homo sapiens<220> <223> Hammerhead ribozyme recognition site
for cdc 2 kinase
<400> 4268
ctcttgattg atgacaaa                                                18

<212> DNA<213> Homo sapiens<220> <223> Hammerhead ribozyme recognition site
for cdc 2 kinase
<400> 4269
ggaacaatta aactggct                                                18

<212> DNA<213> Homo sapiens<220> <223> Hammerhead ribozyme recognition site
for cdc 2 kinase
<400> 4270
tggctgattt tggccttg                                                18

<212> DNA<213> Homo sapiens<220> <223> Hammerhead ribozyme recognition site
for cdc 2 kinase
<400> 4271
ggctgatttt ggccttgc                                                18

<212> DNA<213> Homo sapiens<220> <223> Hammerhead ribozyme recognition site
for cdc 2 kinase
<400> 4272
gctgattttg gccttgcc                                                18

<212> DNA<213> Homo sapiens<220> <223> Hammerhead ribozyme recognition site
for cdc 2 kinase
<400> 4273
tttggccttg ccagagct                                                18

<212> DNA<213> Homo sapiens<220> <223> Hammerhead ribozyme recognition site
for cdc 2 kinase
<400> 4274
ccagagcttt tggaatac                                                18

<212> DNA<213> Homo sapiens<220> <223> Hammerhead ribozyme recognition site
for cdc 2 kinase
<400> 4275
cagagctttt ggaatacc                                                18

<212> DNA<213> Homo sapiens<220> <223> Hammerhead ribozyme recognition site
for cdc 2 kinase
<400> 4276
agagcttttg gaatacct                                                18

<212> DNA<213> Homo sapiens<220> <223> Hammerhead ribozyme recognition site
for cdc 2 kinase
<400> 4277
tttggaatac ctatcaga                                                18

<212> DNA<213> Homo sapiens<220> <223> Hammerhead ribozyme recognition site
for cdc 2 kinase
<400> 4278

gaatacctat cagagtat                                                    18

<212> DNA<213> Homo sapiens<220> <223> Hammerhead ribozyme recognition site
for cdc 2 kinase
<400> 4279
atacctatca gagtatat                                                    18

<212> DNA<213> Homo sapiens<220> <223> Hammerhead ribozyme recognition site
for cdc 2 kinase
<400> 4280
atcagagtat atacacat                                                    18

<212> DNA<213> Homo sapiens<220> <223> Hammerhead ribozyme recognition site
for cdc 2 kinase
<400> 4281
cagagtatat acacatga                                                    18

<212> DNA<213> Homo sapiens<220> <223> Hammerhead ribozyme recognition site
for cdc 2 kinase
<400> 4282
gagtatatac acatgagg                                                    18

<212> DNA<213> Homo sapiens<220> <223> Hammerhead ribozyme recognition site
for cdc 2 kinase
<400> 4283
catgaggtag taacactc                                                    18

<212> DNA<213> Homo sapiens<220> <223> Hammerhead ribozyme recognition site
for cdc 2 kinase
<400> 4284
gaggtagtaa cactctgg                                                    18

<212> DNA<213> Homo sapiens<220> <223> Hammerhead ribozyme recognition site
for cdc 2 kinase
<400> 4285
actctggtac agatctcc                                                    18

<212> DNA<213> Homo sapiens<220> <223> Hammerhead ribozyme recognition site
for cdc 2 kinase
<400> 4286
gtacagatct ccagaagt                                                    18

<212> DNA<213> Homo sapiens<220> <223> Hammerhead ribozyme recognition site
for cdc 2 kinase
<400> 4287
acagatctcc agaagtat                                                    18

<212> DNA<213> Homo sapiens<220> <223> Hammerhead ribozyme recognition site
for cdc 2 kinase
<400> 4288
ccagaagtat tgctgggg                                                    18

<212> DNA<213> Homo sapiens<220> <223> Hammerhead ribozyme recognition site
for cdc 2 kinase
<400> 4289

agaagtattg ctggggtc 18

<212> DNA<213> Homo sapiens<220> <223> Hammerhead ribozyme recognition site
for cdc 2 kinase
<400> 4290
gctggggtca gctcgtta 18

<212> DNA<213> Homo sapiens<220> <223> Hammerhead ribozyme recognition site
for cdc 2 kinase
<400> 4291
ggtcagctcg ttactcaa 18

<212> DNA<213> Homo sapiens<220> <223> Hammerhead ribozyme recognition site
for cdc 2 kinase
<400> 4292
cagctcgtta ctcaactc 18

<212> DNA<213> Homo sapiens<220> <223> Hammerhead ribozyme recognition site
for cdc 2 kinase
<400> 4293
agctcgttac tcaactcc 18

<212> DNA<213> Homo sapiens<220> <223> Hammerhead ribozyme recognition site
for cdc 2 kinase
<400> 4294
tcgttactca actccagt 18

<212> DNA<213> Homo sapiens<220> <223> Hammerhead ribozyme recognition site
for cdc 2 kinase
<400> 4295
actcaactcc agttgaca 18

<212> DNA<213> Homo sapiens<220> <223> Hammerhead ribozyme recognition site
for cdc 2 kinase
<400> 4296
actccagttg acatttgg 18

<212> DNA<213> Homo sapiens<220> <223> Hammerhead ribozyme recognition site
for cdc 2 kinase
<400> 4297
gttgacattt ggagtata 18

<212> DNA<213> Homo sapiens<220> <223> Hammerhead ribozyme recognition site
for cdc 2 kinase
<400> 4298
ttgacatttg gagtatag 18

<212> DNA<213> Homo sapiens<220> <223> Hammerhead ribozyme recognition site
for cdc 2 kinase
<400> 4299
tttggagtat aggcacca 18

<212> DNA<213> Homo sapiens<220> <223> Hammerhead ribozyme recognition site
for cdc 2 kinase
<400> 4300

tggagtatag gcaccata 18

<212> DNA<213> Homo sapiens<220> <223> Hammerhead ribozyme recognition site
for cdc 2 kinase
<400> 4301
ggcaccatat ttgctgaa 18

<212> DNA<213> Homo sapiens<220> <223> Hammerhead ribozyme recognition site
for cdc 2 kinase
<400> 4302
caccatattt gctgaact 18

<212> DNA<213> Homo sapiens<220> <223> Hammerhead ribozyme recognition site
for cdc 2 kinase
<400> 4303
accatatttg ctgaacta 18

<212> DNA<213> Homo sapiens<220> <223> Hammerhead ribozyme recognition site
for cdc 2 kinase
<400> 4304
gctgaactag caactaag 18

<212> DNA<213> Homo sapiens<220> <223> Hammerhead ribozyme recognition site
for cdc 2 kinase
<400> 4305
tagcaactaa gaaaccat 18

<212> DNA<213> Homo sapiens<220> <223> Hammerhead ribozyme recognition site
for cdc 2 kinase
<400> 4306
gaaaccattt tccatggg 18

<212> DNA<213> Homo sapiens<220> <223> Hammerhead ribozyme recognition site
for cdc 2 kinase
<400> 4307
aaaccatttt ccatgggg 18

<212> DNA<213> Homo sapiens<220> <223> Hammerhead ribozyme recognition site
for cdc 2 kinase
<400> 4308
aaccattttc catgggga 18

<212> DNA<213> Homo sapiens<220> <223> Hammerhead ribozyme recognition site
for cdc 2 kinase
<400> 4309
accattttcc atggggat 18

<212> DNA<213> Homo sapiens<220> <223> Hammerhead ribozyme recognition site
for cdc 2 kinase
<400> 4310
atggggattc agaaattg 18

<212> DNA<213> Homo sapiens<220> <223> Hammerhead ribozyme recognition site
for cdc 2 kinase
<400> 4311

tggggattca gaaattga                                                    18

<212> DNA<213> Homo sapiens<220> <223> Hammerhead ribozyme recognition site
for cdc 2 kinase
<400> 4312
aaattgatca actcttca                                                    18

<212> DNA<213> Homo sapiens<220> <223> Hammerhead ribozyme recognition site
for cdc 2 kinase
<400> 4313
gatcaactct tcaggatt                                                    18

<212> DNA<213> Homo sapiens<220> <223> Hammerhead ribozyme recognition site
for cdc 2 kinase
<400> 4314
tcaactcttc aggatttt                                                    18

<212> DNA<213> Homo sapiens<220> <223> Hammerhead ribozyme recognition site
for cdc 2 kinase
<400> 4315
ttcaggattt tcagagct                                                    18

<212> DNA<213> Homo sapiens<220> <223> Hammerhead ribozyme recognition site
for cdc 2 kinase
<400> 4316
tcaggatttt cagagctt                                                    18

<212> DNA<213> Homo sapiens<220> <223> Hammerhead ribozyme recognition site
for cdc 2 kinase
<400> 4317
caggattttc agagcttt                                                    18

<212> DNA<213> Homo sapiens<220> <223> Hammerhead ribozyme recognition site
for cdc 2 kinase
<400> 4318
aggattttca gagctttg                                                    18

<212> DNA<213> Homo sapiens<220> <223> Hammerhead ribozyme recognition site
for cdc 2 kinase
<400> 4319
tcagagcttt gggcactc                                                    18

<212> DNA<213> Homo sapiens<220> <223> Hammerhead ribozyme recognition site
for cdc 2 kinase
<400> 4320
cagagctttg ggcactcc                                                    18

<212> DNA<213> Homo sapiens<220> <223> Hammerhead ribozyme recognition site
for cdc 2 kinase
<400> 4321
tgggcactcc caataatg                                                    18

<212> DNA<213> Homo sapiens<220> <223> Hammerhead ribozyme recognition site
for cdc 2 kinase
<400> 4322

ctcccaataa tgaagtgt 18

<212> DNA<213> Homo sapiens<220> <223> Hammerhead ribozyme recognition site
for cdc 2 kinase
<400> 4323
agtggaatct ttacagga 18

<212> DNA<213> Homo sapiens<220> <223> Hammerhead ribozyme recognition site
for cdc 2 kinase
<400> 4324
tggaatcttt acaggact 18

<212> DNA<213> Homo sapiens<220> <223> Hammerhead ribozyme recognition site
for cdc 2 kinase
<400> 4325
ggaatcttta caggacta 18

<212> DNA<213> Homo sapiens<220> <223> Hammerhead ribozyme recognition site
for cdc 2 kinase
<400> 4326
gaatctttac aggactat 18

<212> DNA<213> Homo sapiens<220> <223> Hammerhead ribozyme recognition site
for cdc 2 kinase
<400> 4327
acaggactat aagaatac 18

<212> DNA<213> Homo sapiens<220> <223> Hammerhead ribozyme recognition site
for cdc 2 kinase
<400> 4328
aggactataa gaatacat 18

<212> DNA<213> Homo sapiens<220> <223> Hammerhead ribozyme recognition site
for cdc 2 kinase
<400> 4329
ataagaatac atttccca 18

<212> DNA<213> Homo sapiens<220> <223> Hammerhead ribozyme recognition site
for cdc 2 kinase
<400> 4330
gaatacattt cccaaatg 18

<212> DNA<213> Homo sapiens<220> <223> Hammerhead ribozyme recognition site
for cdc 2 kinase
<400> 4331
aatacatttc ccaaatgg 18

<212> DNA<213> Homo sapiens<220> <223> Hammerhead ribozyme recognition site
for cdc 2 kinase
<400> 4332
atacatttcc caaatgga 18

<212> DNA<213> Homo sapiens<220> <223> Hammerhead ribozyme recognition site
for cdc 2 kinase
<400> 4333

ggaagcctag catcccat                                                    18

<212> DNA<213> Homo sapiens<220> <223> Hammerhead ribozyme recognition site
for cdc 2 kinase
<400> 4334
cctagcatcc catgtcaa                                                    18

<212> DNA<213> Homo sapiens<220> <223> Hammerhead ribozyme recognition site
for cdc 2 kinase
<400> 4335
tcccatgtca aaaacttg                                                    18

<212> DNA<213> Homo sapiens<220> <223> Hammerhead ribozyme recognition site
for cdc 2 kinase
<400> 4336
caaaaacttg gatgaaaa                                                    18

<212> DNA<213> Homo sapiens<220> <223> Hammerhead ribozyme recognition site
for cdc 2 kinase
<400> 4337
aaatggcttg gatttgct                                                    18

<212> DNA<213> Homo sapiens<220> <223> Hammerhead ribozyme recognition site
for cdc 2 kinase
<400> 4338
gcttggattt gctctcga                                                    18

<212> DNA<213> Homo sapiens<220> <223> Hammerhead ribozyme recognition site
for cdc 2 kinase
<400> 4339
cttggatttg ctctcgaa                                                    18

<212> DNA<213> Homo sapiens<220> <223> Hammerhead ribozyme recognition site
for cdc 2 kinase
<400> 4340
gatttgctct cgaaaatg                                                    18

<212> DNA<213> Homo sapiens<220> <223> Hammerhead ribozyme recognition site
for cdc 2 kinase
<400> 4341
tttgctctcg aaaatgtt                                                    18

<212> DNA<213> Homo sapiens<220> <223> Hammerhead ribozyme recognition site
for cdc 2 kinase
<400> 4342
gaaaatgtta atctatga                                                    18

<212> DNA<213> Homo sapiens<220> <223> Hammerhead ribozyme recognition site
for cdc 2 kinase
<400> 4343
aaaatgttaa tctatgat                                                    18

<212> DNA<213> Homo sapiens<220> <223> Hammerhead ribozyme recognition site
for cdc 2 kinase
<400> 4344

atgttaatct atgatcca 18

<212> DNA<213> Homo sapiens<220> <223> Hammerhead ribozyme recognition site
for cdc 2 kinase
<400> 4345
gttaatctat gatccagc 18

<212> DNA<213> Homo sapiens<220> <223> Hammerhead ribozyme recognition site
for cdc 2 kinase
<400> 4346
tctatgatcc agccaaac 18

<212> DNA<213> Homo sapiens<220> <223> Hammerhead ribozyme recognition site
for cdc 2 kinase
<400> 4347
aaacgaattt ctggcaaa 18

<212> DNA<213> Homo sapiens<220> <223> Hammerhead ribozyme recognition site
for cdc 2 kinase
<400> 4348
aacgaatttc tggcaaaa 18

<212> DNA<213> Homo sapiens<220> <223> Hammerhead ribozyme recognition site
for cdc 2 kinase
<400> 4349
acgaatttct ggcaaaat 18

<212> DNA<213> Homo sapiens<220> <223> Hammerhead ribozyme recognition site
for cdc 2 kinase
<400> 4350
cactgaatca tccatatt 18

<212> DNA<213> Homo sapiens<220> <223> Hammerhead ribozyme recognition site
for cdc 2 kinase
<400> 4351
tgaatcatcc atatttta 18

<212> DNA<213> Homo sapiens<220> <223> Hammerhead ribozyme recognition site
for cdc 2 kinase
<400> 4352
tcatccatat tttaatga 18

<212> DNA<213> Homo sapiens<220> <223> Hammerhead ribozyme recognition site
for cdc 2 kinase
<400> 4353
atccatattt taatgatt 18

<212> DNA<213> Homo sapiens<220> <223> Hammerhead ribozyme recognition site
for cdc 2 kinase
<400> 4354
tccatatttt aatgattt 18

<212> DNA<213> Homo sapiens<220> <223> Hammerhead ribozyme recognition site
for cdc 2 kinase
<400> 4355

ccatatttta atgatttg 18

<212> DNA<213> Homo sapiens<220> <223> Hammerhead ribozyme recognition site
for cdc 2 kinase
<400> 4356
catattttaa tgatttgg 18

<212> DNA<213> Homo sapiens<220> <223> Hammerhead ribozyme recognition site
for cdc 2 kinase
<400> 4357
ttaatgattt ggacaatc 18

<212> DNA<213> Homo sapiens<220> <223> Hammerhead ribozyme recognition site
for cdc 2 kinase
<400> 4358
taatgatttg gacaatca 18

<212> DNA<213> Homo sapiens<220> <223> Hammerhead ribozyme recognition site
for cdc 2 kinase
<400> 4359
tggacaatca gattaaga 18

<212> DNA<213> Homo sapiens<220> <223> Hammerhead ribozyme recognition site
for cdc 2 kinase
<400> 4360
gaagatgtag ctttctga 18

<212> DNA<213> Homo sapiens<220> <223> Hammerhead ribozyme recognition site
for cyclin B1
<400> 4361
tccgagtcac caggaa 16

<212> DNA<213> Homo sapiens<220> <223> Hammerhead ribozyme recognition site
for cyclin B1
<400> 4362
ccagtgtctg agccag 16

<212> DNA<213> Homo sapiens<220> <223> Hammerhead ribozyme recognition site
for cyclin B1
<400> 4363
cctgtgtcag gctttc 16

<212> DNA<213> Homo sapiens<220> <223> Hammerhead ribozyme recognition site
for cyclin B1
<400> 4364
aagcagtcag accaaa 16

<212> DNA<213> Homo sapiens<220> <223> Hammerhead ribozyme recognition site
for cyclin B1
<400> 4365
actgggtcgg gaagtc 16

<212> DNA<213> Homo sapiens<220> <223> Hammerhead ribozyme recognition site
for cyclin B1
<400> 4366

tgactgtctc cattat 16

<212> DNA<213> Homo sapiens<220> <223> Hammerhead ribozyme recognition site
for PCNA
<400> 4367
gcctggtcca gggctc 16

<212> DNA<213> Homo sapiens<220> <223> Hammerhead ribozyme recognition site
for PCNA
<400> 4368
gactcgtccc acgtct 16

<212> DNA<213> Homo sapiens<220> <223> Hammerhead ribozyme recognition site
for PCNA
<400> 4369
ctgcggtctg agggct 16

<212> DNA<213> Homo sapiens<220> <223> Hammerhead ribozyme recognition site
for PCNA
<400> 4370
tttctgtcac caaatt 16

<212> DNA<213> Homo sapiens<220> <223> Hammerhead ribozyme recognition site
for lysyl oxidase
<400> 4371
ccgccgtccc tggtgc 16

<212> DNA<213> Homo sapiens<220> <223> Hammerhead ribozyme recognition site
for lysyl oxidase
<400> 4372
ctggagtcac cgctgg 16

<212> DNA<213> Homo sapiens<220> <223> Hammerhead ribozyme recognition site
for lysyl oxidase
<400> 4373
cgcccgtcac tggttc 16

<212> DNA<213> Homo sapiens<220> <223> Hammerhead ribozyme recognition site
for lysyl oxidase
<400> 4374
gtacggtctc ccagac 16

<212> DNA<213> Homo sapiens<220> <223> Hammerhead ribozyme recognition site
for lysyl oxidase
<400> 4375
caggcgtcca cgtacg 16

<212> DNA<213> Homo sapiens<220> <223> Hammerhead ribozyme recognition site
for lysyl oxidase
<400> 4376
aaactgtctg gccagt 16

<212> DNA<213> Homo sapiens<220> <223> Hammerhead ribozyme recognition site
for lysyl oxidase
<400> 4377

tttctgtctt gaagac                                                16

<212> DNA<213> Homo sapiens<220> <223> Representative Haipin Ribozyme
<400> 4378
aacgagctag aaccagacca gagaaacaca cgttgtggta tattacctgg ta          52

<212> DNA<213> Homo sapiens<220> <223> Representative Haipin Ribozyme
<400> 4379
ctggctcaag aactggacca gagaaacaca cgttgtggta tattacctgg ta          52

<212> DNA<213> Homo sapiens<220> <223> Representative Haipin Ribozyme
<400> 4380
ccagcggtag aaccagacca gagaaacaca cgttgtggta tattacctgg ta          52

<212> DNA<213> Homo sapiens<220> <223> Representative Haipin Ribozyme
<400> 4381
agccctcaag aagcagacca gagaaacaca cgttgtggta tattacctgg ta          52

<212> DNA<213> Artificial Sequence<220> <223> Synthetic human hammerhead
ribozymes targeting PCNA
<400> 4382
gagccctgcu gaugagcaat tttttgcgaa aaccaggcgc

<212> DNA<213> Artificial Sequence<220> <223> Synthetic human hammerhead
ribozymes targeting PCNA
<400> 4383
agcccugcug augaggccgt aaggccgaaa  ccaggcgc

<212> DNA<213> Artificial Sequence<220> <223> Synthetic human hammerhead
ribozymes targeting PCNA
<400> 4384
agcccugcug augaggccgt aaggccgaaa  ccaggcgc

<212> DNA<213> Homo sapien<220> <223> General structure of chimeric DNA/RNA
ribozyme
<400> 4385
nnnnnnnncu gaugagcaat tttttgcgaa aannnnnnn

<212> DNA<213> Homo sapiens<220> <223> General target RNA of chimeric
DNA/RNA ribozyme
<400> 4386
nnnnnnnnhu nnnnnn

<210> 4387<212> DNA<213> Homo sapiens<220> <223> Representative hairpin
ribozyme
<400> 4387
nnnnnnnnag aannnnacca gagaaacaca cguuguggua uauuaccugg ua

<212> DNA<213> Homo sapiens<220> <223> Target RNA of representative hairpin
ribozyme
<400> 4388
nnnsngucnn nnnnnn

<210> 4389          <212> DNA       <213> Homo sapien       <220>

<223> MMP-1 ribozyme recognition site
<400> 4389
gtggtgtctc acagct 16

<210> 4390 <212> DNA <213> Homo sapien <220>
<223> MMP-1 ribozyme recognition site
<400> 4390
agtttgtcct cactga 16

<210> 4391 <212> DNA <213> Homo sapien <220>
<223> MMP-1 ribozyme recognition site
<400> 4391
ccaaggtctc tgaggg 16

<210> 4392 <212> DNA <213> Homo sapien <220>
<223> MMP-1 ribozyme recognition site
<400> 4392
tgagggtcaa gcagac 16

<210> 4393 <212> DNA <213> Homo sapien <220>
<223> MMP-1 ribozyme recognition site
<400> 4393
atcctgtcca gcccat 16

<210> 4394 <212> DNA <213> Homo sapien <220>
<223> MMP-1 ribozyme recognition site
<400> 4394
tgtgagtcca aagaag 16

<210> 4395 <212> DNA <213> Homo sapien <220>
<223> MMP-1 ribozyme recognition site
<400> 4395
gaactgtcta ttttct 16

<210> 4396 <212> DNA <213> Homo sapien <220>
<223> MMP-1 ribozyme recognition site
<400> 4396
tctcagtcat ttttaa 16

<210> 4397 <212> DNA <213> Homo sapien <220>
<223> MMP-1 ribozyme recognition site
<400> 4397
ctagagtcac tgatac 16

<210> 4398 <212> DNA <213> Homo sapien <220>
<223> MMP-1 ribozyme recognition site
<400> 4398
catgagtctt tgccgg 16

<210> 4399 <212> DNA <213> Homo sapien <220>
<223> IL-1 beta ribozyme recognition site
<400> 4399
caagtgtctg aagcag 16

<210> 4400 <212> DNA <213> Homo sapien <220>

<223> IL-1 beta ribozyme recognition site
<400> 4400
gccgcgtcag ttgttg 16

<210> 4401 <212> DNA <213> Homo sapien <220>
<223> IL-1 beta ribozyme recognition site
<400> 4401
gtgatgtctg gtccat 16

<210> 4402 <212> DNA <213> Homo sapien <220>
<223> IL-1 beta ribozyme recognition site
<400> 4402
gtctggtcca tatgaa 16

<210> 4403 <212> DNA <213> Homo sapien <220>
<223> IL-1 beta ribozyme recognition site
<400> 4403
tccatgtcct ttgtac 16

<210> 4404 <212> DNA <213> Homo sapien <220>
<223> IL-1 beta ribozyme recognition site
<400> 4404
tacctgtcct gcgtgt 16

<210> 4405 <212> DNA <213> Homo sapien <220>
<223> IL-1 beta ribozyme recognition site
<400> 4405
gatttgtctt caacaa 16

<210> 4406 <212> DNA <213> Homo sapien <220>
<223> IL-1 beta ribozyme recognition site
<400> 4406
tttgagtctg cccagt 16

<210> 4407 <212> DNA <213> Homo sapien <220>
<223> IL-1 beta ribozyme recognition site
<400> 4407
tgcccgtctt cctggg 16

<210> 4408 <212> DNA <213> Homo sapien <220>
<223> IL-1 beta ribozyme recognition site
<400> 4408
tttgtgtctt cctaaa 16

<210> 4409 <212> DNA <213> Homo sapien <220>
<223> IL-1 beta ribozyme recognition site
<400> 4409
agagagtcct gtgctg 16

<210> 4410 <212> DNA <213> Homo sapien <220>
<223> IL-1 beta ribozyme recognition site
<400> 4410
ctgttgtcta caccaa 16

<210> 4411 <212> DNA <213> Homo sapien <220>

<223> VEGF ribozyme recognition site
<400> 4411
ctgctgtctt gggtgc 16

<210> 4412 <212> DNA <213> Homo sapien <220>
<223> VEGF ribozyme recognition site
<400> 4412
aagtggtccc aggctg 16

<210> 4413 <212> DNA <213> Homo sapien <220>
<223> VEGF ribozyme recognition site
<400> 4413
gaggagtcca acatca 16

<210> 4414 <212> DNA <213> Homo sapien <220>
<223> IL2 ribozyme recognition site
<400> 4414
caactcctgt cttgcattgc a 21

<210> 4415 <212> DNA <213> Homo sapien <220>
<223> IL2 ribozyme recognition site
<400> 4415
ttgcacttgt cacaaacagt g 21

<210> 4416 <212> DNA <213> Homo sapien <220>
<223> IL2 ribozyme recognition site
<400> 4416
gcttcagtgt ctagaagaag a 21

<210> 4417 <212> DNA <213> Homo sapien <220>
<223> IL2 ribozyme recognition site
<400> 4417
tacctttgt caaagcatca t 21

<210> 4418 <212> DNA <213> Homo sapien <220>
<223> INF-gamma ribozyme recognition site
<400> 4418
gccaaattgt ctccttttac t 21

<210> 4419 <212> DNA <213> Homo sapien <220>
<223> INF-gamma ribozyme recognition site
<400> 4419
gctgaactgt cgccagcagc t 21

<210> 4420 <212> DNA <213> Homo sapien <220>
<223> INF-gamma ribozyme recognition site
<400> 4420
aaaaaggagt cagatgctgt t 21

<210> 4421 <212> DNA <213> Homo sapien <220>
<223> INF-gamma ribozyme recognition site
<400> 4421
gtaatggttg tcctgcctgc aa 22

<210> 4422 <212> DNA <213> Homo sapien <220>

<223> INF-gamma ribozyme recognition site
<400> 4422
ctgtgactgt ctcacttaat c 21

<210> 4423 <212> DNA <213> Homo sapien <220>
<223> INF-gamma ribozyme recognition site
<400> 4423
ttgaatgtgt caggtgaccc t 21

<210> 4424 <212> DNA <213> Homo sapien <220>
<223> IL-1 alpha ribozyme recognition site
<400> 4424
gcgtttgagt cagcaaagaa g 21

<210> 4425 <212> DNA <213> Homo sapien <220>
<223> IL-1 alpha ribozyme recognition site
<400> 4425
gatcatctgt ctctgaatca g 21

<210> 4426 <212> DNA <213> Homo sapien <220>
<223> IL-1 alpha ribozyme recognition site
<400> 4426
caatctgtgt ctctgagtat c 21

<210> 4427 <212> DNA <213> Homo sapien <220>
<223> IL-1 alpha ribozyme recognition site
<400> 4427
ggtctggagt ctcacttgtc t 21

<210> 4428 <212> DNA <213> Homo sapien <220>
<223> IL-1 alpha ribozyme recognition site
<400> 4428
tctcacttgt ctcacttgtg c 21

<210> 4429 <212> DNA <213> Homo sapien <220>
<223> IL-1 alpha ribozyme recognition site
<400> 4429
tactgttagt catttgctga g 21

<210> 4430 <212> DNA <213> Homo sapien <220>
<223> IL-1 alpha ribozyme recognition site
<400> 4430
gcattttggt ccaagttgtg c 21

<210> 4431 <212> DNA <213> Homo sapien <220>
<223> IL-1 alpha ribozyme recognition site
<400> 4431
agtaattggt ccgatctttg a 21

<210> 4432 <212> DNA <213> Homo sapien <220>
<223> IL-1 alpha ribozyme recognition site
<400> 4432
ccgtgctggt ctcgaacttc t 21

<210> 4433 <212> DNA <213> Homo sapien <220>

<223> IL-1 alpha ribozyme recognition site
<400> 4433
aatcctcagt cagccgtgtt t 21

<210> 4434 <212> DNA <213> Homo sapien <220>
<223> IL-1 alpha ribozyme recognition site
<400> 4434
ttcttgtccc aaataaaa 18

<210> 4435 <212> DNA <213> Homo sapien <220>
<223> IL6 ribozyme recognition site
<400> 4435
cgccttcggt ccagttgcct t 21

<210> 4436 <212> DNA <213> Homo sapien <220>
<223> IL6 ribozyme recognition site
<400> 4436
catcactggt cttttggagt t 21

<210> 4437 <212> DNA <213> Homo sapien <220>
<223> IL6 ribozyme recognition site
<400> 4437
ttcctgcagt ccagcctgag g 21

<210> 4438 <212> DNA <213> Homo sapien <220>
<223> IL6 ribozyme recognition site
<400> 4438
ttcttctggt cagaaacctg t 21

<210> 4439 <212> DNA <213> Homo sapien <220>
<223> IL6 ribozyme recognition site
<400> 4439
agaaacctgt ccactgggca c 21

<210> 4440 <212> DNA <213> Homo sapien <220>
<223> IL8 ribozyme recognition site
<400> 4440
ttgtgtgggt ctgttgtagg g 21

<210> 4441 <212> DNA <213> Homo sapien <220>
<223> IL8 ribozyme recognition site
<400> 4441
agagctctgt ctggacccca a 21

<210> 4442 <212> DNA <213> Homo sapien <220>
<223> IL8 ribozyme recognition site
<400> 4442
caaccctagt ctgctagcca g 21

<210> 4443 <212> DNA <213> Homo sapien <220>
<223> MMP-2 ribozyme recognition site
<400> 4443
gccgcgccgt cgcccatcat c 21

<210> 4444 <212> DNA <213> Homo sapien <220>

<223> MMP-2 ribozyme recognition site
<400> 4444
gccaagtggt ccgtgtgaag t 21

<210> 4445 <212> DNA <213> Homo sapien <220>
<223> MMP-2 ribozyme recognition site
<400> 4445
cggcttctgt ccccatgaag c 21

<210> 4446 <212> DNA <213> Homo sapien <220>
<223> MMP-2 ribozyme recognition site
<400> 4446
ccccctgtgt cttccccttc a 21

<210> 4447 <212> DNA <213> Homo sapien <220>
<223> MMP-2 ribozyme recognition site
<400> 4447
gaacttccgt ctgtcccagg a 21

<210> 4448 <212> DNA <213> Homo sapien <220>
<223> MMP-2 ribozyme recognition site
<400> 4448
ttccgtctgt cccaggatga c 21

<210> 4449 <212> DNA <213> Homo sapien <220>
<223> MMP-2 ribozyme recognition site
<400> 4449
tgggccctgt cactcctgag a 21

<210> 4450 <212> DNA <213> Homo sapien <220>
<223> MMP-2 ribozyme recognition site
<400> 4450
tggatgccgt cgtggacctg c 21

<210> 4451 <212> DNA <213> Homo sapien <220>
<223> MMP-2 ribozyme recognition site
<400> 4451
gggcggcggt cacagctact t 21

<210> 4452 <212> DNA <213> Homo sapien <220>
<223> MMP-2 ribozyme recognition site
<400> 4452
tggagactgt ctcaagaggg c 21

<210> 4453 <212> DNA <213> Homo sapien <220>
<223> MMP-2 ribozyme recognition site
<400> 4453
ttctttgggt cttgtttttt t 21

<210> 4454 <212> DNA <213> Homo sapien <220>
<223> MMP-3 ribozyme recognition site
<400> 4454
atgaagagtc ttccaatcct 20

<210> 4455 <212> DNA <213> Homo sapien <220>

<223> MMP-3 ribozyme recognition site
<400> 4455
ggacagtggt cctgttgtta a 21

<210> 4456 <212> DNA <213> Homo sapien <220>
<223> MMP-3 ribozyme recognition site
<400> 4456
tgacgttggt cacttcagaa c 21

<210> 4457 <212> DNA <213> Homo sapien <220>
<223> MMP-3 ribozyme recognition site
<400> 4457
ctccctgggt ctctttcact c 21

<210> 4458 <212> DNA <213> Homo sapien <220>
<223> MMP-3 ribozyme recognition site
<400> 4458
ttccgcctgt ctcaagatga t 21

<210> 4459 <212> DNA <213> Homo sapien <220>
<223> MMP-3 ribozyme recognition site
<400> 4459
ggcattcagt ccctctatgg a 21

<210> 4460 <212> DNA <213> Homo sapien <220>
<223> MMP-3 ribozyme recognition site
<400> 4460
cggaacctgt ccctccagaa c 21

<210> 4461 <212> DNA <213> Homo sapien <220>
<223> MMP-3 ribozyme recognition site
<400> 4461
cctgctttgt cctttgatgc t 21

<210> 4462 <212> DNA <213> Homo sapien <220>
<223> MMP-3 ribozyme recognition site
<400> 4462
ttgatgctgt cagcactctg a 21

<210> 4463 <212> DNA <213> Homo sapien <220>
<223> MMP-9 ribozyme recognition site
<400> 4463
agcccctggt cctggtgctc c 21

<210> 4464 <212> DNA <213> Homo sapien <220>
<223> MMP-9 ribozyme recognition site
<400> 4464
cagcgccagt ccacccttgt g 21

<210> 4465 <212> DNA <213> Homo sapien <220>
<223> MMP-9 ribozyme recognition site
<400> 4465
cgtggagagt cgaaatctct g 21

<210> 4466 <212> DNA <213> Homo sapien <220>

<223> MMP-9 ribozyme recognition site
<400> 4466
aagcaactgt ccctgcccga g                                                21

<210> 4467          <212> DNA       <213> Homo sapien <220>
<223> MMP-9 ribozyme recognition site
<400> 4467
ggtgcggggt cccagacctg g                                                21

<210> 4468          <212> DNA       <213> Homo sapien <220>
<223> MMP-9 ribozyme recognition site
<400> 4468
cagacatcgt catccagttt g                                                21

<210> 4469          <212> DNA       <213> Homo sapien <220>
<223> MMP-9 ribozyme recognition site
<400> 4469
agtttggtgt cgcggagcac g                                                21

<210> 4470          <212> DNA       <213> Homo sapien <220>
<223> MMP-9 ribozyme recognition site
<400> 4470
gagttgtggt ccctgggcaa g                                                21

<210> 4471          <212> DNA       <213> Homo sapien <220>
<223> MMP-9 ribozyme recognition site
<400> 4471
gcaagggcgt cgtggttcca a                                                21

<210> 4472          <212> DNA       <213> Homo sapien <220>
<223> MMP-9 ribozyme recognition site
<400> 4472
caccgacggt cgctccgacg g                                                21

<210> 4473          <212> DNA       <213> Homo sapien <220>
<223> MMP-9 ribozyme recognition site
<400> 4473
cacggacggt cgctccgacg g                                                21

<210> 4474          <212> DNA       <213> Homo sapien <220>
<223> MMP-9 ribozyme recognition site
<400> 4474
agctgtgcgt cttccccttc a                                                21

<210> 4475          <212> DNA       <213> Homo sapien <220>
<223> MMP-9 ribozyme recognition site
<400> 4475
cctctatggt cctcgccctg a                                                21

<210> 4476          <212> DNA       <213> Homo sapien <220>
<223> MMP-9 ribozyme recognition site
<400> 4476
ccccgacggt ctgccccacc g                                                21

<210> 4477          <212> DNA       <213> Homo sapien <220>

<223> MMP-9 ribozyme recognition site
<400> 4477
cccccactgt ccacccctca g                                                21

<210> 4478                <212> DNA        <213> Homo sapien <220>
<223> MMP-9 ribozyme recognition site
<400> 4478
gcctttgagt ccggtggacg a                                                21

<210> 4479                <212> DNA        <213> Homo sapien <220>
<223> MMP-9 ribozyme recognition site
<400> 4479
tggactcggt ctttgaggag c                                                21

<210> 4480                <212> DNA        <213> Homo sapien <220>
<223> MMP-9 ribozyme recognition site
<400> 4480
acaggcgcgt cggtgctggg c                                                21

<210> 4481                <212> DNA        <213> Homo sapien <220>
<223> MMP-9 ribozyme recognition site
<400> 4481
cccgaggcgt ctggacaagc t                                                21

<210> 4482                <212> DNA        <213> Homo sapien <220>
<223> MMP-9 ribozyme recognition site
<400> 4482
gggctcccgt cctgctttgc a                                                21

<210> 4483                <212> DNA        <213> Homo sapien <220>
<223> PDGF A ribozyme recognition site
<400> 4483
ccggccgggt cgctcctgaa g                                                21

<210> 4484                <212> DNA        <213> Homo sapien <220>
<223> PDGF A ribozyme recognition site
<400> 4484
ccccttccgt ccccaccccc a                                                21

<210> 4485                <212> DNA        <213> Homo sapien <220>
<223> PDGF A ribozyme recognition site
<400> 4485
cgggcgccgt ccgccagctc c                                                21

<210> 4486                <212> DNA        <213> Homo sapien <220>
<223> PDGF A ribozyme recognition site
<400> 4486
ggcccgcagt cagatccaca g                                                21

<210> 4487                <212> DNA        <213> Homo sapien <220>
<223> PDGF A ribozyme recognition site
<400> 4487
ctcacggggt ccatgccact a                                                21

<210> 4488                <212> DNA        <213> Homo sapien <220>

<223> PDGF A ribozyme recognition site
<400> 4488
aggaagctgt ccccgctgtc t 21

<210> 4489 <212> DNA <213> Homo sapien <220>
<223> PDGF A ribozyme recognition site
<400> 4489
tccccgctgt ctgcaagacc a 21

<210> 4490 <212> DNA <213> Homo sapien <220>
<223> PDGF A ribozyme recognition site
<400> 4490
ccaggacggt catttacgag a 21

<210> 4491 <212> DNA <213> Homo sapien <220>
<223> PDGF A ribozyme recognition site
<400> 4491
tcctcggagt caggtcgacc c 21

<210> 4492 <212> DNA <213> Homo sapien <220>
<223> PDGF A ribozyme recognition site
<400> 4492
cgagcagtgt caagtgccag c 21

<210> 4493 <212> DNA <213> Homo sapien <220>
<223> PDGF A ribozyme recognition site
<400> 4493
cctcccgcgt ccaccaccgc a 21

<210> 4494 <212> DNA <213> Homo sapien <220>
<223> PDGF A ribozyme recognition site
<400> 4494
accgcagcgt caaggtggcc a 21

<210> 4495 <212> DNA <213> Homo sapien <220>
<223> PDGF A ribozyme recognition site
<400> 4495
cctagggagt caggtaaaaa a 21

<210> 4496 <212> DNA <213> Homo sapien <220>
<223> PDGF A ribozyme recognition site
<400> 4496
agtgtgcggt ctttgttctc c 21

<210> 4497 <212> DNA <213> Homo sapien <220>
<223> PDGF A ribozyme recognition site
<400> 4497
aaaactgtgt ccgagaacac t 21

<210> 4498 <212> DNA <213> Homo sapien <220>
<223> PDGF A ribozyme recognition site
<400> 4498
gcttccttgt caaaaagaga g 21

<210> 4499 <212> DNA <213> Homo sapien <220>

<223> PDGF B ribozyme recognition site
<400> 4499
aggttggagt cccctgggcg c 21

<210> 4500 <212> DNA <213> Homo sapien <220>
<223> PDGF B ribozyme recognition site
<400> 4500
ggcccggagt cggcatgaat c 21

<210> 4501 <212> DNA <213> Homo sapien <220>
<223> PDGF B ribozyme recognition site
<400> 4501
ctcttcctgt ctctctgctg c 21

<210> 4502 <212> DNA <213> Homo sapien <220>
<223> PDGF B ribozyme recognition site
<400> 4502
ctacctgcgt ctggtcagcg c 21

<210> 4503 <212> DNA <213> Homo sapien <220>
<223> PDGF B ribozyme recognition site
<400> 4503
tgcgtctggt cagcgccgag g 21

<210> 4504 <212> DNA <213> Homo sapien <220>
<223> PDGF B ribozyme recognition site
<400> 4504
tgcgacctgt ccaggtgaga a 21

<210> 4505 <212> DNA <213> Homo sapien <220>
<223> PDGF B ribozyme recognition site
<400> 4505
cggtgcgagt ccgccggccc c 21

<210> 4506 <212> DNA <213> Homo sapien <220>
<223> PDGF B ribozyme recognition site
<400> 4506
tttccctcgt ccgtctgtct c 21

<210> 4507 <212> DNA <213> Homo sapien <220>
<223> PDGF B ribozyme recognition site
<400> 4507
cctcgtccgt ctgtctcgat g 21

<210> 4508 <212> DNA <213> Homo sapien <220>
<223> PDGF B ribozyme recognition site
<400> 4508
gtccgtctgt ctcgatgcct g 21

<210> 4509 <212> DNA <213> Homo sapien <220>
<223> PDGF B ribozyme recognition site
<400> 4509
ctccacgtgt ccgtccaccc t 21

<210> 4510 <212> DNA <213> Homo sapien <220>

<223> PDGF B ribozyme recognition site
<400> 4510
acgtgtccgt ccacccttcc a 21

<210> 4511 <212> DNA <213> Homo sapien <220>
<223> PDGF B ribozyme recognition site
<400> 4511
atcagcgggt ctcctcccag 20

<210> 4512 <212> DNA <213> Homo sapien <220>
<223> PDGF B ribozyme recognition site
<400> 4512
ctgatggggt cgctctttgg g 21

<210> 4513 <212> DNA <213> Homo sapien <220>
<223> PDGF B ribozyme recognition site
<400> 4513
tgtggactgt cctgaggagc c 21

<210> 4514 <212> DNA <213> Homo sapien <220>
<223> VEGF hammerhead ribozyme recognition site
<400> 4514
tgctgctcta cctccaccat g 21

<210> 4515 <212> DNA <213> Homo sapien <220>
<223> VEGF hammerhead ribozyme recognition site
<400> 4515
tcatggatgt ctatcagcgc 20

<210> 4516 <212> DNA <213> Homo sapien <220>
<223> VEGF hammerhead ribozyme recognition site
<400> 4516
ggacatcttc caggagtac 19

<210> 4517 <212> DNA <213> Homo sapien <220>
<223> VEGF hammerhead ribozyme recognition site
<400> 4517
gtacatcttc aagccatcc 19

<210> 4518 <212> DNA <213> Homo sapien <220>
<223> VEGF hammerhead ribozyme recognition site
<400> 4518
catgcagatt atgcggatc 19

<210> 4519 <212> DNA <213> Homo sapien <220>
<223> VEGF hammerhead ribozyme recognition site
<400> 4519
atcaaacctc accaaggcc 19

<210> 4520 <212> DNA <213> Homo sapien <220>
<223> VEGF hammerhead ribozyme recognition site
<400> 4520
gatgagcttc ctacagcac 19

<210> 4521 <212> DNA <213> Homo sapien <220>

<223> VEGF hammerhead ribozyme recognition site
<400> 4521
agaaaaaaaa tcagttcgag g                                                    21

<210> 4522                <212> DNA        <213> Homo sapien <220>
<223> VEGF hammerhead ribozyme recognition site
<400> 4522
gcaagaaatc ccggtataa                                                       19

<210> 4523                <212> DNA        <213> Homo sapien <220>
<223> VEGF hammerhead ribozyme recognition site
<400> 4523
gtacaagatc cgcagacgt                                                       19

## Claims

1. Use of a chimeric DNA/RNA hammerhead ribozyme in the manufacture of a medicament for treatment of a proliferative skin disease or for treating or preventing scarring, wherein the ribozyme is directed to the sequence of any of SEQ ID NOS: 3855-4115, 4143-4152, wherein these sequences are within the PCNA gene.

2. Use according to claim 1, wherein the ribozyme is directed to SEQ ID NO: 4145.

3. Use according to claim 1 wherein said proliferative skin disease is psoriasis.

4. Use according to claim 1 wherein said proliferative skin disease is atopic dermatitis.

5. Use according to claim 1 wherein said proliferative skin disease is actinic keratosis.

6. Use according to claim 1 wherein said proliferative skin disease is squamous or basal cell carcinoma.

7. Use according to claim 1 wherein said proliferative skin disease is viral or seborrheic wart.

8. Use according to claim 1 wherein said scar is a keloid.

9. Use according to claim 1 wherein said scar is an adhesion.

10. Use according to claim 1 wherein said scar is a hypertrophic or hypertrophic burn scar.

11. A method of treating a proliferative skin disease, of treating or preventing scarring, or of treating a proliferative eye disease, comprising administering to a patient a therapeutically effective amount of ribozyme which cleaves RNA encoding a cytokine involved in inflammation, a matrix metalloproteinase, a cyclin, a cell-cycle dependent kinase, a growth factor, or a reductase, or of nucleic acid molecule comprising a promoter operably linked to a nucleic acid segment encoding said ribozyme, such that said proliferative skin disease is treated.
